# EUROPEAN PATENT SPECIFICATION

(11) **EP 2 513 148 B1**
(45) Date of publication and mention of the grant of the patent: **31.08.2016**
(21) Application number: 10791036.6
(22) Date of filing: 15.12.2010
(51) Int. Cl.: C07K 16/32

(54) **ANTI-HER2 ANTIBODIES AND THEIR USES**
ANTI-HER2-ANTIKÖRPER UND IHRE VERWENDUNG
ANTICORPS ANTI-HER2 ET LEURS UTILISATIONS

(30) Priority: 16.12.2009 US 287155 P
(43) Date of publication of application: 24.10.2012
(73) Proprietor: AbbVie Biotherapeutics Inc., Redwood City, CA 94063 (US)
(72) Inventor: HARDING, Fiona A., Santa Clara California 95050 (US); AKAMATSU, Yoshiko, Palo Alto California 94306 (US); DUBRIDGE, Robert B., Belmont California 94002 (US); POWERS, David B., Fairfax California 94930 (US)
(74) Representative: Roques, Sarah Elizabeth
(86) International application number: PCT/US2010/060533
(87) International publication number: WO 2011/084496

(56) References cited:
- WO-A1-92/22653
- WO-A2-03/068801
- WO-A2-03/074679
- WO-A2-03/087131
- WO-A2-2005/092925
- BOSTROM JENNY ET AL: "Variants of the antibody herceptin that interact with HER2 and VEGF at the antigen binding site", SCIENCE, AMERICAN ASSOCIATION FOR THE ADVANCEMENT OF SCIENCE, WASHINGTON, DC; US, vol. 323, no. 5921, 20 March 2009 (2009-03-20), pages 1610-1614, XP002560553, ISSN: 0036-8075, DOI: DOI:10.1126/SCIENCE.1165480
- LI BOHUA ET AL: "The protein-protein interface evolution acts in a similar way to antibody affinity maturation.", THE JOURNAL OF BIOLOGICAL CHEMISTRY 5 FEB 2010 LNKD- PUBMED:20007707, vol. 285, no. 6, 10 December 2009 (2009-12-10), pages 3865-3871, XP002626385, ISSN: 1083-351X

## Description

### 3. FIELD OF THE INVENTION

The present invention relates to anti-HER2 antibodies, pharmaceutical compositions comprising anti- HER2 antibodies, and therapeutic uses of such antibodies.

### 4. BACKGROUND

Monoclonal antibody therapy has provided an opportunity to target and destroy tumors using antibodies engineered against tumor-specific antigens. In general, monoclonal antibody therapy stimulates a patient's immune system to attack malignant tumor cells or prevents tumor growth by blocking or inhibiting specific cell receptors. Monoclonal antibodies used in cancer therapy generally target tumor-specific antigens on cell-surface molecules. Representative cell-surface molecules targeted in clinical trials include those originating from various lymphomas/leukemias (such as T-cell and/or B-cell lymphomas/leukemias) and solid tumors (such as epithelial tumors of the breast, colon, and lung).

Promising results have been reported in the treatment of breast cancer with humanized monoclonal antibodies, particularly treatments targeting the HER2 (neu/ErbB2) receptor.

HER2 is a transmembrane surface-bound receptor tyrosine kinase and is normally involved in the signal transduction pathways leading to cell growth and differentiation. HER2, also known as epidermal growth factor receptor 2, belongs to a family of epidermal growth factor receptors (EGFRs) including HER1 (ErbB 1), HER3 (ErbB3), and HER4 (ErbB4) (Hudis, 2007, N Engl J Med 357(1):39-51). The ErbB receptors typically dimerize on ligand binding. Although HER2 has no known ligand, it is the preferential dimerization partner of other members of the ErbB family. (Hudis, 2007, N Engl J Med 357(1):39-51). Overexpression of HER2 results in the induction of angiogenesis, a component of cancer growth, and the evocation of an antitumor T-cell response (Ménard et al, 2003, Oncogene 22:6570-6578). HER2 is overexpressed in about one-quarter of breast cancer patients (Bange et al, 2001, Nature Medicine 7:548- 552).

The development of a monoclonal antibody therapy based on the discovery of the role of HER2 in breast cancer first involved the development of a murine-based antibody. Researchers discovered that the murine monoclonal antibody 4D5 had a significant and dose dependent efficacy specifically for HER2 overexpressing cancer cells, while having no effect on cells expressing physiological levels of HER2. However, murine antibodies elicit an immunogenic response in human patients. Murine monoclonal antibodies can be humanized (thereby reducing the murine-induced immune response) by identification of a minimum set of amino acid residues in the complementarity determining regions (CDRs) of the murine antibody required for antigen specificity and antigen binding affinity and substituting these regions into the CDRs of a consensus human IgG framework. The framework regions are the non-CDR regions in the variable chains of the antibody. Accordingly, the murine (4D5) monoclonal antibody was humanized, resulting in a recombinant, humanized monoclonal antibody directed against HER2. This drug is commercially known as Herceptin® (trastuzumab), which gained FDA marketing approval in late 1998.

Herceptin® is known to bind with high affinity to the extracellular domain of the HER2 protein, thereby inhibiting the proliferation of human tumor cells that overexpress HER2. Herceptin® is also a mediator of antibody-dependent cellular cytotoxicity (ADCC) which has been shown to be preferentially exerted on HER2 overexpressing cancer cells compared with cancer cells that do not overexpress HER2.

Herceptin® can elicit an immune response when administered to humans. Such an immune response can result in an immune complex-mediated clearance of the antibodies or fragments from the circulation, and make repeated administration unsuitable for therapy, thereby reducing the therapeutic benefit to the patient and limiting the re-administration of the antibody. Further, Herceptin® may be contraindicated in patients with pre-existing heart disease. Herceptin® has been associated with cardiac dysfunction in 2-7% of cases (Borghesi et al., 2006, Immunol Res 36(1-3):27-32). Also, while up to 70% of HER2-positive breast cancers demonstrate a response to Herceptin®-based therapies, resistance almost inevitably arises within a year of the initial response. Finally, additional problems with tumor-specific or tumor-selective monoclonal antibodies such as Herceptin® as therapeutic agents include antigenic variation of the tumor, inefficient killing of cells after binding the monoclonal antibody, inefficient penetration of the antibody into the tumor mass, and soluble target antigens mopping up the antibody.

Accordingly, there is a need to provide improved monoclonal antibodies that interfere with the HER2 receptor that overcome one or more of these problems, for example, by generating variants with higher affinity than Herceptin® that can be administered at reduced dosages, or variants with reduced immunogenicity and other side-effects as compared to Herceptin®. Furthermore, there is a need to provide variants with increased expression in heterologous hosts, with increased solubility, with decreased heterogeneity due to glycosylation and/or with increased stability, e.g., with respect to oxidation, deamidation and/or cyclization of amino acids.

Citation or identification of any reference in Section 4 or in any other section of this application shall not be construed as an admission that such reference is available as prior art to the present disclosure.

Bostrom et al. (Science, vol. 323, no. 5921, 2009) discloses variants of herceptin that interact with HER2 and VEGF at the antigen binding site.

Li et al (J Biol Chem, vol. 285, no. 6, 2009) discloses protein-protein interface evolution acting in a similar way to antibody affinity maturation.

WO 03/074679 discloses the use of computational screening methods to optimise the physio-chemical properties of antibodies.

WO 92/22653 discloses variant immunoglobulins along with methods for their preparation and use. WO 2005/09295 discloses antibody variants outside the Fc region that alter binding affinity to one or more effector ligands, method for their generation, and their therapeutic application.

WO 03/0688 01 discloses antibody variants with faster antigen association rates.

WO 03/087131 discloses novel antibody variants, having substitutions at positions within the variable domains of the heavy and light chain.

### 5. SUMMARY

The present disclosure relates to improved anti-HER2 antibodies that are related in sequence to the anti-HER2 antibody trastuzumab. In certain aspects, the present disclosure relates to variants of trastuzumab or binding fragments of trastuzumab that are less immunogenic than and/or have binding affinity improvements relative to trastuzumab. Trastuzumab is a humanized version of the antibody produced by the hybridoma 4D5.

The present invention provides for an anti-HER2 antibody or an anti-HER2 binding fragment of an antibody which comprises a VH and a VL that have one or more amino acid substitutions or combinations of amino acid substitutions as compared to an antibody having a VH comprising an amino acid sequence corresponding to positions 1 to 117 of SEQ ID NO:1 and a VL comprising an amino acid sequence corresponding to positions 1 to 103 of SEQ ID NO:2, wherein said one or more amino acid substitutions or combinations of amino acid substitutions are selected from:
(i) the third framework of the VH chain (FR-H3) substitution R83K as compared to a VH of SEQ ID NO:1;
(ii) the first CDR of the VL chain (CDR-L1) substitution D28L + A34D as compared to a VL of SEQ ID NO:2;
(iii) the first CDR of the VL chain (CDR-L1) substitution A34D as compared to a VL of SEQ ID NO:2;
(iv) the first CDR of the VL chain (CDR-L1) substitution A34V as compared to a VL of SEQ ID NO:2;
(v) the first CDR of the VL chain (CDR-L1) substitutions Q27S + A34D as compared to a VL of SEQ ID NO:2;
(vi) the first CDR of the VL chain (CDR-L1) substitutions Q27L + A34V as compared to a VL of SEQ ID NO:2;
(vii) the first CDR of the VL chain (CDR-L1) substitutions Q27F + A34D as compared to a VL of SEQ ID NO:2; and
(viii) the first CDR of the VL chain (CDR-L1) substitutions D28N + A34D as compared to a VL of SEQ ID NO:2.

The present disclosure provides anti-HER2 antibodies that have heavy and light chain CDRs that have at least one amino acid substitution as compared to the heavy and/or light chain CDRs of trastuzumab. The present disclosure also provides anti-HER2 antibodies that have at least one amino acid substitution as compared to the heavy and/or light chain framework regions of trastuzumab.

Accordingly, the present disclosure provides anti-HER2 antibodies having: (1) light chain CDRs with at least one amino acid substitution as compared to the light chain CDRs of trastuzumab; and/or (2) heavy chain CDRs with at least one amino acid substitution as compared to the heavy chain CDRs of trastuzumab; (3) light chain framework regions with at least one amino acid substitution as compared to the framework regions of trastuzumab; and/or (4) heavy chain framework regions with at least one amino acid substitution as compared to the framework regions of trastuzumab.

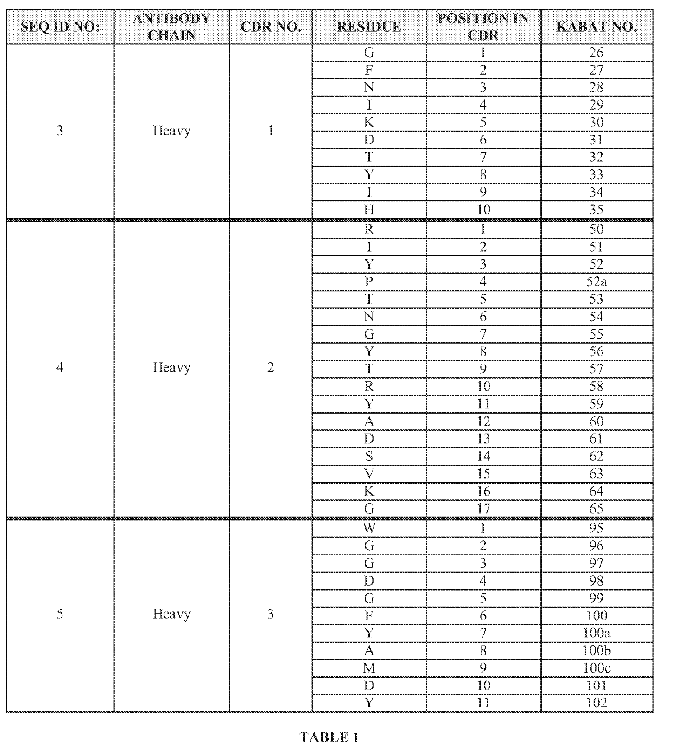

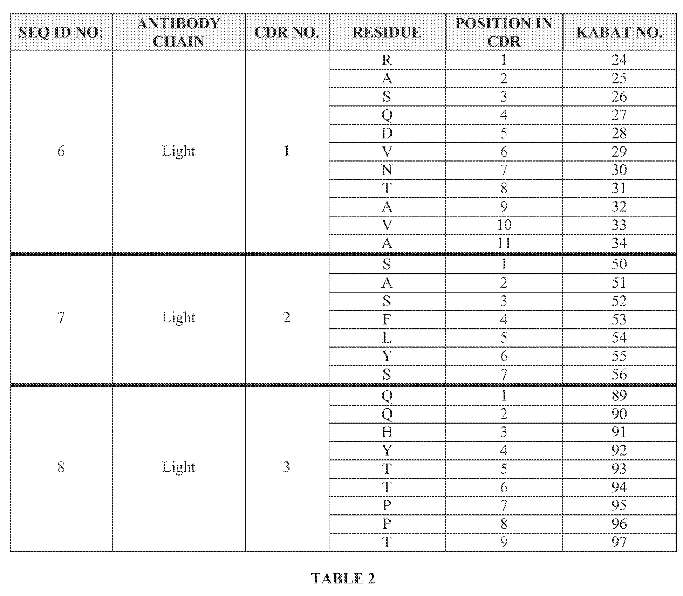

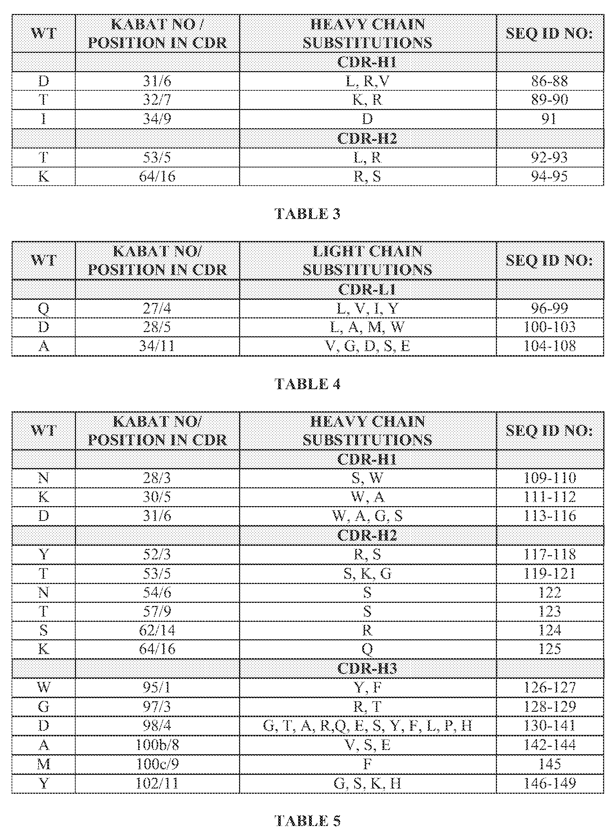

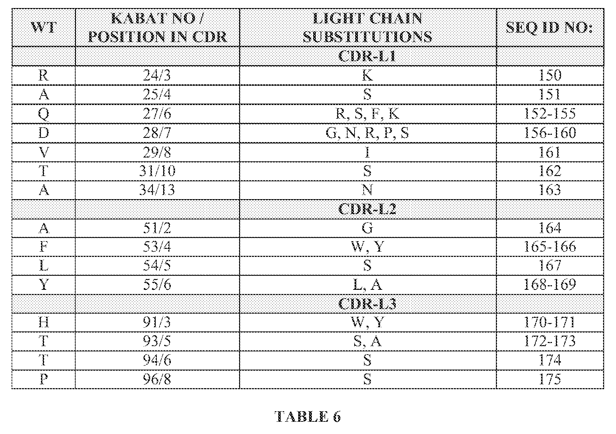

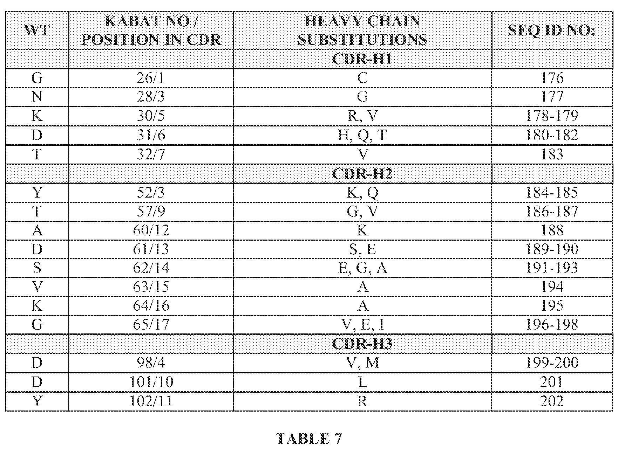

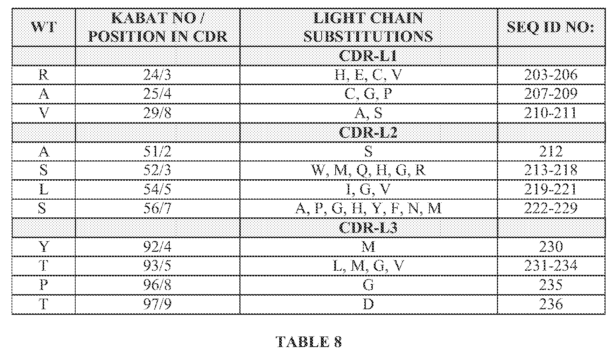

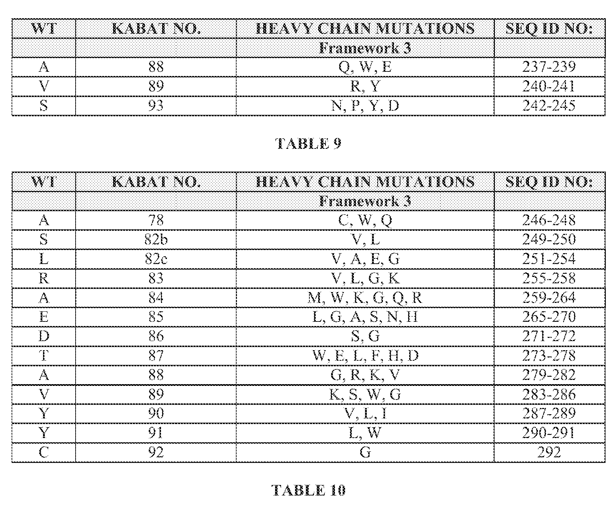

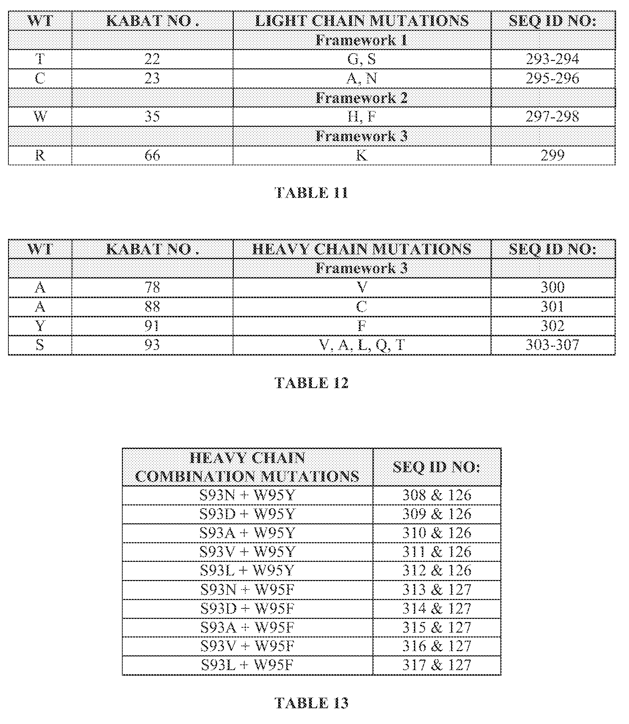

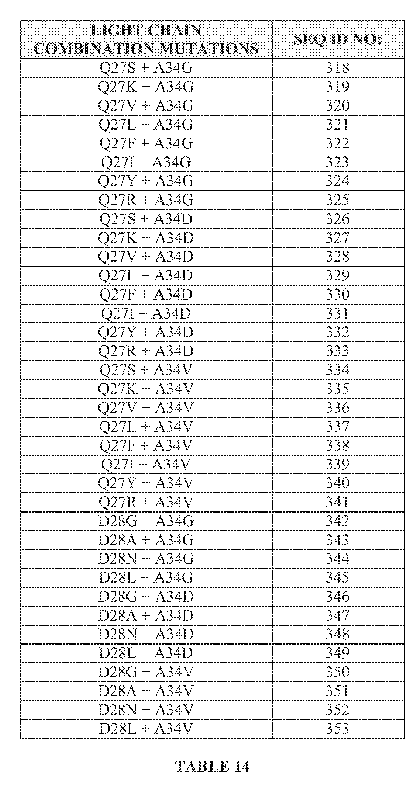

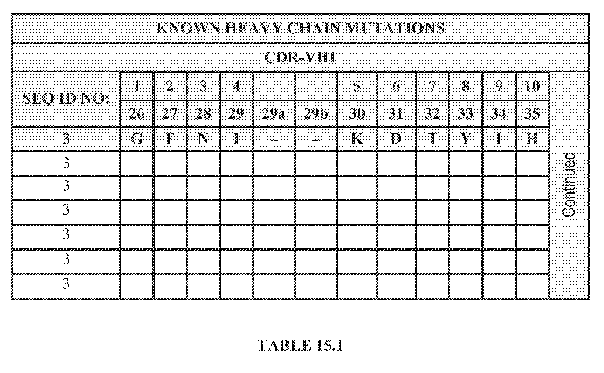

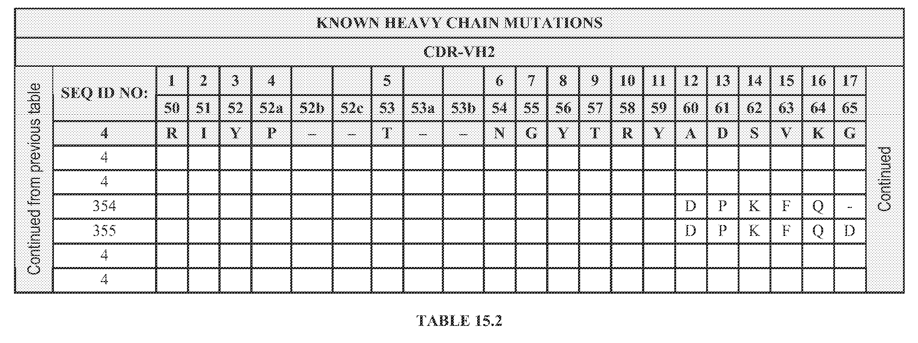

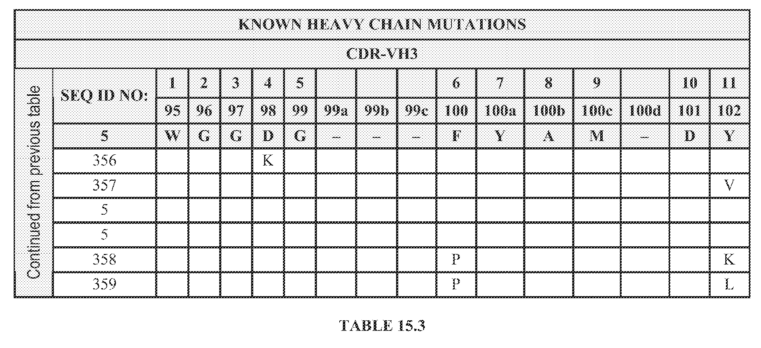

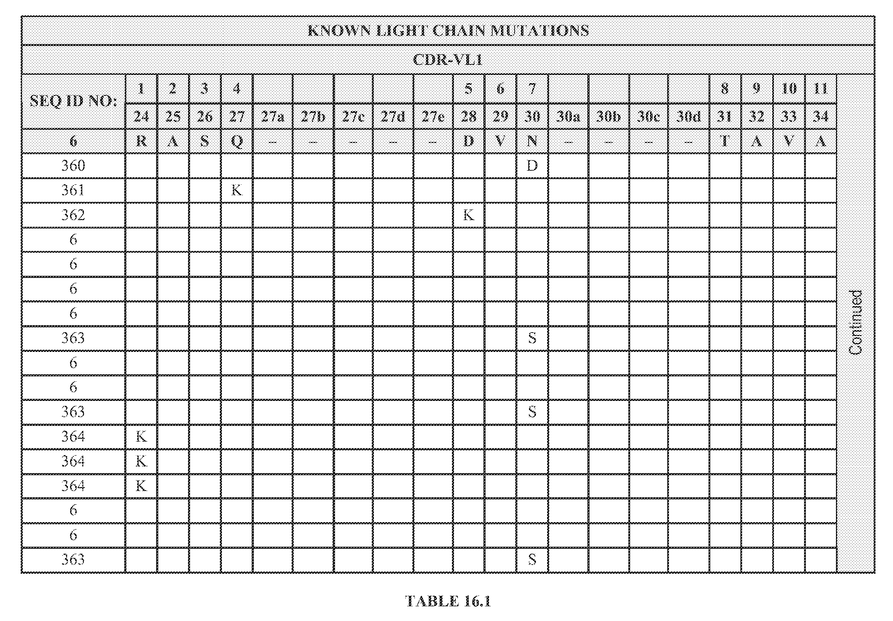

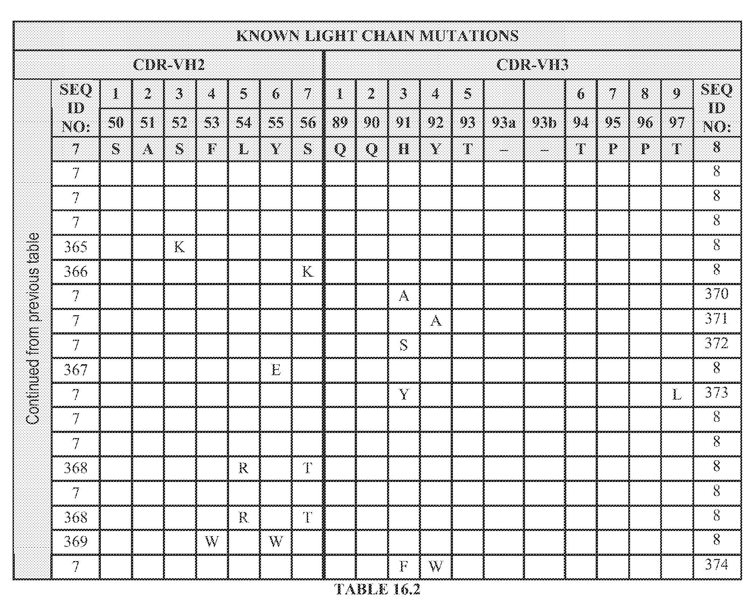

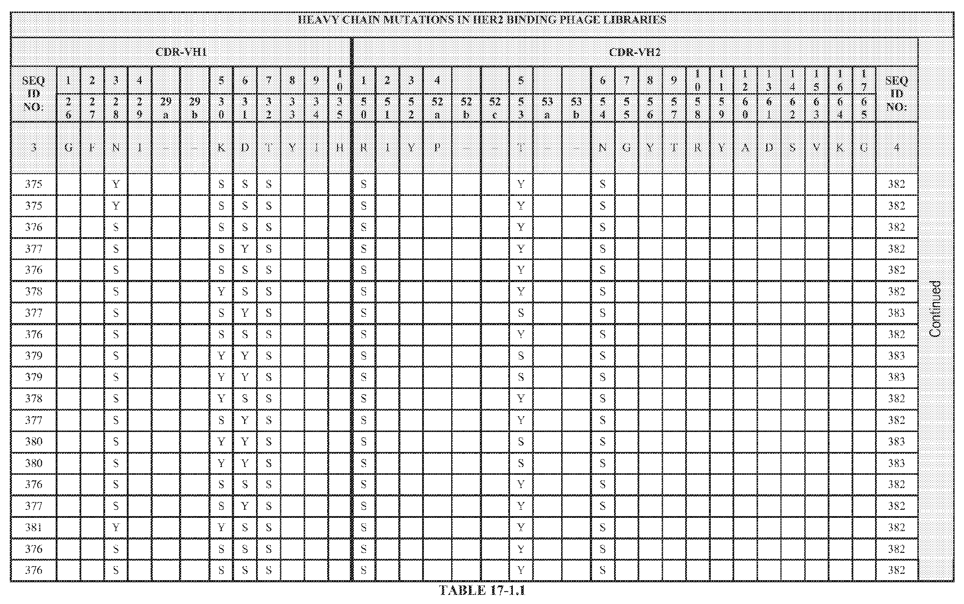

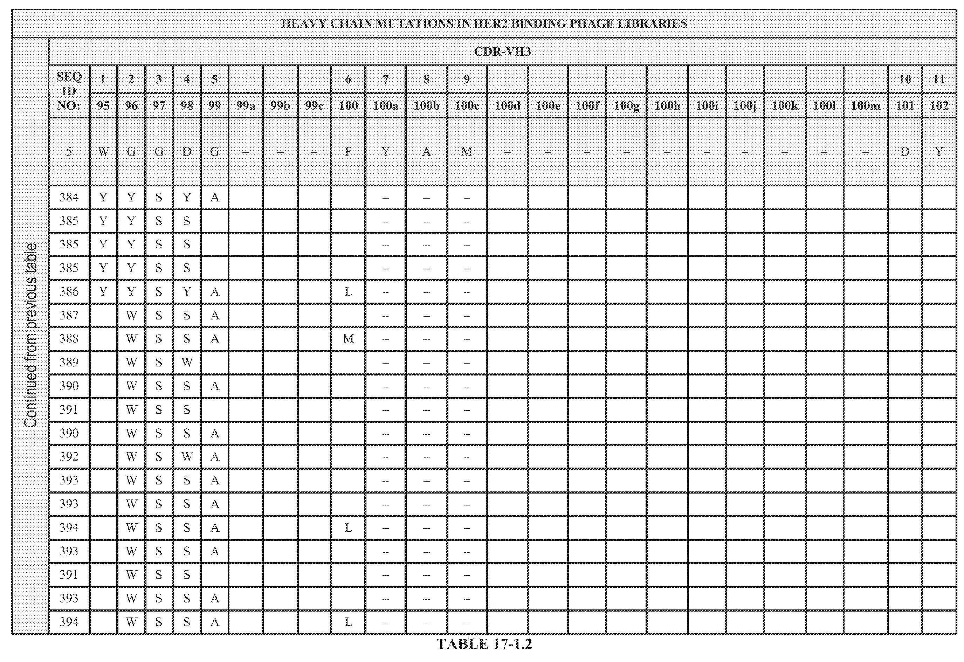

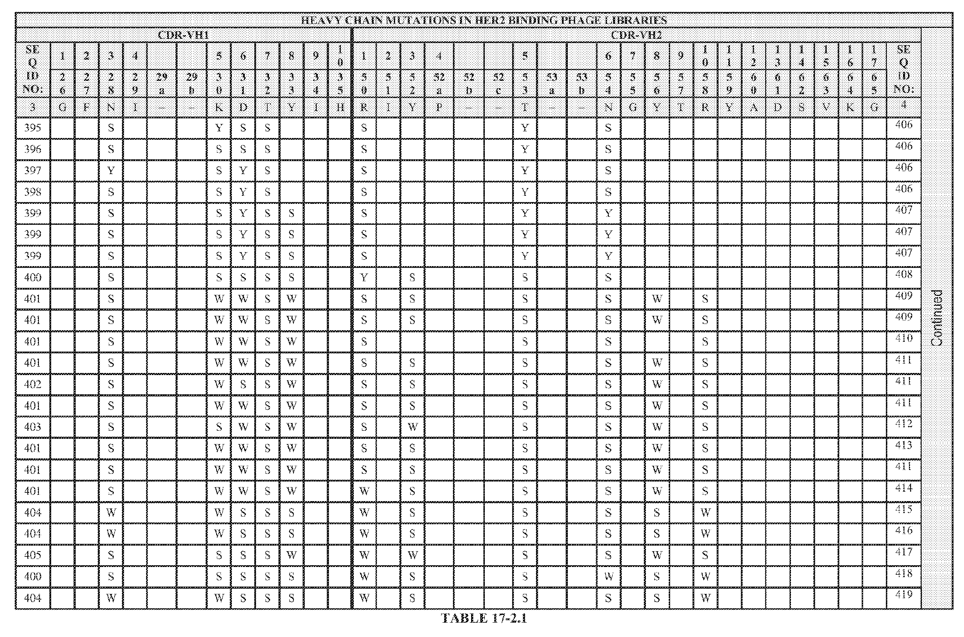

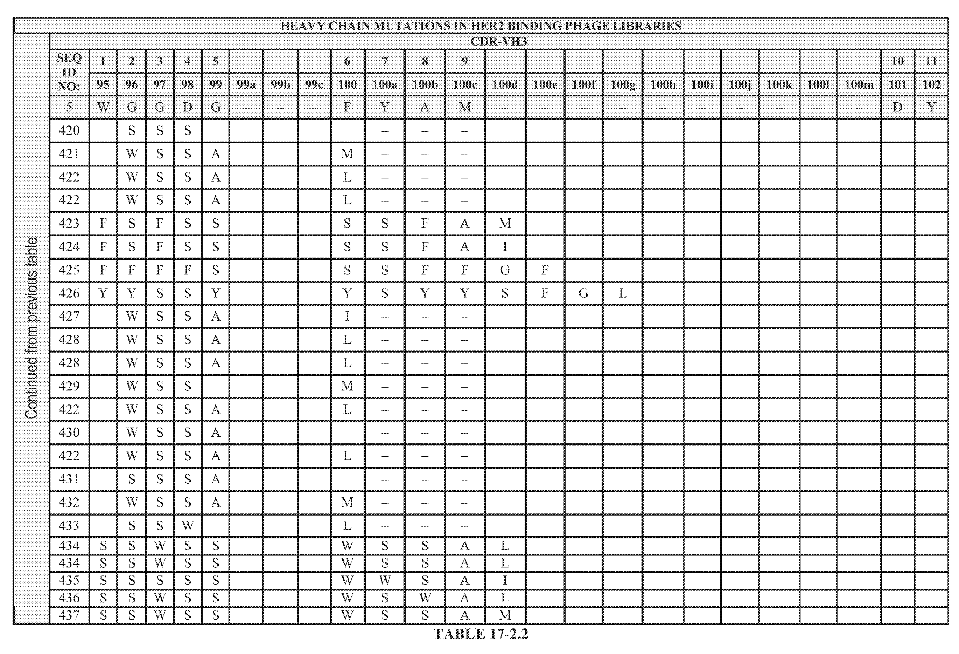

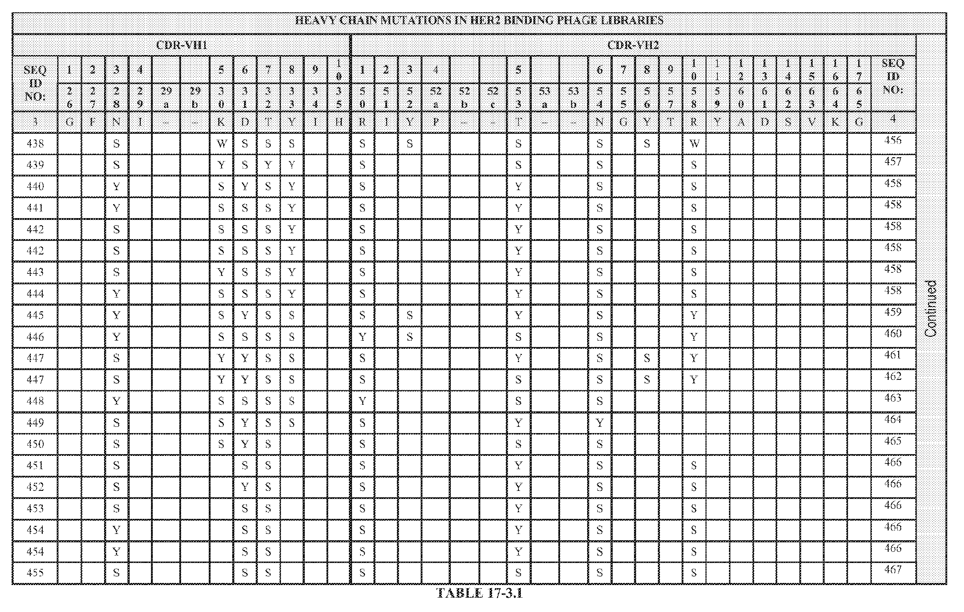

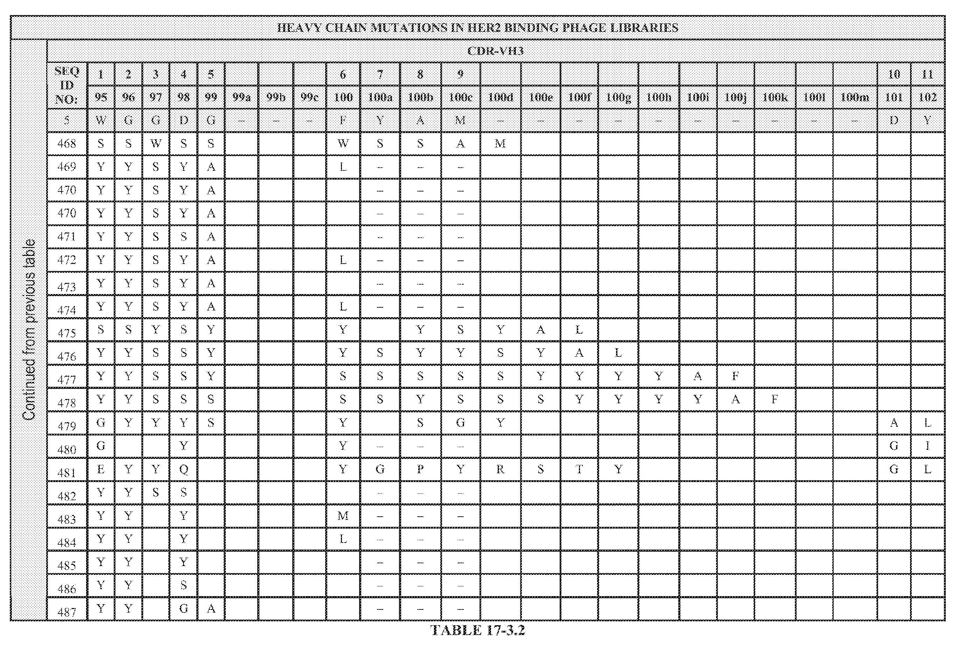

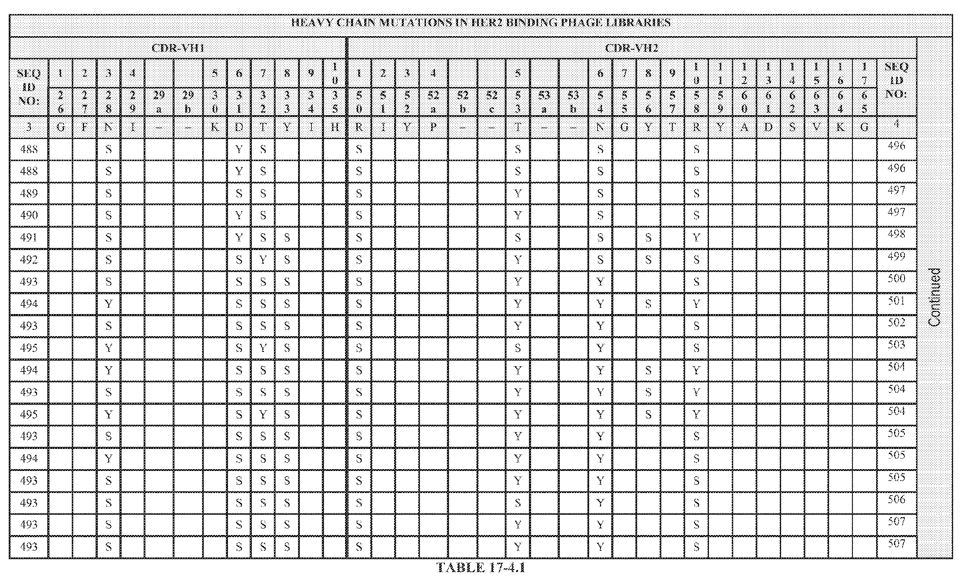

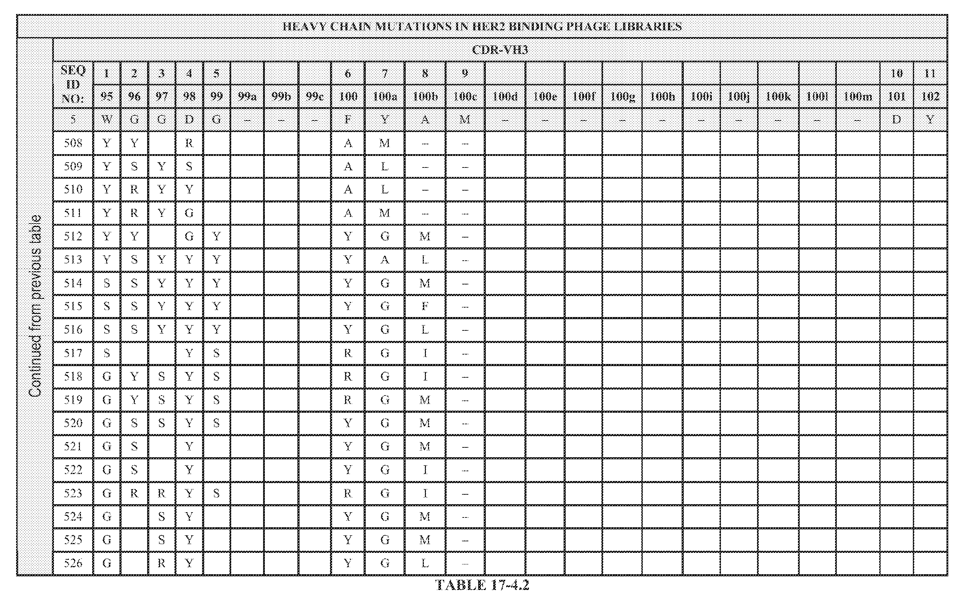

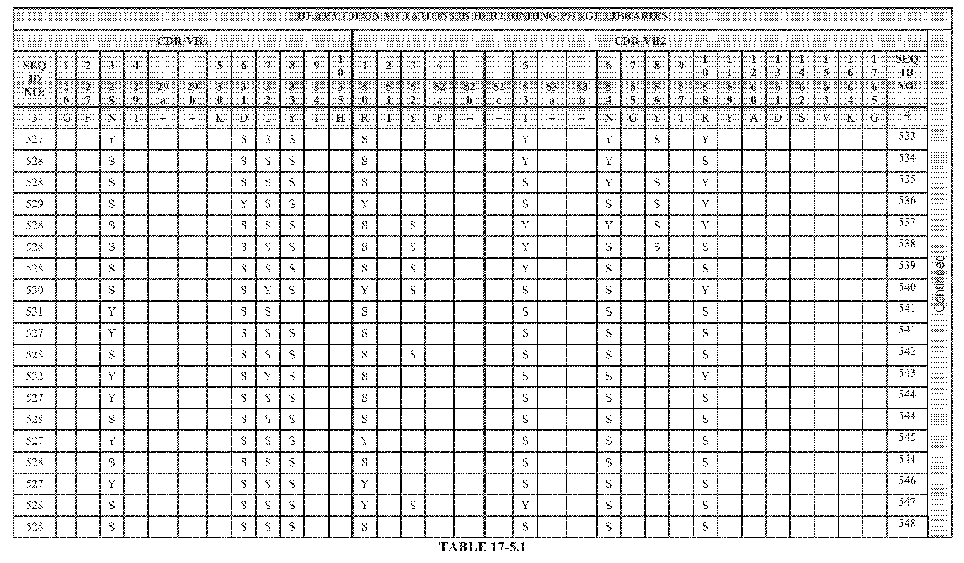

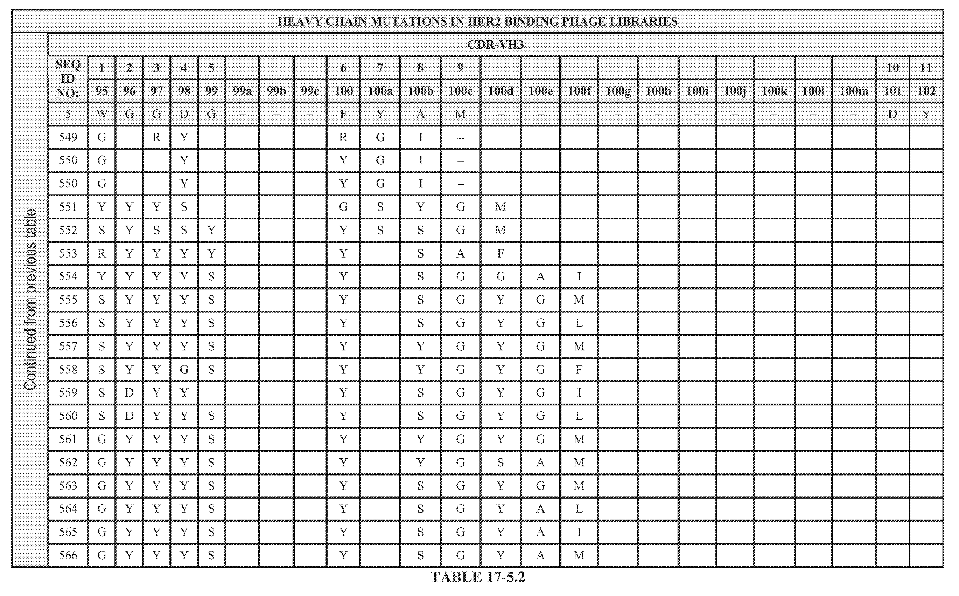

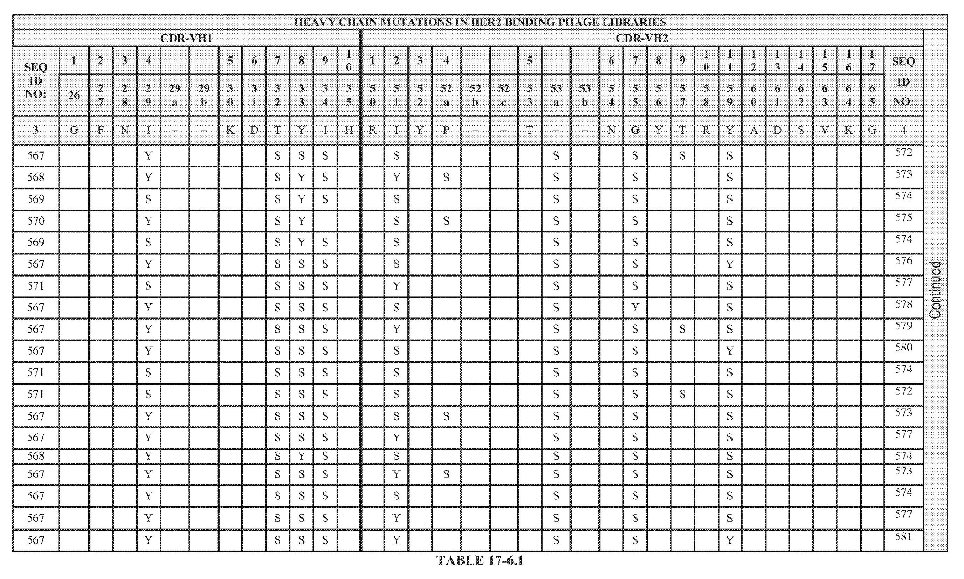

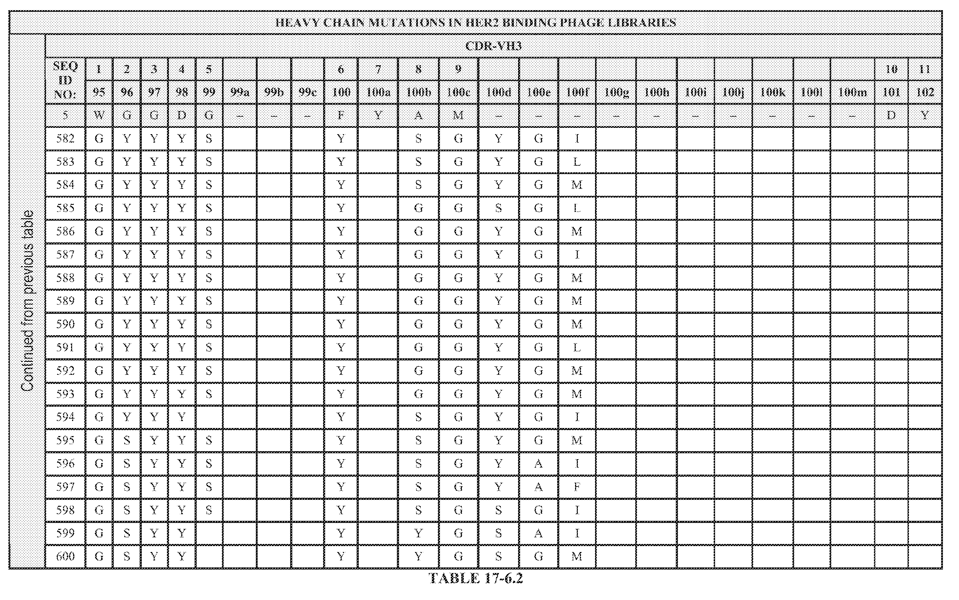

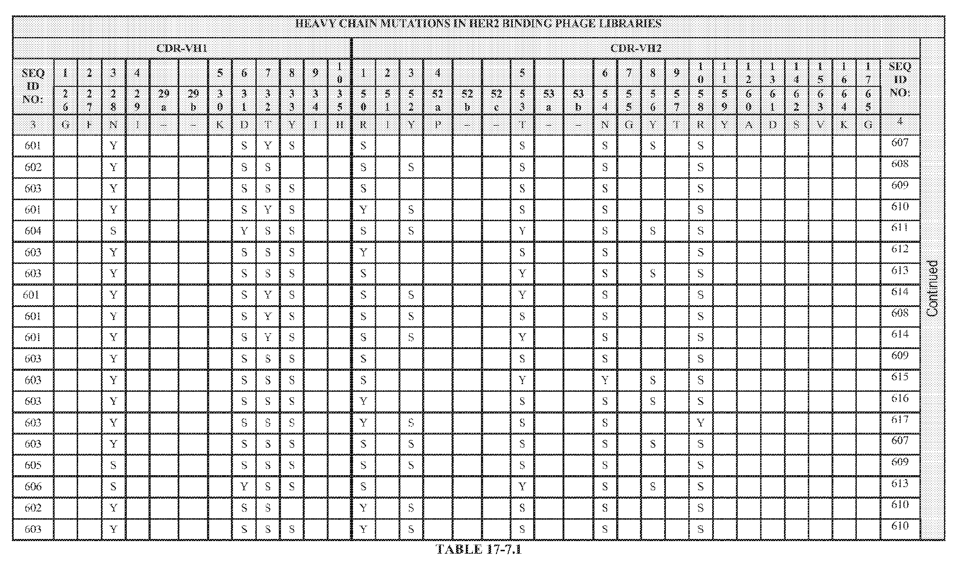

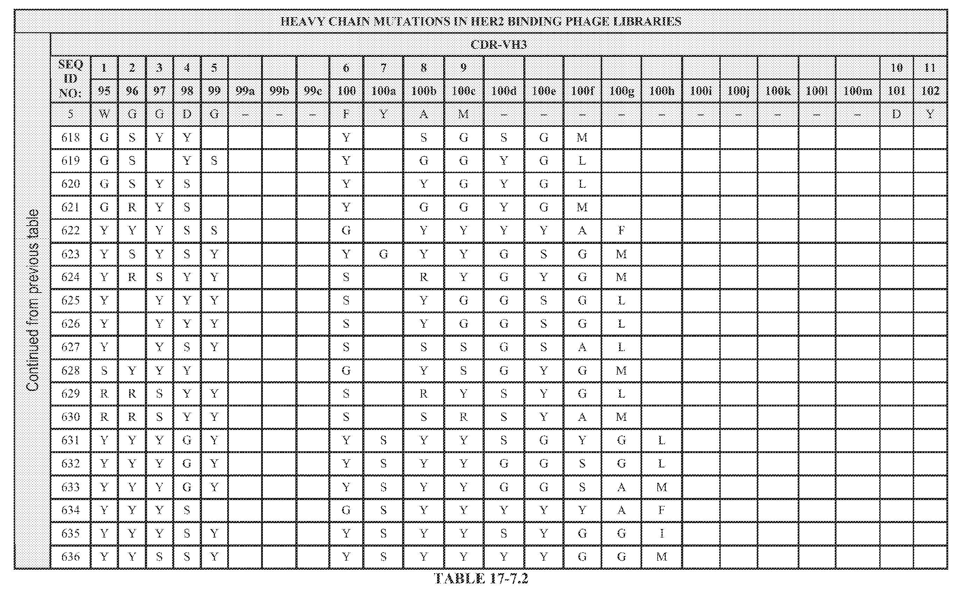

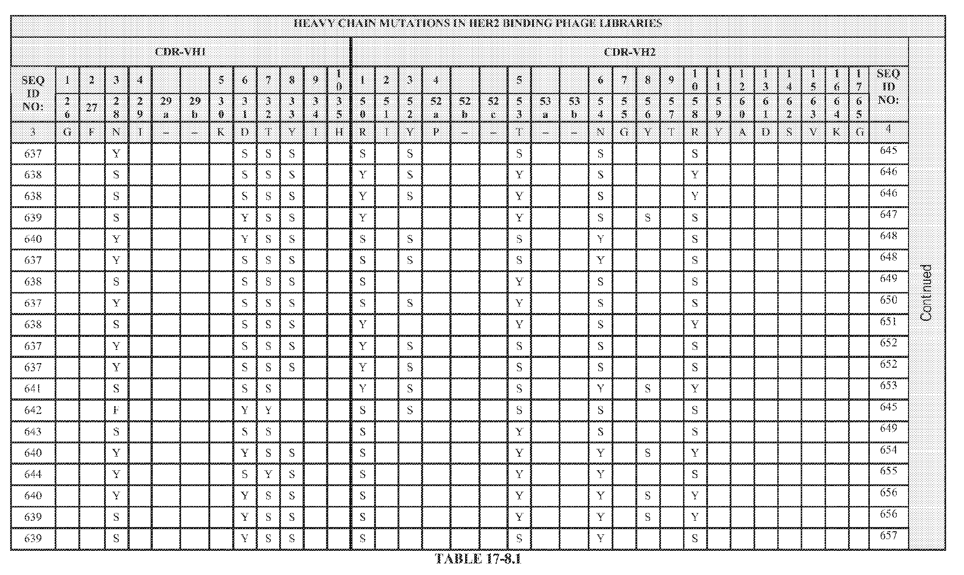

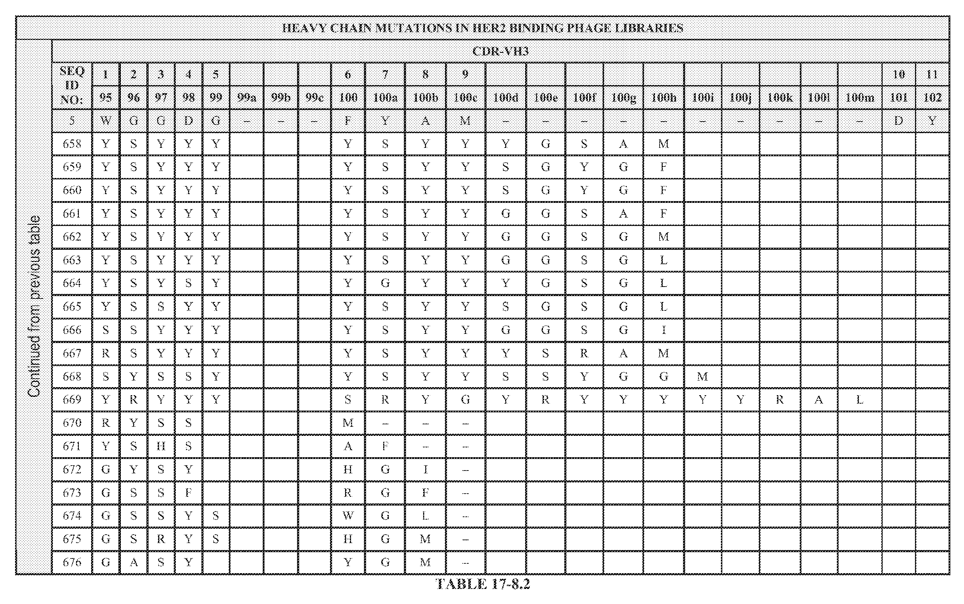

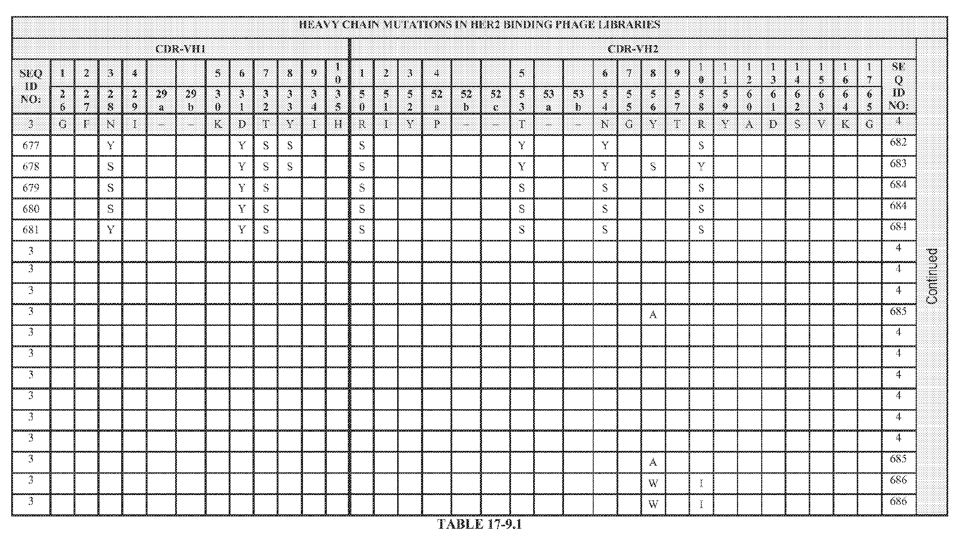

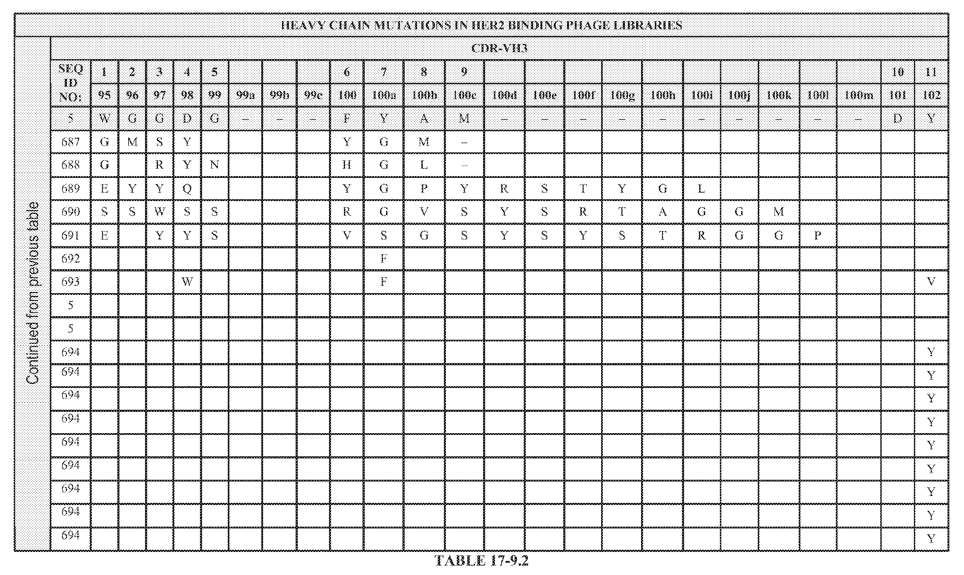

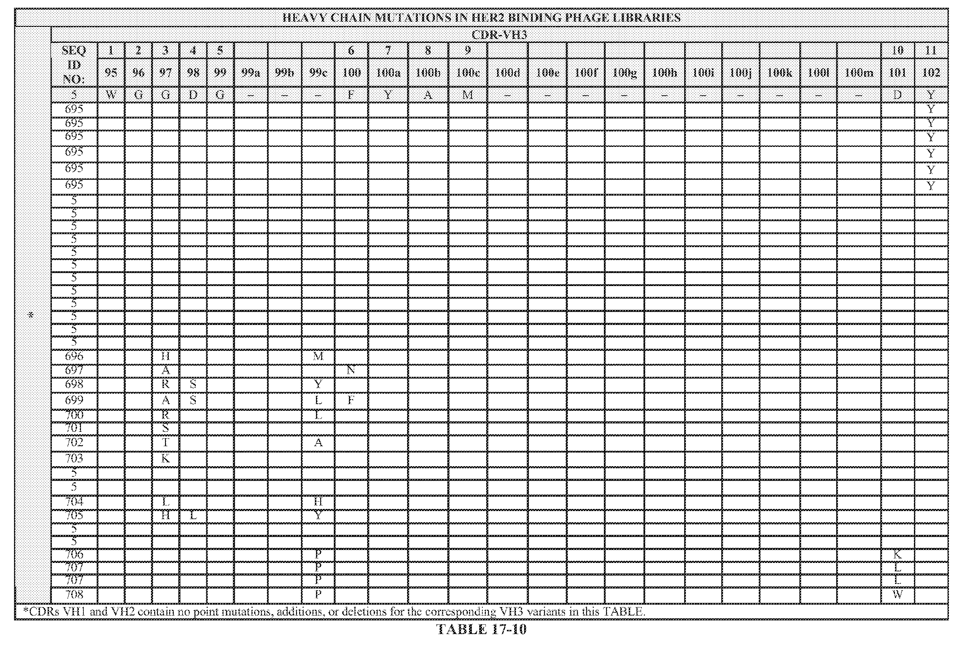

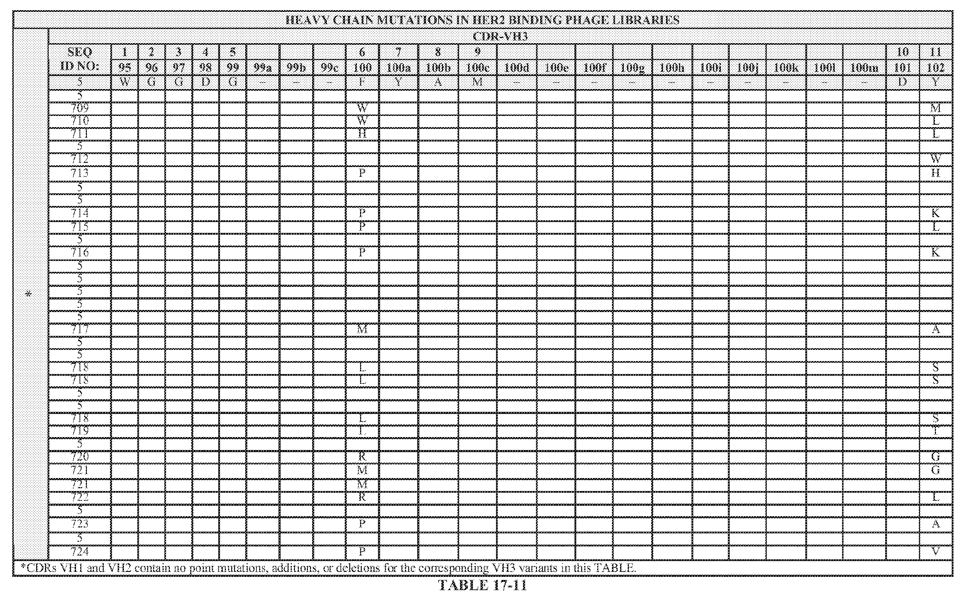

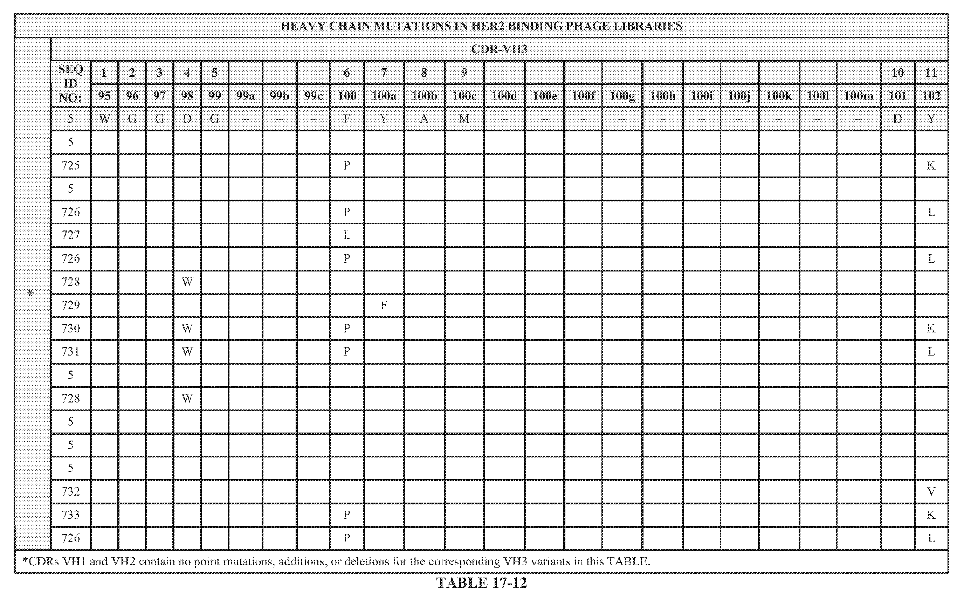

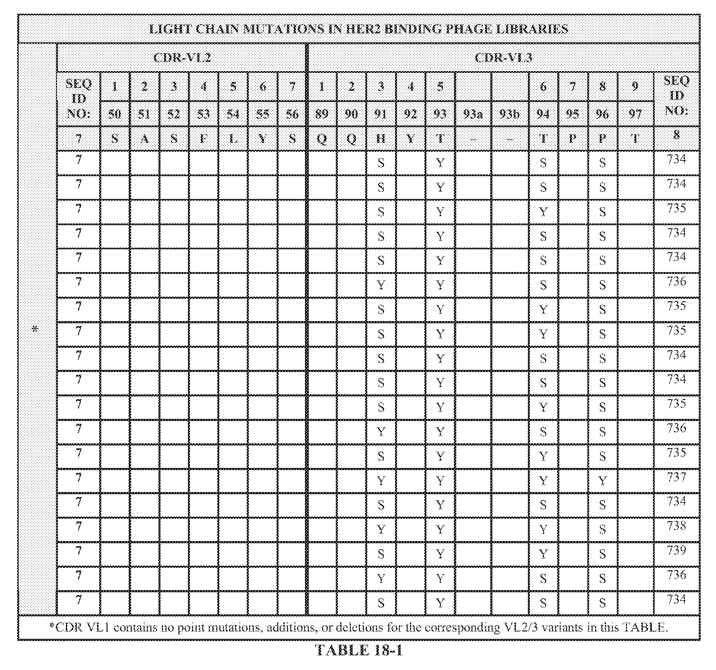

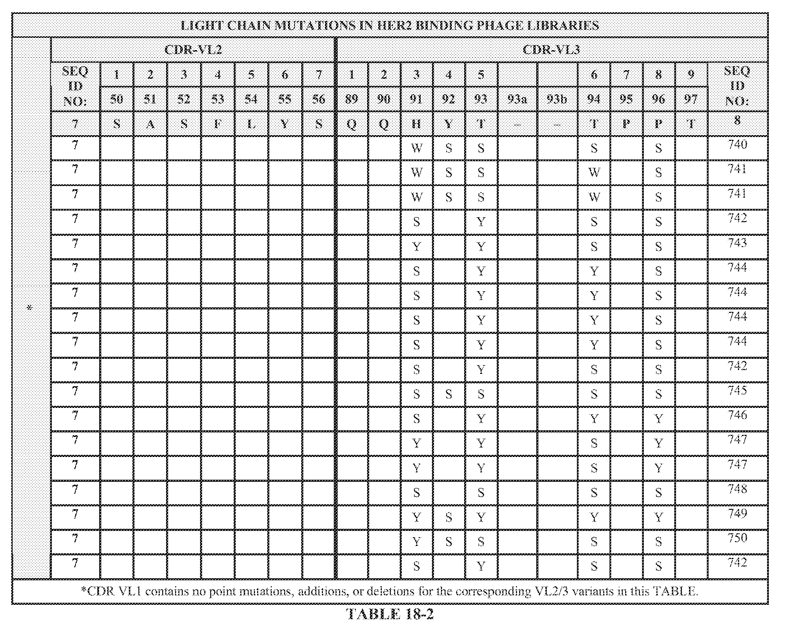

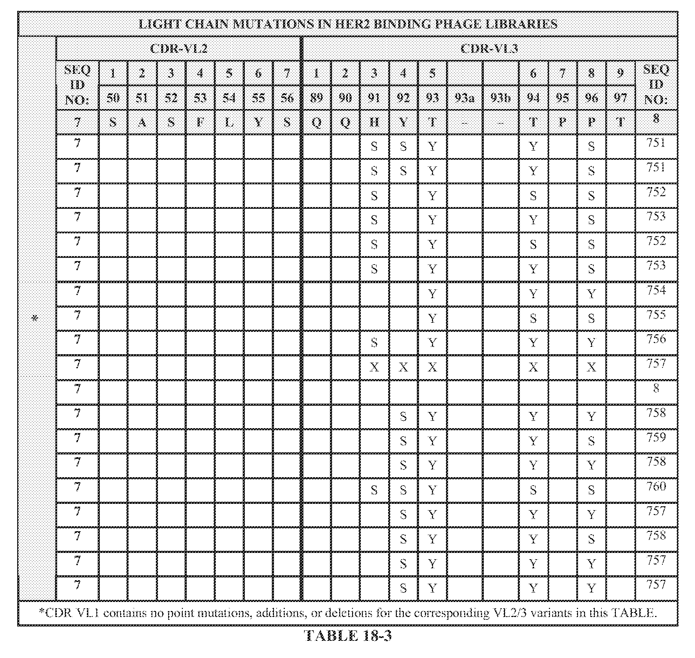

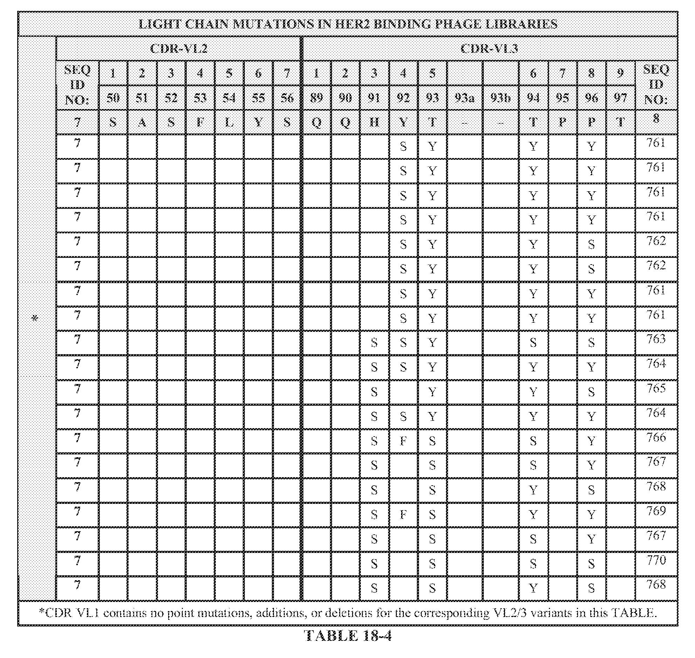

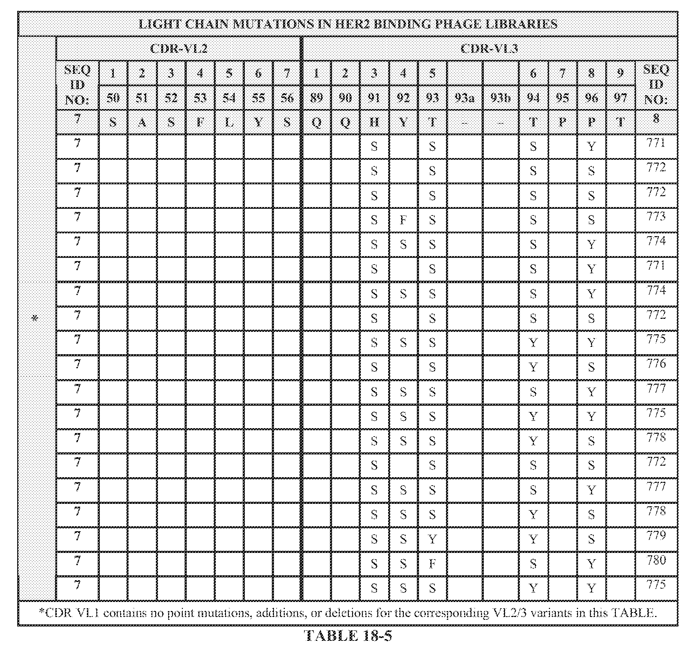

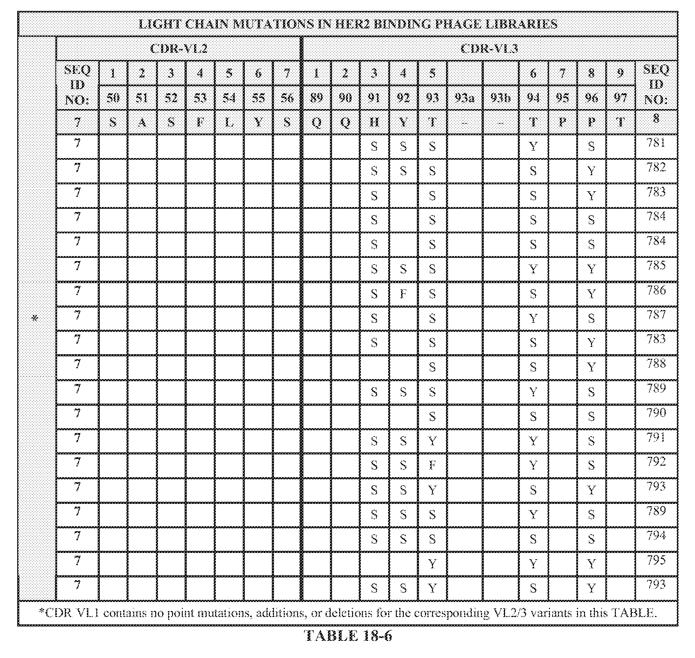

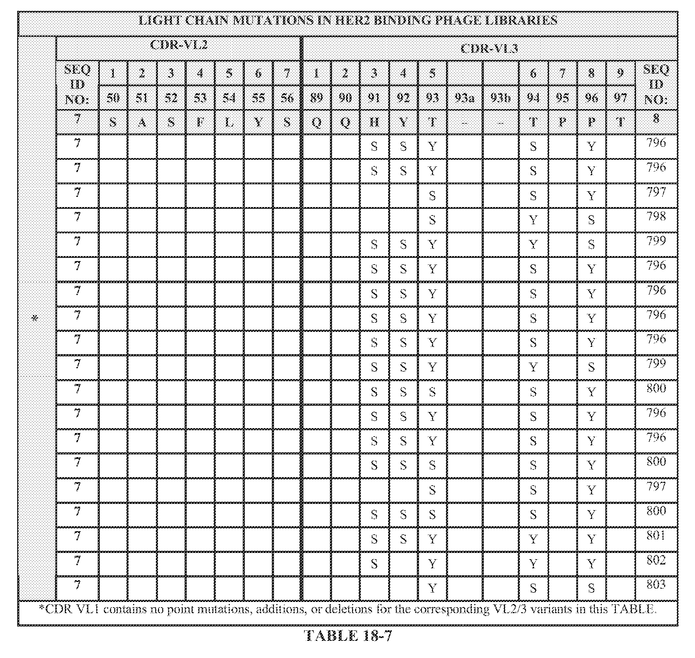

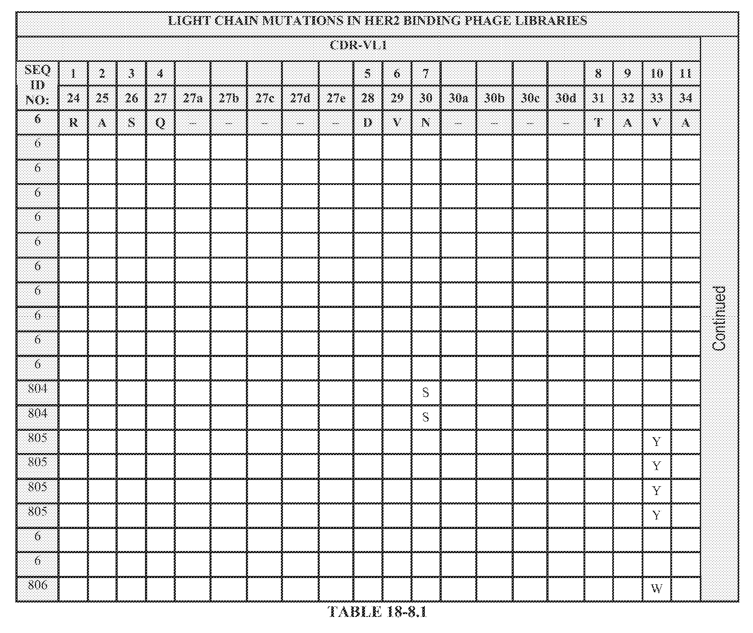

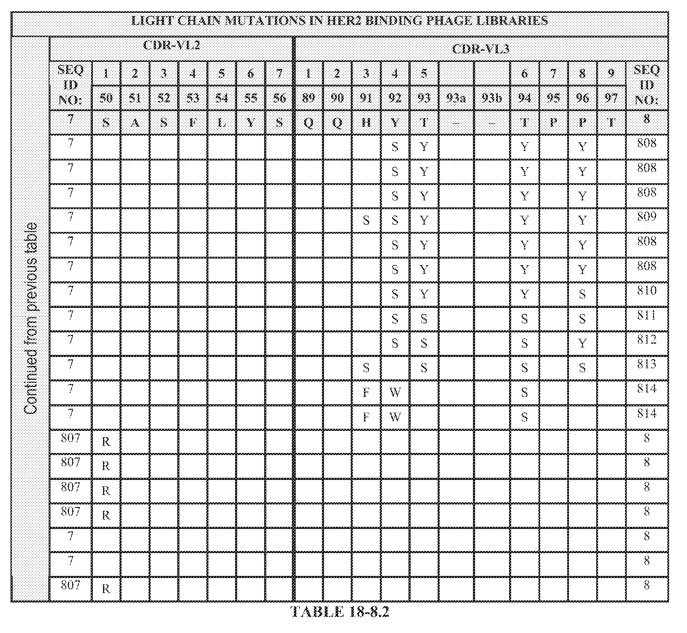

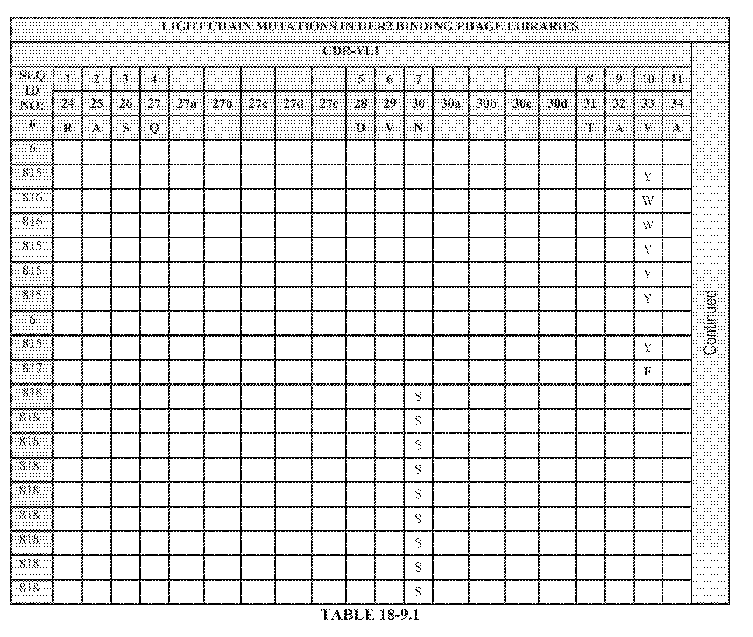

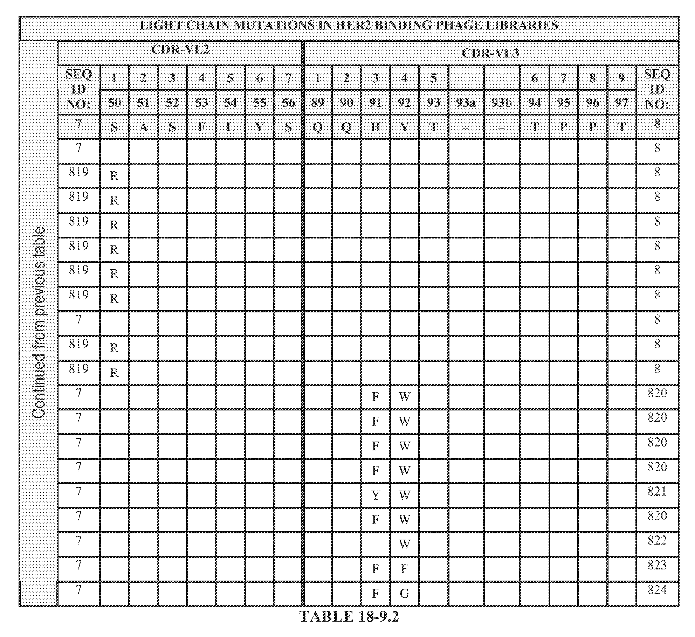

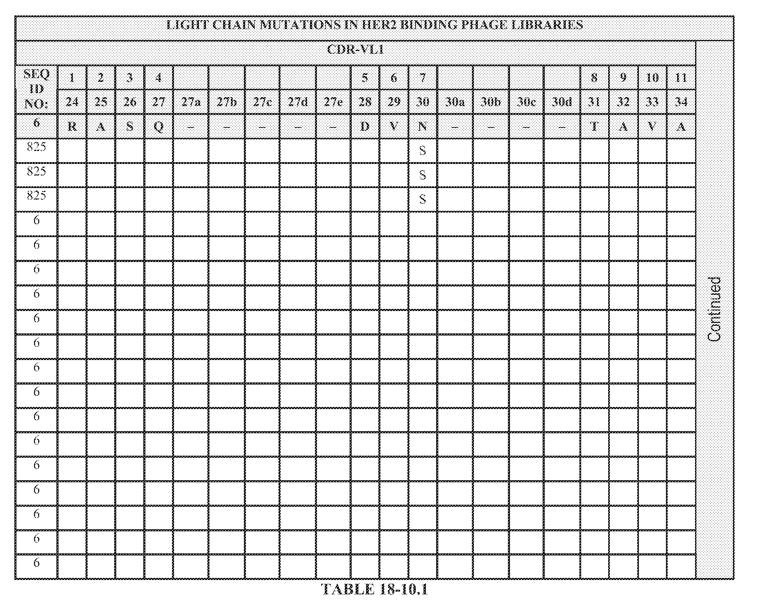

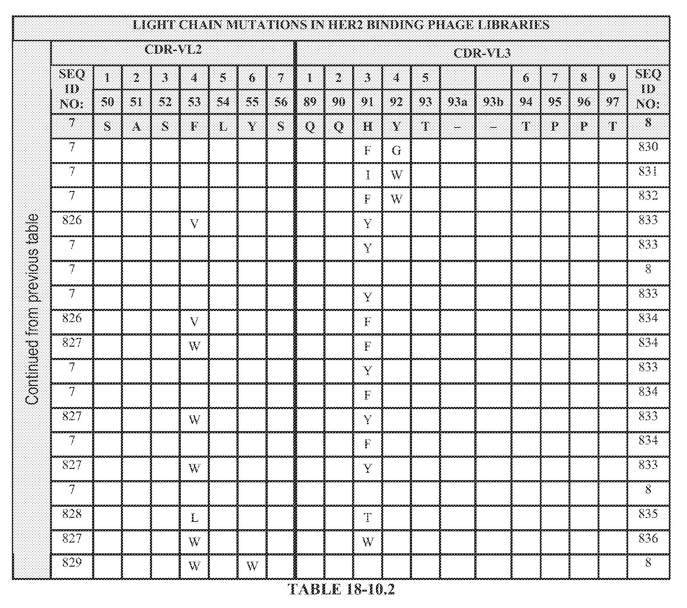

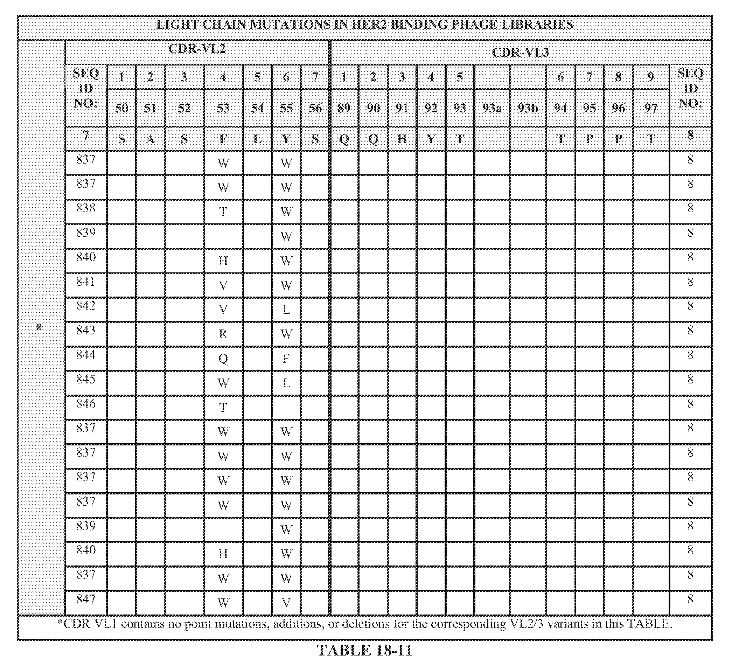

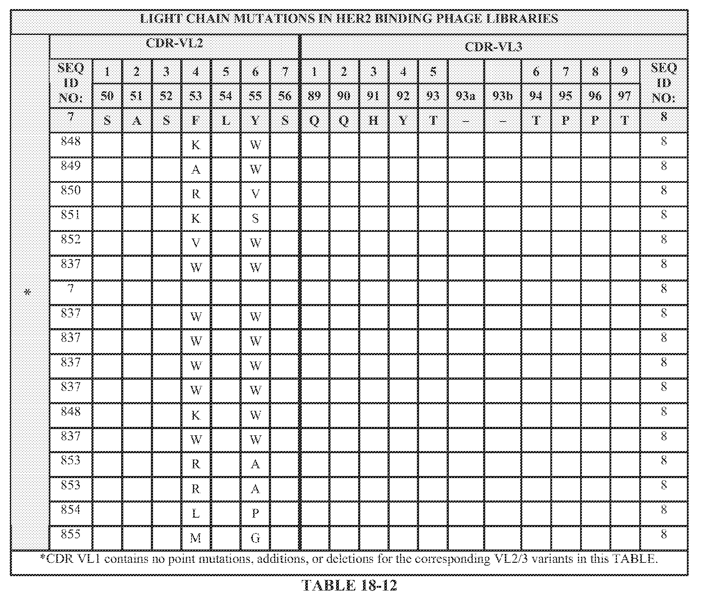

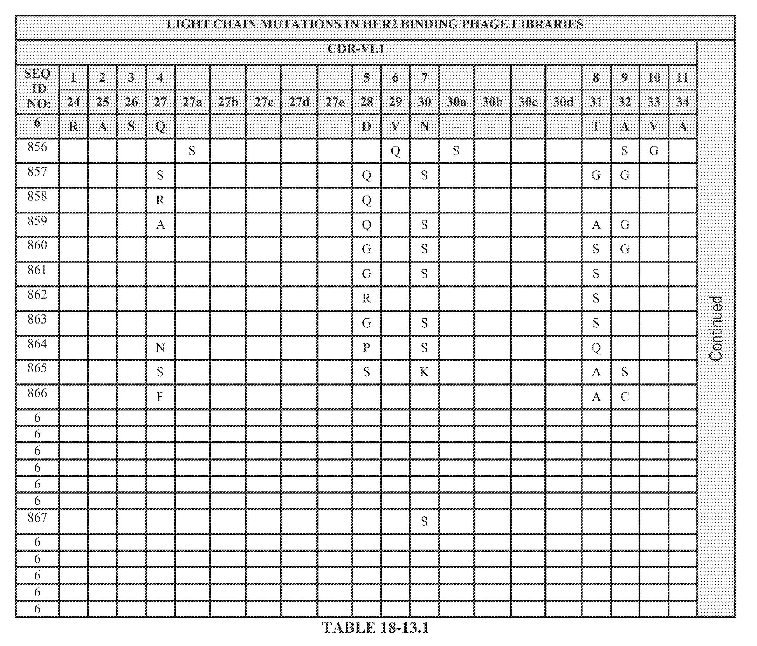

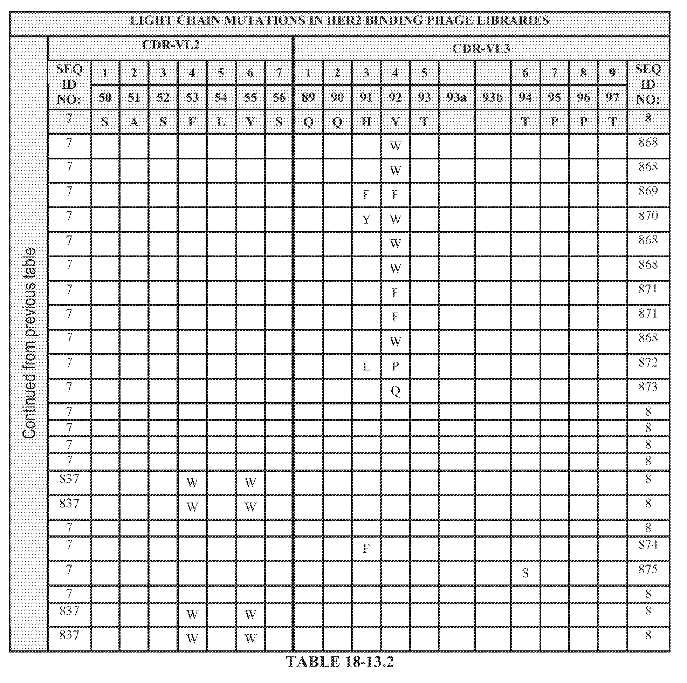

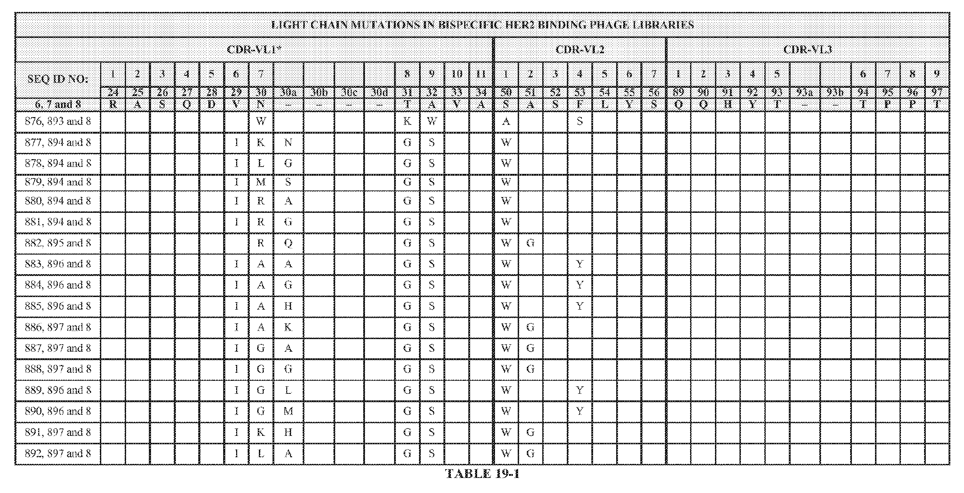

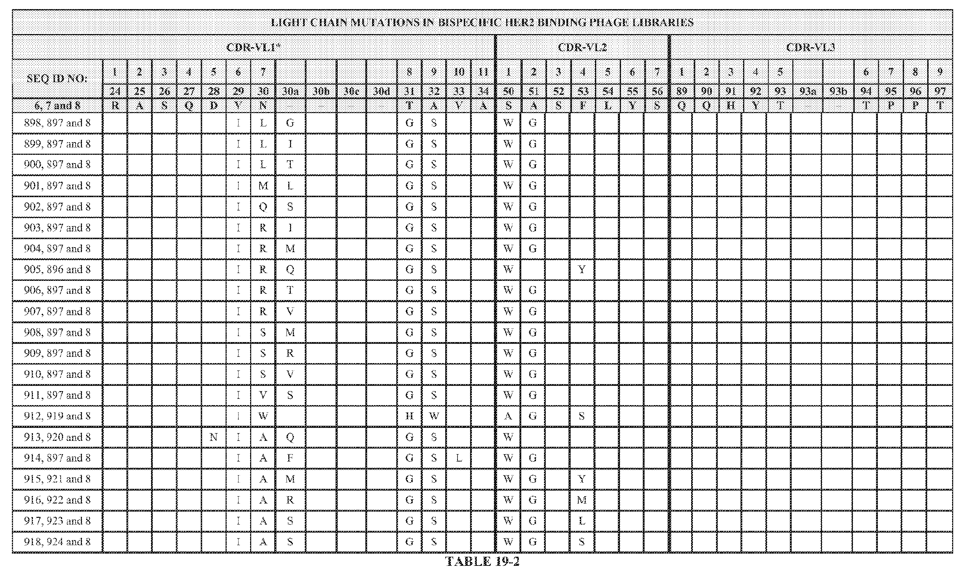

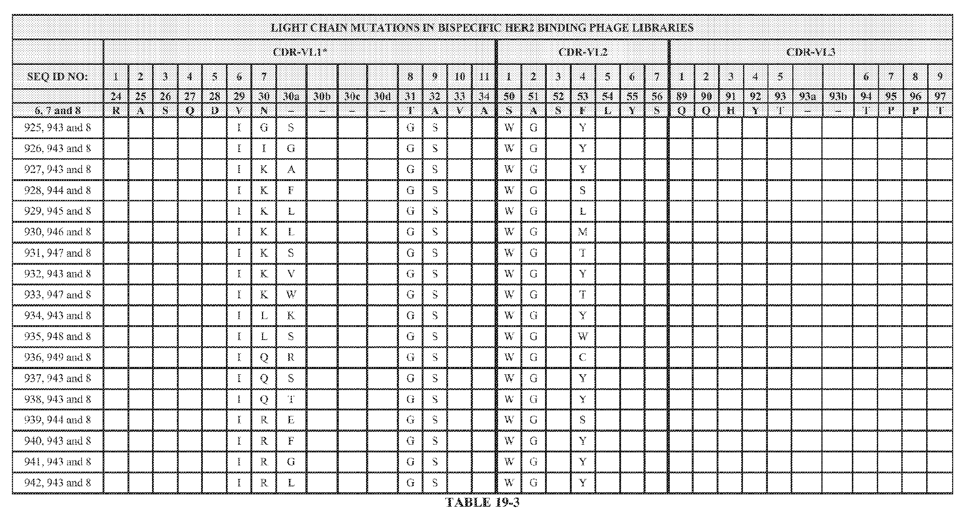

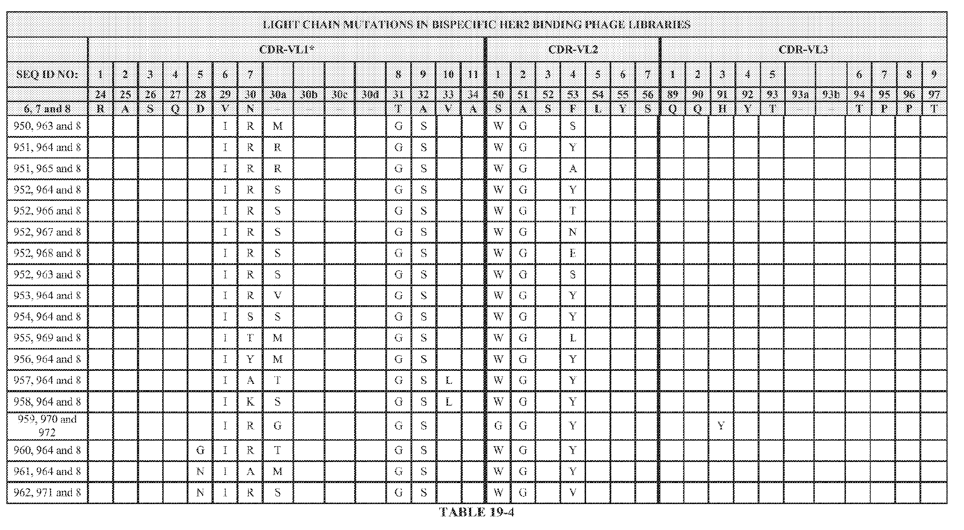

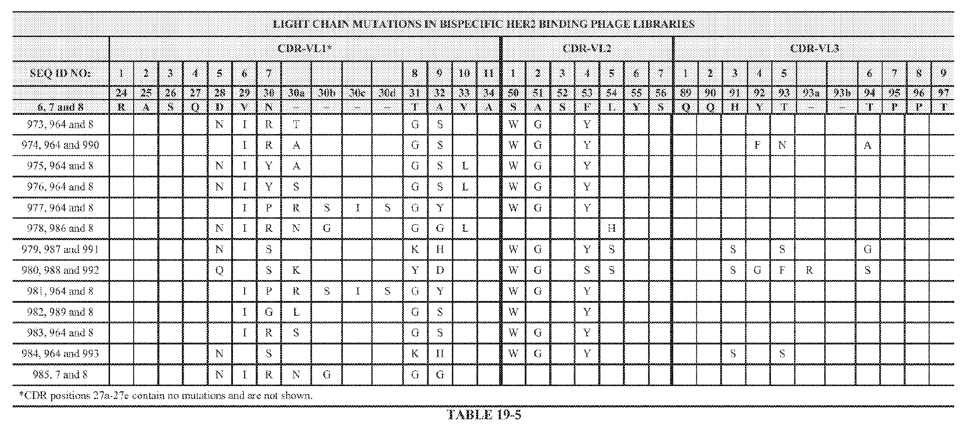

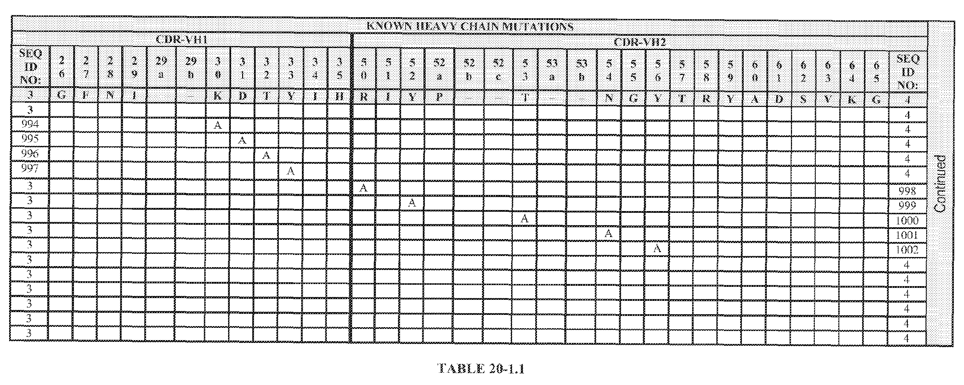

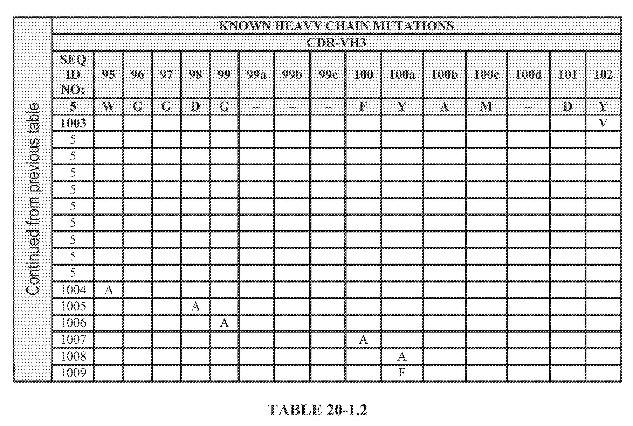

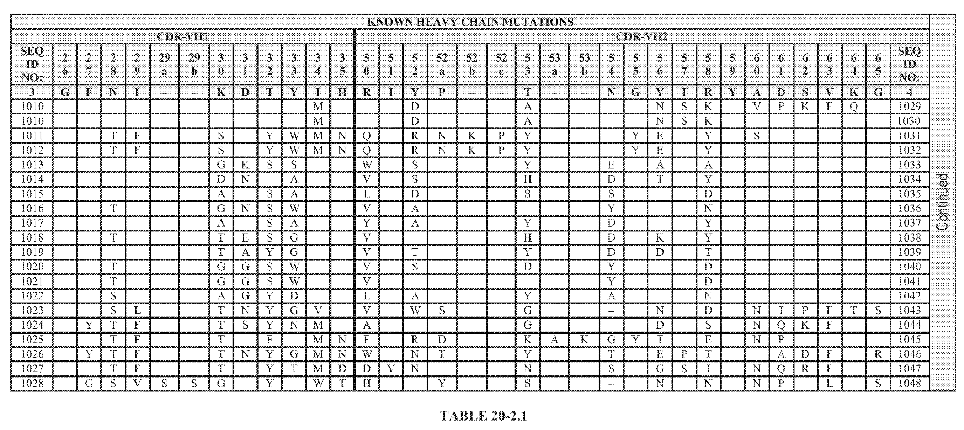

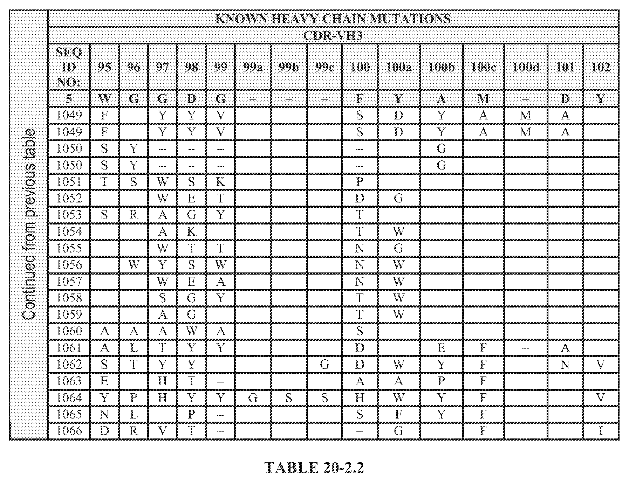

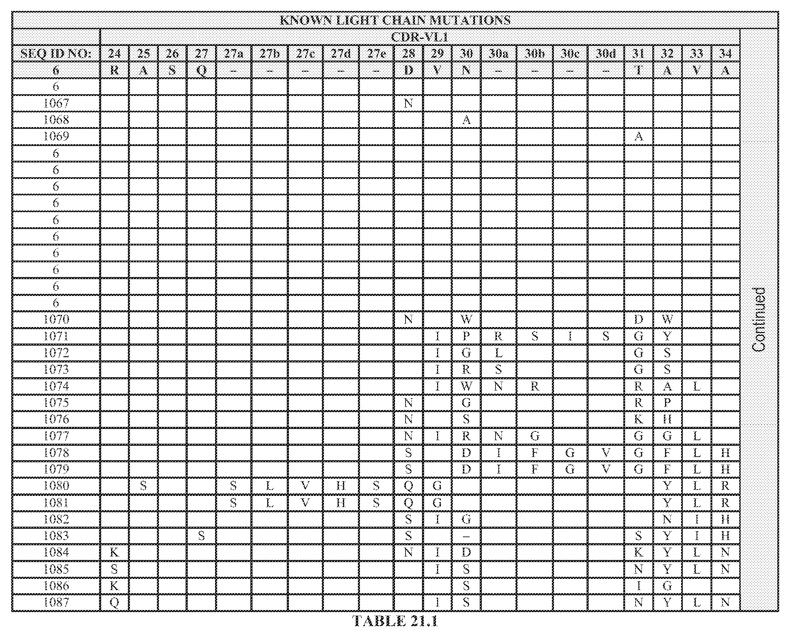

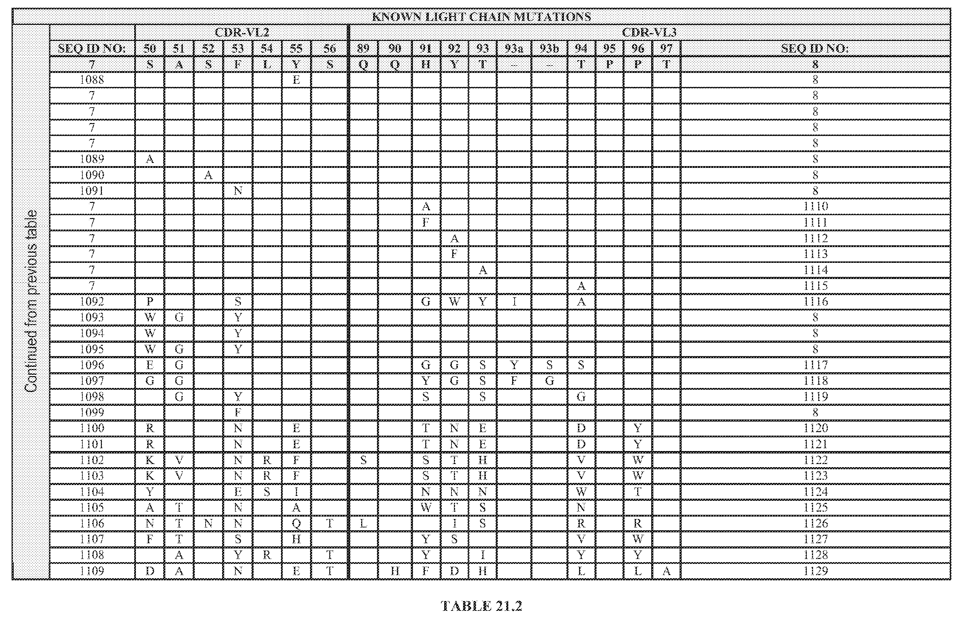

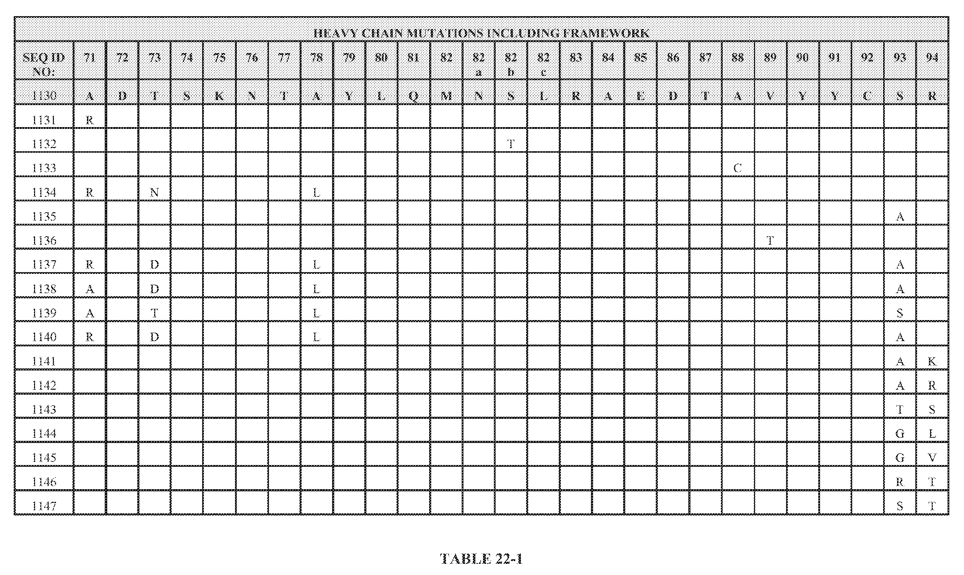

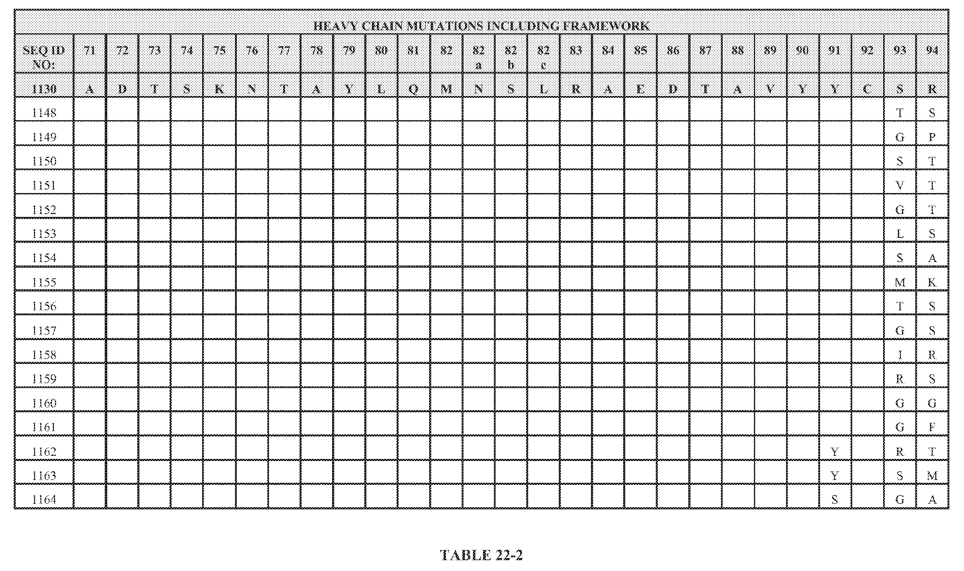

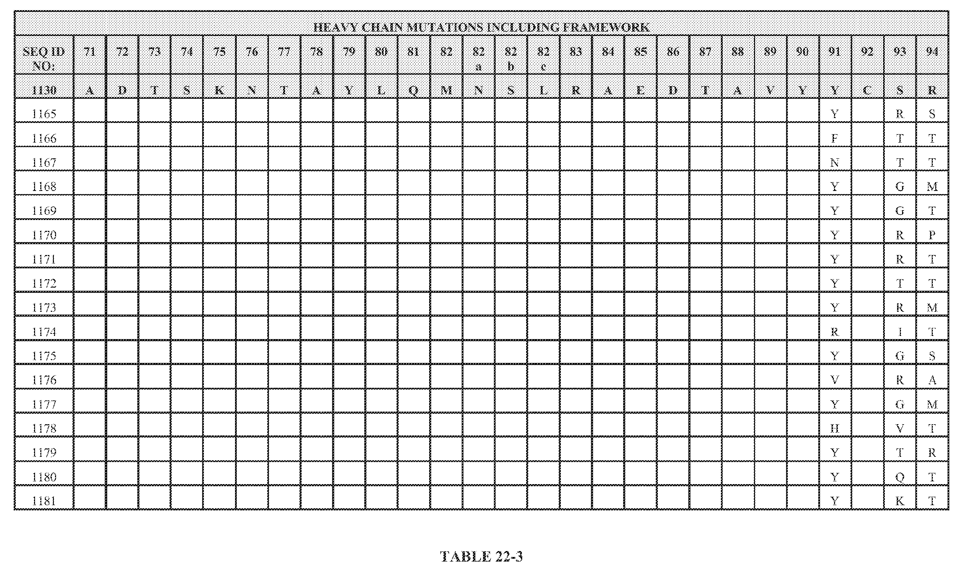

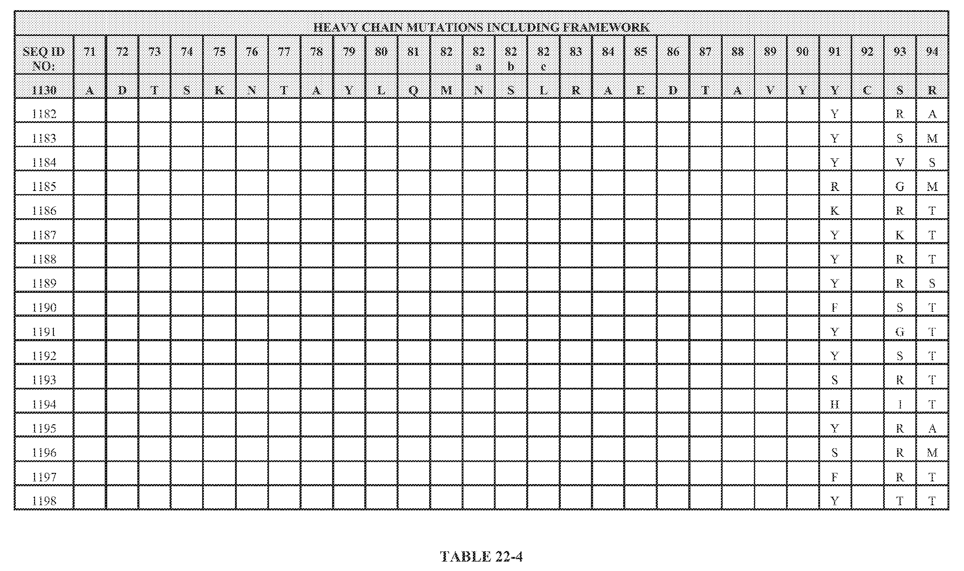

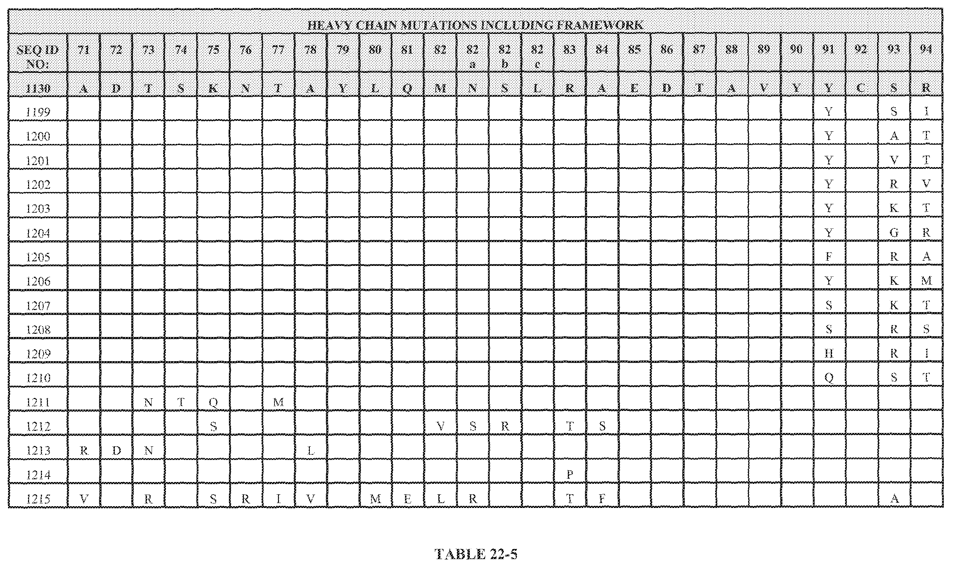

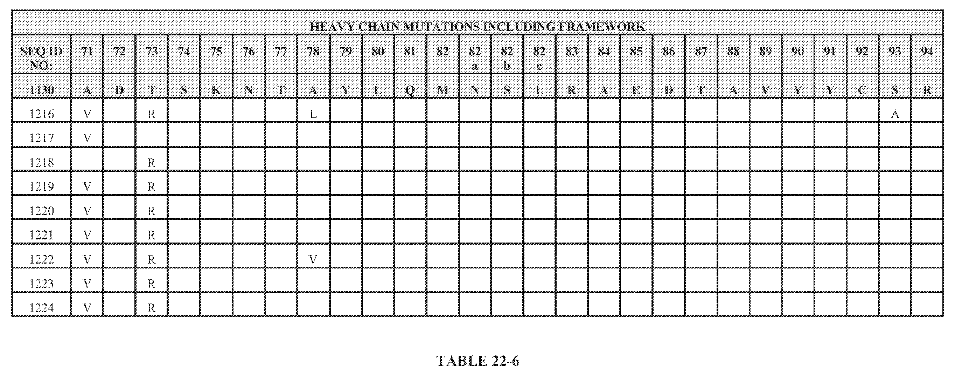

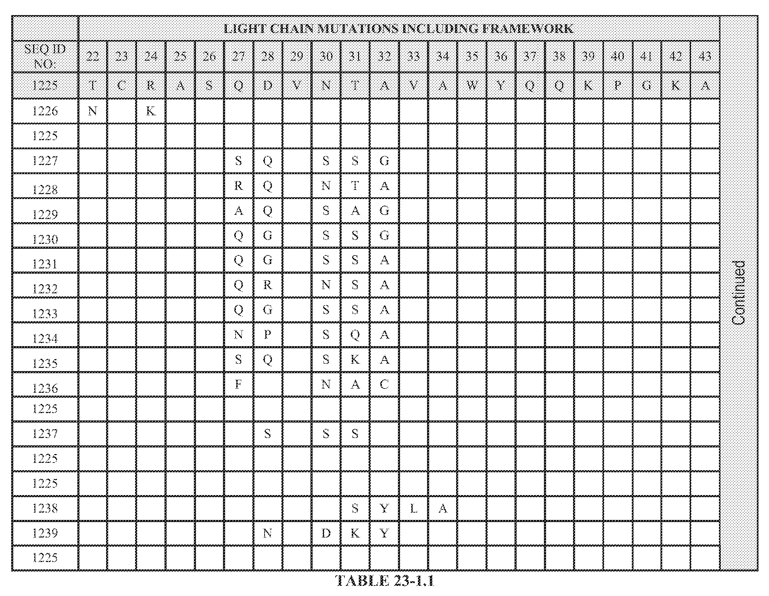

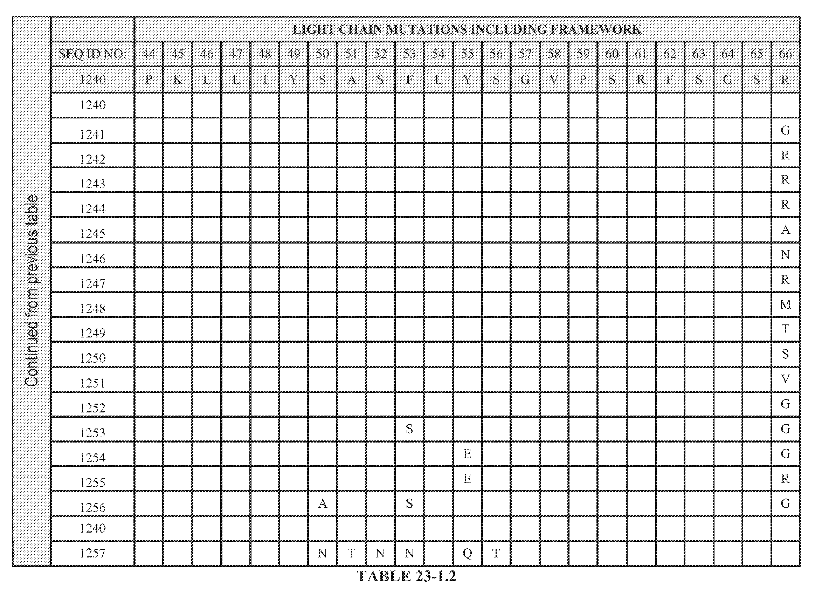

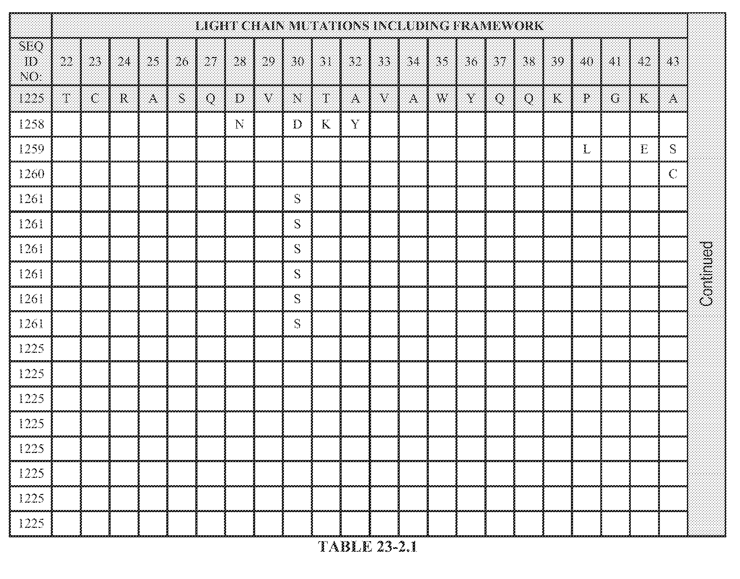

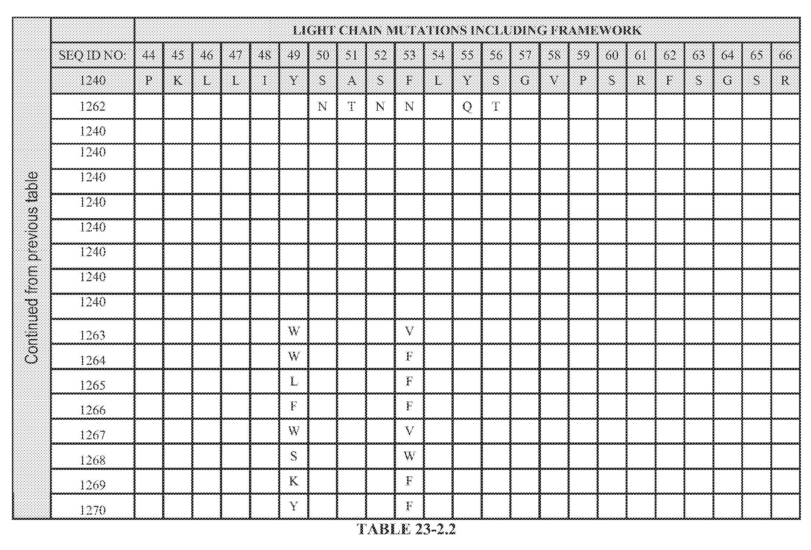

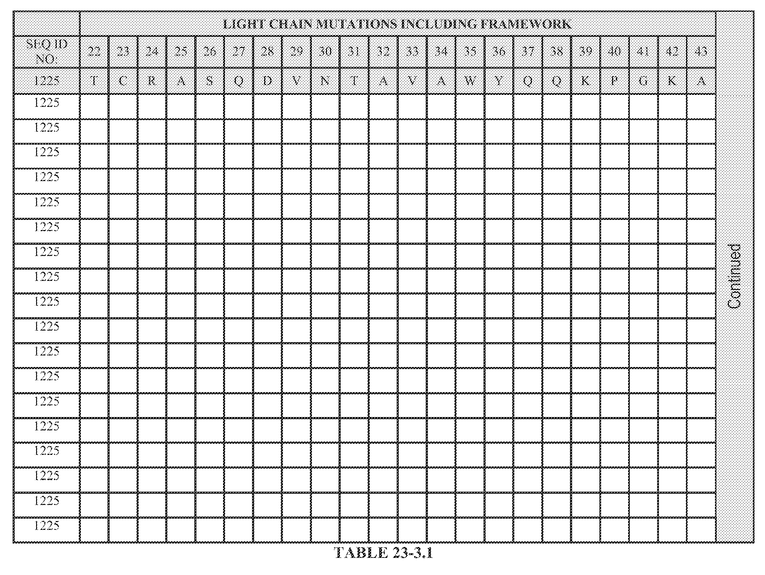

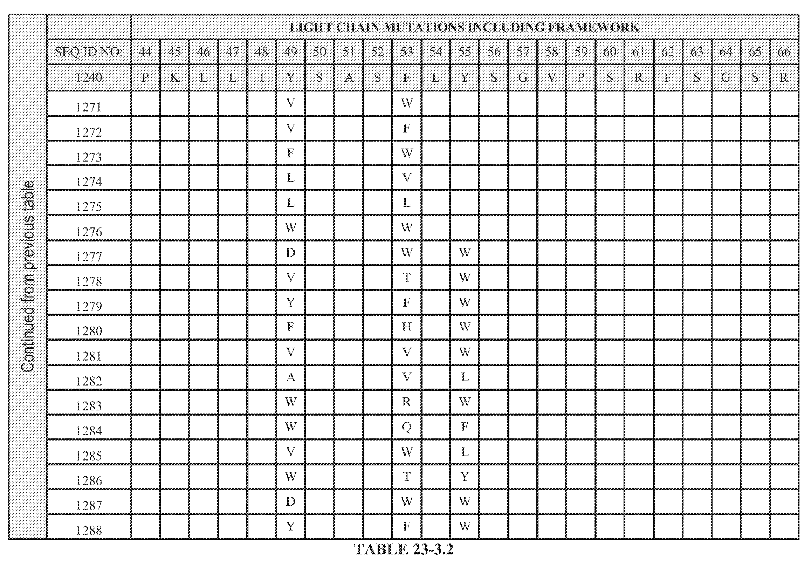

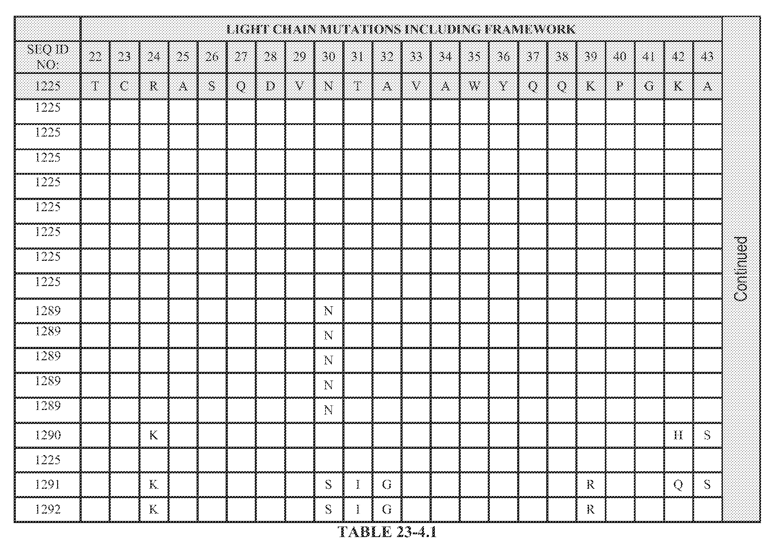

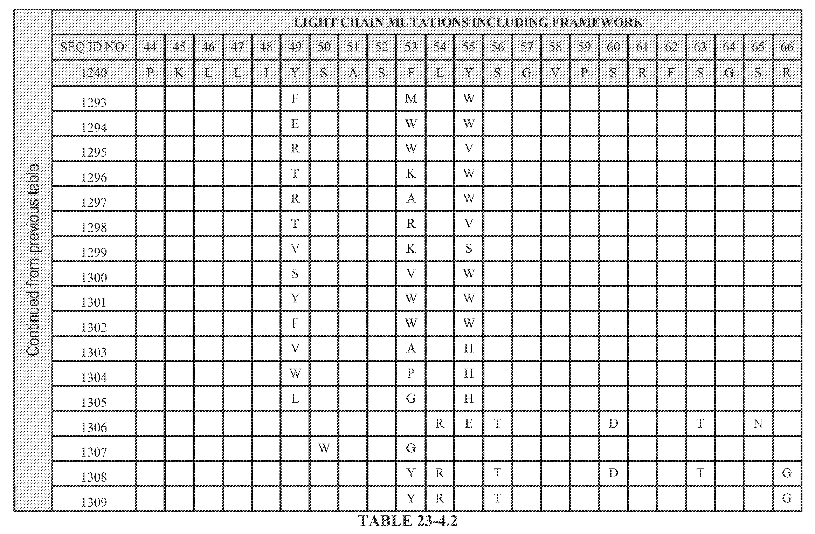

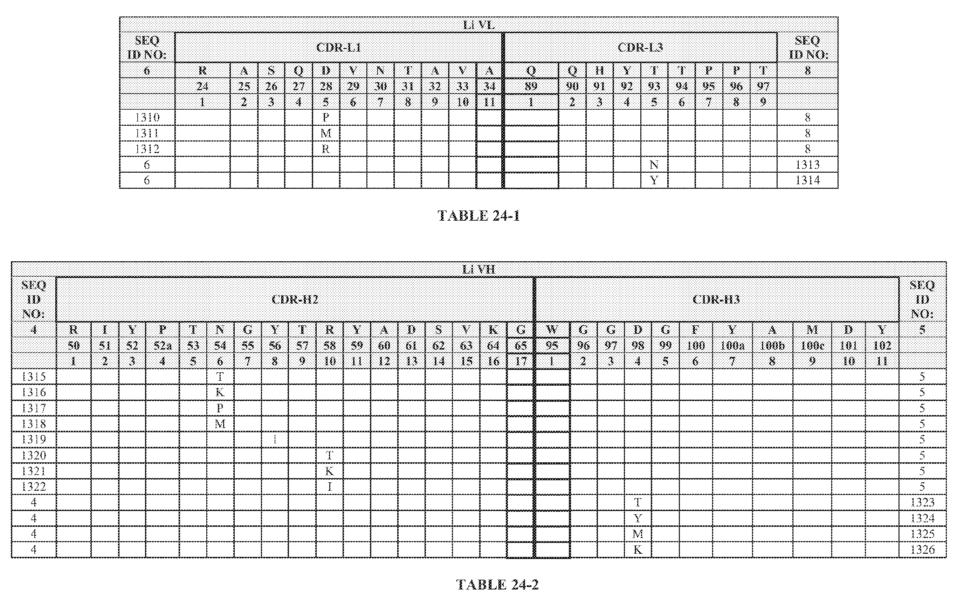

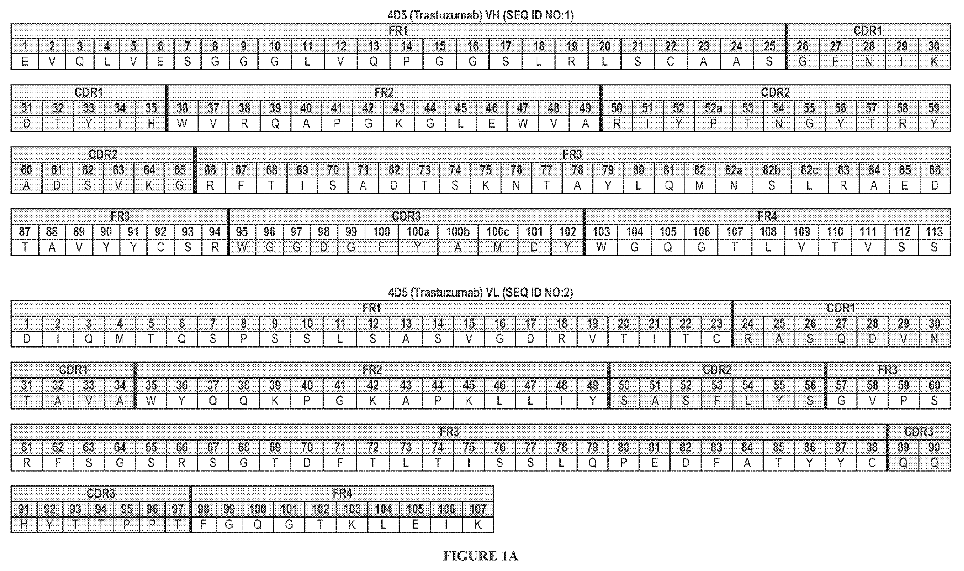

Trastuzumab comprises a heavy chain having a sequence corresponding to SEQ ID NO: 1 and a light chain having a sequence corresponding to SEQ ID NO:2, and has three heavy chain CDRs, referred to herein (in amino- to carboxy-terminal order) as CDR-H1, CDR-H2 and CDR-H3, and three light chain CDRs referred to herein (in amino- to carboxy-terminal order) as CDR-L1, CDR-L2 and CDR-L3. The sequences of the trastuzumab CDRs are shown in Figure 1A and IB, and their numbering is set forth in Table 1 (for heavy chain CDRs) and Table 2 (for light chain CDRs).

While the disclosure refers to antibodies or antibody fragments with reduced immunogenicity and/or improved affinity relative to trastuzumab, it is intended to encompass anti-HER2 antibodies or anti-HER2 binding fragments with reduced immunogenicity and/or improved affinity as compared to (i) trastuzumab; (ii) an antibody comprising the VH and VL regions of SEQ ID NO: 1 and SEQ ID NO:2, respectively; or (iii) an antibody having a VH comprising an amino acid sequence corresponding to positions 1 to 1 17 of SEQ ID NO: 1 and a VL comprising an amino acid sequence corresponding to positions 1 to 103 of SEQ ID NO:2.

As noted above, the present disclosure provides variants of trastuzumab or binding fragments of trastuzumab that are less immunogenic than trastuzumab. For the sake of convenience, such variants are sometimes referred to herein as "deimmunized."

Deimmunized variants of trastuzumab have one or more substitutions in the framework and/or CDR regions. Exemplary positions where one or more substitutions can be made that result in a variant of trastuzumab with reduced immunogenicity relative to trastuzumab include the heavy chain positions R83 and W95 and the light chain positions Q27, D28 and A34.

Exemplary substitutions that can be made at the foregoing positions that result in deimmunized variants of trastuzumab include one or more of the following heavy chain substitutions: R83K or W95F. Exemplary substitutions in the light chain that result in deimmunized variants of trastuzumab include one or more of the following substitutions: Q27S, Q27L, Q27F, D28L, D28N, A34D, and A34V. Combinations of one or more deimmunizing VL chain substitutions and one or more deimmunizing VH chain substitutions can also be made, including, for example, the following VL combinations: D28N + A34D, Q27S + A34D, Q27L + A34V, D28L + A34D or Q27F + A34D.

In one aspect, the anti-HER2 antibody or anti-HER2 binding fragment of an antibody of the disclosure comprises CDRs having overall at least 80% sequence identity to CDRs having amino acid sequences of SEQ ID NO:3 (CDR-H1), SEQ ID NO:4 (CDR-H2), SEQ ID NO:5 (CDR-H3), SEQ ID NO:104 and/or SEQ ID NO:6 (CDR-L1), SEQ ID NO:7 (CDR-L2), and SEQ ID NO:8 (CDR-L3), wherein the anti-HER2 antibody or anti-HER2 binding fragment has reduced immunogenicity as compared to an antibody having a VH of SEQ ID NO:1 and a VL of SEQ ID NO:2.

The anti-HER2 antibody or anti-HER2 binding fragment of an antibody can also comprise a VH and a VL region having amino acid sequences with at least 80% sequence identity to VH and VL regions of trastuzumab, respectively.

In some aspects, the anti-HER2 antibody or anti-HER2 binding fragment of an antibody optionally comprises one or more additional mutations or combinations of mutations as compared to the antibody trastuzumab selected from one or more of Tables 3, 4, 5, 6, 7, 8, 9, 10, 11, 12, 13, 14, 15, 16, 17, 18, 19, 20, 21, 22, and 23, and optionally Table 24.

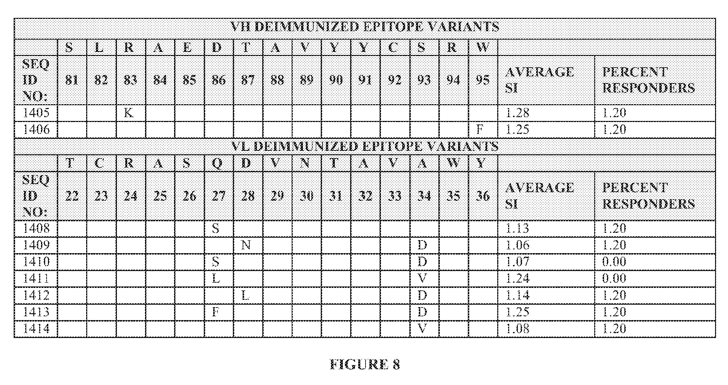

Deimmunizing substitutions are also provided in Figure 8. In addition to deimmunizing substitutions, one or more beneficial or neutral substitutions can also be made including one or more substitutions selected from one or more of Tables 3, 4, 5, 6, 7, 8, 9, 10, 11, 12, 13, 14, 15, 16, 17, 18, 19, 20, 21, 22, 23 and/or 24. In further aspects, the six CDRs can altogether have up to 13 amino acid substitutions as compared to CDR sequences of the antibody trastuzumab.

In another aspect, the anti-HER2 antibodies or anti-HER2 binding fragments of the disclosure comprises CDRs having overall at least 80% sequence identity to CDRs having amino acid sequences of SEQ ID NO:3 (CDR-H1), SEQ ID NO:4 (CDR-H2), SEQ ID NO:5 (CDR-H3), SEQ ID NO:104 and/or SEQ ID NO:6 (CDR-L1), SEQ ID NO:7 (CDR-L2), and SEQ ID NO:8 (CDR-L3), wherein said anti-HER2 antibody or anti-HER2 binding fragment has increased affinity to HER2 as compared to an antibody having a VH of SEQ ID NO:1 and a VL of SEQ ID NO:2.

The anti-HER2 antibodies and anti-HER2 binding fragments of the disclosure can optionally comprise one or more additional mutations or combinations of mutations as compared to the antibody trastuzumab selected from one or more of Tables 3, 4, 5, 6, 7, 8, 9, 10, 11, 12, 13, 14, 15, 16, 17, 18, 19, 20, 21, 22, 23, and optionally Table 24. In particular aspects, the six CDRs altogether have up to 13 amino acid substitutions as compared to CDR sequences of the antibody trastuzumab.

In yet further aspects, the anti-HER2 antibody or anti-HER2 binding fragment of an antibody of the disclosure has increased affinity, as measured by BIAcore, FACS (e.g., one-point FACS or FACS competition with a KS-BR3 cell-line) and/or AlphaLISA, to HER2 as compared to an antibody having a VH of SEQ ID NO:1 and a VL of SEQ ID NO:2.

In a particular aspect, the antibody or binding fragment has an affinity that is 1.1-fold or greater than the affinity of trastuzumab to HER-2. Exemplary antibodies or binding fragments include an anti-HER2 antibody or anti-HER2 binding fragment which has
(i) at least one VL substitution selected from: T22G and A34V as compared a VL of SEQ ID NO:2;
(ii) at least one VL substitution selected from: T22G, T22S, C23A, R24H, R24C, R24V, A25C, A25G, A25P, Q27V, D28A, V29A, V29S, A34D, A34G, A51S, S52W, S52M, S52Q, S52H, S52G, S52R, L54I, L54G, L54V, S56A, S56P, S56G, S56H, S56Y, S56F, S56N, S56M, R66K, Y92M, T93L, T93M, T93V, and P96G as compared a VL of SEQ ID NO:2 and/or at least one VH substitution selected from D31H, Y52K, T53R, K64S, A88E, V89Y, S93D, D98L, D98M and D98V as compared a VH of SEQ ID NO:1;
(iii) at least one VL substitution selected from: T22G, L54I, and S56Y as compared a VL of SEQ ID NO:2; and/or at least one VH substitution selected from D31H, D31T, Y52K, D98M, and D98W, as compared to SEQ ID NO:1;
(iv) at least one VL substitution selected from: T22G, L54I, and S56Y as compared a VL of SEQ ID NO:2; and/or at least one VH substitution selected from D31H, D31T, Y52K, and D98M, as compared to SEQ ID NO:1; or
(v) at least one VL substitution selected from: T22G, T22S, C23A, R24H, R24C, R24V, A25C, A25G, A25P, Q27V, D28A, V29A, V29S, A34D, A34G, A51S, S52W, S52M, S52Q, S52H, S52G, S52R, L54I, L54G, L54V, S56A, S56P, S56G, S56H, S56Y, S56F, S56N, S56M, R66K, Y92M, T93L, T93M, T93V, and P96G as compared a VL of SEQ ID NO:2 and/or at least one VH substitution selected from D31H, Y52K, T53R, K64S, A88E, V89Y, S93D, D98L and D98V as compared a VH of SEQ ID NO:1.

In another particular aspect, the antibody or binding fragment has an affinity that is 1.2-fold or greater than the affinity of trastuzumab to HER-2. Exemplary antibodies or binding fragments include an anti-HER2 antibody or anti-HER2 binding fragment which has
(i) the VL substitution A34V as compared a VL of SEQ ID NO:2;
(ii) at least one VL substitution selected from: T22G, T22S, R24H, R24C, A25G, D28A, V29A, V29S, A34D, A51S, S52W, S52M, S52H, S52G, S52R, L54I, L54G, L54V, S56A, S56P, S56G, S56H, S56Y, S56F, S56N, T93L, T93M, T93V, and P96G as compared a VL of SEQ ID NO:2 and/or at least one VH substitution selected from D31H, T53R, and D98V as compared a VH of SEQ ID NO:1;
(iii) at least one VL substitution selected from: T22G, L54I, and S56Y as compared a VL of SEQ ID NO:2; and/or at least one VH substitution selected from D31T, Y52K, D98M, and D98W, as compared to SEQ ID NO:1; or
(iv) at least one VL substitution selected from: T22G, L54I, and S56Y as compared a VL of SEQ ID NO:2; and/or at least one VH substitution selected from D31T and Y52K, as compared to SEQ ID NO:1.

In still further aspects, the antibody or binding fragment has an affinity that is 1.3-fold or greater than the affinity of trastuzumab to HER-2. Exemplary antibodies or binding fragments include an anti-HER2 antibody or anti-HER2 binding fragment which has
(i) at least the VL substitution A34V as compared a VL of SEQ ID NO:2;
(ii) at least one VL substitution selected from: T22G, D28A, V29S, A34D, A51S, S52G, S52R, L54G, L54V, S56A, S56P, S56Y, S56F, S56N, T93M, T93V, and P96G as compared a VL of SEQ ID NO:2 and/or at the VH substitution D98M as compared a VL of SEQ ID NO:1; or
(iii) at least one VL substitution selected from: T22G and S56Y as compared a VL of SEQ ID NO:2; and/or at least one VH substitution selected from D31T and D98M, as compared to SEQ ID NO:1.

In another aspect, the antibody or binding fragment has an affinity that is 1.4-fold or greater than the affinity of trastuzumab to HER-2. Exemplary antibodies or binding fragments include an anti-HER2 antibody or anti-HER2 binding fragment which has
(i) at least the VL substitution A34V as compared to a VL of SEQ ID NO:2;
(ii) at least one VL substitution selected from: T22G, A34D, A51S, S52R, L54V and S56Y, as compared a VL of SEQ ID NO:2 and/or at least the VH substitution D98M as compared a VL of SEQ ID NO:1; or
(iii) at least the VL substitution T22G as compared a VL of SEQ ID NO:2.

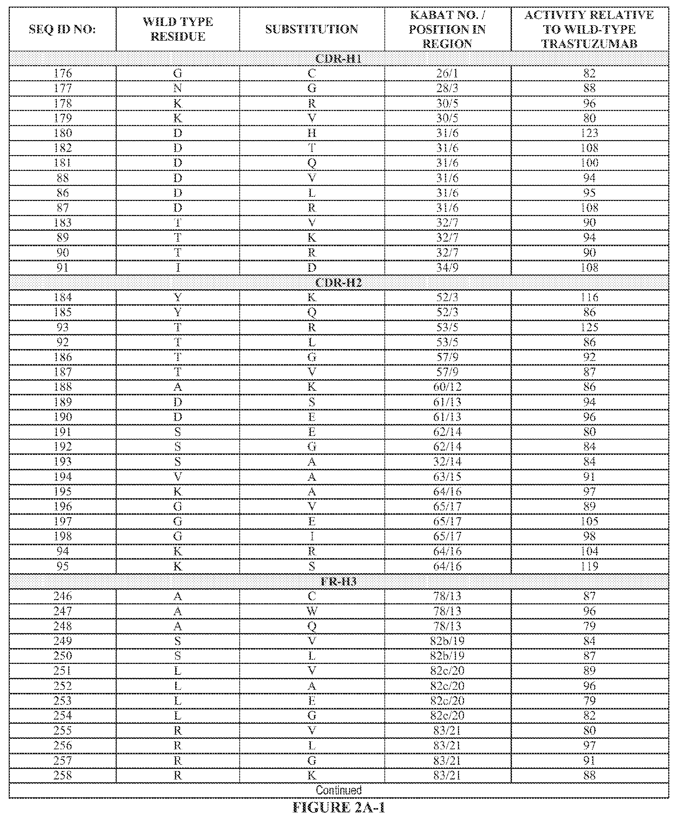

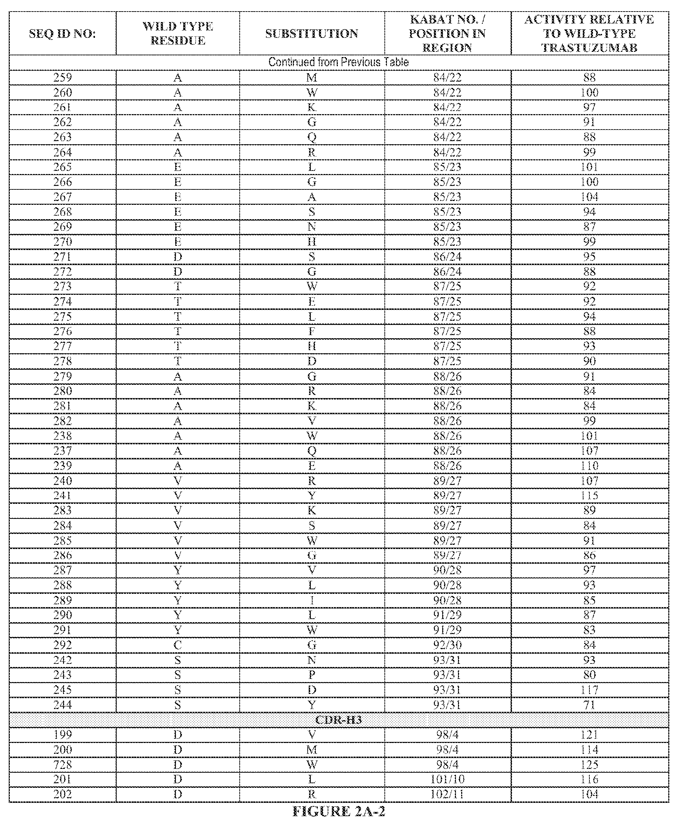

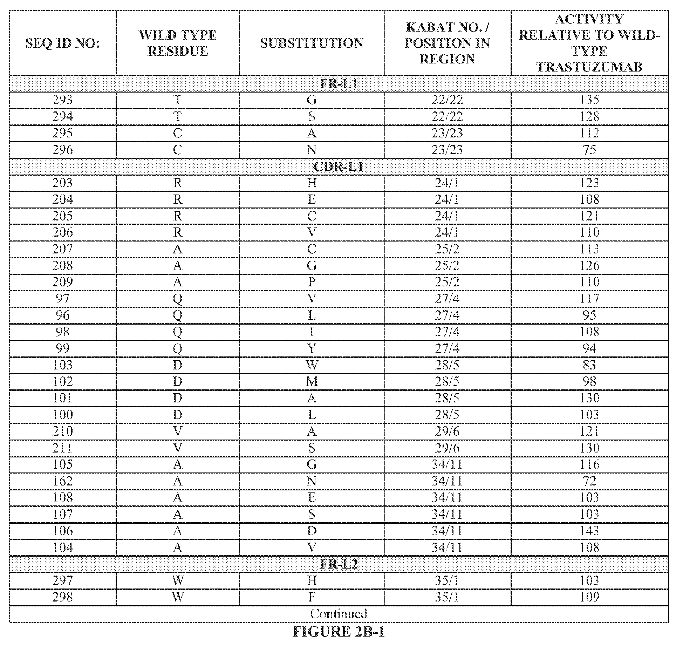

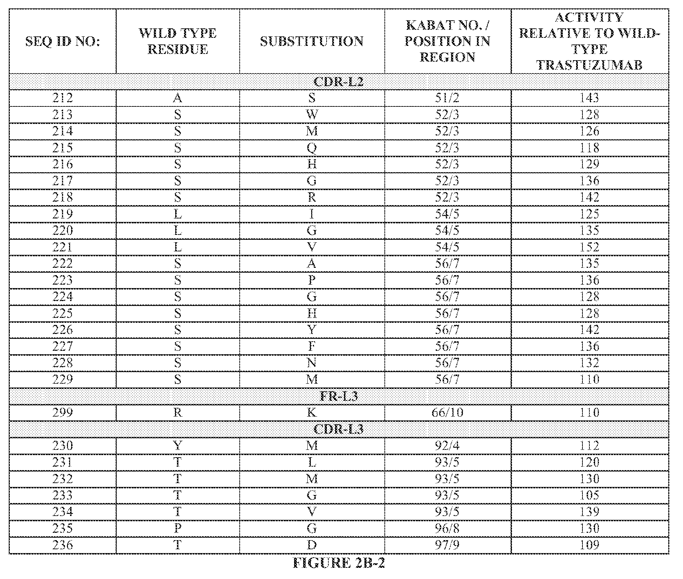

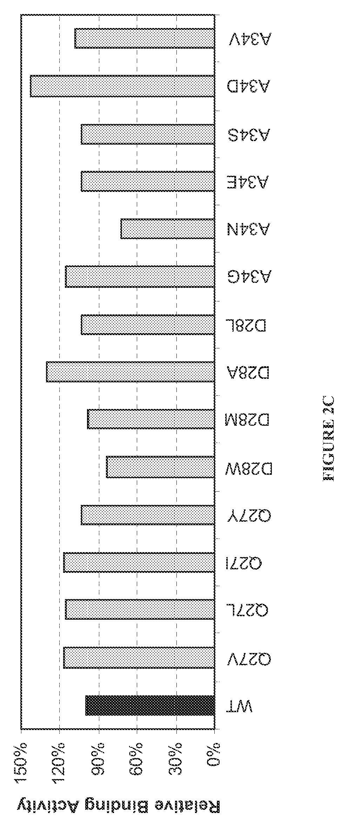

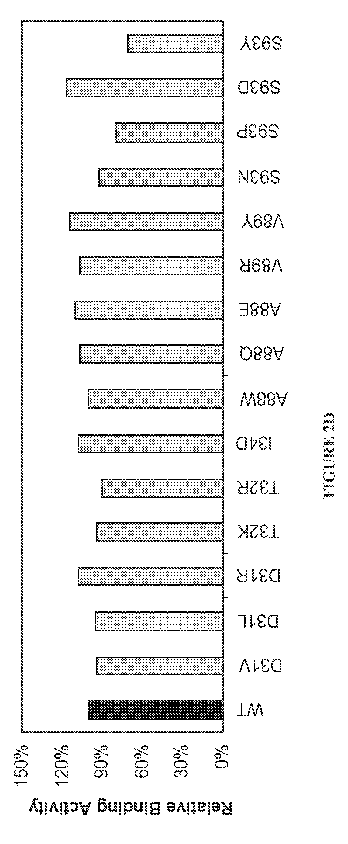

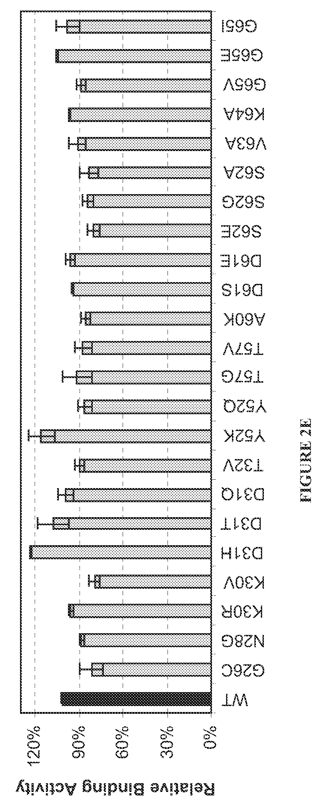

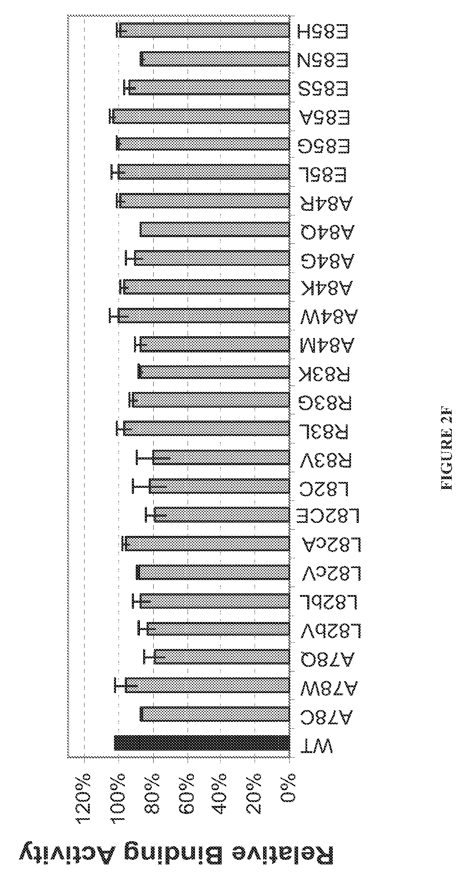

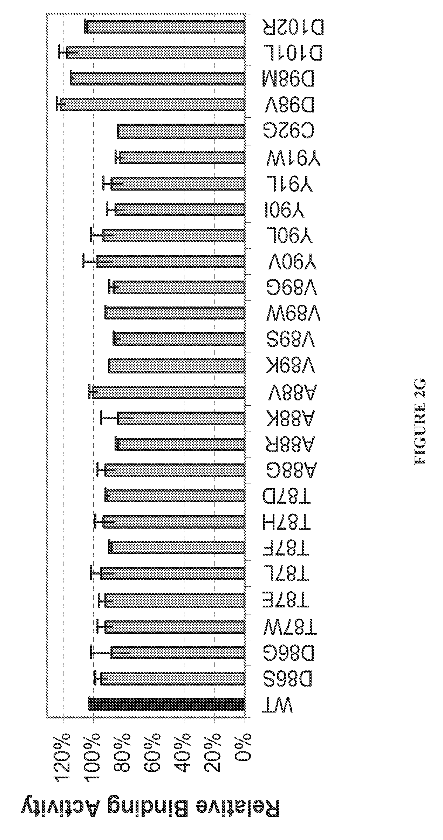

The anti-HER2 antibodies or binding fragments of the disclosure can also contain one or more mutations from any one or more of Figures 2, 3 or 9.

n still other aspects, antibody or binding fragments with increased affinity have at least at least one of the following combinations of substitutions as compared to the corresponding residues of trastuzumab: A34V as compared to a VL of SEQ ID NO:2 and R83K as compared to a VH of SEQ ID NO:1; Q27L and A34V, as compared to a VL of SEQ ID NO:2; T22G as compared to a VL of SEQ ID NO:2 and D31T as compared to a VH of SEQ ID NO:1; T22G as compared to a VL of SEQ ID NO:2 and D31H as compared to a VH of SEQ ID NO:1; D28L and A34D, as compared to a VL of SEQ ID NO:2; D28N and A34D, as compared to a VL of SEQ ID NO:2; Q27L as compared to a VL of SEQ ID NO:2, A34V as compared to a VL of SEQ ID NO:2 and R83K as compared to a VH of SEQ ID NO:1; Q27L as compared to a VL of SEQ ID NO:2, A34V as compared to a VL of SEQ ID NO:2 and W95F as compared to a VH of SEQ ID NO:1; D28N as compared to a VL of SEQ ID NO:2, A34D as compared to a VL of SEQ ID NO:2 and R83K as compared to a VH of SEQ ID NO:1; or D28N as compared to a VL of SEQ ID NO:2, A34D as compared to a VL of SEQ ID NO:2 and W95F as compared to a VH of SEQ ID NO: 1.

In certain aspects, the antibodies of the disclosure have V_{H} and V_{L} sequences having at least 80% sequence identity (and in certain embodiments, at least 85%, at least 90%, at least 95%, at least 98%, or at least 99% sequence identity) to the V_{H} and V_{L} sequences of trastuzumab, and include at least one amino acid substitution in at least one CDR as compared to trastuzumab. In other aspects, the antibodies of the disclosure have V_{H} and V_{L} sequences having at least 80% sequence identity (and in certain embodiments, at least 85%, at least 90%, at least 95%, at least 98%, or at least 99% sequence identity) to the V_{H} and V_{L} sequences of trastuzumab, and include at least one amino acid substitution in at least one framework region as compared to trastuzumab. In specific embodiments, the percentage sequence identity for the heavy chain and the light chain compared to the V_{H} and V_{L} sequences of trastuzumab is independently selected from at least 80%, at least 85%, at least 90%, at least 95% sequence identity, or at least 99% sequence identity. In certain aspects, the antibodies of the disclosure have V_{H} and/or V_{L} sequences having at least 95%, at least 98% or at least 99% sequence identity to the V_{H} and/or V_{L} sequences of trastuzumab.

In other aspects, the CDR regions of antibodies of the disclosure have at least 80% sequence identity overall (and in certain embodiments, at least 85%, at least 90%, at least 95%, at least 98%, or at least 99% sequence identity overall) to the CDR sequences of trastuzumab. In specific embodiments, the percentage sequence identity for the CDRs compared to the CDR sequences of trastuzumab is independently selected from at least 70%, at least 75%, at least 80%, at least 85%, at least 90%, at least 95% sequence identity, or at least 99% sequence identity overall. In certain aspects, the antibodies of the disclosure have CDR sequences having at least 95%, at least 98% or at least 99% sequence identity overall to the CDR sequences of trastuzumab.

The antibodies of the disclosure can have up to 17 amino acid substitutions in their CDRs as compared to trastuzumab. In some aspects, the antibodies of the disclosure have up to 13 amino acid substitutions in their CDRs as compared to trastuzumab. In specific embodiments, the antibodies of the disclosure have up to 2, up to 3, up to 4, up to 5, up to 6, up to 7, up to 8, up to 9, up to 10, up to 11, up to 12, up to 13, up to 14, up to 15, up to 16, or up to 17 amino acid substitutions in their CDRs as compared to trastuzumab.

The antibodies of the disclosure can have up to 10 amino acid substitutions in their framework regions as compared to trastuzumab. In specific embodiments, the antibodies of the disclosure have up to 1, up to 2, up to 3, up to 4, up to 5, up to 6, up to 7, up to 8, up to 9 or up to 10 amino acid substitutions in their CDRs as compared to trastuzumab.

In one or more embodiments, the antibodies or binding fragments of the disclosure do not comprise and/or consist of one or more the variants disclosed in Tables 15, 16, 17, 18, 19, 20, 21, 22, 23, and/or 24. In some embodiments, the anti-HER2 antibodies or binding fragments of the disclosure comprise the VH substitution D98W, as compared to trastuzumab. In other embodiments, the anti-HER2 antibodies or binding fragments of the disclosure exclude the VH substitution D98W, as compared to trastuzumab.

The anti-HER2 antibodies of the disclosure differs in sequence from trastuzumab and can also differ in certain characteristics (*e.g*., stability, ease of expression or production), but retains similar functional characteristics to trastuzumab (*e.g*., binding to HER2) so as to be suitable for use as a "biosimilar" or "bioequivalent" protein.

Variants of trastuzumab with improved affinity to HER2 as compared to trastuzumab can include one or more substitutions in the framework and/or CDR regions of trastuzumab. Exemplary light chain CDR substitutions are disclosed in Tables 4, 6, 8 and 14. Exemplary heavy chain CDR substitutions are disclosed in Tables 3, 5 and 7. Exemplary framework region substitutions are disclosed in Tables 9, 10, 11, 12 and 13. Table 13 recites heavy chain combination mutations comprising a framework mutation and a CDR mutation.

In some aspects, the anti-HER2 antibodies and binding fragments of the disclosure have:
- heavy chain CDRs with at least one amino acid substitution in at least one heavy chain CDR as compared to trastuzumab; and/or
- light chain CDRs with at least one amino acid substitution in at least one light chain CDR as compared to trastuzumab; and/or
- framework regions with at least one amino acid substitution in at least one framework region of a V_{H} region as compared to trastuzumab; and/or
- framework regions with at least one amino acid substitution in at least one framework region of a V_{L} region as compared to trastuzumab.

Additional exemplary embodiments of the disclosure are listed hereinbelow.

In certain aspects, the anti-HER2 antibodies include at least one substitution as compared to trastuzumab selected from D31L, D31R, D31V, T32K, T32R, and I34D in CDR-H1 (SEQ ID NO:3). Additional mutations that can be incorporated into the variant antibodies can be mutations that do not destroy the ability of the antibodies to bind to HER2, including but not limited to the mutations described in Tables 3, 4, 7, 8, 9, 10, 11, 13 and 14, or known mutations such as the mutations described in Tables 15, 16, 17, 18, 19, 20, 21, 22, 23, and, optionally, Table 24.

In certain aspects, the anti-HER2 antibodies include at least one substitution as compared to trastuzumab selected from T53L, T53R, K64R, and K64S in CDR-H2 (SEQ ID NO:4). Additional mutations that can be incorporated into the variant antibodies can be mutations that do not destroy the ability of the antibodies to bind to HER2, including but not limited to the mutations described in Tables 3, 4, 7, 8, 9, 10, 11, 13 and 14, or known mutations such as the mutations described in Tables 5, 6, 15, 16, 17, 18, 19, 20, 21, 22, 23, and, optionally, Table 24.

In various aspects, the anti-HER2 antibodies include at least one substitution as compared to trastuzumab selected from G26C, N28G, K30R, K30V, D31H, D31Q, D31T and T32V in CDR-H1 (SEQ ID NO:3). Additional mutations that can be incorporated into the variant antibodies can be mutations that do not destroy the ability of the antibodies to bind to HER2, including but not limited to the mutations described in Tables 3, 4, 7, 8, 9, 10, 11, 13 and 14, or known mutations such as the mutations described in Tables 5, 6, 15, 16, 17, 18, 19, 20, 21, 22, 23, and, optionally, Table 24.

In other aspects, the anti-HER2 antibodies include at least one substitution as compared to trastuzumab selected from Y52K, Y52Q, T57G, T57V, A60K, D61S, D61E, S62E, S62G, S62A, V63A, K64A, G65V, G65E, and G65I in CDR-H2 (SEQ ID NO:4). Additional mutations that can be incorporated into the variant antibodies can be mutations that do not destroy the ability of the antibodies to bind to HER2, including but not limited to the mutations described in Tables 3, 4, 7, 8, 9, 10, 11, 13 and 14, or known mutations such as the mutations described in Tables 5, 6, 15, 16, 17, 18, 19, 20, 21, 22, 23, and, optionally, Table 24.

In still other aspects, the anti-HER2 antibodies include at least one substitution as compared to trastuzumab selected from D98V, D98M, D101L and Y102R in CDR-H3 (SEQ ID NO:5). Additional mutations that can be incorporated into the variant antibodies can be mutations that do not destroy the ability of the antibodies to bind to HER2, including but not limited to the mutations described in Tables 3, 4, 7, 8, 9, 10, 11, 13 and 14, or known mutations such as the mutations described in Tables 5, 6, 15, 16, 17, 18, 19, 20, 21, 22, 23, and, optionally, Table 24.

In some aspects, the anti-HER2 antibodies include at least one substitution as compared to trastuzumab selected from N28S, N28W, K30W, D31W, D31G and D31S in CDR-H1 (SEQ ID NO:3), wherein CDR-H1 in the HER2 antibodies does not consist of a CDR-H1 sequence set forth in Tables 17 or Table 20. Additional mutations that can be incorporated into the variant antibodies can be mutations that do not destroy the ability of the antibodies to bind to HER2, including but not limited to the mutations described in Tables 3, 4, 7, 8, 9, 10, 11, 13 and 14, or known mutations such as the mutations described in Tables 5, 6, 15, 16, 18, 19, 21, 22, 23, and, optionally, Table 24.

In other embodiments, the anti-HER2 antibodies include at least one substitution selected from Y52R, Y52S, T53S, T53K, T53G, N54S, T57S, S62R and K64Q in CDR-H2 (SEQ ID NO:4), wherein CDR-H2 in the HER2 antibodies does not consist of a CDR-H2 sequence set forth in Tables 17 or Table 20. Additional mutations that can be incorporated into the variant antibodies can be mutations that do not destroy the ability of the antibodies to bind to HER2, including but not limited to the mutations described in Tables 3, 4, 7, 8, 9, 10, 11, 13 and 14, or known mutations such as the mutations described in Tables 5, 6, 15, 16, 18, 19, 21, 22, 23, and, optionally, Table 24.

In still other embodiments, the anti-HER2 antibodies include at least one substitution selected from W95Y, W95F, G97R, G97T, D98G, D98T, D98R, D98Q, D98E, D98S, D98Y, D98F, D98L, D98P, D98H, A100bV, A100bS, A100bE, M100cF, Y102G, Y102S, Y102K and Y102H in CDR-H3 (SEQ ID NO:5), wherein CDR-H3 in the HER2 antibodies does not consist of a CDR-H3 sequence set forth in Tables 15, 17, or 20. Additional mutations that can be incorporated into the variant antibodies can be mutations that do not destroy the ability of the antibodies to bind to HER2, including but not limited to the mutations described in Tables 3, 4, 7, 8, 9, 10, 11, 13 and 14, or known mutations such as the mutations described in Tables 5, 6, 16, 18, 19, 21, 22, 23, and, optionally, Table 24.

In certain aspects, the anti-HER2 antibodies include at least one substitution as compared to trastuzumab selected from Q27L, Q27V, Q27I, Q27Y, D28L, D28A, D28M, D28W, A34V, A34G, A34D, A34S and A34E in CDR-L1 (SEQ ID NO:6). Additional mutations that can be incorporated into the variant antibodies can be mutations that do not destroy the ability of the antibodies to bind to HER2, including but not limited to the mutations described in Tables 3, 4, 7, 8, 9, 10, 11, 13 and 14, or known mutations such as the mutations described in Tables 5, 6, 15, 16, 17, 18, 19, 20, 21, 22, 23, and, optionally, Table 24.

In other aspects, the anti-HER2 antibodies include at least one substitution as compared to trastuzumab selected from R24H, R24E, R24C, R24V, A25C, A25G, A25P, V29A and V29S in CDR-L1 (SEQ ID NO:6). Additional mutations that can be incorporated into the variant antibodies can be mutations that do not destroy the ability of the antibodies to bind to HER2, including but not limited to the mutations described in Tables 3, 4, 7, 8, 9, 10, 11, 13 and 14, or known mutations such as the mutations described in Tables 5, 6, 15, 16, 17, 18, 19, 20, 21, 22, 23, and, optionally, Table 24.

In still other aspects, the anti-HER2 antibodies include at least one substitution as compared to trastuzumab selected from A51S, S52W, S52M, S52Q, S52H, S52G, S52R, L54I, L54G, L54V, S56A, S56P, S56G, S56H, S56Y, S56F, S56N and S56M in CDR-L2 (SEQ ID NO:7). Additional mutations that can be incorporated into the variant antibodies can be mutations that do not destroy the ability of the antibodies to bind to HER2, including but not limited to the mutations described in Tables 3, 4, 7, 8, 9, 10, 11, 13 and 14, or known mutations such as the mutations described in Tables 5, 6, 15, 16, 17, 18, 19, 20, 21, 22, 23, and, optionally, Table 24.

In certain aspects, the anti-HER2 antibodies include at least one substitution as compared to trastuzumab selected from Y92M, T93L, T93M, T93G, T93V, P96G and T97D in CDR-L3 (SEQ ID NO:8). Additional mutations that can be incorporated into the variant antibodies can be mutations that do not destroy the ability of the antibodies to bind to HER2, including but not limited to the mutations described in Tables 3, 4, 7, 8, 9, 10, 11, 13 and 14, or known mutations such as the mutations described in Tables 5, 6, 15, 16, 17, 18, 19, 20, 21, 22, 23, and, optionally, Table 24.

In various aspects, the anti-HER2 antibodies include at least one substitution as compared to trastuzumab selected from A25S, Q27R, Q27S, Q27F, D28G, D28N, D28R, D28P, D28S, V29I, T31S, and A34N in CDR-L1 (SEQ ID NO:6), wherein CDR-L1 in the HER2 antibodies does not consist of a CDR-L1 sequence set forth in Tables 18, 19 or 21. Additional mutations that can be incorporated into the variant antibodies can be mutations that do not destroy the ability of the antibodies to bind to HER2, including but not limited to the mutations described in Tables 3, 4, 7, 8, 9, 10, 11, 13 and 14, or known mutations such as the mutations described in Tables 5, 6, 15, 16, 17, 20, 22, 23, and, optionally, Table 24.

In other aspects, the anti-HER2 antibodies include at least one substitution as compared to trastuzumab selected from A51G, F53W, F53Y, L54S, Y55L and Y55A in CDR-L2 (SEQ ID NO:7), wherein CDR-L2 in the HER2 antibodies does not consist of a CDR-L2 sequence set forth in Tables 16, 18, 19 or 21. Additional mutations that can be incorporated into the variant antibodies can be mutations that do not destroy the ability of the antibodies to bind to HER2, including but not limited to the mutations described in Tables 3, 4, 7, 8, 9, 10, 11, 13 and 14, or known mutations such as the mutations described in Tables 5, 6, 15, 17, 20, 22, 23, and, optionally, Table 24.

In yet other aspects, the anti-HER2 antibodies include at least one substitution as compared to trastuzumab selected from H91W, H91Y, T93S, T94S and P96S in CDR-L3 (SEQ ID NO:8), wherein CDR-L3 in the HER2 antibodies does not consist of a CDR-L3 sequence set forth in Tables 16, 18, 19 or 21. Additional mutations that can be incorporated into the variant antibodies can be mutations that do not destroy the ability of the antibodies to bind to HER2, including but not limited to the mutations described in Tables 3, 4, 7, 8, 9, 10, 11, 13 and 14, or known mutations such as the mutations described in Tables 5, 6, 15, 17, 20, 22, 23, and, optionally, Table 24.

In certain aspects, the anti-HER2 antibodies include at least one substitution as compared to trastuzumab selected from A88Q, A88W, A88E, V89R, V89Y, S93N, S93Y, S93P and S93D in framework region 3 of the heavy chain (FR-H3) (SEQ ID NO:79). Additional mutations that can be incorporated into the variant antibodies can be mutations that do not destroy the ability of the antibodies to bind to HER2, including but not limited to the mutations described in Tables 3, 4, 7, 8, 9, 10, 11, 13 and 14, or known mutations such as the mutations described in Tables 5, 6, 15, 16, 17, 18, 19, 20, 21, 22, 23, and, optionally, Table 24.

In various aspects, the anti-HER2 antibodies include at least one substitution as compared to trastuzumab selected from A78C, A78W, A78Q, S82bV, S82bL, L82cV, L82cA, L82cE, L82cG, R83V, R83L, R83G, R83K, A84M, A84W, A84K, A84G, A84Q, A84R, E85L, E85G, E85A, E85S, E85N, E85H, D86S, D86G, T87W, T87E, T87L, T87F, T87H, T87D, A88G, A88R, A88K, A88V, V89K, V89S, V89W, V89G, Y90V, Y90L, Y90I, Y91L, Y91W and C92G in FR-H3 (SEQ ID NO:79). Additional mutations that can be incorporated into the variant antibodies can be mutations that do not destroy the ability of the antibodies to bind to HER2, including but not limited to the mutations described in Tables 3, 4, 7, 8, 9, 10, 11, 13 and 14, or known mutations such as the mutations described in Tables 5, 6, 15, 16, 17, 18, 19, 20, 21, 22, 23, and, optionally, Table 24.

In various aspects, the anti-HER2 antibodies include at least one substitution as compared to trastuzumab selected from T22G, T22S, C23A and C23N in FR-L1 (SEQ ID NO:81). Additional mutations that can be incorporated into the variant antibodies can be mutations that do not destroy the ability of the antibodies to bind to HER2, including but not limited to the mutations described in Tables 3, 4, 7, 8, 9, 10, 11, 13 and 14, or known mutations such as the mutations described in Tables 5, 6, 15, 16, 17, 18, 19, 20, 21, 22, 23, and, optionally, Table 24.

In other aspects, the anti-HER2 antibodies include at least one substitution as compared to trastuzumab selected from W35H and W35F in FR-L2 (SEQ ID NO:82). Additional mutations that can be incorporated into the variant antibodies can be mutations that do not destroy the ability of the antibodies to bind to HER2, including but not limited to the mutations described in Tables 3, 4, 7, 8, 9, 10, 11, 13 and 14, or known mutations such as the mutations described in Tables 5, 6, 15, 16, 17, 18, 19, 20, 21, 22, 23, and, optionally, Table 24.

In yet other aspects, the anti-HER2 antibodies include the substitution R66K in FR-L3 (SEQ ID NO:83). Additional mutations that can be incorporated into the variant antibodies can be mutations that do not destroy the ability of the antibodies to bind to HER2, including but not limited to the mutations described in Tables 3, 4, 7, 8, 9, 10, 11, 13 and 14, or known mutations such as the mutations described in Tables 5, 6, 15, 16, 17, 18, 19, 20, 21, 22, 23, and, optionally, Table 24.

In some aspects, the anti-HER2 antibodies include at least one substitution as compared to trastuzumab selected from A78V, Y91F, S93V, S93L, S93Q and S93T in FR-H3 (SEQ ID NO:79), wherein FR-H3 in the HER2 antibodies does not consist of a FR-H3 sequence set forth in Tables 22. Additional mutations that can be incorporated into the variant antibodies can be mutations that do not destroy the ability of the antibodies to bind to HER2, including but not limited to the mutations described in Tables 3, 4, 7, 8, 9, 10, 11, 13 and 14, or known mutations such as the mutations described in Tables 5, 6, 15, 16, 17, 18, 19, 20, 21, 23, and, optionally, Table 24.

In some aspects, the anti-HER2 antibodies include a combination of substitutions as compared to trastuzumab selected from S93N in FR-H3 (SEQ ID NO:79) and W95Y in CDR-H3 (SEQ ID NO:5); S93D in FR-H3 and W95Y in CDR-H3; S93A in FR-H3 and W95Y in CDR-H3; S93L in FR-H3 and W95Y in CDR-H3; S93N in FR-H3 and W95F in CDR-H3; S93D in FR-H3 and W95F in CDR-H3; and S93L in FR-H3 and W95F in CDR-H3. Additional mutations that can be incorporated into the variant antibodies can be mutations that do not destroy the ability of the antibodies to bind to HER2, including but not limited to the mutations described in Tables 3, 4, 7, 8, 9, 10, 11, 13 and 14, or known mutations such as the mutations described in Tables 5, 6, 15, 16, 17, 18, 19, 20, 21, 22, 23, and, optionally, Table 24.

In other aspects, the anti-HER2 antibodies include a combination of substitutions as compared to trastuzumab selected from S93V in FR-H3 (SEQ ID NO:79) and W95Y in CDR-H3 (SEQ ID NO:5); S93A in FR-H3 and W95F in CDR-H3; and S93V in FR-H3 and W95F in CDR-H3, wherein position 94 in FR-H3 of said HER2 antibodies is not serine or threonine. Additional mutations that can be incorporated into the variant antibodies can be mutations that do not destroy the ability of the antibodies to bind to HER2, including but not limited to the mutations described in Tables 3, 4, 7, 8, 9, 10, 11, 13 and 14, or known mutations such as the mutations described in Tables 5, 6, 15, 16, 17, 18, 19, 20, 21, 22, 23, and, optionally, Table 24.

In other aspects, the anti-HER2 antibodies include a combination of substitutions as compared to trastuzumab selected from Q27K and A34G; Q27V and A34G; Q27L and A34G; Q27F and A34G; Q27I and A34G; Q27Y and A34G; Q27R and A34G; Q27S and A34D; Q27K and A34D; Q27V and A34D; Q27L and A34D; Q27F and A34D; Q27I and A34D;Q27Y and A34D; Q27R and A34D; Q27S and A34V; Q27K and A34V; Q27V and A34V; Q27L and A34V; Q27F and A34V; Q27I and A34V; Q27Y and A34V; Q27R and A34V; D28A and A34G; D28N and A34G; D28L and A34G; D28G and A34D; D28A and A34D; D28N and A34D; D28L and A34D; D28G and A34V; D28A and A34V; D28N and A34V; and D28L and A34V in CDR-L1 (SEQ ID NO:6). Additional mutations that can be incorporated into the variant antibodies can be mutations that do not destroy the ability of the antibodies to bind to HER2, including but not limited to the mutations described in Tables 3, 4, 7, 8, 9, 10, 11, 13 and 14, or known mutations such as the mutations described in Tables 5, 6, 15, 16, 17, 18, 19, 20, 21, 22, 23, and, optionally, Table 24.

In still other aspects, the anti-HER2 antibodies include a combination of substitutions as compared to trastuzumab selected from Q27S in CDR-L1 and A34G in CDR-L1 (SEQ ID NO:6); and D28G in CDR-L1 and A34G in CDR-L 1, wherein position 9 in CDR-L1 of said anti-HER2 antibodies is not serine. Additional mutations that can be incorporated into the variant antibodies can be mutations that do not destroy the ability of the antibodies to bind to HER2, including but not limited to the mutations described in Tables 3, 4, 7, 8, 9, 10, 11, 13 and 14, or known mutations such as the mutations described in Tables 5, 6, 15, 16, 17, 18, 19, 20, 21, 22, 23, and, optionally, Table 24.

In certain aspects, the present disclosure provides anti-HER2 antibodies that result in a greater adaptive immune response than trastuzumab. Accordingly, the present disclosure provides anti-HER2 antibodies having single or multiple amino acid substitutions in their CDRs and/or framework regions as compared to the CDRs and/or framework regions of trastuzumab, wherein at least one substitution results in an increased adaptive immune response induced by the antibody as compared to trastuzumab.

The present disclosure provides pharmaceutical compositions comprising modified anti-HER2 antibodies.

Nucleic acids comprising nucleotide sequences encoding the anti-HER2 antibodies of the disclosure are provided herein, as are vectors comprising nucleic acids. Additionally, prokaryotic and eukaryotic host cells transformed with a vector comprising a nucleotide sequence encoding an anti-HER2 antibody are provided herein, as well as eukaryotic (such as mammalian) host cells engineered to express the nucleotide sequences. Methods of producing anti-HER2 antibodies by culturing host cells are also provided.

The anti-HER2 antibodies of the disclosure are useful in the treatment of cancers (e.g., breast cancer, ovarian cancer, gastric tumors, colon cancer, non-small cell lung cancer, oral cancer, cervical cancer, osteosarcoma, pancreatic cancer, salivary gland cancer, prostate cancer, endometrial cancer, and bladder cancer), and mammary and extra-mammary Paget' s disease.

It should be noted that the indefinite articles "a" and "an" and the definite article "the" are used in the present application, as is common in patent applications, to mean one or more unless the context clearly dictates otherwise. Further, the term "or" is used in the present application, as is common in patent applications, to mean the disjunctive "or" or the conjunctive "and."

The features and advantages of the disclosure will become further apparent from the following detailed description of embodiments thereof.

### 6. BRIEF DESCRIPTION OF THE TABLES AND FIGURES

**Table 1** shows the numbering of the amino acids in the heavy chain CDRs of trastuzumab.
**Table 2** shows the numbering of the amino acids in the light chain CDRs of trastuzumab.
**Table 3** shows mutations in the trastuzumab heavy chain CDRs that do not substantially negatively impact HER2 binding, *i.e.,* result in comparable or improved affinity relative to trastuzumab, and can be incorporated into the antibodies of the disclosure.
**Table 4** shows mutations in the trastuzumab light chain CDRs that do not substantially negatively impact HER2 binding, *i.e*., result in comparable or improved affinity relative to trastuzumab, and can be incorporated into the antibodies of the disclosure.
**Table 5** shows known mutations in the trastuzumab heavy chain CDRs that can be incorporated into the antibodies of the disclosure.
**Table 6** shows known mutations in the trastuzumab light chain CDRs that can be incorporated into the antibodies of the disclosure.
**Table 7** shows additional mutations in the trastuzumab heavy chain CDRs that do not substantially negatively impact HER2 binding, i.e., result in comparable or improved affinity relative to trastuzumab, and can be incorporated into the antibodies of the disclosure.
**Table 8** shows additional mutations in the trastuzumab light chain CDRs that do not substantially negatively impact HER2 binding, i.e., result in comparable or improved affinity relative to trastuzumab, and can be incorporated into the antibodies of the disclosure.
**Table 9** shows mutations in the trastuzumab heavy chain framework regions that do not substantially negatively impact HER2 binding, i.e., result in comparable or improved affinity relative to trastuzumab, and can be incorporated into the antibodies of the disclosure.
**Table 10** shows additional mutations in the trastuzumab heavy chain framework regions that do not substantially negatively impact HER2 binding, i.e., result in comparable or improved affinity relative to trastuzumab, and can be incorporated into the antibodies of the disclosure.
**Table 11** shows additional mutations in the trastuzumab light chain framework regions that do not substantially negatively impact HER2 binding, i.e., result in comparable or improved affinity relative to trastuzumab, and can be incorporated into the antibodies of the disclosure.
**Table 12** shows known mutations in the trastuzumab heavy chain framework regions that can be incorporated into the antibodies of the disclosure.
**Table 13** shows combinations of mutations that do not substantially negatively impact HER2 binding, *i.e*., result in comparable or improved affinity relative to trastuzumab, and can be incorporated into the antibodies of the disclosure.
**Table 14** shows combinations of mutations that do not substantially negatively impact HER2 binding, *i.e*., result in comparable or improved affinity relative to trastuzumab, and can be incorporated into the antibodies of the disclosure.
**Table 15 (15.1 to 15.3)** shows known mutations in the trastuzumab heavy chain CDRs that can be incorporated into the antibodies of the disclosure.
**Table 16 (16.1 to 16.2)** shows known mutations in the trastuzumab light chain CDRs that can be incorporated into the antibodies of the disclosure.
**Table 17 (17-1.1 to 17-12)** show known mutations in the trastuzumab heavy chain CDRs found in phage binding libraries that can be incorporated into the antibodies of the disclosure.
**Table 18 (18-1 to 18-13.2)** show known mutations in the trastuzumab light chain CDRs found in phage binding libraries that can be incorporated into the antibodies of the disclosure.
**Table 19 (19-1 to 19-5)** show known mutations in the trastuzumab light chain CDRs found in bispecific antibody phage binding libraries that can be incorporated into the antibodies of the disclosure.
**Table 20 (20-1.1 to 20-2.2)** shows known mutations in the trastuzumab heavy chain CDRs that can be incorporated into the antibodies of the disclosure.
**Table 21 (21.1 to 21.2)** shows known mutations in the trastuzumab light chain CDRs that can be incorporated into the antibodies of the disclosure.
**Table 22 (22-1 to 22-6)** shows known mutations in the framework regions of the trastuzumab heavy chain that can be incorporated into the antibodies of the disclosure.
**Table 23 (23-1.1 to 23-4.2)** shows known mutations in the framework and CDR regions of the trastuzumab light chain that can be incorporated into the antibodies of the disclosure.
**Table 24 (24-1 and 24-2)** shows mutations disclosed in Li et al., 2009, Journal of Biological Chemistry 285(6):3865-3871.

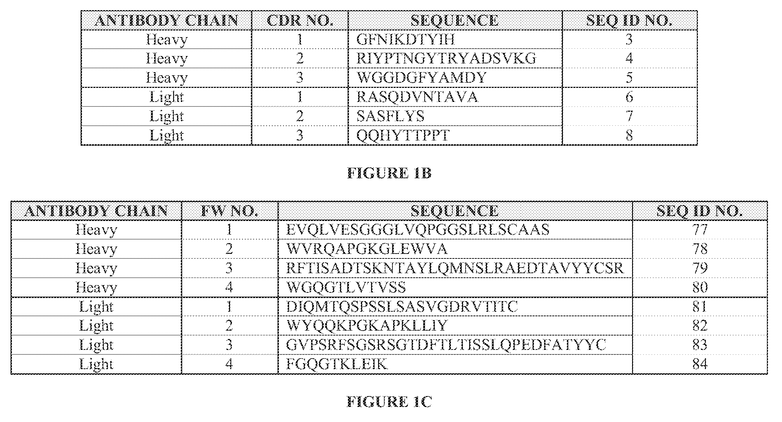

**Figure 1A****-1C.** **Figure 1A** shows the amino acid sequences and numbering of the trastuzumab heavy and light chain variable regions, SEQ ID NO:1 and SEQ ID NO:2, respectively, with CDR regions shaded. **Figure 1B** shows the CDR sequences and corresponding sequence identifiers of trastuzumab. **Figure 1C** shows the framework sequences and corresponding sequence identifiers of trastuzumab.
**Figures 2A-2G** show the binding affinities of trastuzumab variants for HER2 relative to trastuzumab (binding affinity-100) as measured in FACS binding assays with labeled HER2 ECD-Cλ. Experiments were repeated 2-3 times and mean value relative to that of parental trastuzumab were shown as tables (Figures 2A and 2B) and bar graphs of heavy and light chain variants (Figures 2C-2G).

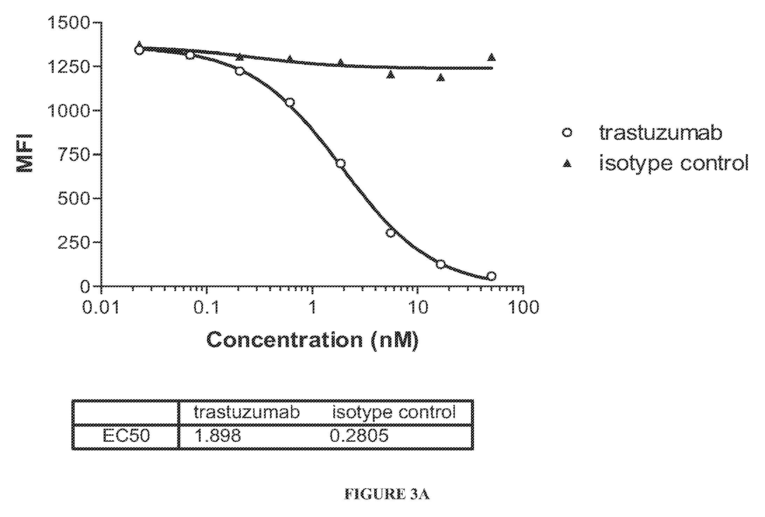

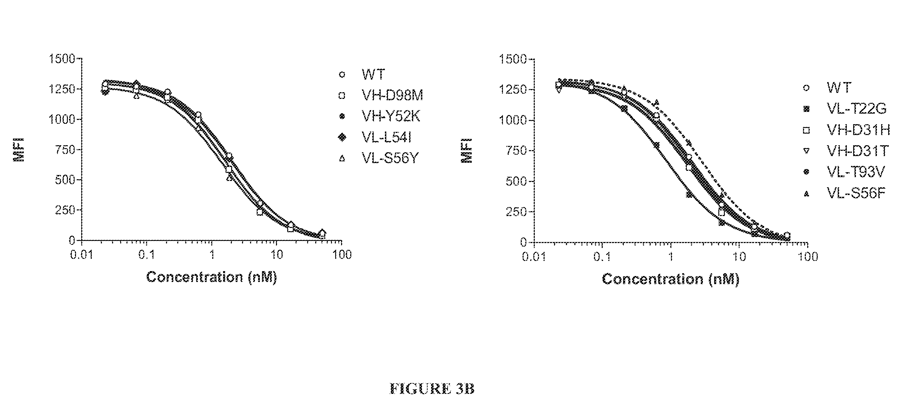

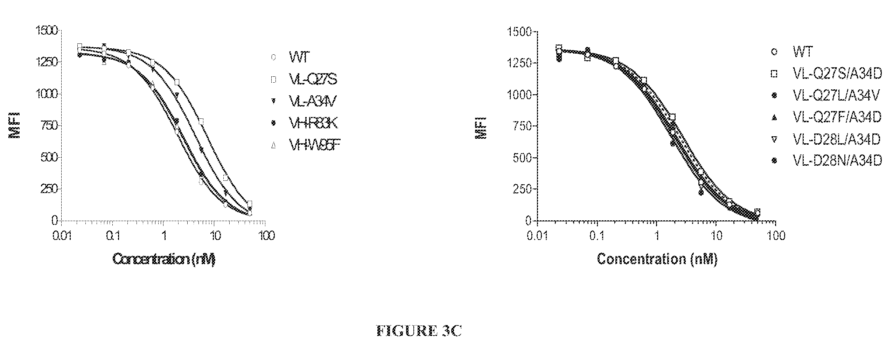

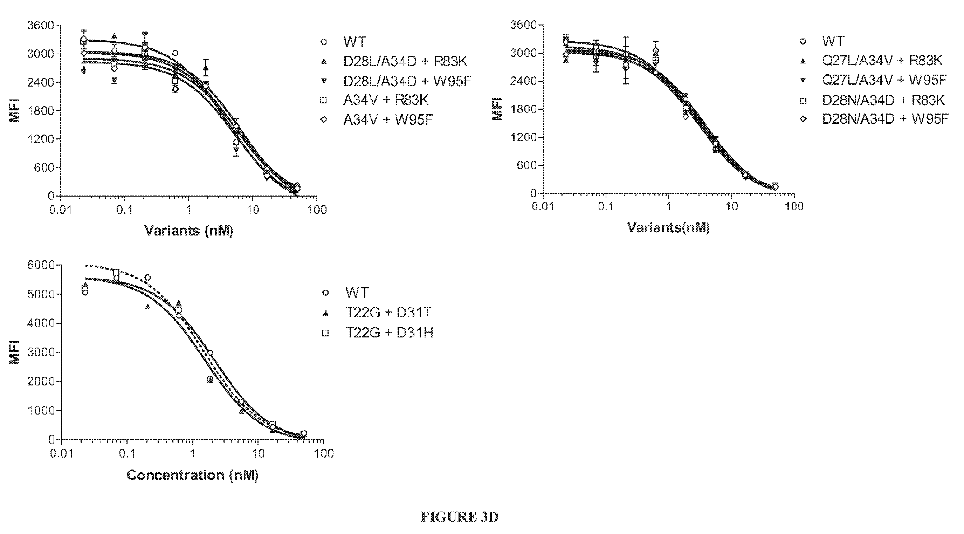

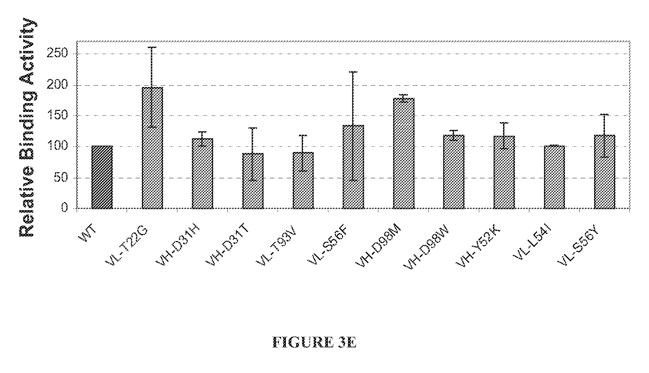

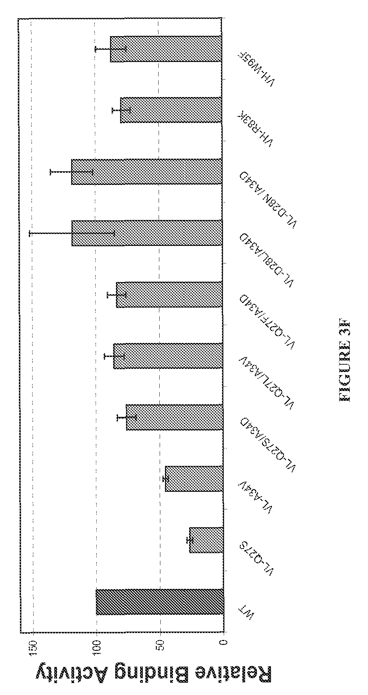

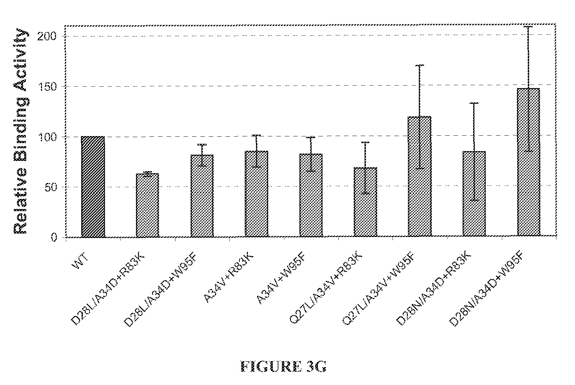

**Figure 3** shows the FACS competition on KS-BR3, a hormone-independent cell line derived from a breast adenocarcinoma expressing high level of HER2 (Koyama et al., Neoplasia 9: 1021-1029 (2007)). Trastuzumab produced in 293c18 competes with Herceptin® while irrelevant IgG1 showed no competition (Figure 3A). Comparison of binding among selected beneficial mutations, deimmunizing mutation and combinatorial mutation are shown in Figure 3B, 3C and 3D, respectively. Bar graph representations of beneficial mutations, deimmunizing mutations and combinatorial mutations are shown in Figures 3E, 3F and 3G, respectively.

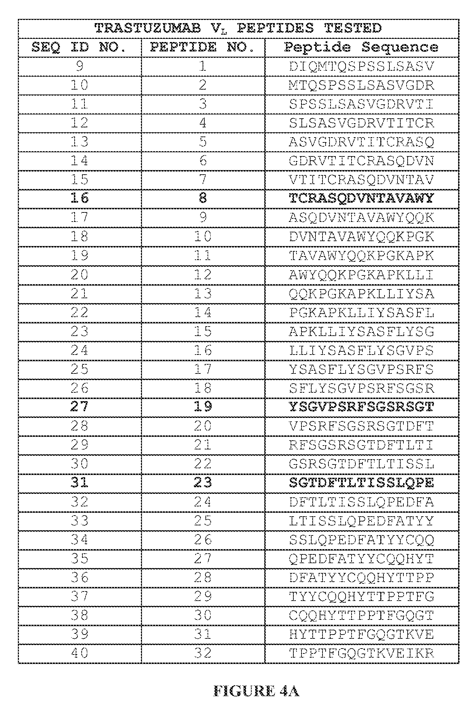

**Figures 4A****-4B** respectively show the amino acid sequences, respectively, of all V_{L} and V_{H} peptides of trastuzumab tested as potential CD4⁺ epitopes.

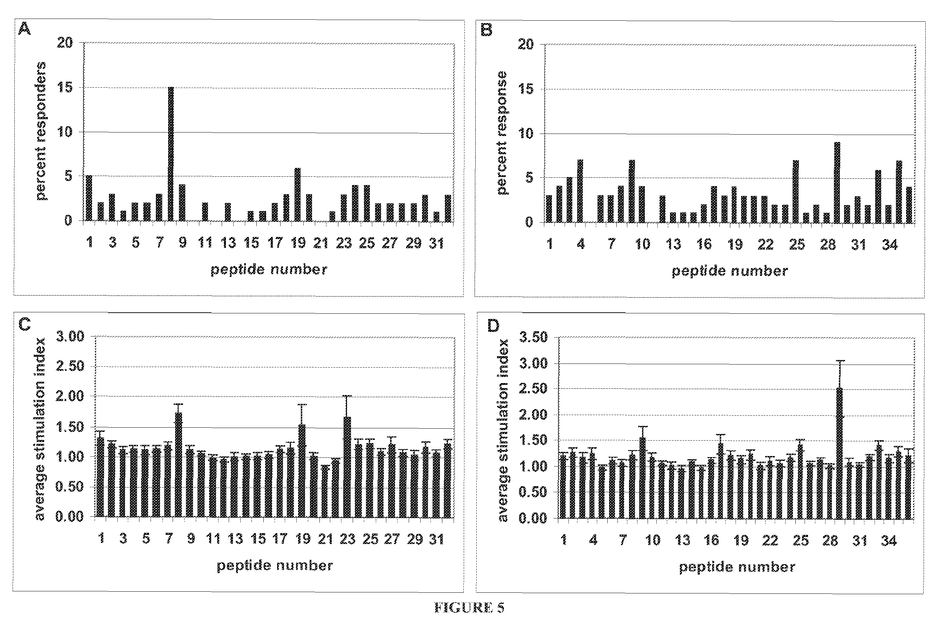

**Figures 5A****-5D** show trastuzumab V_{L} and VH peptide responses. **Figure 5A** and **Figure 5B** respectively show percent of donor responses to each V_{L} and V_{H} peptide with a stimulation index of 2.95 or greater. N=100 donors. **Figure 5C** and **Figure 5D** respectively show the average stimulation index for all 100 donors for each V_{L} and V_{H} peptide plus or minus standard error.

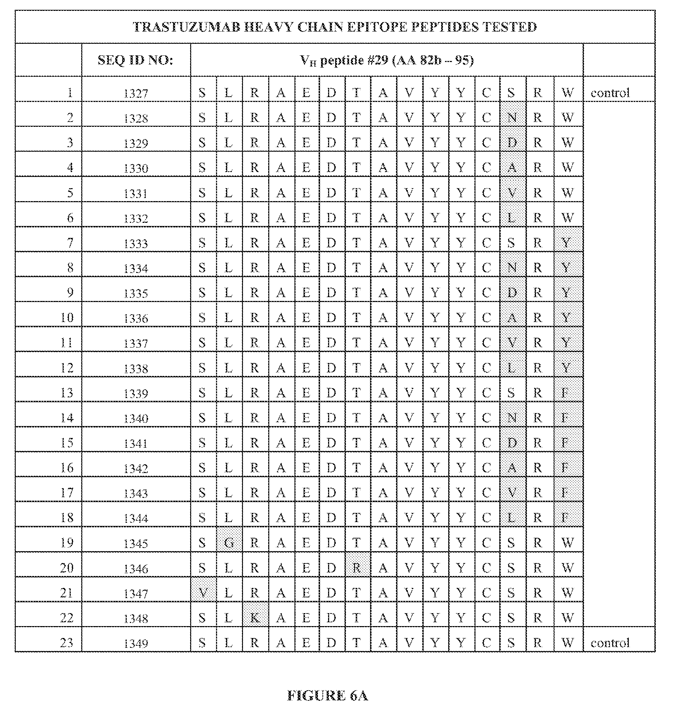

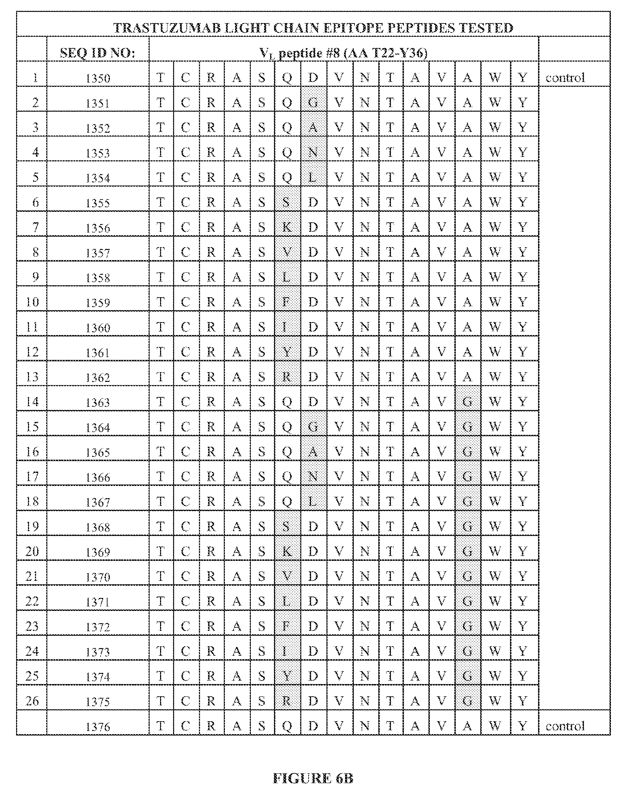

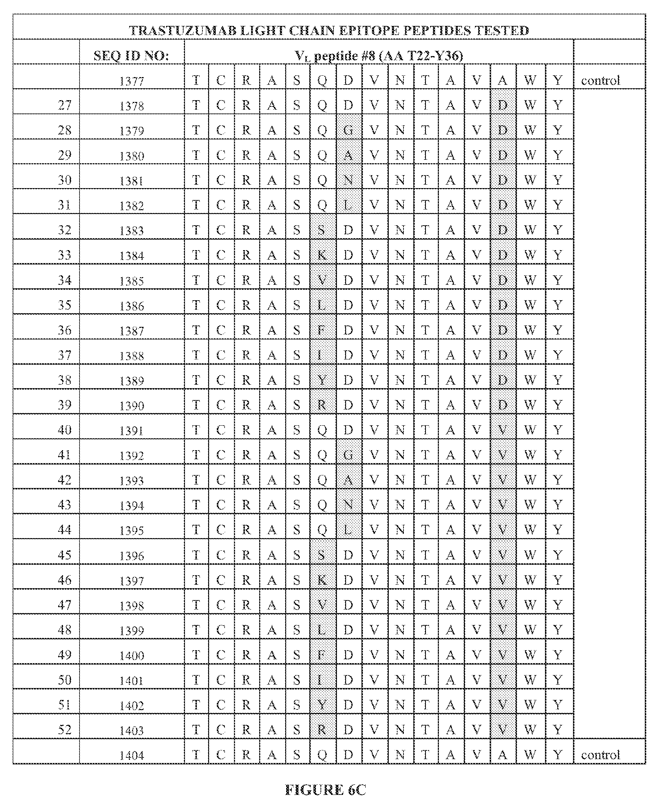

**Figures 6A****-6C** show a series of variant peptides based on the identified CD4⁺ T cell epitope regions, the peptide at position 29 that encompasses V_{H} framework 3 and 3 amino acids of CDR3 and the peptide at position 8 that encompasses V_{L} CDR1 and portions of Framework 1 and 2. **Figure 6A** shows specific amino acid changes in the V_{H} framework 3 and CDR3 (shaded amino acids). **Figures 6B** and **6C** show specific amino acid changes in V_{L} CDR2 and portions of frameworks 1 and 2 (shaded amino acids). The specific amino acid changes shown were confirmed to have no impact on the affinity of antigen binding. All peptides were tested for their ability to induce proliferative responses in human CD4⁺ T cells by the methods detailed in Example 1.

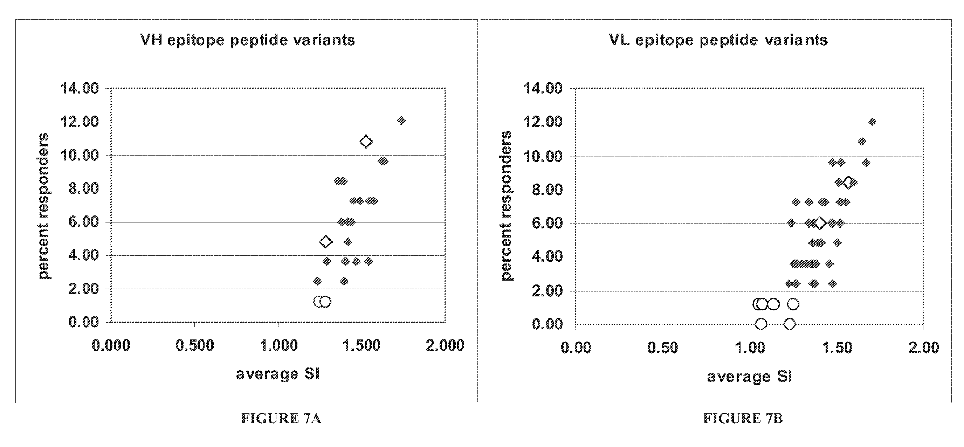

**Figure 7** shows *in vitro* CD4+ T cell proliferation responses of 83 donor samples to trastuzumab variant peptides. **Figure 7A****.** VH epitope peptide variants. **Figure 7B****.** VL epitope variants. (Open diamonds: parent peptides. Closed diamonds: variant peptides as per Figures **6A****-6C.** Open circles: selected deimmunized variants).
**Figure 8** shows exemplary trastuzumab VH and VL epitope variant peptides, wherein "SI" is the stimulation index averaged for all 83 tested donors and "Percent responders" is the percentage of donors with an SI > 2.95.

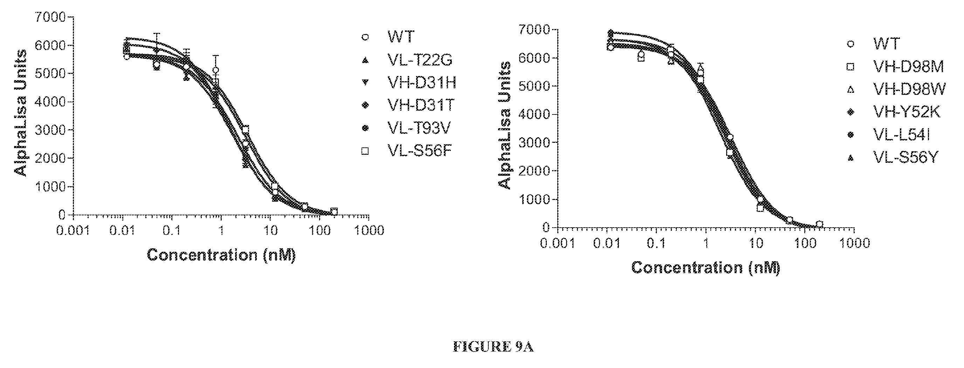

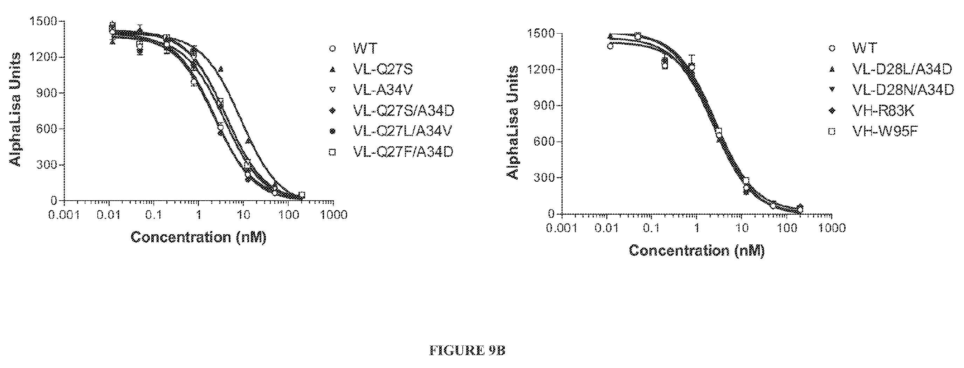

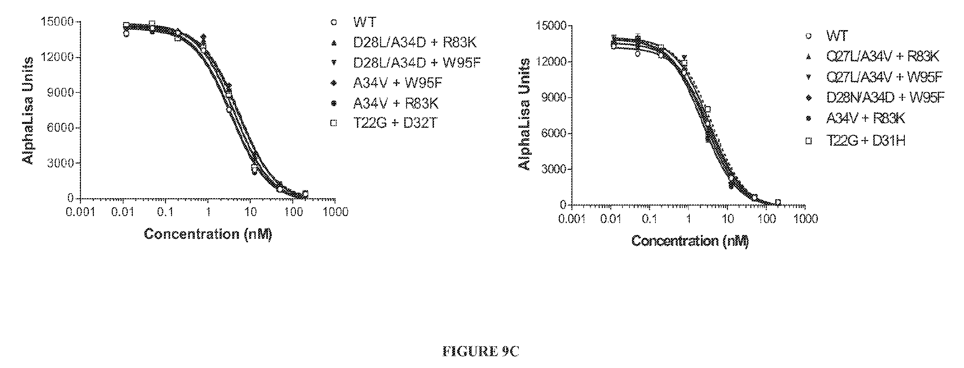

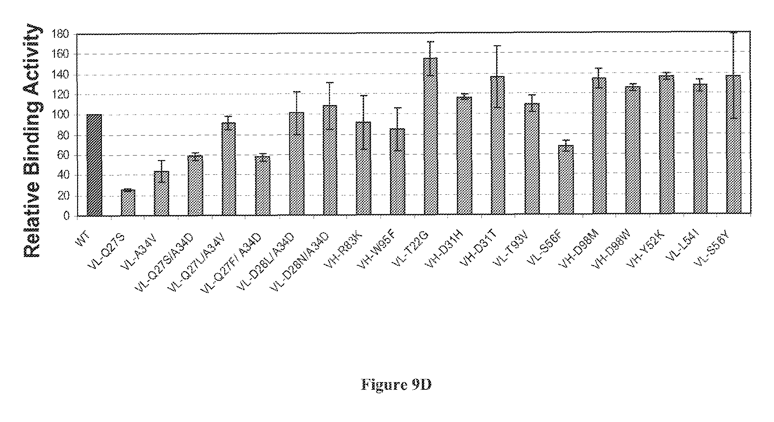

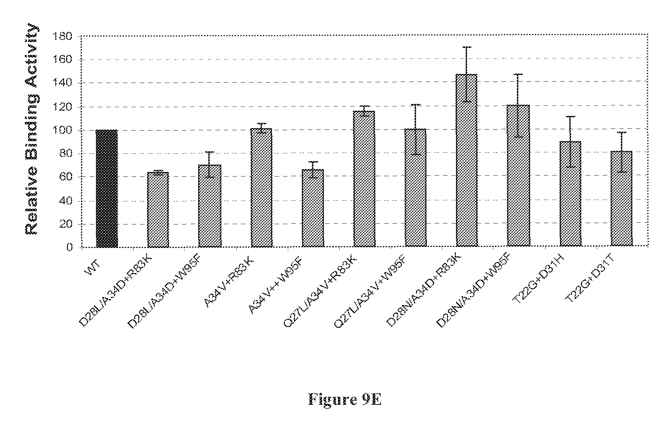

**Figure 9** shows the binding of trastuzumab variants to HER2 as measured by competition AlphaLISA®. Representative results of binding competition are shown in Figure 9A (beneficial single mutations), 9B (deimmunizing mutations) and 9C (combinatorial mutations). Relative binding activities of variants that showed equivalent binding to trastuzumab are shown in Figure 9D (single chain variants) and 9E (combination of heavy and light chain variants). Data are normalized with an IC₅₀ value obtained from wild-type trastuzumab from three independent sets of experiments. Each data point of the dilution curve was duplicated in the assay plate.

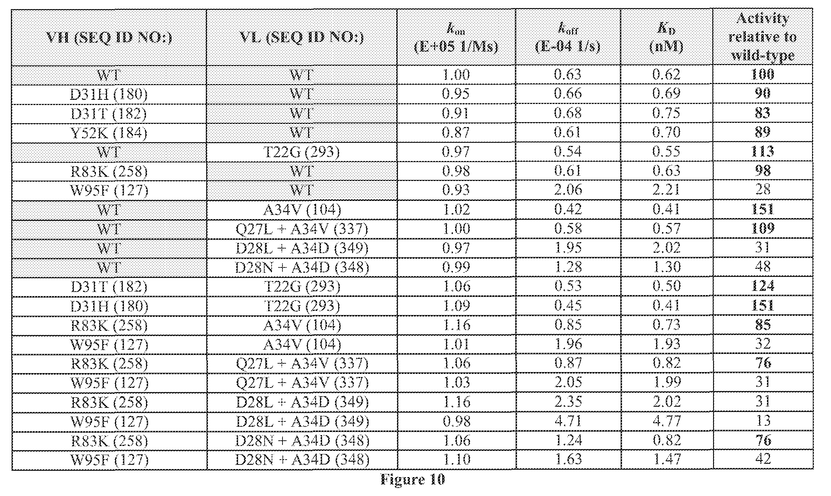

**Figure 10** shows the binding kinetics of trastuzumab variants as measured by BIAcore. Association (*kₒₙ*) and dissociation (*k_{off}*) rate constant of trastuzumab binding against HER2 ECD were determined using surface plasmon resonance in a BIAcore. The dissociation constant (*K_{D}*) was calculated from *k*ₒₙ/*k*_{off}.

### 7. DETAILED DESCRIPTION

### 7.1. Anti-HER2 Antibodies

The present disclosure provides anti-HER2 antibodies. Unless indicated otherwise, the term "antibody" (Ab) refers to an immunoglobulin molecule that specifically binds to, or is immunologically reactive with, a particular antigen, and includes polyclonal, monoclonal, genetically engineered and otherwise modified forms of antibodies, including but not limited to chimeric antibodies, humanized antibodies, heteroconjugate antibodies (e.g., bispecific antibodies, diabodies, triabodies, and tetrabodies), and antigen binding fragments of antibodies, including e.g., Fab', F(ab')₂, Fab, Fv, rIgG, and scFv fragments. Moreover, unless otherwise indicated, the term "monoclonal antibody" (mAb) is meant to include both intact molecules, as well as, antibody fragments (such as, for example, Fab and F(ab')₂ fragments) which are capable of specifically binding to a protein. Fab and F(ab')₂ fragments lack the Fc fragment of intact antibody, clear more rapidly from the circulation of the animal, and may have less non-specific tissue binding than an intact antibody (Wahl et al., 1983, J. Nucl. Med. 24:316).

The term "scFv" refers to a single chain Fv antibody in which the variable domains of the heavy chain and the light chain from a traditional antibody have been joined to form one chain.

References to "VH" refer to the variable region of an immunoglobulin heavy chain of an antibody, including the heavy chain of an Fv, scFv, or Fab. References to "VL" refer to the variable region of an immunoglobulin light chain, including the light chain of an Fv, scFv, dsFv or Fab. Antibodies (Abs) and immunoglobulins (Igs) are glycoproteins having the same structural characteristics. While antibodies exhibit binding specificity to a specific target, immunoglobulins include both antibodies and other antibody-like molecules which lack target specificity. Native antibodies and immunoglobulins are usually heterotetrameric glycoproteins of about 150,000 daltons, composed of two identical light (L) chains and two identical heavy (H) chains. Each heavy chain has at the amino terminus a variable domain (V_{H}) followed by a number of constant domains. Each light chain has a variable domain at the amino terminus (V_{L}) and a constant domain at the carboxy terminus.

The anti-HER2 antibodies of the disclosure bind to human HER2 and inhibit its activity in a cell.

The anti-HER2 antibodies of the disclosure contain complementarity determining regions (CDRs) that are related in sequence to the CDRs of the antibody trastuzumab (also known as Herceptin®).

CDRs are also known as hypervariable regions both in the light chain and the heavy chain variable domains. The more highly conserved portions of variable domains are called the framework (FR). As is known in the art, the amino acid position/boundary delineating a hypervariable region of an antibody can vary, depending on the context and the various definitions known in the art. Some positions within a variable domain may be viewed as hybrid hypervariable positions in that these positions can be deemed to be within a hypervariable region under one set of criteria while being deemed to be outside a hypervariable region under a different set of criteria. One or more of these positions can also be found in extended hypervariable regions. The disclosure provides antibodies comprising modifications in these hybrid hypervariable positions. The variable domains of native heavy and light chains each comprise four FR regions, largely by adopting a β-sheet configuration, connected by three CDRs, which form loops connecting, and in some cases forming part of, the β-sheet structure. The CDRs in each chain are held together in close proximity by the FR regions in the order FR1-CDR1-FR2-CDR2-FR3-CDR3-FR4 and, with the CDRs from the other chain, contribute to the formation of the target binding site of antibodies (see Kabat et al., Sequences of Proteins of Immunological Interest (National Institute of Health, Bethesda, Md. 1987)). As used herein, numbering of immunoglobulin amino acid residues is done according to the immunoglobulin amino acid residue numbering system of Kabat *et al*., unless otherwise indicated.

The sequences of the heavy and light chain variable regions of trastuzumab are represented by SEQ ID NO:1 and SEQ ID NO:2, respectively. The sequences of the heavy and light chain variable regions are also depicted in Figure 1A. The sequences of the CDRs of trastuzumab, and their corresponding identifiers, are presented in Figure 1B. Any nucleotide sequences encoding SEQ ID NO:1 or SEQ ID NO:2 can be used in the compositions and methods of the present disclosure.

The present disclosure further provides anti-HER2 antibody fragments comprising CDR sequences that are related to the CDR sequences of trastuzumab. The term "antibody fragment" refers to a portion of a full-length antibody, generally the target binding or variable region. Examples of antibody fragments include Fab, Fab', F(ab')₂ and Fv fragments. An "Fv" fragment is the minimum antibody fragment which contains a complete target recognition and binding site. This region consists of a dimer of one heavy and one light chain variable domain in a tight, noncovalent association (V_{H} -V_{L} dimer). It is in this configuration that the three CDRs of each variable domain interact to define a target binding site on the surface of the V_{H} -V_{L} dimer. Often, the six CDRs confer target binding specificity to the antibody. However, in some instances even a single variable domain (or half of an Fv comprising only three CDRs specific for a target) can have the ability to recognize and bind target. "Single chain Fv" or "scFv" antibody fragments comprise the V_{H} and V_{L} domains of an antibody in a single polypeptide chain. Generally, the Fv polypeptide further comprises a polypeptide linker between the V_{H} and V_{L} domains which enables the scFv to form the desired structure for target binding. "Single domain antibodies" are composed of a single V_{H} or V_{L} domain which exhibit sufficient affinity to the target. In a specific embodiment, the single domain antibody is a camelid antibody (*see*, *e.g.,* Riechmann, 1999, Journal of Immunological Methods 231:25-38).

The Fab fragment contains the constant domain of the light chain and the first constant domain (CH₁) of the heavy chain. Fab' fragments differ from Fab fragments by the addition of a few residues at the carboxyl terminus of the heavy chain CH₁ domain including one or more cysteines from the antibody hinge region. F(ab') fragments are produced by cleavage of the disulfide bond at the hinge cysteines of the F(ab')₂ pepsin digestion product. Additional chemical couplings of antibody fragments are known to those of ordinary skill in the art.

In certain embodiments, the anti-HER2 antibodies of the disclosure are monoclonal antibodies. The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced. Monoclonal antibodies useful in connection with the present disclosure can be prepared using a wide variety of techniques known in the art including the use of hybridoma, recombinant, and phage display technologies, or a combination thereof. The anti-HER2 antibodies of the disclosure include chimeric, primatized, humanized, or human antibodies.

The anti-HER2 antibodies of the disclosure can be chimeric antibodies. The term "chimeric" antibody as used herein refers to an antibody having variable sequences derived from a non-human immunoglobulin, such as rat or mouse antibody, and human immunoglobulin constant regions, typically chosen from a human immunoglobulin template. Methods for producing chimeric antibodies are known in the art. *See, e.g.,* Morrison, 1985, Science 229(4719): 1202-7; Oi et al, 1986, BioTechniques 4:214-221 ; Gillies et al, 1985, J. Immunol. Methods 125: 191-202; U.S. Pat. Nos. 5,807,715; 4,816,567; and 4,816397.

The anti-HER2 antibodies of the disclosure can be humanized. "Humanized" forms of non-human (*e.g*., murine) antibodies are chimeric immunoglobulins, immunoglobulin chains or fragments thereof (such as Fv, Fab, Fab', F(ab')₂ or other target-binding subdomains of antibodies) which contain minimal sequences derived from non-human immunoglobulin. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin sequence. The humanized antibody can also comprise at least a portion of an immunoglobulin constant region (Fc), typically that of a human immunoglobulin consensus sequence. Methods of antibody humanization are known in the art. *See, e.g.,* Riechmann et al, 1988, Nature 332:323-7; U.S. Patent Nos: 5,530, 101 ; 5,585,089; 5,693,761; 5,693,762; and 6,180,370 to Queen et al; EP239400; PCT publication WO 91/09967; U.S. Patent No. 5,225,539; EP592106; EP519596; Padlan, 1991, Mol. Immunol., 28:489-498; Studnicka et al, 1994, Prot. Eng. 7:805-814; Roguska et al, 1994, Proc. Natl. Acad. Sci. 91 :969-973; and U.S. Patent No. 5,565,332.

The anti-HER2 antibodies of the disclosure can be human antibodies. Completely "human" anti- HER2 antibodies can be desirable for therapeutic treatment of human patients. As used herein, "human antibodies" include antibodies having the amino acid sequence of a human immunoglobulin and include antibodies isolated from human immunoglobulin libraries or from animals transgenic for one or more human immunoglobulin and that do not express endogenous immunoglobulins. Human antibodies can be made by a variety of methods known in the art including phage display methods using antibody libraries derived from human immunoglobulin sequences. *See* U.S. Patent Nos. 4,444,887 and 4,716,111; and PCT publications WO 98/46645; WO 98/50433; WO 98/24893; WO 98/16654; WO 96/34096; WO 96/33735; and WO 91/10741. Human antibodies can also be produced using transgenic mice which are incapable of expressing functional endogenous immunoglobulins, but which can express human immunoglobulin genes. *See, e.g*., PCT publications WO 98/24893; WO 92/01047; WO 96/34096; WO 96/33735; U.S. Patent Nos. 5,413,923; 5,625,126; 5,633,425; 5,569,825; 5,661,016; 5,545,806; 5,814,318; 5,885,793; 5,916,771 ; and 5,939,598. In addition, companies such as Medarex (Princeton, NJ), Astellas Pharma (Deerfield, IL), Amgen (Thousand Oaks, CA) and Regeneron (Tarrytown, NY) can be engaged to provide human antibodies directed against a selected antigen using technology similar to that described above. Completely human antibodies that recognize a selected epitope can be generated using a technique referred to as "guided selection." In this approach a selected non-human monoclonal antibody, *e.g*., a mouse antibody, is used to guide the selection of a completely human antibody recognizing the same epitope (Jespers et al, 1988, Biotechnology 12:899-903).

The anti-HER2 antibodies of the disclosure can be primatized. The term "primatized antibody" refers to an antibody comprising monkey variable regions and human constant regions. Methods for producing primatized antibodies are known in the art. *See e.g.,* U.S. Patent Nos. 5,658,570; 5,681,722; and 5,693,780.

The anti-HER2 antibodies of the disclosure can be bispecific antibodies. Bispecific antibodies are monoclonal, often human or humanized, antibodies that have binding specificities for at least two different antigens. In the present disclosure, one of the binding specificities can be directed towards HER2, the other can be for any other antigen, e.g., for a cell-surface protein, receptor, receptor subunit, tissue-specific antigen, virally derived protein, virally encoded envelope protein, bacterially derived protein, or bacterial surface protein, *etc.*

The anti-HER2 antibodies of the disclosure include derivatized antibodies. For example, but not by way of limitation, derivatized antibodies are typically modified by glycosylation, acetylation, pegylation, phosphorylation, amidation, derivatization by known protecting/blocking groups, proteolytic cleavage, linkage to a cellular ligand or other protein (see Section 7.9 for a discussion of antibody conjugates), *etc.* Any of numerous chemical modifications can be carried out by known techniques, including, but not limited to, specific chemical cleavage, acetylation, formylation, metabolic synthesis of tunicamycin, etc. Additionally, the derivative can contain one or more non-natural amino acids, *e.g.,* using ambrx technology (*see, e.g.,* Wolfson, 2006, Chem. Biol. 13(10):1011-2).

In yet another embodiment of the disclosure, the anti-HER2 antibodies or fragments thereof can be antibodies or antibody fragments whose sequence has been modified to alter at least one constant region-mediated biological effector function relative to the corresponding wild type sequence.

For example, in some embodiments, an anti-HER2 antibody of the disclosure can be modified to reduce at least one constant region-mediated biological effector function relative to an unmodified antibody, e.g., reduced binding to the Fc receptor (FcγR). FcγR binding can be reduced by mutating the immunoglobulin constant region segment of the antibody at particular regions necessary for FcγR interactions *(see e.g.,* Canfield and Morrison, 1991, J. Exp. Med. 173:1483-1491; and Lund et al., 1991, J. Immunol. 147:2657-2662). Reduction in FcγR binding ability of the antibody can also reduce other effector functions which rely on FcγR interactions, such as opsonization, phagocytosis and antigen-dependent cellular cytotoxicity ("ADCC").

In other embodiments, an anti-HER2 antibody of the disclosure can be modified to acquire or improve at least one constant region-mediated biological effector function relative to an unmodified antibody, *e.g.,* to enhance FcγR interactions (*see, e.g.,* US 2006/0134709). For example, an anti-HER2 antibody of the disclosure can have a constant region that binds FcγRIIA, FcγRIIB and/or FcγRIIIA with greater affinity than the corresponding wild type constant region.

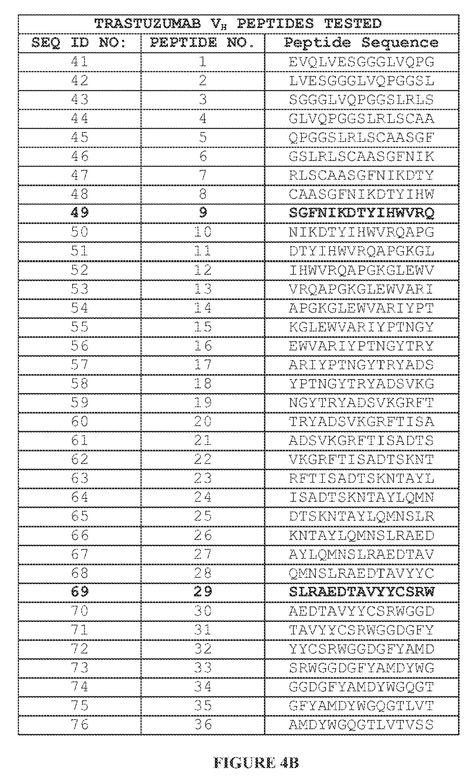

Thus, antibodies of the disclosure can have alterations in biological activity that result in increased or decreased opsonization, phagocytosis, or ADCC. Such alterations are known in the art. For example, modifications in antibodies that reduce ADCC activity are described in U.S. Patent No. 5,834,597. An exemplary ADCC lowering variant corresponds to "mutant 3" shown in Figure 4 of U.S. Patent No. 5,834,597, in which residue 236 is deleted and residues 234, 235 and 237 (using EU numbering) are substituted with alanines.

In some embodiments, the anti-HER2 antibodies of the disclosure have low levels of or lack fucose. Antibodies lacking fucose have been correlated with enhanced ADCC activity, especially at low doses of antibody. *See* Shields et al., 2002, J. Biol. Chem. 277:26733-26740; Shinkawa et al., 2003, J. Biol. Chem. 278:3466-73. Methods of preparing fucose-less antibodies include growth in rat myeloma YB2/0 cells (ATCC CRL 1662). YB2/0 cells express low levels of FUT8 mRNA, which encodes α-1,6-fucosyltransferase, an enzyme necessary for fucosylation of polypeptides.

In yet another aspect, the anti-HER2 antibodies or fragments thereof can be antibodies or antibody fragments that have been modified to increase or reduce their binding affinities to the fetal Fc receptor, FcRn, for example by mutating the immunoglobulin constant region segment at particular regions involved in FcRn interactions (see e.g., WO 2005/123780). In particular embodiments, an anti-HER2 antibody of the IgG class is mutated such that at least one of amino acid residues 250, 314, and 428 of the heavy chain constant region is substituted alone, or in any combinations thereof, such as at positions 250 and 428, or at positions 250 and 314, or at positions 314 and 428, or at positions 250, 314, and 428, with positions 250 and 428 a specific combination. For position 250, the substituting amino acid residue can be any amino acid residue other than threonine, including, but not limited to, alanine, cysteine, aspartic acid, glutamic acid, phenylalanine, glycine, histidine, isoleucine, lysine, leucine, methionine, asparagine, proline, glutamine, arginine, serine, valine, tryptophan, or tyrosine. For position 314, the substituting amino acid residue can be any amino acid residue other than leucine, including, but not limited to, alanine, cysteine, aspartic acid, glutamic acid, phenylalanine, glycine, histidine, isoleucine, lysine, methionine, asparagine, proline, glutamine, arginine, serine, threonine, valine, tryptophan, or tyrosine. For position 428, the substituting amino acid residues can be any amino acid residue other than methionine, including, but not limited to, alanine, cysteine, aspartic acid, glutamic acid, phenylalanine, glycine, histidine, isoleucine, lysine, leucine, asparagine, proline, glutamine, arginine, serine, threonine, valine, tryptophan, or tyrosine. Specific combinations of suitable amino acid substitutions are identified in Table 1 of U.S. Patent No. 7,217,797. Such mutations increase the antibody's binding to FcRn, which protects the antibody from degradation and increases its half-life.

In yet other aspects, an anti-HER2 antibody has one or more amino acids inserted into one or more of its hypervariable regions, for example as described in S. Jung and A. Pluckthun (1997) Improving in vivo folding and stability of a single-chain Fv antibody fragment by loop grafting. Protein Engineering 10:9, 959-966; Yazaki, et al. (2004) Humanization of the anti-CEA T84.66 antibody based on crystal structure data. Protein Eng Des Sel. 17(5):481-9. Epub 2004 Aug 17.

In various embodiments, the anti-HER2 antibodies or fragments thereof can be antibodies or antibody fragments that have been modified for increased expression in heterologous hosts. In certain embodiments, the anti-HER2 antibodies or fragments thereof can be antibodies or antibody fragments that have been modified for increased expression in and/or secretion from heterologous host cells. In some embodiments, the anti-HER2 antibodies or fragments thereof are modified for increased expression in bacteria, such as E. coli. In other embodiments, the anti-HER2 antibodies or fragments thereof are modified for increased expression in yeast. (Kieke et al., 1999, Proc. Nat'l Acad. Sci. USA 96:5651-5656). In still other embodiments, the anti-HER2 antibodies or fragments thereof are modified for increased expression in insect cells. In additional embodiments, the anti-HER2 antibodies or fragments thereof are modified for increased expression in mammalian cells, such as CHO cells.

In certain embodiments, the anti-HER2 antibodies or fragments thereof can be antibodies or antibody fragments that have been modified to increase stability of the antibodies during production. In some embodiments, the antibodies or fragments thereof can be modified to replace one or more amino acids such as asparagine or glutamine that are susceptible to nonenzymatic deamidation with amino acids that do not undergo deamidation. (Huang et al., 2005, Anal. Chem. 77:1432-1439). In other embodiments, the antibodies or fragments thereof can be modified to replace one or more amino acids that is susceptible to oxidation, such as methionine, cysteine or tryptophan, with an amino acid that does not readily undergo oxidation. In still other embodiments, the antibodies or fragments thereof can be modified to replace one or more amino acids that is susceptible to cyclization, such as asparagine or glutamic acid, with an amino acid that does not readily undergo cyclization.

### 7.2. Nucleic Acids and Expression Systems

The present disclosure encompasses nucleic acid molecules and host cells encoding the anti-HER2 antibodies of the disclosure.

An anti-HER2 antibody of the disclosure can be prepared by recombinant expression of immunoglobulin light and heavy chain genes in a host cell. To express an antibody recombinantly, a host cell is transfected with one or more recombinant expression vectors carrying DNA fragments encoding the immunoglobulin light and heavy chains of the antibody such that the light and heavy chains are expressed in the host cell and, optionally, secreted into the medium in which the host cells are cultured, from which medium the antibodies can be recovered. Standard recombinant DNA methodologies are used to obtain antibody heavy and light chain genes, incorporate these genes into recombinant expression vectors and introduce the vectors into host cells, such as those described in Molecular Cloning; A Laboratory Manual, Second Edition (Sambrook, Fritsch and Maniatis (eds), Cold Spring Harbor, N. Y., 1989), Current Protocols in Molecular Biology (Ausubel, F.M. et al., eds., Greene Publishing Associates, 1989) and in U.S. Patent No. 4,816,397.

In one embodiment, the anti-HER2 antibodies are similar to trastuzumab but for changes in one or more CDRs (referred to herein as having "trastuzumab-related" sequences). In another embodiment, the anti-HER2 antibodies are similar to trastuzumab but for changes in one or more framework regions. In yet another embodiment, the anti-HER2 antibodies are similar to trastuzumab but for changes in one or more CDRs and in one or more framework regions. To generate nucleic acids encoding such anti-HER2 antibodies, DNA fragments encoding the light and heavy chain variable regions are first obtained. These DNAs can be obtained by amplification and modification of germline DNA or cDNA encoding light and heavy chain variable sequences, for example using the polymerase chain reaction (PCR). Germline DNA sequences for human heavy and light chain variable region genes are known in the art (*see e.g*., the "VBASE" human germline sequence database; see also Kabat, E. A. et al., 1991 , Sequences of Proteins of Immunological Interest, Fifth Edition, U.S. Department of Health and Human Services, NIH Publication No. 91 -3242; Tomlinson et al., 1992, J. Mol. Biol. 22T: 116-198; and Cox et al, 1994, Eur. J. Immunol. 24:827-836. A DNA fragment encoding the heavy or light chain variable region of trastuzumab can be synthesized and used as a template for mutagenesis to generate a variant as described herein using routine mutagenesis techniques; alternatively, a DNA fragment encoding the variant can be directly synthesized.

Once DNA fragments encoding trastuzumab or trastuzumab-related VH and VL segments are obtained, these DNA fragments can be further manipulated by standard recombinant DNA techniques, for example to convert the variable region genes to full-length antibody chain genes, to Fab fragment genes or to a scFv gene. In these manipulations, a VL- or VH-encoding DNA fragment is operatively linked to another DNA fragment encoding another protein, such as an antibody constant region or a flexible linker. The term "operatively linked," as used in this context, is intended to mean that the two DNA fragments are joined such that the amino acid sequences encoded by the two DNA fragments remain in- frame.

The isolated DNA encoding the VH region can be converted to a full-length heavy chain gene by operatively linking the VH-encoding DNA to another DNA molecule encoding heavy chain constant regions (CH₁, CH₂, CH₃ and, optionally, CH₄). The sequences of human heavy chain constant region genes are known in the art *(see e.g.,* Kabat, E.A., et al., 1991 , Sequences of Proteins of Immunological Interest, Fifth Edition, U.S. Department of Health and Human Services, NIH Publication No. 91-3242) and DNA fragments encompassing these regions can be obtained by standard PCR amplification. The heavy chain constant region can be an IgG₁, IgG₂, IgG₃, IgG₄, IgA, IgE, IgM or IgD constant region, but in certain embodiments is an IgG₁ constant region. For a Fab fragment heavy chain gene, the V_{H}-encoding DNA can be operatively linked to another DNA molecule encoding only the heavy chain CH₁ constant region.

The isolated DNA encoding the V_{L} region can be converted to a full-length light chain gene (as well as a Fab light chain gene) by operatively linking the V_{L}-encoding DNA to another DNA molecule encoding the light chain constant region, C_{L}- The sequences of human light chain constant region genes are known in the art (*see e.g.,* Kabat, E. A., et al., 1991 , Sequences of Proteins of Immunological Interest, Fifth Edition (U.S. Department of Health and Human Services, NIH Publication No. 91-3242)) and DNA fragments encompassing these regions can be obtained by standard PCR amplification. The light chain constant region can be a kappa or lambda constant region, but in certain embodiments is a kappa constant region. To create a scFv gene, the V_{H} and V_{L}-encoding DNA fragments are operatively linked to another fragment encoding a flexible linker, e.g., encoding the amino acid sequence (Gly₄∼Ser)₃, such that the V_{H} and V_{L} sequences can be expressed as a contiguous single-chain protein, with the V_{L} and V_{H} regions joined by the flexible linker (*see e.g.,* Bird et al., 1988, Science 242:423-426; Huston et al., 1988, Proc. Natl. Acad. Sci. USA 85:5879-5883; McCafferty et al., 1990, Nature 348:552-554).

To express the anti-HER2 antibodies of the disclosure, DNAs encoding partial or full-length light and heavy chains, obtained as described above, are inserted into expression vectors such that the genes are operatively linked to transcriptional and translational control sequences. In this context, the term "operatively linked" is intended to mean that an antibody gene is ligated into a vector such that transcriptional and translational control sequences within the vector serve their intended function of regulating the transcription and translation of the antibody gene. The expression vector and expression control sequences are chosen to be compatible with the expression host cell used. The antibody light chain gene and the antibody heavy chain gene can be inserted into separate vectors or, more typically, both genes are inserted into the same expression vector.

The antibody genes are inserted into the expression vector by standard methods (e.g., ligation of complementary restriction sites on the antibody gene fragment and vector, or blunt end ligation if no restriction sites are present). Prior to insertion of the trastuzumab or trastuzumab-related light or heavy chain sequences, the expression vector can already carry antibody constant region sequences. For example, one approach to converting the trastuzumab or trastuzumab-related V_{H} and V_{L} sequences to full-length antibody genes is to insert them into expression vectors already encoding heavy chain constant and light chain constant regions, respectively, such that the V_{H} segment is operatively linked to the C_{H} segment(s) within the vector and the V_{L} segment is operatively linked to the C_{L} segment within the vector. Additionally or alternatively, the recombinant expression vector can encode a signal peptide that facilitates secretion of the antibody chain from a host cell. The antibody chain gene can be cloned into the vector such that the signal peptide is linked in-frame to the amino terminus of the antibody chain gene. The signal peptide can be an immunoglobulin signal peptide or a heterologous signal peptide (*i.e*., a signal peptide from a non-immunoglobulin protein).

In addition to the antibody chain genes, the recombinant expression vectors of the disclosure carry regulatory sequences that control the expression of the antibody chain genes in a host cell. The term "regulatory sequence" is intended to include promoters, enhancers and other expression control elements (*e.g*., polyadenylation signals) that control the transcription or translation of the antibody chain genes. Such regulatory sequences are described, for example, in Goeddel, Gene Expression Technology: Methods in Enzymology 185 (Academic Press, San Diego, CA, 1990). It will be appreciated by those skilled in the art that the design of the expression vector, including the selection of regulatory sequences may depend on such factors as the choice of the host cell to be transformed, the level of expression of protein desired, etc. Suitable regulatory sequences for mammalian host cell expression include viral elements that direct high levels of protein expression in mammalian cells, such as promoters and/or enhancers derived from cytomegalovirus (CMV) (such as the CMV promoter/enhancer), Simian Virus 40 (SV40) (such as the SV40 promoter/enhancer), adenovirus, (*e.g*., the adenovirus major late promoter (AdMLP)) and polyoma. For further description of viral regulatory elements, and sequences thereof, *see e.g.,* U.S. Patent No. 5,168,062 by Stinski, U.S. Patent No. 4,510,245 by Bell et al., and U.S. Patent No. 4,968,615 by Schaffner et al.

In addition to the antibody chain genes and regulatory sequences, the recombinant expression vectors of the disclosure can carry additional sequences, such as sequences that regulate replication of the vector in host cells (*e.g*., origins of replication) and selectable marker genes. The selectable marker gene facilitates selection of host cells into which the vector has been introduced *(see e.g.,* U.S. Patents Nos. 4,399,216, 4,634,665 and 5,179,017, all by Axel et al.). For example, typically the selectable marker gene confers resistance to drugs, such as G418, puromycin, blasticidin, hygromycin or methotrexate, on a host cell into which the vector has been introduced. Suitable selectable marker genes include the dihydrofolate reductase (DHFR) gene (for use in DHFR⁻ host cells with methotrexate selection/amplification) and the neo gene (for G418 selection). For expression of the light and heavy chains, the expression vector(s) encoding the heavy and light chains is transfected into a host cell by standard techniques. The various forms of the term "transfection" are intended to encompass a wide variety of techniques commonly used for the introduction of exogenous DNA into a prokaryotic or eukaryotic host cell, *e.g*., electroporation, lipofection, calcium-phosphate precipitation, DEAE- dextran transfection and the like.

It is possible to express the antibodies of the disclosure in either prokaryotic or eukaryotic host cells. In certain embodiments, expression of antibodies is performed in eukaryotic cells, *e.g*., mammalian host cells, for optimal secretion of a properly folded and immunologically active antibody. Exemplary mammalian host cells for expressing the recombinant antibodies of the disclosure include Chinese Hamster Ovary (CHO cells) (including DHFR⁻ CHO cells, described in Urlaub and Chasin, 1980, Proc. Natl. Acad. Sci. USA 77:4216-4220, used with a DHFR selectable marker, *e.g*., as described in Kaufman and Sharp, 1982, Mol. Biol. 159:601-621), NS0 myeloma cells, COS cells, 293 cells and SP2/0 cells. When recombinant expression vectors encoding antibody genes are introduced into mammalian host cells, the antibodies are produced by culturing the host cells for a period of time sufficient to allow for expression of the antibody in the host cells or secretion of the antibody into the culture medium in which the host cells are grown. Antibodies can be recovered from the culture medium using standard protein purification methods. Host cells can also be used to produce portions of intact antibodies, such as Fab fragments or scFv molecules. It is understood that variations on the above procedure are within the scope of the present disclosure. For example, it can be desirable to transfect a host cell with DNA encoding either the light chain or the heavy chain (but not both) of an anti-HER2 antibody of this disclosure.

Recombinant DNA technology can also be used to remove some or all of the DNA encoding either or both of the light and heavy chains that is not necessary for binding to HER2. The molecules expressed from such truncated DNA molecules are also encompassed by the antibodies of the disclosure.

In addition, bifunctional antibodies can be produced in which one heavy and one light chain are an antibody of the disclosure and the other heavy and light chain are specific for an antigen other than HER2 by crosslinking an antibody of the disclosure to a second antibody by standard chemical crosslinking methods. Bifunctional antibodies can also be made by expressing a nucleic acid engineered to encode a bifunctional antibody.

In certain embodiments, dual specific antibodies, *i.e*., antibodies that bind HER2 and an unrelated antigen using the same binding site, can be produced by mutating amino acid residues in the light chain and/or heavy chain CDRs. In various embodiments, dual specific antibodies that bind two antigens, such as HER2 and VEGF, can be produced by mutating amino acid residues in the periphery of the antigen binding site. (Bostrom et al., 2009, Science 323:1610-1614). Dual functional antibodies can be made by expressing a nucleic acid engineered to encode a dual specific antibody.

For recombinant expression of an anti-HER2 antibody of the disclosure, the host cell can be co-transfected with two expression vectors of the disclosure, the first vector encoding a heavy chain derived polypeptide and the second vector encoding a light chain derived polypeptide. Typically, the two vectors each contain a separate selectable marker. Alternatively, a single vector can be used which encodes both heavy and light chain polypeptides.

Once a nucleic acid encoding one or more portions of trastuzumab or of an anti-HER2 antibody with CDR sequences related to the CDR sequences of trastuzumab is generated, further alterations or mutations can be introduced into the coding sequence, for example to generate nucleic acids encoding antibodies with different CDR sequences, antibodies with reduced affinity to the Fc receptor, or antibodies of different subclasses.

The anti-HER2 antibodies of the disclosure can also be produced by chemical synthesis (*e.g*., by the methods described in Solid Phase Peptide Synthesis, 2nd ed., 1984 The Pierce Chemical Co., Rockford, Ill.). Variant antibodies can also be generated using a cell-free platform (*see, e.g.,* Chu et al., Biochemia No. 2, 2001 (Roche Molecular Biologicals)).

Once an anti-HER2 antibody of the disclosure has been produced by recombinant expression, it can be purified by any method known in the art for purification of an immunoglobulin molecule, for example, by chromatography (e.g., ion exchange, affinity, particularly by affinity for HER2 after Protein A or Protein G selection, and sizing column chromatography), centrifugation, differential solubility, or by any other standard technique for the purification of proteins. Further, the anti-HER2 antibodies of the present disclosure or fragments thereof can be fused to heterologous polypeptide sequences described herein or otherwise known in the art to facilitate purification.

Once isolated, an anti-HER2 antibody can, if desired, be further purified, e.g., by high performance liquid chromatography (*See, e.g.,* Fisher, Laboratory Techniques In Biochemistry And Molecular Biology (Work and Burdon, eds., Elsevier, 1980)), or by gel filtration chromatography on a SuperdexTM 75 column (Pharmacia Biotech AB, Uppsala, Sweden).

### 7.3. Biological Activities of Anti-HER2 Antibodies

In certain embodiments, the anti-HER2 antibodies of the disclosure have certain biological activities, such as competing with trastuzumab for binding to HER2 or neutralizing HER2 activity.

Accordingly, in certain embodiments, anti-HER2 antibodies of the disclosure compete with trastuzumab for binding to HER2. The ability to compete for binding to HER2 can be tested using a competition assay. Anti-HER2 antibodies of the disclosure can be titered in PBS-BSA and tested for the ability to block the binding of a particular concentration of biotinylated trastuzumab (*e.g*., 40nM) to HER2 extracellular domain ("HER2-ECD") immobilized on microtiter plates (*e.g.,* by incubating 5 µg/ml HER2-ECD in 50 mM ammonium bicarbonate, pH 9.3 overnight at 4° C). Following a 1 h incubation with antibody, the plate is washed with PBS-Tween and Strepavidin/HRP conjugate (Streptavidin-POD, Roche Molecular Biochemicals) is added for 30 min. Plates are washed again with PBS-Tween and the bound HRP is assayed using ABTS/H₂O₂ substrate (Kirkegaard & Perry Laboratories) and the absorbance at 405 nm is monitored. The absorbance at 405 nm is plotted versus the concentration of anti-HER2 antibody originally added to the well. Sigmoidal curves are fit to a four parameter equation by nonlinear regression analysis (Marquardt, J. Soc. Indust. Appl. Math. 11:431-441 (1963)); the concentration of anti-HER2 antibody required to give a half-maximal signal in the assay (IC₅₀) can be calculated from the curves. Variations on this competition assay can also be used to test competition between an anti-HER2 antibody of the disclosure and trastuzumab. For example, in certain aspects, the anti-HER2 antibody is used as a reference antibody and trastuzumab is used as a test antibody.

Additionally, instead of soluble HER2, membrane-bound HER2 expressed on the surfaces of cell (for example mammalian cells) in culture can be used.

Other formats for competition assays are known in the art and can be employed, such as, for example, Amplified Luminescence Proximity Homogeneous Assay ("Alpha"), for example the AlphaLISA assay.

AlphaLISA is an analogous screening process to ELISA, which involves the attachment of a capture antibody to a solid phase support, whereupon samples containing antigen are then added in a matrix or buffer adapted to minimize attachment to the solid phase. In AlphaLISA, an analyte is captured by a biotinylated antibody bound to streptavidin-coated donor beads and a second antibody conjugated to AlphaLISA acceptor beads. The binding of the two antibodies to the analyte brings donor and acceptor beads into proximity. Laser irradiation of donor beads at 680 nm generates a flow of singlet oxygen, triggering a cascade of chemical events in nearby acceptor beads, which results in a chemiluminescent emission at 615 nm. In competitive AlphaLISA immunoassays, a biotinylated analyte bound to streptavidin donor beads is used with an antibody conjugated to AlphaLISA acceptor beads. AlphaLISA can also be used to determine the binding affinity of individual variants, *e.g*., anti-HER2 antibodies or antibody binding fragments of the disclosure, to HER2. See, for example, Ullman et al., Clinical Chemistry 42, no. 9:1518-1526, 1996.

In other aspects, an anti-HER2 antibody of the disclosure inhibits (or neutralizes) HER2 activity in a range of *in vitro* assays, such as cell proliferation, tyrosine phosphorylation, inhibition of heregulin binding and apoptosis. For example, in one embodiment, the anti-HER2 antibody is assayed for the ability to inhibit growth of SK-BR-3 human breast cancer tumor cells. (*See* U. S. Patent No. 6,577,171). In particular, SK-BR-3 cells are grown in a 1:1 mixture of F12 and DMEM medium supplemented with 10% fetal bovine serum, glutamine and penicillin streptomycin. The SK-BR-3 cells are plated at 20,000 cells in a 35 mm cell culture dish (2 mls/35 mm dish) and 0.5 to 30 µg/ml of the anti-HER2 antibody is added per dish. After six days, the number of cells in the wells is counted using an electronic COULTER™ counter and compared to the number of cells in untreated wells. The antibodies that inhibit the growth of SK-BR-3 cells by about 20-100%, and preferably by about 50-100% may be selected as growth inhibitory antibodies.

In various embodiments, an anti-HER2 antibody of the disclosure reduces the binding of labeled trastuzumab by at least 30%, by at least 40%, by at least 50%, by at least 60%, by at least 70%, by at least 80%, by at least 90%, by at least 95%, by at least 99%, or by a percentage ranging between any of the foregoing values (*e.g*., an anti-HER2 antibody of the disclosure reduces the binding of labeled trastuzumab by 50% to 70%) when the anti-HER2 antibody is used at a concentration of 0.08 µg/ml, 0.4 µg/ml, 2 µg/ml, 10 µg/ml, 50 µg/ml, 100 µg/ml or at a concentration ranging between any of the foregoing values (*e.g*., at a concentration ranging from 2 µg/ml to 10 µg/ml).

In certain embodiments, the activity assayed is phosphorylation of the HER2 receptor tyrosine kinase. In particular embodiments, the assay screens anti-HER2 antibodies for inhibition of heregulin stimulation of p180 tyrosine phosphorylation in MCF7 cells. Specifically, MCF7 cells are plated in 24-well plates and an antibody to be tested is added to each well and incubated with the cells for 30 minutes at room temperature. Subsequent to the incubation step, rHRGβ1₁₇₇₋₂₄₄ is added to each well to a final concentration of 0.2 nM and the incubation is continued for 8 minutes. Media is then aspirated from each well and reactions are stopped by the addition of 100 µl of SDS sample buffer (5% SDS, 25 mM DTT, and 25 mM Tris-HCl, pH 6.8). Each sample is then electrophoreses on a 4-12% gradient gel (Novex) and then electrophoretically transferred to polyvinylidene difluoride membrane. Antiphosphotyrosine (1 µg/ml) immunoblots are developed and the intensity of the predominant reactive band a Mᵣ∼ 180,000 quantified by reflectance densitometry.

In various embodiments, the anti-HER2 antibody activity is assayed by inhibition of heregulin binding to MCF7 breast tumor cells. MCF7 cells are cultured in 24-well-plates and then put on ice. An anti-HER2 antibody is added to each well and incubated for 30 minutes. ¹²⁵I_labeled rHRGβ1₁₇₇₋₂₂₄(25 pm) are then added and the incubation continued for 4 to 16 hours. Dose response curves are prepared and an IC₅₀ value is calculated for the anti-HER2 antibody. In certain embodiments, the antibody that blocks ligand activation of a EGF receptor will have an IC₅₀ for inhibiting heregulin binding to MCF7 cells in the assay of about 100 nM or less, such as about 75 nM or less, such as about 50 nM or less, such as about 25 nM or less, such as about 10 nM or less, or such as about 1 nM or less.

In other embodiments, the anti-HER2 antibody activity is assayed by induction of apoptosis. In certain embodiments, an assay for propidium iodide uptake using BT474 cells is used to identify antibodies that induce apoptosis. BT474 cells (from the American Type Culture Collection (Rockville, Md.)) are cultured in Dulbecco's Modified Eagle Medium:Ham's F12 (50:50) supplemented with 10% heat-inactivated FBS (Hyclone) and 2 mM L-glutamine. BT474 cells are seeded at a density of 3x10⁶ per dish in 100x20 mm dishes and allowed to attach overnight. Medium is then removed and replaced with fresh medium alone or medium containing 10 µg/ml of antibody. After a 3-day incubation, monolayers are washed with PBS and detached by trypsinization. Cells are centrifuged at 1200 rpm for 5 minutes at 4° C, and the pellet is resuspended in 3 ml ice cold Ca²⁺binding buffer (10 mM Hepes, pH 7.4, 140 mM NaCl, 2.5 mM CaCl₂) and aliquoted into 35 mm strainer-capped 12x75 tubes (1 ml per tube, 3 tubes per treatment group) to remove cell clumps. Tubes then receive 10/µg/ml propidium iodide and samples are analyzed by flow cytometry. Antibodies that induce statistically significant levels of cell death as determined by propidium iodide uptake relative to control are apoptosis-inducing antibodies.

Alternatively, an annexin binding assay using BT474 cells is used to identify apoptosis inducing antibodies. In this assay, cells are grown and treated as detailed in the previous paragraph. Tubes then received labeled annexin (*e.g*., annexin V-FTIC) (1µg/ml) and are then assayed by flow cytometry. In other embodiments, a DNA staining assay using BT474 cells is used to identify anti-HER2 antibodies that induce apoptosis. BT474 cells that have been grown and treated as detained in the previous paragraph are incubated with 9 µg/ml HOECHST 33342™ for 2 hours at 37° C, and then analyzed by flow cytometry. Antibodies that induce a greater than 2-fold change in the percentage of apoptotic cells as compared to untreated cells are apoptosis-inducing antibodies.

Other formats for HER2 neutralization assays are known in the art and can be employed.

In various embodiments, an anti-HER2 antibody of the disclosure neutralizes HER2 by at least 30%, by at least 40%, by at least 50%, by at least 60%, by at least 70%, by at least 80%, by at least 90%, or by a percentage ranging between any of the foregoing values (*e.g*., an anti-HER2 antibody of the disclosure neutralizes HER2 activity by 50% to 70%) when the anti-HER2 antibody is used at a concentration of 2 ng/ml, 5 ng/ml, 10 ng/ml, 20 ng/ml, 0.1 µg/ml, 0.2 µg/ml, 1 µg/ml, 2 µg/ml, 5 µg/ml, 10 µg/ml, 20 µg/ml, or at a concentration ranging between any of the foregoing values (*e.g*., at a concentration ranging from 1 µg/ml to 5 µg/ml).

In some embodiments, an anti-HER2 antibody of the disclosure is at least 0.7-fold as effective, 0.8-fold as effective, at least 0.9-fold as effective, at least 1-fold as effective, at least 1.1-fold as effective, at least 1.25-fold as effective, at least 1.5-fold as effective, at least 2-fold as effective, at least 5-fold as effective, at least 10-fold as effective, at least 20-fold as effective, at least 50-fold as effective, at least 100-fold as effective, at least 200-fold as effective, at least 500-fold as effective, at least 1000-fold as effective as trastuzumab at neutralizing HER2, or having an effectiveness at neutralizing HER2 relative to trastuzumab ranging between any pair of the foregoing values (*e.g*., 0.9-fold to 5-fold as effective as trastuzumab or 2-fold to 50-fold as effective as trastuzumab in neutralizing HER2).

### 7.4. Kinetic Properties of Anti-HER2 Antibodies

In certain embodiments, the anti-HER2 antibodies of the disclosure have a high binding affinity for HER2. In specific embodiments, the anti-HER2 antibodies of the present disclosure have specific association rate constants (kₒₙ or kₐ values), dissociation rate constants (k_{off} or k_{d} values), affinity constants (K_{A} values), dissociation constants (K_{D} values) and/or IC₅₀ values. In various embodiments, binding constants for the interaction of the anti-HER2 antibodies with HER2 receptor extracellular domain (p185^{HER2-ECD}) can be determined using surface plasmon resonance according to the method disclosed in Karlsson et al. (1991) J. Immunol. Methods 145:229-240. In certain aspects, such values are selected from the following embodiments.

In some embodiments, an anti-HER2 antibody of the disclosure binds to HER2 with a K_{A} (kₒₙ/k_{off}) of at least 10⁸ M⁻¹, at least 4 X 10⁸ M⁻¹, at least 10⁹ M⁻¹, at least 4 X 10⁹ M⁻¹, at least 10¹⁰ M⁻¹, at least 4 X 10¹¹ M⁻¹, at least 10¹¹ M⁻¹, at least 4 X10¹² M⁻¹, at least 10¹² M⁻¹, at least 4 X 10¹³ M⁻¹, at least 10¹³ M⁻¹, at least 4 X 10¹⁴ M⁻¹, at least 10¹⁴ M⁻¹, at least 4 X 10¹⁵ M⁻¹, at least 10¹⁵ M⁻¹, or with a K_{A} of any range between any pair of the foregoing values (e.g., 4 X 10⁸ M⁻¹, to 4 X 10¹³ M⁻¹, or 4 X 10¹² M⁻¹, to 4 X 10¹⁵ M⁻¹).

In certain embodiments, an anti-HER2 antibody of the disclosure binds to HER2 with a K_{D} (k_{off}/kₒₙ) of 5 X 10⁻⁸ M or less, of 10⁻⁸ M or less, of 5X 10⁻⁹ M or less, of 10⁻⁹ M or less, of 5 X 10⁻¹⁰ M or less, 10⁻¹⁰ M or less, 5 X 10⁻¹¹ M or less, 10⁻¹¹ M or less, 4 X 10⁻¹² M or less, 10⁻¹² M or less, 5 X 10⁻¹³ M or less, 10⁻¹³ M or less, 5 X 10¹⁴ M or less, 10⁻¹⁴ M or less, 5 X 10⁻¹⁵ M or less, 10⁻¹⁵ M or less, or with a K_{D} of any range between any pair of the foregoing values (*e.g*., 5 X 10⁻¹¹ M to 5 X 10⁻¹³ M or 5 X 10⁻¹² M to 10⁻¹⁵ M).

In specific embodiments, the K_{D} (k_{off}/kₒₙ) value is determined by assays well known in the art, e.g., ELISA, isothermal titration calorimetry (ITC), fluorescent polarization assay or any other biosensors such as BIAcore.

In some embodiments, an anti-HER2 antibody of the disclosure binds to HER2 and inhibits cell growth at an IC₅₀ less than 3 nM, less than 2 nM, less than 1 nM, less than 0.5 nM , less than 0.2 nM , less than 0.1 nM , less than 0.05 nM , less than 0.02 nM, less than 0.01 nM, less than 0.005 nM, less than 0.002 nM, less than 0.001 nM, less than 5 X 10⁻⁴ nM, less than 2 X 10⁻⁴ nM, less than 1 X 10⁻⁴ nM, less than 5 X 10⁻⁵ nM, less than 2 X 10⁻⁵ nM, less than 1 X 10⁻⁴ nM, less than 5 X 10⁻⁶ nM, less than 2 X 10⁻⁶ nM, less than 1 X 10⁻⁶ nM, less than 5 X 10⁻⁷ nM, less than 2 X 10⁻⁷ nM, less than 1 X 10⁻⁷ nM, or with an IC₅₀ of any range between any pair of the foregoing values (e.g., 0.02 nM to 2 X 10⁻⁵ nM, or 5 X 10⁻⁵ nM to 1 X 10⁻⁷ nM). IC₅₀ can be measured according to methods well known in the art, e.g., ELISA.

In certain embodiments, an anti-HER2 antibody of the disclosure binds to HER2 at a minimum effective concentration (EC₅₀) less than 10 nM, less than 9 nM, less than 8 nM, less than 7 nM, less than 6 nM, less than 5 nM, less than 4 nM, less than 3 nM, less than 2 nM, less than 1 nM, less than 0.5 nM, less than 0.2 nM, less than 0.1 nM, less than 0.05 nM, less than 0.02 nM, less than 0.01 nM, less than 0.005 nM, less than 0.002 nM, less than 0.001 nM, less than 5 X 10⁻⁴ nM, less than 2 X 10⁻⁴ nM, less than 1 X 10⁻⁴ nM, less than 5 X 10⁻⁵ nM, less than 2 X 10⁻⁵ nM, less than 1 X 10⁻⁵ nM, less than 5 X 10⁻⁶ nM, less than 2 X 10⁻⁶ nM, less than 1 X 10⁻⁶ nM, less than 5 X 10⁻⁷ nM, less than 2 X 10⁻⁷ nM, less than 1 X 10⁻⁷ nM, or with an EC₅₀ of any range between any pair of the foregoing values (*e.g*., 5 nM to 5 X 10⁻² nM, or 1 X 10⁻⁴ nM to 1 X 10⁻⁷ nM). EC₅₀ can be measured according to methods well known in the art, e.g., FACS competition binding assay.

In certain embodiments, the kinetic properties of an antibody of the disclosure are comparable to, or improved relative to, trastuzumab in a comparable assay. For example, in certain embodiments, an anti-HER2 antibody of the disclosure binds to HER2 with a kₒₙ rate ranging from approximately 0.5x to 1000x of the kₒₙ of trastuzumab, for example a kₒₙ of 0.75 of the kₒₙ of trastuzumab, a kₒₙ of 1 x of the kₒₙ of trastuzumab, a kₒₙ of 1.1x of the kₒₙ of trastuzumab, a kₒₙ of 1.2x of the kₒₙ of trastuzumab, a kₒₙ of 1.3x of the kₒₙ of trastuzumab, a kₒₙ of 1.4x of the kₒₙ of trastuzumab, a kₒₙ of 1.5x of the kₒₙ of trastuzumab, a kₒₙ of 1.6x of the kₒₙ of trastuzumab, a kₒₙ of 1.6x of the kₒₙ of trastuzumab, a kₒₙ of 1.7x of the kₒₙ of trastuzumab, a kₒₙ of 1.8x of the kₒₙ of trastuzumab, a kₒₙ of 1.9x of the kₒₙ of trastuzumab, a kₒₙ of 2x of the kₒₙ of trastuzumab, a kₒₙ of 2.25x of the kₒₙ of trastuzumab, a kₒₙ of 2.5x of the kₒₙ of trastuzumab, a kₒₙ of 2.75x of the kₒₙ of traztuzumab, a kₒₙ of 3x of the kₒₙ of trastuzumab, a kₒₙ of 4x of the kₒₙ of trastuzumab, a kₒₙ of 5x of the kₒₙ of trastuzumab, a kₒₙ of 6x of the kₒₙ of trastuzumab, a kₒₙ of 7x of the kₒₙ of trastuzumab, a kₒₙ of 8x of the kₒₙ of trastuzumab, a kₒₙ of 9x of the kₒₙ of trastuzumab, a kₒₙ of 10x of the kₒₙ of trastuzumab, a kₒₙ of 15x of the kₒₙ of trastuzumab, a kₒₙ of 20x of the kₒₙ of trastuzumab, a kₒₙ of 50x of the kₒₙ of trastuzumab, a kₒₙ of 75x of the kₒₙ of trastuzumab, a kₒₙ of 100x of the kₒₙ of trastuzumab, a kₒₙ of 150x of the kₒₙ of trastuzumab, a kₒₙ of 200x of the kₒₙ of trastuzumab, or a kₒₙ ranging between any pair of the foregoing values, e.g., a kₒₙ of 2x-75x of the kₒₙ of trastuzumab, a kₒₙ of 5x-100x of the kₒₙ of trastuzumab, a kₒₙ of 0.05x-1000x of the kₒₙ of trastuzumab, a kₒₙ of 0.75x-250x of the kₒₙ of trastuzumab, *etc.*

In various embodiments, an anti-HER2 antibody of the disclosure binds to HER2 with a k_{off} rate ranging from about 0.001x to about 3x of the k_{off} of trastuzumab, for example a k_{off} of 0.002x of the k_{off} of trastuzumab, a k_{off} of 0.003x of the k_{off} of trastuzumab, a k_{off} of 0.004x of the k_{off} of trastuzumab, a k_{off} of 0.005x of the k_{off} of trastuzumab, a k_{off} of 0.006x of the k_{off} of trastuzumab, a k_{off} of 0.0075x of the k_{off} of trastuzumab, a k_{off} of 0.01x of the k_{off} of trastuzumab, a k_{off} of 0.025x of the k_{off} of trastuzumab, a k_{off} of 0.05x of the k_{off} of trastuzumab, a k_{off} of 0.075x of the k_{off} of trastuzumab, a k_{off} of 0.1x of the k_{off} of trastuzumab, a k_{off} of 0.25x of the k_{off} of trastuzumab, a k_{off} of 0.5x of the k_{off} of trastuzumab, a k_{off} of 0.75x of the k_{off} of trastuzumab, a k_{off} of 1x of the k_{off} of trastuzumab, a k_{off} of 1.25x of the k_{off} of trastuzumab, a k_{off} of 1.5x of the k_{off} of trastuzumab, a k_{off} of 1.75x of the k_{off} of trastuzumab, a k_{off} of 2x of the k_{off} of trastuzumab, a k_{off} of 2.25x of the k_{off} of trastuzumab, a k_{off} of 2.5x of the k_{off} of trastuzumab, a k_{off} of 3x of the k_{off} of trastuzumab, or a k_{off} ranging between any pair of the foregoing values, e.g., a k_{off} of 0.01x to 1.25x of the k_{off} of trastuzumab, a k_{off} of 0.05x to 2.5x of the k_{off} of trastuzumab, or a k_{off} of 0.006x to 0.1x of the k_{off} of trastuzumab, *etc.*

In other embodiments, an anti-HER2 antibody of the disclosure binds to HER2 with a K_{A} (kₒₙ/k_{off}) ranging from about 0.25x to about 1000x of the K_{A} of trastuzumab, for example a K_{A} of 0.5x of the K_{A} of trastuzumab, a K_{A} of 0.75 of the K_{A} of trastuzumab, a K_{A} of 1x of the K_{A} of trastuzumab, a K_{A} of 2x of the K_{A} of trastuzumab, a K_{A} of 3x of the K_{A} of trastuzumab, a K_{A} of 4x of the K_{A} of trastuzumab, a K_{A} of 5x of the K_{A} of trastuzumab, a K_{A} of 10x of the K_{A} of trastuzumab, a K_{A} of 15x of the K_{A} of trastuzumab, a K_{A} of 20x of the K_{A} of trastuzumab, a K_{A} of 30x of the K_{A} of trastuzumab, a K_{A} of 40x of the K_{A} of trastuzumab, a K_{A} of 50x of the K_{A} of trastuzumab, a K_{A} of 75x of the K_{A} of trastuzumab, a K_{A} of 100x of the K_{A} of trastuzumab, a K_{A} of 200x of the K_{A} of trastuzumab, a K_{A} of 250x of the K_{A} of trastuzumab, a K_{A} of 300x of the K_{A} of trastuzumab, a K_{A} of 350X of the K_{A} of trastuzumab, a K_{A} of 400x of the K_{A} of trastuzumab, a K_{A} of 500x of the K_{A} of trastuzumab, a K_{A} of 750x of the K_{A} of trastuzumab, a K_{A} of 100x of the K_{A} of trastuzumab, or a K_{A} ranging between any pair of the foregoing values, *e.g*., a K_{A} of 0.75x to 100x of the K_{A} of trastuzumab, a K_{A} of 10x to 50x of the K_{A} of trastuzumab, or a K_{A} of 5x to 50x of the K_{A} of trastuzumab, *etc.*

In still other embodiments, an anti-Her2 antibody of the disclosure binds to HER2 with a K_{D} (k_{off}/kₒₙ) ranging from about 0.001x to 10x of the K_{D} of trastuzumab, or example a K_{D} of 0.001x of the K_{D} of trastuzumab, a K_{D} of 0.002x of the K_{D} of trastuzumab, a K_{D} of 0.003x of the K_{D} of trastuzumab, a K_{D} of 0.004x of the K_{D} of trastuzumab, a K_{D} of 0.005x of the K_{D} of trastuzumab, a K_{D} of 0.0075x of the K_{D} of trastuzumab, a K_{D} of 0.01x of the K_{D} of trastuzumab, a K_{D} of 0.025x of the K_{D} of trastuzumab, a K_{D} of 0.05x of the K_{D} of trastuzumab, a K_{D} of 0.075x of the K_{D} of trastuzumab, a K_{D} of 0.1x of the K_{D} of trastuzumab, a K_{D} of 0.2x of the K_{D} of trastuzumab, a K_{D} of 0.3x of the K_{D} of trastuzumab, a K_{D} of 0.4x of the K_{D} of trastuzumab, a K_{D} of 0.5x of the K_{D} of trastuzumab, a K_{D} of 0.75x of the K_{D} of trastuzumab, a K_{D} of 1x of the K_{D} of trastuzumab, a K_{D} of 1.5x of the K_{D} of trastuzumab, a K_{D} of 2x of the K_{D} of trastuzumab, a K_{D} of 3x of the K_{D} of trastuzumab, a K_{D} of 4x of the K_{D} of trastuzumab, a K_{D} of 5x of the K_{D} of trastuzumab, a K_{D} of 7.5x of the K_{D} of trastuzumab, a K_{D} of 10x of the K_{D} of trastuzumab, or a K_{D} ranging between any pair of the foregoing values, *e.g*., a K_{D} of 0.001x o 0.5x of the K_{D} of trastuzumab, a K_{D} of 0.1x to 4x of the K_{D} of trastuzumab, a K_{D} of 0.05 to 1x of the K_{D} of trastuzumab, *etc.*

In certain embodiments, an anti-HER2 antibody of the disclosure binds to HER2 and inhibits cell growth or neutralizes the activity of HER2 at a IC₅₀ value ranging from about 0.001x to 10x of the IC₅₀ of trastuzumab, for example at an IC₅₀ value of 0.01x of the IC₅₀ of trastuzumab, at an IC₅₀ of 0.05x of the IC₅₀ of trastuzumab, at an IC₅₀ of 0.1x of the IC₅₀ of trastuzumab, at an IC₅₀ of 0.2x of the IC₅₀ of trastuzumab, at an IC₅₀ of 0.3x of the IC₅₀ of trastuzumab, at an IC₅₀ of 0.4x of the IC₅₀ of trastuzumab, at an IC₅₀ of 0.5x of the IC₅₀ of trastuzumab, at an IC₅₀ of 0.6x of the IC₅₀ of trastuzumab, at an IC₅₀ of 0.7x of the IC₅₀ of trastuzumab, at an IC₅₀ of 0.8x of the IC₅₀ of trastuzumab, at an IC₅₀ of 0.9x of the IC₅₀ of trastuzumab, at an IC₅₀ of 1x of the IC₅₀ of trastuzumab, at an IC₅₀ of 1.5x of the IC₅₀ of trastuzumab, at an IC₅₀ of 2x of the IC₅₀ of trastuzumab, at an IC₅₀ of 3x of the IC₅₀ of trastuzumab, at an IC₅₀ of 4x of the IC₅₀ of trastuzumab, at an IC₅₀ of 5x of the IC₅₀ of trastuzumab, at an IC₅₀ of 7.5x of the IC₅₀ of trastuzumab, at an IC₅₀ of 10x of the IC₅₀ of trastuzumab, or an IC₅₀ ranging between any pair of the foregoing values, e.g., an IC₅₀ of 0.01 to 0.2 of the IC₅₀ of trastuzumab, an IC₅₀ of 0.1x to 1.5x of the IC₅₀ of trastuzumab, an IC₅₀ of 0.2x to 2x of the IC₅₀ of trastuzumab, *etc.* In certain embodiments, a single CDR substitution can result in the foregoing differences in IC₅₀ as compared to trastuzumab, whereas an anti-HER2 antibody of the disclosure can comprise such substitution and up to 16 additional CDR substitutions as compared to trastuzumab.

### 7.5. Improved Expression of Anti-HER2 Antibodies

In certain aspects, the present disclosure provides anti-HER2 antibodies having improved expression as compared to trastuzumab. The present disclosure provides anti-HER2 antibodies having single or multiple amino acid substitutions in their CDRs and/or framework regions as compared to the CDRs and/or framework regions of trastuzumab, wherein at least one substitution improves the expression of the antibody as compared to trastuzumab. In certain embodiments, the improved expression is an increase in overall expression levels. In other embodiments, the improved expression results in a more homogenous population of antibodies with respect to molecular weight, glycosylation, or other property of the molecule. Without being bound by any theory, the improvement in expression levels can result from improved folding, reduced glycosylation (e.g., due elimination of a glycosylation site) and/or reduced proteolysis (e.g., due to elimination of a protease recognition motif) as compared to trastuzumab.

In certain embodiments, the expression of an anti-HER2 antibody of the disclosure can be at least 10% greater, at least 20% greater, at least 30% greater, at least 40% greater, at least 50% greater, at least 75% greater, at least 100% greater, at least 125% greater, at least 150% greater or at least 200% greater than the expression of trastuzumab in any given prokaryotic or eukaryotic (e.g., yeast, insect or mammalian) host cell as described in Section 7.2 above. In certain embodiments, the expression of an anti-HER2 antibody of the disclosure can be increased relative to trastuzumab in any given prokaryotic or eukaryotic (e.g., yeast, insect or mammalian) host cell in a range between any of the foregoing values, e.g., 20%-75% greater, 50%-100% greater, 30%-125% greater, or the like. In various specific embodiment, the host cell is E. coli, Sf9, S. cereviseae, or CHO cells.

In other embodiments, the homogeneity of an anti-HER2 antibody of the disclosure upon expression in a given prokaryotic or eukaryotic (e.g., yeast, insect or mammalian) host cell (as described in Section 7.2 above) and, optionally isolation and/or purification (e.g., by passage on an affinity column) can be at least 10% greater, at least 20% greater, at least 30% greater, at least 40% greater, at least 50% greater, at least 60% greater, at least 70% greater, at least 80% greater, at least 90% greater, at least 100% greater, at least 125% greater, at least 150% greater or at least 200% greater than the homogeneity of trastuzumab when expressed in the same host cell given host cell an subjected to the same treatment (e.g., isolation and/or purification). In certain embodiments, the homogeneity of an anti-HER2 antibody of the disclosure can be increased relative to trastuzumab in a range between any of the foregoing values, e.g., 20%-80% greater, 50%-100% greater, 30%-125% greater, or the like. In various specific embodiment, the host cell is *E. coli,* Sf9, *S. cereviseae*, or CHO, and the homogeneity is assayed in a composition after the anti-HER2 antibody is purified such that various anti-HER2 antibody species in the composition represent at least 70%, at least 80%, at least 90%, or at least 95% of proteins in the composition.

The present disclosure further provides methods for screening anti-HER2 antibodies for improved expression relative to trastuzumab. In specific embodiments, the methods comprise the steps of (a) determining an amount of the anti-HER2 antibody or anti-HER2 antibody binding fragment expressed in the host cell; and (b) comparing the amount of anti-HER2 antibody or anti-HER2 binding fragment produced in the host cell to an amount of trastuzumab produced in the host cell.

Methods of assaying for improved antibody expression are known in the art. In certain embodiments, an improvement in antibody expression is tested according to the methods referenced in Section 7.1 above.

### 7.6. Increased Adaptive Immune Response of Anti-HER2 Antibodies

As described in Section 4, anti-HER2 antibody therapy has been shown to induce tumor regression of HER-2 expressing tumors. The mechanisms of tumor regression by anti-HER2 therapy include induction of the adaptive immune response, *i.e.*, an increase in the innate immune response against HER-2 tumor cells. See Park et al., 2010, Cancer Cell 18:160-170.

In certain aspects, the present disclosure provides anti-HER2 antibodies that result in a greater adaptive immune response than trastuzumab. Accordingly, the present disclosure provides anti-HER2 antibodies having single or multiple amino acid substitutions in their CDRs and/or framework regions as compared to the CDRs and/or framework regions of trastuzumab, wherein at least one substitution results in an increased adaptive immune response induced by the antibody as compared to trastuzumab.

In certain embodiments, anti-HER2 antibodies of the disclosure induce an adaptive immune response that is at least 10% greater, at least 20% greater, at least 30% greater, at least 40% greater, at least 50% greater, at least 75% greater, at least 100% greater, at least 125% greater, at least 150% greater or at least 200% greater than the adaptive immune response induced by trastuzumab. In certain embodiments, the adaptive immune response induced by an anti-HER2 antibody of the disclosure is greater than the adaptive immune response induced by trastuzumab in an amount ranging between any of the foregoing values, *e.g.*, 20%-75% greater, 50%-100% greater, 30%-125% greater, or the like.

### 7.7. Increased Stability of Anti-HER2 Antibodies

In certain aspects, the present disclosure provides anti-HER2 antibodies having increased protein stability as compared to trastuzumab. The present disclosure provides anti-HER2 antibodies having single or multiple amino acid substitutions in their CDRs and/or framework regions as compared to the CDRs and/or framework regions of trastuzumab, wherein at least one substitution results in increased stability of the antibody as compared to trastuzumab. In certain embodiments, the increased stability is upon storage of the antibody. In other embodiments, the increased stability is due to resistance to degradation by proteases and the like.

In certain embodiments, the stability of an anti-HER2 antibody of the disclosure can be at least 10% greater, at least 20% greater, at least 30% greater, at least 40% greater, at least 50% greater, at least 75% greater, at least 100% greater, at least 125% greater, at least 150% greater or at least 200% greater than the stability of trastuzumab. In certain embodiments, the stability of an anti-HER2 antibody of the disclosure is increased relative to the stability of trastuzumab in a range between any of the foregoing values, e.g., 20%-75% greater, 50%-100% greater, 30%-125% greater, or the like. In certain aspects, the stability of an anti-HER2 antibody of the disclosure in a sample is greater than the stability of trastuzumab in a sample where the samples are substantially identical (*e.g.*, are both in pharmaceutical compositions containing the same ingredients) and/or are processed or handled in substantially the same way (*e.g.*, are stored under the same conditions of temperature and humidity for the same period of time).

In certain aspects, the increased stability of an anti-HER2 antibody of the disclosure is an increase in shelf-life. An increased shelf life can result from a decrease in amino acid oxidation (for example because an amino acid residue susceptible to oxidation, such as methionine, cysteine or tryptophan, in a CDR or FR has been substituted relative to trastuzumab). An increased shelf life can also result from a decrease in amino acid cyclization (for example because an amino acid residue susceptible to cyclization, such as asparagines or glutamic acid) in a CDR or FR has been substituted relative to trastuzumab. An increased shelf life can also result from the elimination of an amino acid motif that is recognized by proteases that may be in antibody purifications at residual levels. The increase in shelf life or decrease in oxidation, cyclization or proteolysis levels relative to trastuzumab can be at least 10%, at least 20%, at least 30%, at least 40%, at least 50%, at least 75%, at least 100%, at least 125%, at least 150% or at least 200%. In certain embodiments, increase in shelf life or decrease in oxidation, cyclization or proteolysis levels relative to trastuzumab is in a range between any of the foregoing values, e.g., 20%-75% greater, 50%-100% greater, 30%-125% greater, or the like.

The present disclosure further provides methods for screening anti-HER2 antibodies for increased stability relative to trastuzumab. In specific embodiments, the methods comprise the steps of: (a) determining the amount of full-length anti-HER2 antibody or anti-HER2 binding fragment in a first sample; and (b) comparing the amount of full-length anti-HER2 antibody or anti-HER2 binding fragment in the first sample with an amount of full-length trastuzumab in a second sample.

Methods of assaying for antibody stability are known in the art. In certain embodiments, an increase in antibody stability is tested according to the methods referenced in Section 7.1 above.

### 7.8. Reduced Immunogenicity of Anti-HER2 Antibodies

In certain aspects, the present disclosure provides anti-HER2 antibodies having reduced immunogenicity as compared to trastuzumab. The present disclosure provides anti-HER2 antibodies having single or multiple amino acid substitutions in their CDRs and/or framework regions as compared to the CDRs and/or framework regions of trastuzumab, wherein at least one substitution reduces the immunogenicity of the antibody as compared to trastuzumab. In certain embodiments, the reduced immunogenicity results from one or more amino acid substitutions that result in eliminating or mitigating one or more T cell epitopes.

In certain aspects, the anti-HER2 antibodies of the disclosure having reduced immunogenicity have comparable or improved biological activity as compared to trastuzumab, *e.g.*, affinity towards HER2 or neutralization of HER2 activity. Such properties can be tested, for example, by the methods described in Section 7.3 above.

In certain embodiments, the immunogenicity of an anti-HER2 antibody of the disclosure is reduced relative to trastuzumab. Such antibodies generally have variant sequences relative to the heavy and/or light chain variable region in regions corresponding to SEQ ID NOS:16, 27, 31, 49 and/or 69. The antibodies will generally have one, two or three amino acid substitutions in one or more of the sequences corresponding to SEQ ID NOS:16, 27, 31, 49 and/or 69, although up to four or five substitutions in one or more of the regions are contemplated herein.

As used in the present disclosure, a variant with "reduced immunogenicity" refers to an anti-HER2 antibody with a variant sequence in a region corresponding to SEQ ID NOS:16, 27, 31, 49 or 69, and wherein a peptide having the variant sequence as compared to SEQ ID NOS:16, 27, 31, 49 or 69 elicits a reduced proliferative response in peripheral blood mononuclear cells as compared to a peptide of SEQ ID NOS:16, 27, 31, 49 or 69, respectively. An exemplary proliferation assay that can be used to evaluate the proliferative response is set forth in Section 9 below. The reduced proliferative response can be reflected in terms of the percentage of responders, the stimulation index, or both.

In other embodiments, as compared to a peptide having the sequence of SEQ ID NOS:16, 27, 31, 49 or 69, the variant sequence results in at least 25% fewer responders, in at least 30% fewer responders, in at least 35% fewer responders, in at least 40% fewer responders, in at least 45% fewer responders, in at least 50% fewer responders, in at least 60% fewer responders, in at least 65% fewer responders, in at least 70% fewer responders, in at least 75% fewer responders, in at least 80% fewer responders, in at least 85% fewer responders, in at least 90% fewer responders, in at least 95% fewer responders, in at least 100% fewer responders, or a reduction in responders in a range between any of the foregoing values, e.g., 25%-75% fewer responders, 50%-90% fewer responders, 60%-100% fewer responders, 70%-90% fewer responders, or the like.

In other embodiments, the variant sequence results in a stimulation index that is at least 5% less, at least 10% less, at least 15% less, at least 20% less, at least 25% less, at least 30% less, at least 35% less, or at least 40% less than the stimulation index elicited by a peptide of SEQ ID NOS:16, 27, 31, 49 or 69, respectively, or results in a stimulation reduced by a range between any of the foregoing values as compared to a peptide of SEQ ID NOS:16, 27, 31, 49 or 69, *e.g.,* 5%-20% less, 10%-30% less, 30%-40% less, or the like.

Exemplary embodiments of anti-HER2 antibodies with reduced immunogenicity as compared to trastuzumab comprise one or more of the CDR and/or framework substitutions or combination of substitutions set forth in Tables 4, 6, 8, 9, 10, 11, 12, 13 and 14. Optionally, anti-HER2 antibodies with reduced immunogenicity as compared to trastuzumab comprise one or more additional substitutions, such as the CDR mutations in any of Tables 3, 5 and 7, singly or in combination.

### 7.9. Antibody Conjugates

The anti-HER2 antibodies of the disclosure include antibody conjugates that are modified, *e.g.*, by the covalent attachment of any type of molecule to the antibody, such that covalent attachment does not interfere with binding to HER2.

In certain aspects, an anti-HER2 antibody of the disclosure can be conjugated to an effector moiety or a label. The term "effector moiety" as used herein includes, for example, antineoplastic agents, drugs, toxins, biologically active proteins, for example enzymes, other antibody or antibody fragments, synthetic or naturally occurring polymers, nucleic acids (*e.g.*, DNA and RNA), radionuclides, particularly radioiodide, radioisotopes, chelated metals, nanoparticles and reporter groups such as fluorescent compounds or compounds which can be detected by NMR or ESR spectroscopy.

In one example, anti-HER2 antibodies can be conjugated to an effector moiety, such as a cytotoxic agent, a radionuclide or drug moiety to modify a given biological response. The effector moiety can be a protein or polypeptide, such as, for example and without limitation, a toxin (such as abrin, ricin A, Pseudomonas exotoxin, or Diphtheria toxin), a signaling molecule (such as α-interferon, β-interferon, nerve growth factor, platelet derived growth factor or tissue plasminogen activator), a thrombotic agent or an anti-angiogenic agent (*e.g.*, angiostatin or endostatin) or a biological response modifier such as a cytokine or growth factor (*e.g.*, interleukin-1 (IL-I), interleukin-2 (IL- 2), interleukin-6 (IL-6), granulocyte macrophage colony stimulating factor (GM-CSF), granulocyte colony stimulating factor (G-CSF), or nerve growth factor (NGF)).

In another example the effector moieties can be cytotoxins or cytotoxic agents. Examples of cytotoxins and cytotoxic agents include taxol, cytochalasin B, gramicidin D, ethidium bromide, emetine, mitomycin, etoposide, tenoposide, vincristine, vinblastine, colchicin, doxorubicin, daunorabicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof.

Effector moieties also include, but are not limited to, antimetabolites (*e.g.* methotrexate, 6-mercaptopurine, 6-thioguanine, cytarabine, 5-fluorouracil decarbazine), alkylating agents (*e.g.,* mechlorethamine, thioepa chlorambucil, melphalan, carmustine (BSNU) and lomustine (CCNU), cyclothosphamide, busulfan, dibromomannitol, streptozotocin, mitomycin C5 and cisdichlorodiamine platinum (II) (DDP) cisplatin), anthracyclines (*e.g.*, daunorubicin (formerly daunomycin) and doxorubicin), antibiotics (e.g., dactinomycin (formerly actinomycin), bleomycin, mithramycin, anthramycin (AMC), calicheamicins or duocarmycins), and anti-mitotic agents (*e.g.*, vincristine and vinblastine).

Other effector moieties can include radionuclides such as, but not limited to, ¹¹¹In and ⁹⁰Y, Lu¹⁷⁷, Bismuth²¹³, Californiu²⁵², Iridium¹⁹² and Tungsten^{18s} /Rhenium¹⁸⁸ and drugs such as, but not limited to, alkylphosphocholines, topoisomerase I inhibitors, taxoids and suramin.

Techniques for conjugating such effector moieties to antibodies are well known in the art *(see, e.g.,* Hellstrom et al, Controlled Drug Delivery, 2nd Ed., at pp. 623-53 (Robinson et al, eds., 1987)); Thorpe et al, 1982, Immunol. Rev. 62: 1 19-58 and Dubowchik et al, 1999, Pharmacology and Therapeutics 83:67- 123).

In one example, the anti-HER2 antibody or fragment thereof is fused via a covalent bond *(e.g.,* a peptide bond), through the antibody's N-terminus or C-terminus or internally, to an amino acid sequence of another protein (or portion thereof; for example at least a 10, 20 or 50 amino acid portion of the protein). The antibody, or fragment thereof, can linked to the other protein at the N-terminus of the constant domain of the antibody. Recombinant DNA procedures can be used to create such fusions, for example as described in WO 86/01533 and EP0392745. In another example the effector molecule can increase half-life *in vivo,* and/or enhance the delivery of an antibody across an epithelial barrier to the immune system. Examples of suitable effector molecules of this type include polymers, albumin, albumin binding proteins or albumin binding compounds such as those described in WO 2005/117984.

In certain aspects, an anti-HER2 antibody is conjugated to a small molecule toxin. In certain exemplary embodiments, an anti-HER2 antibody of the disclosure is conjugated to a dolastatin or a dolostatin peptidic analogs or derivatives, *e.g.*, an auristatin (U.S. Pat. Nos. 5,635,483 and 5,780,588). The dolastatin or auristatin drug moiety may be attached to the antibody through its N (amino) terminus, C (carboxyl) terminus or internally (WO 02/088172). Exemplary auristatin embodiments include the N- terminus linked monomethylauristatin drug moieties DE and DF, as disclosed in U.S. Patent No. 7,498,298 (disclosing, *e.g.*, linkers and methods of preparing monomethylvaline compounds such as MMAE and MMAF conjugated to linkers).

In other exemplary embodiments, small molecule toxins include but are not limited to calicheamicin, maytansine (U.S. Pat. No. 5,208,020), trichothene, and CC1065. In one embodiment of the disclosure, the antibody is conjugated to one or more maytansine molecules (*e.g.*, about 1 to about 10 maytansine molecules per antibody molecule). Maytansine may, for example, be converted to May-SS-Me which may be reduced to May-SH3 and reacted with an antibody (Chari et al., 1992, Cancer Research 52: 127-131) to generate a maytansinoid-antibody or maytansinoid-Fc fusion conjugate. Structural analogues of calicheamicin that can also be used include but are not limited to γ₁¹, γ₃¹, γ₃¹, N-acetyl- γ₁¹, PSAG, and θ₁¹, (Hinman et al., 1993, Cancer Research 53:3336-3342; Lode et al., 1998, Cancer Research 58:2925-2928; U.S. Patent No. 5,714,586; U.S. Patent No. 5,712,374; U.S. Patent No. 5,264,586; U.S. Patent No. 5,773,001).

Antibodies of the disclosure can also be conjugated to liposomes for targeted delivery *(See, e.g.,* Park et al., 1997, Adv. Pharmacol. 40:399-435; Marty & Schwendener, 2004, Methods in Molecular Medicine 109:389-401).

In one example antibodies of the present disclosure can be attached to poly(ethyleneglycol) (PEG) moieties. In one particular example the antibody is an antibody fragment and the PEG moieties can be attached through any available amino acid side-chain or terminal amino acid functional group located in the antibody fragment, for example any free amino, imino, thiol, hydroxyl or carboxyl group. Such amino acids can occur naturally in the antibody fragment or can be engineered into the fragment using recombinant DNA methods. *See* for example U.S. Patent No. 5,219,996. Multiple sites can be used to attach two or more PEG molecules. PEG moieties can be covalently linked through a thiol group of at least one cysteine residue located in the antibody fragment. Where a thiol group is used as the point of attachment, appropriately activated effector moieties, for example thiol selective derivatives such as maleimides and cysteine derivatives, can be used.

In a specific example, an anti-HER2 antibody conjugate is a modified Fab' fragment which is PEGylated, *i.e.*, has PEG (poly(ethyleneglycol)) covalently attached thereto, *e.g.*, according to the method disclosed in EP0948544. *See also* Poly(ethyleneglycol) Chemistry, Biotechnical and Biomedical Applications, (J. Milton Harris (ed.), Plenum Press, New York, 1992); Poly(ethyleneglycol) Chemistry and Biological Applications, (J. Milton Harris and S. Zalipsky, eds., American Chemical Society, Washington D.C., 1997); and Bioconjugation Protein Coupling Techniques for the Biomedical Sciences, (M. Aslam and A. Dent, eds., Grove Publishers, New York, 1998); and Chapman, 2002, Advanced Drug Delivery Reviews 54:531-545. PEG can be attached to a cysteine in the hinge region. In one example, a PEG-modified Fab' fragment has a maleimide group covalently linked to a single thiol group in a modified hinge region. A lysine residue can be covalently linked to the maleimide group and to each of the amine groups on the lysine residue can be attached a methoxypoly(ethyleneglycol) polymer having a molecular weight of approximately 20,000 Da. The total molecular weight of the PEG attached to the Fab' fragment can therefore be approximately 40,000 Da.

The word "label" when used herein refers to a detectable compound or composition which can be conjugated directly or indirectly to an anti-HER2 antibody of the disclosure. The label can itself be detectable (e.g., radioisotope labels or fluorescent labels) or, in the case of an enzymatic label, can catalyze chemical alteration of a substrate compound or composition which is detectable. Useful fluorescent moieties include, but are not limited to, fluorescein, fluorescein isothiocyanate, rhodamine, 5- dimethylamine-1- napthalenesulfonyl chloride, phycoerythrin and the like. Useful enzymatic labels include, but are not limited to, alkaline phosphatase, horseradish peroxidase, glucose oxidase and the like.

Additional anti-HER2 antibody conjugates that are useful for, inter alia, diagnostic purposes, are described in Section 7.10 below.

### 7.10. Diagnostic Uses of Anti-HER2 Antibodies

The anti-HER2 antibodies of the disclosure, including those antibodies that have been modified, *e.g.*, by biotinylation, horseradish peroxidase, or any other detectable moiety (including those described in Section 7.9), can be advantageously used for diagnostic purposes.

In particular, the anti-HER2 antibodies can be used, for example, but not limited to, to purify or detect HER2, including both *in vitro* and *in vivo* diagnostic methods. For example, the antibodies have use in immunoassays for qualitatively and quantitatively measuring levels of HER2 in biological samples. *See, e.g.,* Harlow et al.,Antibodies: A Laboratory Manual, Second Edition (Cold Spring Harbor Laboratory Press, 1988). In a specific embodiment, the anti-HER2 antibodies can be used for detecting and quantitating levels of HER2 in the serum, *i.e.,* levels of HER2 extracellular domain that has been shed from the surface of cells.

The present disclosure further encompasses antibodies or fragments thereof conjugated to a diagnostic agent. The antibodies can be used diagnostically, for example, to detect expression of a target of interest in specific cells, tissues, or serum; or to monitor the development or progression of an immunologic response as part of a clinical testing procedure to, *e.g.*, determine the efficacy of a given treatment regimen. Detection can be facilitated by coupling the antibody to a detectable substance. Examples of detectable substances include various enzymes, prosthetic groups, fluorescent materials, luminescent materials, bioluminescent materials, radioactive materials, positron emitting metals using various positron emission tomographies, and nonradioactive paramagnetic metal ions. The detectable substance can be coupled or conjugated either directly to the antibody (or fragment thereof) or indirectly, through an intermediate (such as, for example, a linker known in the art) using techniques known in the art. Examples of enzymatic labels include luciferases (*e.g.*, firefly luciferase and bacterial luciferase; U.S. Patent No. 4,737,456), luciferin, 2,3- dihydrophthalazinediones, malate dehydrogenase, urease, peroxidase such as horseradish peroxidase (HRPO), alkaline phosphatase, β- galactosidase, acetylcholinesterase, glucoamylase, lysozyme, saccharide oxidases (e.g., glucose oxidase, galactose oxidase, and glucose-6-phosphate dehydrogenase), heterocyclic oxidases (such as uricase and xanthine oxidase), lactoperoxidase, microperoxidase, and the like. Examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin; and examples of suitable radioactive material include ¹²⁵I, ¹³¹I, ¹¹¹In or ⁹⁹Tc.

The disclosure provides for the detection of expression of HER2 comprising contacting a biological sample (cells, tissue, or body fluid of an individual) using one or more anti-HER2 antibodies of the disclosure (optionally conjugated to detectable moiety), and detecting whether or not the sample is positive for HER2 expression, or whether the sample has altered (e.g., reduced or increased) expression as compared to a control sample.

The overexpression of HER2 can be determined by routine clinical laboratory testing, usually immunohistochemistry (IHC) and silver, chromogenic or fluorescent *in situ* hybridization (SISH/CISH/FISH). FISH testing is considered the gold-standard technique for identifying patients who would benefit from Herceptin® treatment but its use is limited by its cost. Other techniques include virtual karyotyping of formalin-fixed paraffin embedded tumors.

Diseases that can be diagnosed using the present methods include, but are not limited to, the diseases described herein. In certain embodiments, the tissue or body fluid is peripheral blood, peripheral blood leukocytes, biopsy tissues such as breast or lymph node biopsies, and tissue.

### 7.11. Therapeutic Methods Using Anti-HER2 Antibodies

### 7.11.1. Clinical Benefits

The anti-HER2 antibodies of the disclosure can be used to treat various neoplasms.

The antibodies of the disclosure are useful in the treatment of tumors, including cancers and benign tumors. Examples of cancer to be treated herein include, but are not limited to, carcinoma, lymphoma, blastoma, sarcoma, and leukemia. More particular examples of such cancers include squamous cell cancer, lung cancer (including small-cell lung cancer, non-small cell lung cancer, adenocarcinoma of the lung, and squamous carcinoma of the lung), cancer of the peritoneum, hepatocellular cancer, gastric or stomach cancer (including gastrointestinal cancer), pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney or renal cancer, liver cancer, prostate cancer, vulval cancer, thyroid cancer, hepatic carcinoma and various types of head and neck cancer, as well as B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; and post-transplant lymphoproliferative disorder (PTLD), as well as abnormal vascular proliferation associated with phakomatoses, edema (such as that associated with brain tumors), and Meigs' syndrome.

More particularly, cancers that are amenable to treatment by the antibodies of the disclosure include those that overexpress HER2. In certain embodiments, cancers that are amenable to treatment by the antibodies disclosed herein include breast cancer, ovarian cancer, gastric cancer, colon cancer, non-small cell lung cancer, oral cancer, cervical cancer, osteosarcoma, pancreatic cancer, salivary gland cancer, prostate cancer, endometrial cancer, and bladder cancer. In some embodiments, the anti-HER2 antibodies of the disclosure are used to treat breast cancer in a human patient. In other embodiments, the anti-HER2 antibodies of the disclosure are used to treat mammary and extra-mammary Paget's disease.

In certain embodiments, the present disclosure encompasses anti-angiogenic therapy, a cancer treatment strategy aimed at inhibiting the development of tumor blood vessels required for providing nutrients to support tumor growth. Because angiogenesis is involved in both primary tumor growth and metastasis, the antiangiogenic treatment provided by the disclosure is capable of inhibiting the neoplastic growth of tumor at the primary site as well as preventing metastasis of tumors at the secondary sites.

Accordingly, the present disclosure provides methods of treating any of the foregoing diseases in a patient in need thereof, comprising: administering to the patient an anti-HER2 antibody of the disclosure. Optionally, said administration is repeated, e.g., after one day, two days, three days, five days, one week, two weeks, three weeks, one month, five weeks, six weeks, seven weeks, eight weeks, two months or three months. The repeated administration can be at the same dose or at a different dose. The administration can be repeated once, twice, three times, four times, five times, six times, seven times, eight times, nine times, ten times, or more. For example, according to certain dosage regimens a patient receives anti-HER2 therapy for a prolonged period of time, *e.g.,* 6 months, 1 year or more. The amount of anti-HER2 antibody administered to the patient is in certain embodiments a therapeutically effective amount. As used herein, a "therapeutically effective" amount of HER2 antibody can be administered as a single dose or over the course of a therapeutic regimen, e.g., over the course of a week, two weeks, three weeks, one month, three months, six months, one year, or longer. Exemplary therapeutic regimens are described in Section 7.14 below.

In certain aspects, the methods include before the step of administering to the patient an anti-HER2 antibody of the disclosure, a step of detecting HER2 overexpression. Said detecting step can be accomplished by any method known in the art, including, but not limited to, an immunohistochemistry assay to measure HER2 protein levels (such as HercepTest™ or Pathway®), and a fluorescence *in situ* hybridization (FISH) assay to measure HER2 gene amplification (such as PathVysion® and pharmDx™). In certain embodiments, immunohistochemistry assays can be performed using a labeled antibody of the disclosure, such as those described in Section 7.9, above.

According to the present disclosure, treatment of a disease encompasses the treatment of patients already diagnosed as having any form of the disease at any clinical stage or manifestation; the delay of the onset or evolution or aggravation or deterioration of the symptoms or signs of the disease; and/or preventing and/or reducing the severity of the disease.

A "subject" or "patient" to whom the anti-HER2 antibody of the disclosure is administered is preferably a mammal such as a non-primate (e.g., cow, pig, horse, cat, dog, rat, etc.) or a primate (e.g., monkey or human). In certain embodiments, the subject or patient is a human. In certain aspects, the human is an adult patient. In other aspects, the human is a pediatric patient.

### 7.12. Pharmaceutical Compositions and Routes of Administration

Compositions comprising an anti-HER2 antibody of the disclosure and, optionally one or more additional therapeutic agents, such as the combination therapeutic agents described in Section 7.13 below, are provided herein. The compositions will usually be supplied as part of a sterile, pharmaceutical composition that will normally include a pharmaceutically acceptable carrier. This composition can be in any suitable form (depending upon the desired method of administering it to a patient).

The anti-HER2 antibodies of the disclosure can be administered to a patient by a variety of routes such as orally, transdermally, subcutaneously, intranasally, intravenously, intramuscularly, intraocularly, topically, intrathecally and intracerebroventricularly. The most suitable route for administration in any given case will depend on the particular antibody, the subject, and the nature and severity of the disease and the physical condition of the subject.

For treatment of indications described herein, the effective dose of an anti-HER2 antibody of the disclosure can range from about 0.1 to about 75 mg/kg per single (e.g., bolus) administration, multiple administrations or continuous administration, or to achieve a serum concentration of 0.01-5000 µg/ml serum concentration per single (e.g., bolus) administration, multiple administrations or continuous administration, or any effective range or value therein depending on the condition being treated, the route of administration and the age, weight and condition of the subject. In certain embodiments, e.g. for the treatment of cancer, each dose can range from about 0.2 mg to about 50 mg per kilogram of body weight, for example from about 0.5 mg to about 20 mg per kilogram body weight. The antibody can be formulated as an aqueous solution and administered by subcutaneous injection.

Pharmaceutical compositions can be conveniently presented in unit dose forms containing a predetermined amount of an anti-HER2 antibody of the disclosure per dose. Such a unit can contain for example but without limitation 0.1 mg to 5 g, for example 1 mg to 1 g, or 10 to 50 mg. Pharmaceutically acceptable carriers for use in the disclosure can take a wide variety of forms depending, e.g., on the condition to be treated or route of administration.

Therapeutic formulations of the anti-HER2 antibodies of the disclosure can be prepared for storage as lyophilized formulations or aqueous solutions by mixing the antibody having the desired degree of purity with optional pharmaceutically-acceptable carriers, excipients or stabilizers typically employed in the art (all of which are referred to herein as "carriers"), *i.e.,* buffering agents, stabilizing agents, preservatives, isotonifiers, non-ionic detergents, antioxidants, and other miscellaneous additives. *See,* Remington's Pharmaceutical Sciences, 16th edition (Osol, ed. 1980). Such additives must be nontoxic to the recipients at the dosages and concentrations employed.

Buffering agents help to maintain the pH in the range which approximates physiological conditions. They can be present at concentration ranging from about 2 mM to about 50 mM. Suitable buffering agents for use with the present disclosure include both organic and inorganic acids and salts thereof such as citrate buffers (*e.g.*, monosodium citrate-disodium citrate mixture, citric acid-trisodium citrate mixture, citric acid-monosodium citrate mixture, etc.), succinate buffers (*e.g.*, succinic acid monosodium succinate mixture, succinic acid-sodium hydroxide mixture, succinic acid disodium succinate mixture, etc.), tartrate buffers (*e.g.*, tartaric acid-sodium tartrate mixture, tartaric acid-potassium tartrate mixture, tartaric acid-sodium hydroxide mixture, etc.), fumarate buffers (e.g., fumaric acid-monosodium fumarate mixture, fumaric acid disodium fumarate mixture, monosodium fumarate-disodium fumarate mixture, etc.), gluconate buffers (*e.g.*, gluconic acid-sodium glyconate mixture, gluconic acid-sodium hydroxide mixture, gluconic acid-potassium glyuconate mixture, etc.), oxalate buffer (*e.g.*, oxalic acid-sodium oxalate mixture, oxalic acid-sodium hydroxide mixture, oxalic acid-potassium oxalate mixture, etc.), lactate buffers (*e.g.*, lactic acid-sodium lactate mixture, lactic acid-sodium hydroxide mixture, lactic acid-potassium lactate mixture, etc.) and acetate buffers (*e.g.*, acetic acid-sodium acetate mixture, acetic acid-sodium hydroxide mixture, etc.). Additionally, phosphate buffers, histidine buffers and trimethylamine salts such as Tris can be used.

Preservatives can be added to retard microbial growth, and can be added in amounts ranging from 0.2%-1% (w/v). Suitable preservatives for use with the present disclosure include phenol, benzyl alcohol, meta-cresol, methyl paraben, propyl paraben, octadecyldimethylbenzyl ammonium chloride, benzalconium halides (*e.g.*, chloride, bromide, and iodide), hexamethonium chloride, and alkyl parabens such as methyl or propyl paraben, catechol, resorcinol, cyclohexanol, and 3-pentanol. Isotonicifiers sometimes known as "stabilizers" can be added to ensure isotonicity of liquid compositions of the present disclosure and include polhydric sugar alcohols, for example trihydric or higher sugar alcohols, such as glycerin, erythritol, arabitol, xylitol, sorbitol and mannitol. Stabilizers refer to a broad category of excipients which can range in function from a bulking agent to an additive which solubilizes the therapeutic agent or helps to prevent denaturation or adherence to the container wall. Typical stabilizers can be polyhydric sugar alcohols (enumerated above); amino acids such as arginine, lysine, glycine, glutamine, asparagine, histidine, alanine, ornithine, L-leucine, 2-phenylalanine, glutamic acid, threonine, etc., organic sugars or sugar alcohols, such as lactose, trehalose, stachyose, mannitol, sorbitol, xylitol, ribitol, myoinisitol, galactitol, glycerol and the like, including cyclitols such as inositol; polyethylene glycol; amino acid polymers; sulfur containing reducing agents, such as urea, glutathione, thioctic acid, sodium thioglycolate, thioglycerol, α-monothioglycerol and sodium thio sulfate; low molecular weight polypeptides (*e.g.*, peptides of 10 residues or fewer); proteins such as human serum albumin, bovine serum albumin, gelatin or immunoglobulins; hydrophylic polymers, such as polyvinylpyrrolidone monosaccharides, such as xylose, mannose, fructose, glucose; disaccharides such as lactose, maltose, sucrose and trisaccacharides such as raffinose; and polysaccharides such as dextran. Stabilizers can be present in the range from 0.1 to 10,000 weights per part of weight active protein.

Non-ionic surfactants or detergents (also known as "wetting agents") can be added to help solubilize the therapeutic agent as well as to protect the therapeutic protein against agitation-induced aggregation, which also permits the formulation to be exposed to shear surface stressed without causing denaturation of the protein. Suitable non-ionic surfactants include polysorbates (20, 80, etc.), polyoxamers (184, 188 etc.), Pluronic polyols, polyoxyethylene sorbitan monoethers (TWEEN®-20, TWEEN®-80, etc.). Nonionic surfactants can be present in a range of about 0.05 mg/ml to about 1.0 mg/ml, for example about 0.07 mg/ml to about 0.2 mg/ml.

Additional miscellaneous excipients include bulking agents *(e.g.,* starch), chelating agents (*e.g.,* EDTA), antioxidants (*e.g.*, ascorbic acid, methionine, vitamin E), and cosolvents.

The formulation herein can also contain a combination therapeutic agent in addition to the anti-HER2 antibody of the disclosure. Examples of suitable combination therapeutic agents are provided in Section 7.13 below.

The dosing schedule for subcutaneous administration can vary from once every six months to daily depending on a number of clinical factors, including the type of disease, severity of disease, and the patient's sensitivity to the anti-HER2 antibody.

The dosage of an anti-HER2 antibody of the disclosure to be administered will vary according to the particular antibody, the type of cancer, the subject, and the severity of the disease, the physical condition of the subject, the therapeutic regimen (*e.g.*, whether a combination therapeutic agent is used), and the selected route of administration; the appropriate dosage can be readily determined by a person skilled in the art.

It will be recognized by one of skill in the art that the optimal quantity and spacing of individual dosages of an anti-HER2 antibody of the disclosure will be determined by the nature and extent of the condition being treated, the form, route and site of administration, and the age and condition of the particular subject being treated, and that a physician will ultimately determine appropriate dosages to be used. This dosage can be repeated as often as appropriate. If side effects develop, the amount and/or frequency of the dosage can be altered or reduced, in accordance with normal clinical practice.

### 7.13. Combination Therapy

Described below are combinatorial methods in which the anti-HER2 antibodies of the disclosure can be utilized. The combinatorial methods of the disclosure involve the administration of at least two agents to a patient, the first of which is an anti- HER2 antibody of the disclosure, and the second of which is a combination therapeutic agent. The anti-HER2 antibody and the combination therapeutic agent can be administered simultaneously, sequentially or separately.

The combinatorial therapy methods of the present disclosure can result in a greater than additive effect, providing therapeutic benefits where neither the anti-HER2 antibody or combination therapeutic agent administered in an amount that is alone therapeutically effective.

In the present methods, the anti-HER2 antibody of the disclosure and the combination therapeutic agent can be administered concurrently, either simultaneously or successively. As used herein, the anti-HER2 antibody of the disclosure and the combination therapeutic agent are said to be administered successively if they are administered to the patient on the same day, for example during the same patient visit. Successive administration can occur 1, 2, 3, 4, 5, 6, 7 or 8 hours apart. In contrast, the anti-HER2 antibody of the disclosure and the combination therapeutic agent are said to be administered separately if they are administered to the patient on the different days, for example, the anti-HER2 antibody of the disclosure and the combination therapeutic agent can be administered at a 1-day, 2-day or 3-day, one-week, 2-week or monthly intervals. In the methods of the present disclosure, administration of the anti-HER2 antibody of the disclosure can precede or follow administration of the combination therapeutic agent.

As a non-limiting example, the anti-HER2 antibody of the disclosure and combination therapeutic agent can be administered concurrently for a period of time, followed by a second period of time in which the administration of the anti-HER2 antibody of the disclosure and the combination therapeutic agent is alternated.

Because of the potentially synergistic effects of administering an anti-HER2 antibody of the disclosure and a combination therapeutic agent, such agents can be administered in amounts that, if one or both of the agents is administered alone, is/are not therapeutically effective.

In certain aspects, the combination therapeutic agent is a chemotherapeutic agent, an anti-angiogenic agent, an anti-inflammatory agent, a radiotherapeutic, an immunosuppressive agent, or a cytotoxic drug.

It is contemplated that when used to treat various diseases, the anti-HER2 antibodies of the disclosure can be combined with other therapeutic agents suitable for the same or similar diseases. When used for treating cancer, antibodies of the present disclosure may be used in combination with conventional cancer therapies, such as surgery, radiotherapy, chemotherapy or combinations thereof.

In some other aspects, other therapeutic agents useful for combination tumor therapy with the antibody of the disclosure include antagonists, *e.g.*, antibodies, of other factors that are involved in tumor growth, such as EGFR, HER3, HER4, VEGF, or α5β1 integrin.

Sometimes, for treatment of cancers it may be beneficial to also administer one or more cytokines to the patient. In a preferred embodiment, the HER2 antibody is co-administered with a growth inhibitory agent.

Suitable dosages for the growth inhibitory agent are those presently used and may be lowered due to the combined action (synergy) of the growth inhibitory agent and anti-HER2 antibody.

For treatment of cancers, anti-inflammatory agents can suitably be used in combination with the anti-HER2 antibodies of the disclosure. Anti-inflammatory agents include, but are not limited to, acetaminophen, diphenhydramine, meperidine, dexamethasone, pentasa, mesalazine, asacol, codeine phosphate, benorylate, fenbufen, naprosyn, diclofenac, etodolac and indomethacin, aspirin and ibuprofen.

For treatment of cancers, chemotherapeutic agents can suitably be used in combination with the anti-HER2 antibodies of the disclosure. Chemotherapeutic agents include, but are not limited to, radioactive molecules, toxins, also referred to as cytotoxins or cytotoxic agents, which includes any agent that is detrimental to the viability of cells, agents, and liposomes or other vesicles containing chemotherapeutic compounds. Examples of suitable chemotherapeutic agents include but are not limited to 1-dehydrotestosterone, 5-fluorouracil decarbazine, 6-mercaptopurine, 6-thioguanine, actinomycin D, adriamycin, aldesleukin, an anti-α5β1 integrin antibody, alkylating agents, allopurinol sodium, altretamine, amifostine, anastrozole, anthramycin (AMC)), anti-mitotic agents, cisdichlorodiamine platinum (II) (DDP) cisplatin), diamino dichloro platinum, anthracyclines, antibiotics, antimetabolites, asparaginase, BCG live (intravesical), betamethasone sodium phosphate and betamethasone acetate, bicalutamide, bleomycin sulfate, busulfan, calcium leucouorin, calicheamicin, capecitabine, carboplatin, lomustine (CCNU), carmustine (BSNU), Chlorambucil, Cisplatin, Cladribine, Colchicin, conjugated estrogens, Cyclophosphamide, Cyclothosphamide, Cytarabine, Cytarabine, cytochalasin B, Cytoxan, Dacarbazine, Dactinomycin, dactinomycin (formerly actinomycin), daunirubicin HCL, daunorucbicin citrate, denileukin diftitox, Dexrazoxane, Dibromomannitol, dihydroxy anthracin dione, Docetaxel, dolasetron mesylate, doxorubicin HCL, dronabinol, E. coli L-asparaginase, eolociximab, emetine, epoetin-α, Erwinia Lasparaginase, esterified estrogens, estradiol, estramustine phosphate sodium, ethidium bromide, ethinyl estradiol, etidronate, etoposide citrororum factor, etoposide phosphate, filgrastim, floxuridine, fluconazole, fludarabine phosphate, fluorouracil, flutamide, folinic acid, gemcitabine HCL, glucocorticoids, goserelin acetate, gramicidin D, granisetron HCL, hydroxyurea, idarubicin HCL, ifosfamide, interferon α-2b, irinotecan HCL, letrozole, leucovorin calcium, leuprolide acetate, levamisole HCL, lidocaine, lomustine, maytansinoid, mechlorethamine HCL, medroxyprogesterone acetate, megestrol acetate, melphalan HCL, mercaptipurine, mesna, methotrexate, methyltestosterone, mithramycin, mitomycin C, mitotane, mitoxantrone, nilutamide, octreotide acetate, ondansetron HCL, paclitaxel, pamidronate disodium, pentostatin, pilocarpine HCL, plimycin, polifeprosan 20 with carmustine implant, porfimer sodium, procaine, procarbazine HCL, propranolol, rituximab, sargramostim, streptozotocin, tamoxifen, taxol, teniposide, tenoposide, testolactone, tetracaine, thioepa chlorambucil, thioguanine, thiotepa, topotecan HCL, toremifene citrate, tretinoin, valrubicin, vinblastine sulfate, vincristine sulfate, and vinorelbine tartrate.

Any anti-angiogenic agent can be used in conjunction with the anti-HER2 antibodies of the disclosure, including those listed by Carmeliet and Jain, 2000, Nature 407:249-257. In certain embodiments, the anti-angiogenic agent is a VEGF antagonist or a VEGF receptor antagonist such as VEGF variants, soluble VEGF receptor fragments, aptamers capable of blocking VEGF or VEGFR, neutralizing anti-VEGFR antibodies, low molecule weight inhibitors of VEGFR tyrosine kinases and any combinations thereof. Alternatively, or in addition, an anti-VEGF antibody may be co-administered to the patient.

In certain embodiments, hormone therapy can be used in conjunction with anti-HER2 antibodies of the disclosure. In some embodiments, the hormone therapy includes one or more agents that inhibit estrogen and/or progesterone from promoting cancer cell growth, *e.g.*, a selective estrogen-receptor modulator such as tamoxifen, an aromatase inhibitor such as anastrozole (Arimidex®) or letrozole (Femara), an aromatase inactivator such as exemestane (Aromasin®), or an agent that inhibits estrogen production such as goserelin (Zoladex). In other embodiments, the hormone therapy is one or more agents that inhibit production of hormones from the ovaries.

In some aspects, an anti-HER2 antibody can be used in conjunction with a small molecule protein tyrosine kinase (PTK) inhibitor. In some embodiments, the PTK inhibitor is specific for the HER3 tyrosine kinase. In other embodiments, the PTK inhibitor binds to more than one of the HER family of tyrosine kinases (*e.g.*, EGFR, HER3 and/or HER4). In still other embodiments, the PTK inhibitors bind to and inhibit the tyrosine kinases of one or more proteins that interact with or are regulated by one or more HER family members, *e.g.*, proteins involved in one or more signaling cascades that originate with one or more HER family members. In other embodiments, protein tyrosine kinase inhibitors useful in the compositions and methods of the invention include PTK inhibitors that do not bind selectively to the HER family of receptor tyrosine kinases, but also bind to the tyrosine kinase domains of other families of proteins such as VEGFR, PDGFR, and/or Raf.

In some embodiments, the tyrosine kinase is a receptor tyrosine kinase, i.e., is an intra-cellular domain of a larger protein that has an extra-cellular ligand binding domain and is activated by the binding of one or more ligands. In certain embodiments, the protein tyrosine kinase is a non-receptor tyrosine kinase. PTK inhibitors for use in the methods of the present disclosure include, but are not limited to, gefitinib (ZD-1839, Iressa®), erlotinib (OSI-1774, Tarceva™), canertinib (CI-1033), vandetanib (ZD6474, Zactima®), tyrphostin AG-825 (CAS 149092-50-2), lapatinib (GW-572016), sorafenib (BAY43-9006), AG-494 (CAS 133550-35-3), RG-13022 (CAS 149286-90-8), RG-14620 (CAS 136831-49-7), BIBW 2992 (Tovok), tyrphostin 9 (CAS 136831-49-7), tyrphostin 23 (CAS 118409-57-7), tyrphostin 25 (CAS 118409-58-8), tyrphostin 46 (CAS 122520-85-8), tyrphostin 47 (CAS 122520-86-9), tyrphostin 53 (CAS 122520-90-5), butein (1-(2,4-dihydroxyphenyl)-3-(3,4-dihydroxyphenyl)-2-propen-1-one 2',3,4,4'-Tetrahydroxychalcone; CAS 487-52-5), curcumin ((E,E)-1,7-bis(4-Hydroxy-3-methoxyphenyl)-1,6-heptadiene-3,5-dione; CAS 458-37-7), N4-(1-Benzyl-1H-indazol-5-yl)-N6,N6-dimethyl-pyrido-[3,4-d]-pyrimidine-4,6-diamine (202272-68-2), AG-1478, AG-879, Cyclopropanecarboxylic acid-(3-(6-(3-trifluoromethyl-phenylamino)-pyrimidin-4-ylamino)-phenyl)-amide (CAS 879127-07-8), N8-(3-Chloro-4-fluorophenyl)-N2-(1-methylpiperidin-4-yl)-pyrimido[5,4-d]pyrimidine-2,8-diamine, 2HCl (CAS 196612-93-8), 4-(4-Benzyloxyanilino)-6,7-dimethoxyquinazoline (CAS 179248-61-4), N-(4-((3-Chloro-4-fluorophenyl)amino)pyrido[3,4-d]pyrimidin-6-yl)2-butynamide (CAS 881001-19-0), EKB-569, HKI-272, and HKI-357.

In a specific embodiment, an anti-HER2 antibody of the disclosure is used as part of a treatment regimen consisting of doxorubicin, cyclophosphamide and either paclitaxel or docetaxel. This combination is suitable for, *inter alia,* adjuvant treatment of patients with HER2-overexpressing node positive or node negative breast cancer.

In another specific embodiment, an anti-HER2 antibody of the disclosure is used in combination with docetaxel and carboplatin. This combination is suitable for, *inter alia,* adjuvant treatment of patients with HER2-overexpressing node positive or node negative breast cancer.

In yet another specific embodiment, an anti-HER2 antibody of the disclosure is used as a single agent following multi-modality anthracycline based therapy. This combination is suitable for, *inter alia,* adjuvant treatment of patients with HER2-overexpressing node positive or node negative breast cancer.

In another specific embodiment, the anti-HER2 antibody of the disclosure is used in combination with paclitaxel. This combination is suitable for, *inter alia,* first-line treatment of HER2-overexpressing metastatic breast cancer.

In another specific embodiment, an anti-HER2 antibody of the disclosure is used as a single agent for treatment of HER2-overexpressing breast cancer in patients who have received one or more chemotherapy regimens for metastatic disease.

### 7.14. Therapeutic Regimens

The present disclosure provides therapeutic regimens involving the administration of the anti-HER2 antibodies of the disclosure. The therapeutic regimen will vary depending on the patient's age, weight, and disease condition. The therapeutic regimen can continue for 2 weeks to indefinitely. In specific embodiments, the therapeutic regimen is continued for 2 weeks to 6 months, from 3 months to 5 years, from 6 months to 1 or 2 years, from 8 months to 18 months, or the like. The therapeutic regimen can be a non-variable dose regimen or a multiple-variable dose regimen.

For the dosage exemplary regimens described below, the anti-HER2 antibody can be administered as a sterile, preservative-free solution for subcutaneous administration.

For adjuvant breast cancer treatment during and/or following treatment with paclitaxel, docetaxel or with a combination of docetaxel and carboplatin, an anti-HER2 antibody of the disclosure is administered intravenously at an initial dose of 0.1 to 10 mg/kg. In specific embodiments, the initial dose is 0.2-8 mg/kg, 0.5-8 mg/kg, 1-6 mg/kg, 1.5-5 mg/kg, 2-5 mg/kg, or 1-4.5 mg/kg. Following the initial dose, an anti-HER2 antibody of the disclosure is administered intravenously for the first 12 weeks of chemotherapy (treatment with paclitaxel or docetaxel) or for the first 18 weeks of chemotherapy (treatment with a combination of docetaxel and carboplatin) at a dose of 0.05 to 10 mg/kg, such as 0.1-10 mg/kg, such as 0.3-8 mg/kg, such as 0.5-6 mg/kg, such as 0.8-6.5 mg/kg, such as 1-5 mg/kg, such as 1.5-3.5 mg/kg. One week after the last weekly dose of an anti-HER2 antibody of the present disclosure, an anti-HER2 antibody of the present disclosure is administered intravenously every three weeks at a dose of 0.1-12 mg/kg. In specific embodiments, the dose is 0.1-10 mg/kg, 0.5-8 mg/kg, 1-6.5 mg/kg, 1.5-6 mg/kg, 2-6 mg/kg, 0.1-5.5 mg/kg, or 1-4 mg/kg.

For adjuvant breast cancer treatment within three weeks following completion of a multi-modality, anthracycline-base chemotherapy regimen, an anti-HER2 antibody of the disclosure is administered intravenously as a single agent at an initial dose of 0.1 to 15 mg/kg. In specific embodiments, the initial dose is 0.2-12.5 mg/kg, 0.5-12 mg/kg, 1-10.5 mg/kg, 2-10 mg/kg, 3-9 mg/kg, or 4-8.5 mg/kg. Subsequent doses of an anti-HER2 antibody of the present disclosure are administered intravenously every three weeks and are from 0.1-12 mg/kg. In specific embodiments, each subsequent dose is 0.1-10 mg/kg, 0.5-8 mg/kg, 1-6.5 mg/kg, 1.5-6 mg/kg, 2-6 mg/kg, 0.1-5.5 mg/kg, or 1-4 mg/kg.

For treatment of metastatic breast cancer treatment, an anti-HER2 antibody of the disclosure is administered intravenously either alone or in combination with paclitaxel at an initial dose of 0.1 to 10 mg/kg. In specific embodiments, the initial dose is 0.2-8 mg/kg, 0.5-8 mg/kg, 1-6 mg/kg, 1.5-5 mg/kg, 2-5 mg/kg, or 1-4.5 mg/kg. Following the initial dose, an anti-HER2 antibody of the disclosure is administered intravenously once weekly at a dose of 0.05 to 10 mg/kg, such as 0.1-10 mg/kg, such as 0.3-8 mg/kg, such as 0.5-6 mg/kg, such as 0.8-6.5 mg/kg, such as 1-5 mg/kg, such as 1.5-3.5 mg/kg.

### 7.15. Diagnostic and Pharmaceutical Kits

Encompassed by the present disclosure are pharmaceutical kits containing the anti-HER2 antibodies (including antibody conjugates) of the disclosure. The pharmaceutical kit is a package comprising the anti-HER2 antibody of the disclosure (e.g., either in lyophilized form or as an aqueous solution) and one or more of the following:
- A combination therapeutic agent, for example as described in Section 7.13 above;
- A device for administering the anti-HER2 antibody, for example a pen, needle and/or syringe; and
- Pharmaceutical grade water or buffer to resuspend the antibody if the antibody is in lyophilized form.

In certain aspects, each unit dose of the anti-HER2 antibody is packaged separately, and a kit can contain one or more unit doses (*e.g.*, two unit doses, three unit doses, four unit doses, five unit doses, eight unit doses, ten unit doses, or more). In a specific embodiment, the one or more unit doses are each housed in a syringe or pen.

Diagnostic kits containing the anti-HER2 antibodies (including antibody conjugates) of the disclosure are also encompassed herein. The diagnostic kit is a package comprising the anti-HER2 antibody of the disclosure (*e.g.*, either in lyophilized form or as an aqueous solution) and one or more reagents useful for performing a diagnostic assay. Where the anti-HER2 antibody is labeled with an enzyme, the kit can include substrates and cofactors required by the enzyme (*e.g.*, a substrate precursor which provides the detectable chromophore or fluorophore). In addition, other additives can be included, such as stabilizers, buffers (*e.g.*, a block buffer or lysis buffer), and the like. In certain embodiments, the anti-HER2 antibody included in a diagnostic kit is immobilized on a solid surface, or a solid surface (*e.g.*, a slide) on which the antibody can be immobilized is included in the kit. The relative amounts of the various reagents can be varied widely to provide for concentrations in solution of the reagents which substantially optimize the sensitivity of the assay. In a specific embodiment, the antibody and one or more reagents can be provided (individually or combined) as dry powders, usually lyophilized, including excipients which on dissolution will provide a reagent solution having the appropriate concentration.

### 8. EXAMPLE 1: IDENTIFICATION OF VARIANTS OF TRASTUZUMAB WITH AFFINITY TO HER2

Trastuzumab was subjected to comprehensive mutational analysis to identify mutants that had affinity to HER2 as compared to wild-type trastuzumab. Mutants were analyzed by FACS to confirm their binding affinity to HER2 as compared to trastuzumab.

### 8.1. Materials and Methods

To determine binding of individual variants to HER2, cell surface displayed trastuzumab immunoglobulin variants were incubated with soluble HER2 at sub-saturating conditions (below K_{D}), and the degree of binding was quantitated by FACS. Individual trastuzumab variants were constructed in the mammalian cell surface display vector (Akamatsu et al., 2007, J. Immunol. Methods 327(1-2):40-52), and transfected into a human cell line. 400ng of plasmid DNA in 50µl Hybridoma-Serum Free Medium (SFM) were mixed with 1µl of Lipofectamine 2000 in 50ul Hybridoma-SFM and incubated for 20 minutes at room temperature. This mixture was then added to one well of a 24-well plate, previously seeded 24 hours earlier with 2 x 10⁵ cells of the human embryonic kidney-derived cell line 293c18 in 0.5ml of DME medium supplemented with 10% Fetal Bovine Serum and 0.25mg/ml G418. After 48 hours, the cells were harvested and ready for FACS staining.

For FACS staining, approximately 5 x 10⁵ cells were incubated with 1nM HER2-C Lambda-AF647 (HER2 extracellular domain fused to C Lambda and directly conjugated with Alexa Fluor 647 dye) and 1/500 dilution of Goat anti-Human Kappa-PE (Southern Biotech #2060-09) in 1ml of Phosphate Buffered Saline (PBS) plus 0.5% Bovine Serum Albumin (BSA), and incubated at room temperature for 1 hour. Cells were washed 3 times with 1ml of cold PBS+0.5% BSA, resuspended in 200 µl of PBS+1% Formaldehyde, and analyzed on a BD FACS Calibur. Cells were gated to only include the IgG expressing population, and the mean fluorescence intensity (MFI) of the binding (Alexa Fluor 647) channel was determined. The MFI for binding of each variant was compared to wild-type trastuzumab for each sample set to normalize for experiment-to-experiment variability.

### 8.2. Results

Figures 2A-2D show exemplary data for the binding affinity of trastuzumab variants to labeled HER2 extracellular domain as compared to wild-type trastuzumab (binding affinity=100). The EC₅₀ of wild-type trastuzumab was determined by a FACS competition binding assay to be 2.638 µg/ml (Figure 3). All variants were found to have a binding affinity to HER2 extracellular domain that is comparable to the binding affinity of wild-type trastuzumab.

### 9. EXAMPLE 2: TESTING FOR CD4+ T CELL EPITOPE REGIONS IN TRASTUZUMAB 9.1. Methods

The testing for T cell epitopes detailed below was performed in accordance with US Patent No. 6,838,269.

Peptides. Peptides were synthesized using a multi-pin format by Mimotopes (Adelaide, Australia). The sequences of the trastuzumab light and heavy chain variable regions were synthesized as 15-mer peptides overlapping by 12 amino acids for a total of 68 peptides. (Figures 4A and 4B; SEQ ID NOs: 9-76) Peptides arrived lyophilized and were resuspended in DMSO (Sigma-Aldrich) at approximately 1-2 mg/ml. Stock peptides were kept frozen at -20°C.

Peripheral blood mononuclear cells. Community donor buffy coat products were purchased from the Stanford Blood Center, Palo Alto, CA. Buffy coat material was diluted 1:1 v:v with DPBS containing no calcium or magnesium. Diluted buffy coat material (25-35 mls) was underlayed in 50 ml conical centrifuge tubes (Sarsted or Costar) with 12.5 mls of FicollPaque-PLUS (GE Healthcare). The samples were centrifuged at 900 g for 30 minutes at room temperature. Peripheral blood mononuclear cells (PBMC) were collected from the interface. DPBS was added to bring the final volume to 50 mls and the cells were centrifuged at 350 g for 5 minutes. Pelleted cells were resuspended in DPBS and counted.

Dendritic cells. For isolation of dendritic cells, T75 culture flasks (Costar) were seeded with 10⁸ freshly isolated PBMC in a total volume of 30 mls AIM V media (Invitrogen). Excess PBMC were frozen at -80°C in 90% fetal calf serum (FCS), 10% DMSO at 5 x 10⁷ cells/ml. T75 flasks were incubated at 37°C in 5% CO₂ for 2 hours. Nonadherent cells were removed, and the adherent monolayer was washed with DPBS. To differentiate dendritic cells from monocytes, 30 mls of AIM V media containing 800 units/ml of GM-CSF (R and D Systems) and 500 units/ml IL-4 (R and D Systems) were added. Flasks were incubated for 5 days. On day 5 IL-1α (Endogen) and TNFα (Endogen) were added to 50 pg/ml and 0.2 ng/ml, respectively. Flasks were incubated for two more days. On day 7, dendritic cells were collected by the addition of 3 mls of 100 mM EDTA containing 0.5 to 1.0 mg Mitomycin C (Sigma-Aldrich) for a final concentration of 10 mM EDTA and 16.5 to 33 µg/ml Mitomycin C. Flasks were incubated an additional hour at 37°C and 5% CO₂. Dendritic cells were collected, and washed in AIM V media 2-3 times.

Cell culture. On day 7, previously frozen autologous PBMC were thawed quickly in a 37°C water bath. Cells were immediately diluted into DPBS or AIM V media and centrifuged at 350g for 5 minutes. CD4⁺ cells were enriched by negative selection using magnetic beads (Easy-Sep CD4⁺ kit, Stem Cell Technologies). Autologous CD4⁺ T cells and dendritic cells were cocultured at 2 x 10⁵ CD4⁺ T cells per 2 x 10⁴ dendritic cells per well in 96 well round bottomed plates (Costar 9077). Peptides were added at approximately 5 µg/ml. Control wells contained the DMSO (Sigma) vehicle alone at 0.25% v:v. Positive control wells contained DMSO at 0.25% and tetanus toxoid (List Biologicals or CalBioChem) at 1 µg/ml. Cultures were incubated for 5 days. On day 5, 0.25 µCi per well of tritiated thymidine (Amersham or GE Healthcare) was added. Cultures were harvested on day 6 to filtermats using a Packard Filtermate Cell harvester. Scintillation counting was performed using a Wallac MicroBeta 1450 scintillation counter (Perkin Elmer).

Data analyses. Average background CPM values were calculated by averaging individual results from 6 to 12 replicates. The CPM values of the four positive control wells were averaged. Replicate or triplicate wells for each peptide were averaged. Stimulation index values for the positive control and the peptide wells were calculated by dividing the average experimental CPM values by the average control values. In order to be included in the dataset, a stimulation index of greater than 3.0 in the tetanus toxoid positive control wells was required. A response was noted for any peptide resulting in a stimulation index of 2.95 or greater. Peptides were tested using peripheral blood samples from a group of 100 donors. Responses to all peptides were compiled. For each peptide tested, the percentage of the donor set that responded with a stimulation index of 2.95 or greater was calculated. In addition, the average stimulation index for all donors was calculated.

### 9.2. Results

Identification of a CD4⁺ T cell epitopes in the trastuzumab V_{H} and V_{L} regions. CD4⁺ T cell epitope peptides were identified by an analysis of the percent responses to the peptides within the set of 100 donors. The average percent response and standard deviation were calculated for all peptides tested describing the trastuzumab heavy chain and light chain variable regions. A response rate greater than or equal to the average background response plus three standard deviations was considered a potential CD4⁺ T cell epitope. For the trastuzumab light chain variable region, 32 peptides were tested (Figure 4A) which resulted in an average background percent response of 2.63 ± 2.67%. Three standard deviations above background was determined to be 10.6%. One light chain peptide at position 8 (T22-Y36; SEQ ID NO:16) displayed this level of response in the trastuzumab light chain peptide dataset, with a response rate of 15.0% (Figure 5A). For the trastuzumab heavy chain variable region, 36 peptides were tested (Figure 4B). The average background percent response was 3.28 ± 2.14%. Three standard deviations above background was 9.7%. No peptides within the trastuzumab heavy chain dataset achieved epitope status. (Figure 5B) However, the peptide at position 29 (S85-W99; SEQ ID NO:69) in the heavy chain dataset achieved a response rate of 9.0%, and was considered an epitope due to an increase stimulation index (see below).

The average stimulation index was calculated for all peptides in the dataset (light chain and heavy change stimulation indices are set forth respectively in Figures 5C and 5D). The light chain peptide at position 8 (SEQ ID NO:16) had a high average stimulation index of 1.73. The heavy chain peptide at position 29 (SEQ ID NO:69) returned an average stimulation index of 2.51. Due to an elevated average stimulation index and an above average response rate, the heavy chain peptide at position 29 was included when determining CD4⁺ T cell epitope content of this antibody variable region. All of these stimulation index values are significantly higher than the average stimulation index for all peptides in the two datasets (1.17 ± 0.03 for all 68 heavy chain and light chain peptides).

Two CD4⁺ T cell epitope regions are present in the trastuzumab variable regions (Figure 1A). In the V_{L} region, an epitope was found in the peptide at position 8 that encompasses CDR1 and portions of framework 1 and 2. The CDR-derived amino acids are in bold underlined type in Figure 1A. In the heavy chain, an epitope peptide region was identified in the peptide at position 29 that encompasses V_{H} framework 3 and 3 amino acids of CDR3. This epitope peptide also encompasses a back-mutation at S97 that was incorporated during humanization.

A series of variant peptides were selected for additional testing based on the PxP analysis of trastuzumab CDR regions. (Figures 6A, 6B and 6C) The specific amino acid changes selected for incorporation into the variant peptides were confirmed to have no impact on the affinity of antigen binding. Variant peptides were designed to have a single amino acid modification, or to have two amino acid substitutions. All peptides were synthesized via pin synthesis, and will be tested for their ability to induce proliferative responses in human CD4⁺ T cells by the methods detailed above.

### 10. EXAMPLE 3: PRODUCTION AND PURIFICATION OF HER2 PROTEIN

HER2 protein was expressed as monomeric secreted fusion proteins. The extracellular domain (ECD) of HER2 was fused with human lambda constant region (Cλ) containing a Cys to Ser mutation to disrupt the intermolecular disulfide bond (Akamatsu et al., 2007, J. Immunol. Methods 327:40). The HER2 ECD-Cλ fusion protein was expressed in the culture supernatant of 293S stable transfectant. The fusion protein was purified over an affinity column with Herceptin® linked to sepharose beads. After binding at neutral pH, the protein was eluted with 20mM sodium acetate pH 2.5. Eluted protein was dialyzed into PBS (pH 7.4).

### 11. EXAMPLE 4: PRODUCTION AND PURIFICATION OF TRASTUZUMAB AND ITS VARIANTS

To produce the soluble trastuzumab and its variants, 293c18 in DME medium containing 2% ultra low Ig FBS was transfected with Lipofectamine 2000 (Invitrogen). After 7 days, culture supernatants containing soluble antibodies were harvested. Transiently expressed antibody variants were purified using protein G (GE Healthcare, Uppsala, Sweden). Antibodies were eluted with 20mM sodium citrate (pH 2.5), neutralized with 1M Tris (pH 8.0), and buffer exchanged into PBS (pH 7.4) by centrifugal filter (Vivaspin 50kDa MWCO, GE Healthcare). Purity was evaluated by SDS-PAGE.

### 12. EXAMPLE 5: BINDING OF TRASTUZUMAB VARIANTS WITH PURIFIED HER2 PROTEIN

HER2 ECD fused with a Cλ tag was bound to AlphaLISA® acceptor beads conjugated with goat anti-human Cλ antibody and biotinylated Herceptin® was bound to AlphaScreen® streptavidin-coated donor beads (Perkin Elmer, Waltham, MA). Herceptin® was biotinylated with Sulfo-NHS-Biotin using standard methods and dialyzed in PBS. Conjugation of AlphaLISA® acceptor beads was performed by following the manufacturer's instructions ("AlphaLISA® Assay Development Guide", Perkin Elmer). The binding assay was performed in a 96-well AlphaPlate (Perkin Elmer) in assay buffer (0.5% BSA, 0.01% Tween20 in PBS (pH 7.1)). Each well contained 1.25 nM biotinylated Herceptin®, 1:4 serially diluted unlabeled trastuzumab variant protein starting from 200 nM, 1.25 µg/mL HER2 ECD-Cλ, and 5 µg/ml goat anti-human Cλ-conjugated acceptor beads. The plate was incubated in the dark for 1 h at room temperature. Streptavidin donor beads were subsequently added to each well at 5 µg/mL. The plate was incubated in the dark at room temperature for an additional 30 min, after which it was read on an EnVision reader (Perkin Elmer). Data were fit using nonlinear regression with the software GRAPHPAD PRISM (GraphPad, San Diego, CA).

### 13. EXAMPLE 6: FACS COMPETITION OF TRASTUZUMAB VARIANTS WITH HER2 EXPRESSED ON CELLS

The relative binding affinities of Herceptin® and trastuzumab to native HER2 expressed on breast cancer cell line were determined by a competition assay using FACS. KS-BR3 cells were washed with FACS buffer containing 0.5% FBS in PBS. Biotinylated Herceptin® diluted to a final concentration of 1.0 µg/mL was mixed with competitor antibodies at 1:3 serial dilution starting with 50 µg/ml (at final concentration) and the mixture were transferred to the 96-well plates containing the cells at 1 x 10⁵ per well. The plate were incubated at 4°C for 1 hour, and then washed twice with FACS buffer. 100 µL of Streptavidin-RPE conjugate (Invitrogen, Carlsbad, CA) diluted to 2.5 µg/mL in FACS buffer were added to the wells, and the plates were incubated 4°C for another 30 minutes in the dark. Cells were washed twice with FACS buffer and resuspended with 200 µL of fixing buffer (1% paraformaldehyde in PBS). Samples were analyzed on a FACS Calibur (BD, Franklin Lakes, NJ)

### 14. EXAMPLE 7: BINDING KINETICS OF TRASTUZUMAB VARIANTS WITH PURIFIED HER2 PROTEIN

Binding kinetics of trastuzumab variants were measured by using a BIAcore T100 system (GE Healthcare Life Sciences, Piscataway, NJ). Approx. 5000 RU of polyclonal goat anti-human Fc antibody (Thermo Fisher Scientific, Rockford, IL) was immobilized on a CM5 chip according to the manufacturer's instructions. Binding assays to study the binding of trastuzumab and HER2 were run at a flow rate of 50 µL/min at room temperature. HER2 ECD-Cλ in 5 different concentrations between 6.25-100 nM were injected over surfaces where trastuzumab and its variants were captured, with a 5 min association phase followed by 15 min dissociation phase. Binding data were fit to the 1:1 Langmuir model to extract binding constants from the BIAevaluation T100 software.

### 15. EXAMPLE 8: DEIMMUNIZATION STUDY

A series of variant peptides were selected for additional testing based on the PxP analysis of trastuzumab CDR regions. *See* Figures 6A to 6C. The specific amino acid changes incorporated into the variant peptides were selected for minimal impact on antigen binding. Variant peptides were designed to have a single amino acid modification, or to have two amino acid substitutions. All peptides were synthesized via pin synthesis, and were tested for this ability to induce a CD4+ T cell proliferative response. A total of 76 peptides, including two replicates of each parent peptide, were tested using cells from 83 community donors. The results are shown in Figure 7. The epitope peptide identified in trastuzumab VH (peptide #29) was tested twice in this replicate of donors Figure 7A (open triangles), and the percent responses were 10.8% and 4.8% . The first value is consistent with the response rate seen in the initial screening (9.0%), and the second value is low. Within the set of 21 VH epitope variant peptides, there were two variants with response rates of 1.20% (Figure 7). One of these peptides (SEQ ID NO: 1405) contained an amino acid modification within the framework region. The epitope peptides identified in trastuzumab VL (peptide # 8) was also tested twice in this replicate of donors (Figure 7B open triangles), and the percent responses were 8.4% and 6.0%. These results compare unfavorably to the initial screening, where 15% of the tested donors responded to the peptide. However, within this dataset of 51 variant peptides, two variants did not induce responses in any of the tested donors, and 5 peptides induced proliferative responses in 1.2% of the donors (Figure 8). The variant peptides selected for further characterization are shown in Figure 8.

## Claims

1. An anti-HER2 antibody or an anti-HER2 binding fragment of an antibody which comprises a VH and a VL that have one or more amino acid substitutions or combinations of amino acid substitutions as compared to an antibody having a VH comprising an amino acid sequence corresponding to positions 1 to 117 of SEQ ID NO:1 and a VL comprising an amino acid sequence corresponding to positions 1 to 103 of SEQ ID NO:2, wherein said one or more amino acid substitutions or combinations of amino acid substitutions are selected from:
(i) the third framework of the VH chain (FR-H3) substitution R83K as compared to a VH of SEQ ID NO:1;
(ii) the first CDR of the VL chain (CDR-L1) substitution D28L + A34D as compared to a VL of SEQ ID NO:2;
(iii) the first CDR of the VL chain (CDR-L1) substitution A34D as compared to a VL of SEQ ID NO:2;
(iv) the first CDR of the VL chain (CDR-L1) substitution A34V as compared to a VL of SEQ ID NO:2;
(v) the first CDR of the VL chain (CDR-L1) substitutions Q27S + A34D as compared to a VL of SEQ ID NO:2;
(vi) the first CDR of the VL chain (CDR-L1) substitutions Q27L + A34V as compared to a VL of SEQ ID NO:2;
(vii) the first CDR of the VL chain (CDR-L1) substitutions Q27F + A34D as compared to a VL of SEQ ID NO:2; and
(viii) the first CDR of the VL chain (CDR-L1) substitutions D28N + A34D as compared to a VL of SEQ ID NO:2.

2. The anti-HER2 antibody or anti-HER2 binding fragment of an antibody of claim 1, wherein FR-H3 includes the substitution R83K as compared to a VH of SEQ ID NO:1.

3. The anti-HER2 antibody or anti-HER2 binding fragment of an antibody of claim 1, wherein CDR-L1 includes the substitution A34V as compared to CDR-L1 of a VL of SEQ ID NO:2.

4. The anti-HER2 antibody or anti-HER2 binding fragment of an antibody of claim 1, wherein CDR-L1 includes at least one of the combination of substitutions selected from D28N + A34D and D28L + A34D as compared to CDR-L1 of a VL of SEQ ID NO:2.

5. The anti-HER2 antibody or anti-HER2 binding fragment of an antibody of claim 1, wherein CDR-L1 includes at least one of the combination of substitutions selected from Q27S + A34D , Q27L + A34V, and Q27F + A34D as compared to CDR-L1 of a VL of SEQ ID NO:2.

6. The anti-HER2 antibody or anti-HER2 binding fragment of an antibody of claim 1, wherein CDR-H3 further includes the substitution W95F as compared to CDR-H3 of a VH of SEQ ID NO:1.

7. The anti-HER2 antibody or anti-HER2 binding fragment of any one of claims 1 to 6 which has an affinity to HER-2 that is at least 1.1-fold that of an antibody having a VH comprising an amino acid sequence corresponding to positions 1 to 117 of SEQ ID NO:1 and a VL comprising an amino acid sequence corresponding to positions 1 to 103 of SEQ ID NO:2, as assayed by BIAcore, AlphaLISA or by FACS.

8. The anti-HER2 antibody or anti-HER2 binding fragment of any preceding claim which is:
(i) a monoclonal antibody or anti-HER2 binding fragment of a monoclonal antibody, respectively;
and/or
(ii) an IgG, preferably an IgG1, an IgG2 or an IgG4.

9. The anti-HER2 antibody or anti-HER2 binding fragment of any preceding claim which;
(a) includes one or more mutations in the Fc region that increases ADCC activity;
(b) is non-fucosylated;
(c) includes one or more mutations in the Fc region that increases binding to FcγR;
(d) includes one or more mutations in the Fc region that decreases binding to FcγR;
(e) includes one or more mutations in the Fc region that increases binding to FcRn;
(f) includes one or more mutations in the Fc region that decrease ADCC activity; and/or
(g) is purified, preferably wherein the anti-HER2 antibody or anti-HER2 binding fragment is purified to at least 85%, at least 90%, at least 95% or at least 98% homogeneity.

10. An antibody-drug conjugate comprising an anti-HER2 antibody or anti-HER2 binding fragment according to any preceding claim.

11. A pharmaceutical composition comprising an anti-HER2 antibody or anti-HER2 binding fragment according to any one of claims 1 to 9 or an antibody-drug conjugate according to claim 10, and a pharmaceutically acceptable carrier.

12. A nucleic acid comprising a nucleotide sequence encoding an anti-HER2 antibody or anti-HER2 binding fragment according to any one of claims 1 to 9.

13. A vector comprising the nucleic acid of claim 12.

14. A prokaryotic host cell transformed with a vector according to claim 13.

15. A therapeutically effective amount of an anti-HER2 antibody or anti-HER2 binding fragment according to any one of claims 1 to 9, an antibody-drug conjugate according to claim 10, or a pharmaceutical composition according to claim 11, for use in treating cancer, or mammary or extra-mammary Paget's disease, in a human.

16. The anti-HER2 antibody or anti-HER2 binding fragment, antibody-drug conjugate or pharmaceutical composition for use according to claim 15 wherein:
(i) the cancer is breast cancer; and/or
(ii) HER2 overexpression has been detected in said cancer.

17. A method of determining whether a host cell produces a greater amount of an anti-HER2 antibody or an anti-HER2 binding fragment according to any one of claims 1 to 9 as compared to an antibody having a VH of SEQ ID NO: 1 and a VL of SEQ ID NO:2, comprising the steps of:
(a) optionally isolating the anti-HER2 antibody or anti-HER2 binding fragment, or isolating and purifying the anti-HER2 antibody or anti-HER2 binding fragment;
(b) determining the amount of the anti-HER2 antibody or anti-HER2 antibody binding fragment produced by a first cell, wherein said first cell is said host cell engineered to express said anti-HER2 antibody or anti-HER2 antibody binding fragment;
(c) optionally determining the amount of an antibody having a VH of SEQ ID NO: 1 and a VL of SEQ ID NO:2 produced by a second cell; and
(d) comparing the amount of anti-HER2 antibody or anti-HER2 binding fragment produced by said first cell to the amount of an antibody having a VH of SEQ ID NO:1 and a VL of SEQ ID NO:2 produced by the second cell,
wherein said second cell is said host cell engineered to express an antibody having a VH of SEQ ID NO: 1 and a VL of SEQ ID NO:2, such that greater production of said anti-HER2 antibody or anti-HER2 antibody binding fragment by the first cell relative to the amount of an antibody having a VH of SEQ ID NO: 1 and a VL of SEQ ID NO:2 produced by the second cell indicates that the host cell produces a greater amount of said anti-HER2 antibody or anti-HER2 binding fragment as compared to an antibody having a VH of SEQ ID NO: 1 and a VL of SEQ ID NO:2, thereby determining whether the host cell produces a greater amount of said anti-HER2 antibody or anti-HER2 binding fragment as compared to an antibody having a VH of SEQ ID NO:1 and a VL of SEQ ID NO:2.

18. The method of claim 17, wherein the host cell is either:
(i) a prokaryotic cell, preferably *E. coli*; or
(ii) a eukaryotic cell, preferably, wherein the host cell is selected from Sf9, *S*. *cereviseae*, and CHO.

19. The method of claims 17 or 18, wherein the production of said anti-HER2 antibody or anti-HER2 binding fragment is at least 20% greater than the production of an antibody having a VH of SEQ ID NO: 1 and a VL of SEQ ID NO:2 in the host cell, preferably wherein the production of said anti-HER2 antibody or anti-HER2 binding fragment is at least 50% greater, and further preferably wherein the production of anti-HER2 antibody or anti-HER2 binding fragment is at least 100% greater.

20. A method of identifying an anti-HER2 antibody or an anti-HER2 binding fragment that is produced at a greater amount than an antibody having a VH of SEQ ID NO: 1 and a VL of SEQ ID NO:2 by a host cell, comprising:
(a) determining the amount of anti-HER2 antibody or anti-HER2 binding fragment produced by a plurality of host cells, each engineered to express a different anti-HER2 antibody or anti-HER2 binding fragment of any one of claims 1 to 11 ; and
(b) identifying a host cell that produces an anti-HER2 antibody or anti-HER2 binding fragment in a greater amount than an antibody having a VH of SEQ ID NO:1 and a VL of SEQ ID NO:2 is produced by a control host cell engineered to express an antibody having a VH of SEQ ID NO:1 and a VL of SEQ ID NO:2,
wherein a host cell identified in step (b) produces an anti-HER2 antibody or an anti-HER2 binding fragment at a greater amount than an antibody having a VH of SEQ ID NO: 1 and a VL of SEQ ID NO:2, thereby identifying an anti-HER2 antibody or an anti-HER2 binding fragment that is produced at a greater amount than an antibody having a VH of SEQ ID NO:1 1 and a VL of SEQ ID NO:2 is produced by said host cell.

21. The method of claim 20, wherein:
(i) said plurality of host cells is at least 5 host cells, at least 10 host cells or at least 20 host cells; and/or
(ii) said host cell is *E. coli*, Sf9, *S. cereviseae*, or CHO.

22. The method of claim 20 or 21, wherein:
(i) a host cell is identified in step (b) that produces an anti-HER2 antibody or anti-HER2 binding fragment in an amount that is at least 20%, at least 50% or at least 100% greater than an antibody having a VH of SEQ ID NO:1 and a VL of SEQ ID NO:2 is produced by a host cell engineered to express an antibody having a VH of SEQ ID NO:1 and a VL of SEQ ID NO:2; and/or
(ii) the amount of anti-HER2 antibody or anti-HER2 binding fragment produced by a plurality of host cells in step (a) is determined simultaneously or successively.

23. A method of determining whether an anti-HER2 antibody or an anti-HER2 binding fragment according to any one of claims 1 to 9 exhibits greater stability as compared to an antibody having a VH of SEQ ID NO: 1 and a VL of SEQ ID NO:2, comprising the steps of:
(a) determining stability of an anti-HER2 antibody or anti-HER2 binding fragment in a first sample;
(b) optionally determining the stability of an antibody having a VH of SEQ ID NO:1 and a VL of SEQ ID NO:2 in the second sample; and
(c) comparing the amount of full-length anti-HER2 antibody or anti-HER2 binding fragment in the first sample with an amount of full-length an antibody having a VH of SEQ ID NO:1 and a VL of SEQ ID NO:2 in a second sample, wherein the second sample is subjected to substantially the same conditions as the first sample, thereby determining whether an anti-HER2 antibody or anti-HER2 antibody binding fragment exhibits greater stability as compared to an antibody having a VH of SEQ ID NO:1 1 and a VL of SEQ ID NO:2.

## Patentansprüche

1. Anti-HER2-Antikörper oder Anti-HER2-Bindungsfragment eines Antikörpers, der/das eine VH und eine VL umfasst, die eine oder mehrere Aminosäuresubstitution(en) oder Kombinationen von Aminosäuresubstitutionen hat, wenn man Vergleiche mit einem Antikörper anstellt, der eine VH, die eine Aminosäuresequenz umfasst, die Positionen 1 bis 117 von SEQ ID NO:1 entspricht, und eine VL, die eine Aminosäuresequenz umfasst, die Positionen 1 bis 103 von SEQ ID NO:2 entspricht, hat, wobei die eine oder mehrere Aminosäuresubstitution(en) oder Kombinationen von Aminosäuresubstitutionen ausgewählt sind aus:
(i) der Substitution R83K des dritten Gerüsts der VH-Kette (FR-H3) im Vergleich zu einer VH von SEQ ID NO:1;
(ii) der Substitution D28L + A34D der ersten CDR der VL-Kette (CDR-L1) im Vergleich zu einer VL von SEQ ID NO:2;
(iii) der Substitution A34D der ersten CDR der VL-Kette (CDR-L1) im Vergleich zu einer VL von SEQ ID NO:2;
(iv) der Substitution A34V der ersten CDR der VL-Kette (CDR-L1) im Vergleich zu einer VL von SEQ ID NO:2;
(v) den Substitutionen Q27S + A34D der ersten CDR der VL-Kette (CDR-L1) im Vergleich zu einer VL von SEQ ID NO:2;
(vi) den Substitutionen Q27L + A34V der ersten CDR der VL-Kette (CDR-L1) im Vergleich zu einer VL von SEQ ID NO:2;
(vii) den Substitutionen Q27F + A34D der ersten CDR der VL-Kette (CDR-L1) im Vergleich zu einer VL von SEQ ID NO:2 und
(viii) den Substitutionen D28N + A34D der ersten CDR der VL-Kette (CDR-L1) im Vergleich zu einer VL von SEQ ID NO:2.

2. Anti-HER2-Antikörper oder Anti-HER2-Bindungsfragment eines Antikörpers gemäß Anspruch 1, wobei FR-H3 die Substitution R83K im Vergleich zu einer VH von SEQ ID NO:1 umfasst.

3. Anti-HER2-Antikörper oder Anti-HER2-Bindungsfragment eines Antikörpers gemäß Anspruch 1, wobei CDR-L1 die Substitution A34V im Vergleich zu CDR-L1 einer VL von SEQ ID NO:2 umfasst.

4. Anti-HER2-Antikörper oder Anti-HER2-Bindungsfragment eines Antikörpers gemäß Anspruch 1, wobei CDR-L1 wenigstens eine der Kombinationen von Substitutionen, ausgewählt aus D28N + A34D und D28L + A34D, im Vergleich zu CDR-L1 einer VL von SEQ ID NO:2 umfasst.

5. Anti-HER2-Antikörper oder Anti-HER2-Bindungsfragment eines Antikörpers gemäß Anspruch 1, wobei CDR-L1 wenigstens eine der Kombinationen von Substitutionen, ausgewählt aus Q27S + A34D, Q27L + A34V und Q27F + A34D, im Vergleich zu CDR-L1 einer VL von SEQ ID NO:2 umfasst.

6. Anti-HER2-Antikörper oder Anti-HER2-Bindungsfragment eines Antikörpers gemäß Anspruch 1, wobei CDR-H3 außerdem die Substitution W95F im Vergleich zu CDR-H3 einer VH von SEQ ID NO:1 umfasst.

7. Anti-HER2-Antikörper oder Anti-HER2-Bindungsfragment gemäß einem der Ansprüche 1 bis 6, der/das eine Affinität zu HER-2 hat, die das wenigstens 1,1-Fache derjenigen eines Antikörpers ist, der eine VH, die eine Aminosäuresequenz umfasst, die Positionen 1 bis 117 von SEQ ID NO:1 entspricht, und eine VL, die eine Aminosäuresequenz umfasst, die Positionen 1 bis 103 von SEQ ID NO:2 entspricht, hat, wie sie durch BIAcore, AlphaLISA oder durch FACS analysiert wird.

8. Anti-HER2-Antikörper oder Anti-HER2-Bindungsfragment gemäß einem vorangehenden Anspruch, der/das ist:
(i) ein monoklonaler Antikörper bzw. ein Anti-HER2-Bindungsfragment eines monoklonalen Antikörpers und/oder
(ii) ein IgG, vorzugsweise ein IgG1, ein IgG2 oder ein IgG4.

9. Anti-HER2-Antikörper oder Anti-HER2-Bindungsfragment gemäß einem vorangehenden Anspruch, der/das:
(a) eine oder mehrere Mutation(en) in der Fc-Region umfasst, welche die ADCC-Aktivität erhöht/erhöhen;
(b) nicht-fucosyliert ist;
(c) eine oder mehrere Mutation(en) in der Fc-Region umfasst, die eine Bindung an FcγR erhöht/erhöhen;
(d) eine Mutation oder mehrere Mutationen in der Fc-Region umfasst, die eine Bindung an FcγR verringert/verringern;
(e) eine oder mehrere Mutation(en) in der Fc-Region umfasst, die eine Bindung an FcRn erhöht/erhöhen;
(f) eine Mutation oder mehrere Mutationen in der Fc-Region umfasst, die die ADCC-Aktivität verringert/verringern, und/oder
(g) gereinigt ist, wobei vorzugsweise der Anti-HER2-Antikörper oder das Anti-HER2-Bindungsfragment zu wenigstens 85%, wenigstens 90%, wenigstens 95% oder wenigstens 98% Homogenität gereinigt ist.

10. Antikörper-Wirkstoff-Konjugat, das einen Anti-HER2-Antikörper oder ein Anti-HER2-Bindungsfragment gemäß einem vorangehenden Anspruch umfasst.

11. Pharmazeutische Zusammensetzung, umfassend einen Anti-HER2-Antikörper oder ein Anti-HER2-Bindungsfragment gemäß einem der Ansprüche 1 bis 9 oder ein Antikörper-Wirkstoff-Konjugat gemäß Anspruch 10 und einen pharmazeutisch annehmbaren Träger.

12. Nucleinsäure, die eine Nucleotidsequenz umfasst, die einen Anti-HER2-Antikörper oder ein Anti-HER2-Bindungsfragment gemäß einem der Ansprüche 1 bis 9 codiert.

13. Vektor, der die Nucleinsäure gemäß Anspruch 12 umfasst.

14. Prokaryotische Wirtszelle, die mit einem Vektor gemäß Anspruch 13 transformiert ist.

15. Therapeutisch wirksame Menge eines Anti-HER2-Antikörpers oder eines Anti-HER2-Bindungsfragmentes gemäß einem der Ansprüche 1 bis 9, Antikörper-Wirkstoff-Konjugat gemäß Anspruch 10 oder pharmazeutische Zusammensetzung gemäß Anspruch 11 zur Verwendung bei der Behandlung von Krebs oder mammärer oder extra-mammärer Paget-Erkrankung bei einem Menschen.

16. Anti-HER2-Antikörper oder Anti-HER2-Bindungsfragment, Antikörper-Wirkstoff-Konjugat oder pharmazeutische Zusammensetzung zur Verwendung gemäß Anspruch 15, wobei:
(i) der Krebs Brustkrebs ist und/oder
(ii) HER2-Überexpression in dem Krebs detektiert worden ist.

17. Verfahren zur Bestimmung, ob eine Wirtszelle eine größere Menge eines Anti-HER2-Antikörpers oder eines Anti-HER2-Bindungsfragments gemäß einem der Ansprüche 1 bis 9 im Vergleich zu einem Antikörper, der eine VH von SEQ ID NO:1 und eine VL von SEQ ID NO:2 hat, produziert, umfassend die Schritte:
(a) gegebenenfalls Isolieren des Anti-HER2-Antikörpers oder Anti-HER2-Bindungsfragments oder Isolieren und Reinigen des Anti-HER2-Antikörpers oder Anti-HER2-Bindungsfragments;
(b) Bestimmen der Menge des Anti-HER2-Antikörpers oder Anti-HER2-Antikörper-Bindungsfragments, die durch eine erste Zelle produziert wurde, wobei die erste Zelle die Wirtszelle ist, die manipuliert wurde, um den Anti-HER2-Antikörper oder das Anti-HER2-Antikörper-Bindungsfragment zu exprimieren;
(c) gegebenenfalls Bestimmen der Menge eines Antikörpers, der eine VH von SEQ ID NO:1 und eine VL von SEQ ID NO:2 hat, der durch eine zweite Zelle produziert wurde, und
(d) Vergleichen der Menge eines Anti-HER2-Antikörpers oder Anti-HER2-Bindungsfragments, produziert durch die erste Zelle, mit der Menge eines Antikörpers, der eine VH von SEQ ID NO:1 und eine VL von SEQ ID NO:2 hat, produziert durch die zweite Zelle,
wobei die zweite Zelle die Wirtszelle ist, die gentechnisch manipuliert wurde, um einen Antikörper, der eine VH von SEQ ID NO:1 und eine VL von SEQ ID NO:2 hat, zu exprimieren, so dass eine größere Produktion des Anti-HER2-Antikörpers oder Anti-HER2-Antikörper-Bindungsfragments durch die erste Zelle relativ zu der Menge eines Antikörpers, der eine VH von SEQ ID NO:1 und eine VL von SEQ ID NO:2 hat, produziert durch die zweite Zelle, anzeigt, dass die Wirtszelle eine größere Menge des Anti-HER2-Antikörpers oder Anti-HER2-Bindungsfragments im Vergleich zu einem Antikörper, der eine VH von SEQ ID NO:1 und eine VL von SEQ ID NO:2 hat, produziert, wodurch bestimmt wird, ob die Wirtszelle eine größere Menge des Anti-HER2-Antikörpers oder Anti-HER2-Bindungsfragments im Vergleich zu der eines Antikörpers, der eine VH von SEQ ID NO:1 und eine VL von SEQ ID NO:2 hat, produziert.

18. Verfahren gemäß Anspruch 17, wobei die Wirtszelle ist, entweder:
(i) eine prokaryotische Zelle, vorzugsweise *E. coli,* oder
(ii) eine eukaryotische Zelle, wobei die Wirtszelle vorzugsweise aus Sf9, *S. cerevisiae* und CHO ausgewählt wird.

19. Verfahren gemäß Anspruch 17 oder 18, wobei in der Wirtszelle die Produktion des Anti-HER2-Antikörpers oder Anti-HER2-Bindungsfragments wenigstens 20% höher ist als die Produktion eines Antikörpers, der eine VH von SEQ ID NO:1 und eine VL von SEQ ID NO:2 hat, wobei vorzugsweise die Produktion des Anti-HER2-Antikörpers oder Anti-HER2-Bindungsfragments wenigstens 50% höher ist und wobei außerdem vorzugsweise die Produktion von Anti-HER2-Antikörper oder Anti-HER2-Bindungsfragment wenigstens 100% höher ist.

20. Verfahren zur Identifizierung eines Anti-HER2-Antikörpers oder eines Anti-HER2-Bindungsfragments, der/das in größerer Menge als ein Antikörper, der eine VH von SEQ ID NO:1 und eine VL von SEQ ID NO:2 hat, durch eine Wirtszelle produziert wird, umfassend:
(a) Bestimmen der Menge eines Anti-HER2-Antikörpers oder Anti-HER2-Bindungsfragments, der/das durch eine Vielzahl von Wirtszellen produziert wird, die jeweils manipuliert sind, um einen unterschiedlichen Anti-HER2-Antikörper oder ein unterschiedliches Anti-HER2-Bindungsfragment gemäß einem der Ansprüche 1 bis 11 zu exprimieren, und
(b) Identifizieren einer Wirtszelle, die einen Anti-HER2-Antikörper oder ein Anti-HER2-Bindungsfragment in größerer Menge produziert als die eines Antikörpers, der eine VH von SEQ ID NO:1 und eine VL von SEQ ID NO:2 hat, die durch eine Kontrollwirtszelle produziert wird, welche manipuliert worden ist, um einen Antikörper, der eine VH von SEQ ID NO:1 und eine VL von SEQ ID NO:2 hat, zu exprimieren,
wobei eine Wirtszelle, die in Schritt (b) identifiziert wurde, einen Anti-HER2-Antikörper oder ein Anti-HER2-Bindungsfragment in einer größeren Menge als einen Antikörper, der eine VH von SEQ ID NO:1 und eine VL von SEQ ID NO:2 hat, produziert, wodurch ein Anti-HER2-Antikörper oder ein Anti-HER2-Bindungsfragment identifiziert wird, der/das durch die Wirtszelle in einer größeren Menge produziert wird als ein Antikörper, der eine VH von SEQ ID NO:1 und eine VL von SEQ ID NO:2 hat, produziert wird.

21. Verfahren gemäß Anspruch 20, wobei:
(i) die Vielzahl von Wirtszellen wenigstens 5 Wirtszellen, wenigstens 10 Wirtszellen oder wenigstens 20 Wirtszellen ist und/oder
(ii) die Wirtszelle *E. coli*, Sf9, *S. cerevisiae* oder CHO ist.

22. Verfahren gemäß Anspruch 20 oder 21, wobei:
(i) eine Wirtszelle in Schritt (b) identifiziert wird, welche einen Anti-HER2-Antikörper oder ein Anti-HER2-Bindungsfragment in einer Menge produziert, die wenigstens 20%, wenigstens 50% oder wenigstens 100% höher ist als die eines Antikörpers, der eine VH von SEQ ID NO:1 und eine VL von SEQ ID NO:2 hat, die durch eine Wirtszelle produziert wird, die manipuliert ist, um einen Antikörper, der eine VH von SEQ ID NO:1 und eine VL von SEQ ID NO:2 hat, zu exprimieren, und/oder
(ii) die Menge an Anti-HER2-Antikörper oder Anti-HER2-Bindungsfragment, die durch eine Vielzahl von Wirtszellen in Schritt (a) produziert wird, gleichzeitig oder aufeinanderfolgend bestimmt wird.

23. Verfahren zum Bestimmen, ob ein Anti-HER2-Antikörper oder ein Anti-HER2-Bindungsfragment gemäß einem der Ansprüche 1 bis 9 im Vergleich zu einem Antikörper, der eine VH von SEQ ID NO:1 und eine VL von SEQ ID NO:2 hat, größere Stabilität aufweist, umfassend die Schritte:
(a) Bestimmen der Stabilität eines Anti-HER2-Antikörpers oder eines Anti-HER2-Bindungsfragments in einer ersten Probe;
(b) gegebenenfalls Bestimmen der Stabilität eines Antikörpers, der eine VH von SEQ ID NO:1 und eine VL von SEQ ID NO:2 hat, in der zweiten Probe und
(c) Vergleichen der Menge an Volllängen-Anti-HER2-Antikörper oder Anti-HER2-Bindungsfragment in der ersten Probe mit der Menge an Volllängen-Antikörper, der eine VH von SEQ ID NO:1 und eine VL von SEQ ID NO:2 hat, in einer zweiten Probe, wobei die zweite Probe im Wesentlichen denselben Bedingungen wie die erste Probe unterworfen wird, wodurch bestimmt wird, ob ein Anti-HER2-Antikörper oder Anti-HER2-Antikörperbindungsfragment im Vergleich zu einem Antikörper, der eine VH von SEQ ID NO:1 und eine VL von SEQ ID NO:2 hat, größere Stabilität aufweist.

## Revendications

1. Anticorps anti-HER2 ou fragment d'un tel anticorps se liant à HER2, comprenant une région VH et une région VL qui présente une ou plusieurs substitution(s) d'acide aminé ou combinaison(s) de substitutions d'acide aminé par rapport à un anticorps comportant une région VH qui comprend une séquence d'acides aminés correspondant à celle des numéros de position 1 à 117 de la Séquence N° 1 et une région VL qui comprend une séquence d'acides aminés correspondant à celle des numéros de position 1 à 103 de la Séquence N° 2, étant entendu que ladite ou lesdites substitution(s) d'acide aminé ou combinaison(s) de substitutions d'acide aminé est ou sont choisie(s) parmi les suivantes :
i) par rapport à la région VH de Séquence N° 1, substitution R83K dans FR-H3, troisième région charpente de la chaîne de VH ;
ii) par rapport à la région VL de Séquence N° 2, substitutions D28L et A34D dans CDR-L1, première région CDR de la chaîne de VL ;
iii) par rapport à la région VL de Séquence N° 2, substitution A34D dans CDR-L1, première région CDR de la chaîne de VL ;
iv) par rapport à la région VL de Séquence N° 2, substitution A34V dans CDR-L1, première région CDR de la chaîne de VL ;
v) par rapport à la région VL de Séquence N° 2, substitutions Q27S et A34D dans CDR-L1, première région CDR de la chaîne de VL ;
vi) par rapport à la région VL de Séquence N° 2, substitutions Q27L et A34V dans CDR-L1, première région CDR de la chaîne de VL ;
vii) par rapport à la région VL de Séquence N° 2, substitutions Q27F et A34D dans CDR-L1, première région CDR de la chaîne de VL ;
viii) par rapport à la région VL de Séquence N° 2, substitutions D28N et A34D dans CDR-L1, première région CDR de la chaîne de VL.

2. Anticorps anti-HER2 ou fragment d'un tel anticorps se liant à HER2, conforme à la revendication 1, dans lequel la région FR-H3 présente la substitution R83K par rapport à la région VH de Séquence N° 1.

3. Anticorps anti-HER2 ou fragment d'un tel anticorps se liant à HER2, conforme à la revendication 1, dans lequel la région CDR-L1 présente la substitution A34V par rapport à la région CDR-L1 de la région VL de Séquence N° 2.

4. Anticorps anti-HER2 ou fragment d'un tel anticorps se liant à HER2, conforme à la revendication 1, dans lequel la région CDR-L1 présente au moins l'une des combinaisons de substitutions D28N + A34D et D28L + A34D par rapport à la région CDR-L1 de la région VL de Séquence N° 2.

5. Anticorps anti-HER2 ou fragment d'un tel anticorps se liant à HER2, conforme à la revendication 1, dans lequel la région CDR-L1 présente au moins l'une des combinaisons de substitutions Q27S + A34D, Q27L + A34V et Q27F + A34D, par rapport à la région CDR-L1 de la région VL de Séquence N° 2.

6. Anticorps anti-HER2 ou fragment d'un tel anticorps se liant à HER2, conforme à la revendication 1, dans lequel, en outre, la région CDR-H3 présente la substitution W95F par rapport à la région CDR-H3 de la région VH de Séquence N° 1.

7. Anticorps anti-HER2 ou fragment d'un tel anticorps se liant à HER2, conforme à l'une des revendications 1 à 6, qui présente pour HER2 une affinité qui, déterminée au moyen d'une technique BIAcore, AlphaLISA ou FACS, vaut au moins 1,1 fois celle d'un anticorps comportant une région VH qui comprend une séquence d'acides aminés correspondant à celle des numéros de position 1 à 117 de la Séquence N° 1 et une région VL qui comprend une séquence d'acides aminés correspondant à celle des numéros de position 1 à 103 de la Séquence N° 2.

8. Anticorps anti-HER2 ou fragment d'un tel anticorps se liant à HER2, conforme à l'une des revendications précédentes, qui est :
i) un anticorps monoclonal ou un fragment se liant à HER2 d'un anticorps monoclonal, respectivement,
ii) et/ou une immunoglobuline IgG, de préférence une IgG1, une IgG2 ou une IgG4.

9. Anticorps anti-HER2 ou fragment d'un tel anticorps se liant à HER2, conforme à l'une des revendications précédentes, qui :
a) porte dans le fragment Fc une ou plusieurs mutation(s) qui font que l'activité d'ADCC augmente,
b) n'est pas fucosylé,
c) porte dans le fragment Fc une ou plusieurs mutation(s) qui font que la liaison aux récepteurs FcγR augmente,
d) porte dans le fragment Fc une ou plusieurs mutation(s) qui font que la liaison aux récepteurs FcγR diminue,
e) porte dans le fragment Fc une ou plusieurs mutation(s) qui font que la liaison aux récepteurs FcRn augmente,
f) porte dans le fragment Fc une ou plusieurs mutation(s) qui font que l'activité d'ADCC diminue,
g) et/ou est purifié, étant entendu que de préférence, l'anticorps anti-HER2 ou le fragment d'un tel anticorps se liant à HER2 est purifié jusqu'à homogénéité d'au moins 85 %, d'au moins 90 %, d'au moins 95 %, ou d'au moins 98 %.

10. Conjugué anticorps-médicament comprenant un anticorps anti-HER2, ou un fragment d'un tel anticorps se liant à HER2, conforme à l'une des revendications précédentes.

11. Composition pharmaceutique comprenant un anticorps anti-HER2, ou un fragment d'un tel anticorps se liant à HER2, conforme à l'une des revendications 1 à 9, ou un conjugué anticorps-médicament conforme à la revendication 10, et un véhicule pharmacologiquement admissible.

12. Acide nucléique comprenant une séquence de nucléotides codant un anticorps anti-HER2, ou un fragment d'un tel anticorps se liant à HER2, conforme à l'une des revendications 1 à 9.

13. Vecteur comprenant un acide nucléique conforme à la revendication 12.

14. Cellule hôte procaryote transformée avec un vecteur conforme à la revendication 13.

15. Quantité à effet thérapeutique d'un anticorps anti-HER2 ou d'un fragment d'un tel anticorps se liant à HER2, conforme à l'une des revendications 1 à 9, d'un conjugué anticorps-médicament conforme à la revendication 10, ou d'une composition pharmaceutique conforme à la revendication 11, pour utilisation dans le traitement d'un cancer ou d'une maladie de Paget mammaire ou extra-mammaire chez un humain.

16. Anticorps anti-HER2 ou fragment d'un tel anticorps se liant à HER2, conjugué anticorps-médicament ou composition pharmaceutique pour utilisation conforme à la revendication 15 dans laquelle :
i) le cancer est un cancer du sein,
ii) et/ou une surexpression de HER2 a été détectée dans ledit cancer.

17. Procédé permettant de déterminer si une cellule hôte produit un anticorps anti-HER2, ou un fragment d'un tel anticorps se liant à HER2, conforme à l'une des revendications 1 à 9, en plus grande quantité qu'un anticorps comportant une région VH de Séquence N° 1 et une région VL de Séquence N° 2, lequel procédé comporte les étapes suivantes :
a) en option, isoler l'anticorps anti-HER2 ou le fragment se liant à HER2, ou isoler et purifier l'anticorps anti-HER2 ou le fragment se liant à HER2 ;
b) déterminer la quantité d'anticorps anti-HER2 ou de fragment d'anticorps se liant à HER2 produite par une première cellule, laquelle première cellule est ladite cellule hôte modifiée pour exprimer ledit anticorps anti-HER2 ou fragment d'anticorps se liant à HER2 ;
c) en option, déterminer la quantité d'anticorps comportant une région VH de Séquence N° 1 et une région VL de Séquence N° 2 produite par une deuxième cellule ;
d) et comparer la quantité d'anticorps anti-HER2 ou de fragment se liant à HER2 produite par ladite première cellule avec la quantité d'anticorps comportant une région VH de Séquence N° 1 et une région VL de Séquence N° 2 produite par la deuxième cellule;
étant entendu que ladite deuxième cellule est ladite cellule hôte modifiée pour exprimer un anticorps comportant une région VH de Séquence N° 1 et une région VL de Séquence N° 2, de sorte qu'une production plus importante dudit anticorps anti-HER2 ou fragment d'anticorps se liant à HER2 par la première cellule, par rapport à la quantité d'anticorps comportant une région VH de Séquence N° 1 et une région VL de Séquence N° 2 produite par la deuxième cellule, indique que la cellule hôte produit ledit anticorps anti-HER2 ou fragment se liant à HER2 en plus grande quantité que l'anticorps comportant une région VH de Séquence N° 1 et une région VL de Séquence N° 2, permettant ainsi de déterminer si la cellule hôte produit ledit anticorps anti-HER2 ou fragment se liant à HER2 en plus grande quantité qu'un anticorps comportant une région VH de Séquence N° 1 et une région VL de Séquence N° 2.

18. Procédé conforme à la revendication 17, dans lequel la cellule hôte est :
i) soit une cellule procaryote, de préférence une cellule *E. coli*,
ii) soit une cellule eucaryote, la cellule hôte étant alors choisie de préférence parmi les cellules Sf9, *S. cerevisiae* et CHO.

19. Procédé conforme à la revendication 17 ou 18, dans lequel la production dudit anticorps anti-HER2 ou fragment se liant à HER2 est d'au moins 20 % plus importante que la production d'anticorps comportant une région VH de Séquence N° 1 et une région VL de Séquence N° 2 chez la cellule hôte, et dans lequel, de préférence, la production dudit anticorps anti-HER2 ou fragment se liant à HER2 est d'au moins 50 % plus importante, et mieux encore, la production dudit anticorps anti-HER2 ou fragment se liant à HER2 est d'au moins 100 % plus importante.

20. Procédé permettant d'identifier un anticorps anti-HER2 ou un fragment se liant à HER2 qui est produit par une cellule hôte en plus grande quantité qu'un anticorps comportant une région VH de Séquence N° 1 et une région VL de Séquence N° 2, lequel procédé comporte les étapes suivantes :
a) déterminer la quantité d'anticorps anti-HER2 ou de fragment se liant à HER2 produite par plusieurs cellules hôtes, dont chacune est modifiée pour exprimer un anticorps anti-HER2 ou fragment se liant à HER2 différent, conforme à l'une des revendications 1 à 11 ;
b) et identifier une cellule hôte qui produit un anticorps anti-HER2 ou un fragment se liant à HER2 en une quantité plus grande que celle en laquelle un anticorps comportant une région VH de Séquence N° 1 et une région VL de Séquence N° 2 est produit par une cellule hôte témoin modifiée pour exprimer un anticorps comportant une région VH de Séquence N° 1 et une région VL de Séquence N° 2 ;
étant entendu qu'une cellule hôte identifiée dans l'étape (b) produit un anticorps anti-HER2 ou un fragment se liant à HER2 en plus grande quantité qu'un anticorps comportant une région VH de Séquence N° 1 et une région VL de Séquence N° 2, ce qui permet d'identifier un anticorps anti-HER2 ou un fragment se liant à HER2 qui est produit en plus grande quantité que ne l'est un anticorps comportant une région VH de Séquence N° 1 et une région VL de Séquence N° 2 par ladite cellule hôte.

21. Procédé conforme à la revendication 20, dans lequel :
i) lesdites plusieurs cellules hôtes sont au moins 5 cellules hôtes, au moins 10 cellules hôtes ou au moins 20 cellules hôtes,
ii) et/ou ladite cellule hôte est une cellule *E.coli,* Sf9, *S. cerevisiae* ou CHO.

22. Procédé conforme à la revendication 20 ou 21, dans lequel :
i) est identifiée dans l'étape (b) une cellule hôte qui produit un anticorps anti-HER2 ou un fragment se liant à HER2 en une quantité qui est plus grande d'au moins 20 %, d'au moins 50 % ou d'au moins 100 % que celle en laquelle un anticorps comportant une région VH de Séquence N° 1 et une région VL de Séquence N° 2 est produit par une cellule hôte modifiée pour exprimer un anticorps comportant une région VH de Séquence N° 1 et une région VL de Séquence N° 2,
ii) et/ou dans l'étape (a), la quantité d'anticorps anti-HER2 ou de fragment se liant à HER2 produite par les plusieurs cellules hôtes est déterminée simultanément ou successivement.

23. Procédé permettant de déterminer si un anticorps anti-HER2 ou un fragment se liant à HER2, conforme à l'une des revendications 1 à 9, possède une plus grande stabilité qu'un anticorps comportant une région VH de Séquence N° 1 et une région VL de Séquence N° 2, lequel procédé comporte les étapes suivantes :
a) déterminer la stabilité d'un anticorps anti-HER2 ou d'un fragment se liant à HER2, dans un premier échantillon ;
b) en option, déterminer la stabilité d'un anticorps comportant une région VH de Séquence N° 1 et une région VL de Séquence N° 2, dans un deuxième échantillon ;
c) et comparer la quantité d'anticorps anti-HER2 entier ou de fragment se liant à HER2 entier, dans le premier échantillon, avec la quantité d'anticorps entier comportant une région VH de Séquence N° 1 et une région VL de Séquence N° 2, dans le deuxième échantillon, lequel deuxième échantillon a été soumis pratiquement aux mêmes conditions que le premier échantillon, ce qui permet de déterminer si un anticorps anti-HER2 ou un fragment d'anticorps se liant à HER2 possède une plus grande stabilité qu'un anticorps comportant une région VH de Séquence N° 1 et une région VL de Séquence N° 2.


### SEQUENCE LISTING

<110> HARDING, FIONA A. AKAMATSU, YOSHIKO DUBRIDGE, ROBERT B. POWERS, DAVID B.
<120> ANTI-HER2 ANTIBODIES AND THEIR USES
<130> 381493-236US (104690)
<140>
   <141>
<150> 61/287,155
   <151> 2009-12-16
<160> 1414
<170> PatentIn version 3.5
<210> 1
   <211> 120
   <212> PRT
   <213> Artificial Sequence
<220>
   <223> Description of Artificial Sequence: Synthetic polypeptide
<400> 1
<210> 2
   <211> 107
   <212> PRT
   <213> Artificial Sequence
<220>
   <223> Description of Artificial Sequence: Synthetic polypeptide
<400> 2
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Asp Val Asn Thr Ala
   20 25 30
   Val Ala Trp Tyr Gln Gln Lys Pro Gly Lys Ala Pro Lys Leu Leu Ile
   35 40 45
   Tyr Ser Ala Ser Phe Leu Tyr Ser Gly Val Pro Ser Arg Phe Ser Gly
   50 55 60
   Ser Arg Ser Gly Thr Asp Phe Thr Leu Thr Ile Ser Ser Leu Gln Pro
   65 70 75 80
   Glu Asp Phe Ala Thr Tyr Tyr Cys Gln Gln His Tyr Thr Thr Pro Pro
   85 90 95
   Thr Phe Gly Gln Gly Thr Lys Leu Glu Ile Lys
   100 105
<210> 3
   <211> 10
   <212> PRT
   <213> Artificial Sequence
<220>
   <223> Description of Artificial Sequence: Synthetic peptide
<400> 3
   Gly Phe Asn Ile Lys Asp Thr Tyr Ile His
   1 5 10
<210> 4
   <211> 17
   <212> PRT
   <213> Artificial Sequence
<220>
   <223> Description of Artificial Sequence: Synthetic peptide
<400> 4
   Arg Ile Tyr Pro Thr Asn Gly Tyr Thr Arg Tyr Ala Asp Ser Val Lys
   1 5 10 15
   Gly
<210> 5
   <211> 11
   <212> PRT
   <213> Artificial Sequence
<220>
   <223> Description of Artificial Sequence: Synthetic peptide
<400> 5
   Trp Gly Gly Asp Gly Phe Tyr Ala Met Asp Tyr
   1               5                   10
<210> 6
   <211> 11
   <212> PRT
   <213> Artificial Sequence
<220>
   <223> Description of Artificial Sequence: Synthetic peptide
<400> 6
   Arg Ala Ser Gln Asp Val Asn Thr Ala Val Ala
   1               5                   10
<210> 7
   <211> 7
   <212> PRT
   <213> Artificial Sequence
<220>
   <223> Description of Artificial Sequence: Synthetic peptide
<400> 7
   Ser Ala Ser Phe Leu Tyr Ser
   1               5
<210> 8
   <211> 9
   <212> PRT
   <213> Artificial Sequence
<220>
   <223> Description of Artificial Sequence: Synthetic peptide
<400> 8
   Gln Gln His Tyr Thr Thr Pro Pro Thr
   1               5
<210> 9
   <211> 15
   <212> PRT
   <213> Artificial Sequence
<220>
   <223> Description of Artificial Sequence: Synthetic peptide
<400> 9
   Asp Ile Gln Met Thr Gln Ser Pro Ser Ser Leu Ser Ala Ser Val
   1               5                   10                  15
<210> 10
   <211> 15
   <212> PRT
   <213> Artificial Sequence
<220>
   <223> Description of Artificial Sequence: Synthetic peptide
<400> 10
   Met Thr Gln Ser Pro Ser Ser Leu Ser Ala Ser Val Gly Asp Arg
   1               5                   10                  15
<210> 11
   <211> 15
   <212> PRT
   <213> Artificial Sequence
<220>
   <223> Description of Artificial Sequence: Synthetic peptide
<400> 11
   Ser Pro Ser Ser Leu Ser Ala Ser Val Gly Asp Arg Val Thr Ile
   1               5                   10                  15
<210> 12
   <211> 15
   <212> PRT
   <213> Artificial Sequence
<220>
   <223> Description of Artificial Sequence: Synthetic peptide
<400> 12
   Ser Leu Ser Ala Ser Val Gly Asp Arg Val Thr Ile Thr Cys Arg
   1               5                   10                  15
<210> 13
   <211> 15
   <212> PRT
   <213> Artificial Sequence
<220>
   <223> Description of Artificial Sequence: Synthetic peptide
<400> 13
   Ala Ser Val Gly Asp Arg Val Thr Ile Thr Cys Arg Ala Ser Gln
   1               5                   10                  15
<210> 14
   <211> 15
   <212> PRT
   <213> Artificial Sequence
<220>
   <223> Description of Artificial Sequence: Synthetic peptide
<400> 14
   Gly Asp Arg Val Thr Ile Thr Cys Arg Ala Ser Gln Asp Val Asn
   1               5                   10                  15
<210> 15
   <211> 15
   <212> PRT
   <213> Artificial Sequence
<220>
   <223> Description of Artificial Sequence: Synthetic peptide
<400> 15
   Val Thr Ile Thr Cys Arg Ala Ser Gln Asp Val Asn Thr Ala Val
   1               5                   10                  15
<210> 16
   <211> 15
   <212> PRT
   <213> Artificial Sequence
<220>
   <223> Description of Artificial Sequence: Synthetic peptide
<400> 16
   Thr Cys Arg Ala Ser Gln Asp Val Asn Thr Ala Val Ala Trp Tyr
   1               5                   10                  15
<210> 17
   <211> 15
   <212> PRT
   <213> Artificial Sequence
<220>
   <223> Description of Artificial Sequence: Synthetic peptide
<400> 17
   Ala Ser Gln Asp Val Asn Thr Ala Val Ala Trp Tyr Gln Gln Lys
   1               5                   10                  15
<210> 18
   <211> 15
   <212> PRT
   <213> Artificial Sequence
<220>
   <223> Description of Artificial Sequence: Synthetic peptide
<400> 18
   Asp Val Asn Thr Ala Val Ala Trp Tyr Gln Gln Lys Pro Gly Lys
   1               5                   10                  15
<210> 19
   <211> 15
   <212> PRT
   <213> Artificial Sequence
<220>
   <223> Description of Artificial Sequence: Synthetic peptide
<400> 19
   Thr Ala Val Ala Trp Tyr Gln Gln Lys Pro Gly Lys Ala Pro Lys
   1               5                   10                  15
<210> 20
   <211> 15
   <212> PRT
   <213> Artificial Sequence
<220>
   <223> Description of Artificial Sequence: Synthetic peptide
<400> 20
   Ala Trp Tyr Gln Gln Lys Pro Gly Lys Ala Pro Lys Leu Leu Ile
   1               5                   10                  15
<210> 21
   <211> 15
   <212> PRT
   <213> Artificial Sequence
<220>
   <223> Description of Artificial Sequence: Synthetic peptide
<400> 21
   Gln Gln Lys Pro Gly Lys Ala Pro Lys Leu Leu Ile Tyr Ser Ala
   1               5                   10                  15
<210> 22
   <211> 15
   <212> PRT
   <213> Artificial Sequence
<220>
   <223> Description of Artificial Sequence: Synthetic peptide
<400> 22
   Pro Gly Lys Ala Pro Lys Leu Leu Ile Tyr Ser Ala Ser Phe Leu
   1               5                   10                  15
<210> 23
   <211> 15
   <212> PRT
   <213> Artificial Sequence
<220>
   <223> Description of Artificial Sequence: Synthetic peptide
<400> 23
   Ala Pro Lys Leu Leu Ile Tyr Ser Ala Ser Phe Leu Tyr Ser Gly
   1               5                   10                  15
<210> 24
   <211> 15
   <212> PRT
   <213> Artificial Sequence
<220>
   <223> Description of Artificial Sequence: Synthetic peptide
<400> 24
   Leu Leu Ile Tyr Ser Ala Ser Phe Leu Tyr Ser Gly Val Pro Ser
   1               5                   10                  15
<210> 25
   <211> 15
   <212> PRT
   <213> Artificial Sequence
<220>
   <223> Description of Artificial Sequence: Synthetic peptide
<400> 25
   Tyr Ser Ala Ser Phe Leu Tyr Ser Gly Val Pro Ser Arg Phe Ser
   1               5                   10                  15
<210> 26
   <211> 15
   <212> PRT
   <213> Artificial Sequence
<220>
   <223> Description of Artificial Sequence: Synthetic peptide
<400> 26
   Ser Phe Leu Tyr Ser Gly Val Pro Ser Arg Phe Ser Gly Ser Arg
   1               5                   10                  15
<210> 27
   <211> 15
   <212> PRT
   <213> Artificial Sequence
<220>
   <223> Description of Artificial Sequence: Synthetic peptide
<400> 27
   Tyr Ser Gly Val Pro Ser Arg Phe Ser Gly Ser Arg Ser Gly Thr
   1               5                   10                  15
<210> 28
   <211> 15
   <212> PRT
   <213> Artificial Sequence
<220>
   <223> Description of Artificial Sequence: Synthetic peptide
<400> 28
   Val Pro Ser Arg Phe Ser Gly Ser Arg Ser Gly Thr Asp Phe Thr
   1               5                   10                  15
<210> 29
   <211> 15
   <212> PRT
   <213> Artificial Sequence
<220>
   <223> Description of Artificial Sequence: Synthetic peptide
<400> 29
   Arg Phe Ser Gly Ser Arg Ser Gly Thr Asp Phe Thr Leu Thr Ile
   1               5                   10                  15
<210> 30
   <211> 15
   <212> PRT
   <213> Artificial Sequence
<220>
   <223> Description of Artificial Sequence: Synthetic peptide
<400> 30
   Gly Ser Arg Ser Gly Thr Asp Phe Thr Leu Thr Ile Ser Ser Leu
   1               5                   10                  15
<210> 31
   <211> 15
   <212> PRT
   <213> Artificial Sequence
<220>
   <223> Description of Artificial Sequence: Synthetic peptide
<400> 31
   Ser Gly Thr Asp Phe Thr Leu Thr Ile Ser Ser Leu Gln Pro Glu
   1               5                   10                  15
<210> 32
   <211> 15
   <212> PRT
   <213> Artificial Sequence
<220>
   <223> Description of Artificial Sequence: Synthetic peptide
<400> 32
   Asp Phe Thr Leu Thr Ile Ser Ser Leu Gln Pro Glu Asp Phe Ala
   1               5                   10                  15
<210> 33
   <211> 15
   <212> PRT
   <213> Artificial Sequence
<220>
   <223> Description of Artificial Sequence: Synthetic peptide
<400> 33
   Leu Thr Ile Ser Ser Leu Gln Pro Glu Asp Phe Ala Thr Tyr Tyr
   1               5                   10                  15
<210> 34
   <211> 15
   <212> PRT
   <213> Artificial Sequence
<220>
   <223> Description of Artificial Sequence: Synthetic peptide
<400> 34
   Ser Ser Leu Gln Pro Glu Asp Phe Ala Thr Tyr Tyr Cys Gln Gln
   1               5                   10                  15
<210> 35
   <211> 15
   <212> PRT
   <213> Artificial Sequence
<220>
   <223> Description of Artificial Sequence: Synthetic peptide
<400> 35
   Gln Pro Glu Asp Phe Ala Thr Tyr Tyr Cys Gln Gln His Tyr Thr
   1               5                   10                  15
<210> 36
   <211> 15
   <212> PRT
   <213> Artificial Sequence
<220>
   <223> Description of Artificial Sequence: Synthetic peptide
<400> 36
   Asp Phe Ala Thr Tyr Tyr Cys Gln Gln His Tyr Thr Thr Pro Pro
   1               5                   10                  15
<210> 37
   <211> 15
   <212> PRT
   <213> Artificial Sequence
<220>
   <223> Description of Artificial Sequence: Synthetic peptide
<400> 37
   Thr Tyr Tyr Cys Gln Gln His Tyr Thr Thr Pro Pro Thr Phe Gly
   1               5                   10                  15
<210> 38
   <211> 15
   <212> PRT
   <213> Artificial Sequence
<220>
   <223> Description of Artificial Sequence: Synthetic peptide
<400> 38
   Cys Gln Gln His Tyr Thr Thr Pro Pro Thr Phe Gly Gln Gly Thr
   1               5                   10                  15
<210> 39
   <211> 15
   <212> PRT
   <213> Artificial Sequence
<220>
   <223> Description of Artificial Sequence: Synthetic peptide
<400> 39
   His Tyr Thr Thr Pro Pro Thr Phe Gly Gln Gly Thr Lys Val Glu
   1               5                   10                  15
<210> 40
   <211> 15
   <212> PRT
   <213> Artificial Sequence
<220>
   <223> Description of Artificial Sequence: Synthetic peptide
<400> 40
   Thr Pro Pro Thr Phe Gly Gln Gly Thr Lys Val Glu Ile Lys Arg
   1               5                   10                  15
<210> 41
   <211> 15
   <212> PRT
   <213> Artificial Sequence
<220>
   <223> Description of Artificial Sequence: Synthetic peptide
<400> 41
   Glu Val Gln Leu Val Glu Ser Gly Gly Gly Leu Val Gln Pro Gly
   1               5                   10                  15
<210> 42
   <211> 15
   <212> PRT
   <213> Artificial Sequence
<220>
   <223> Description of Artificial Sequence: Synthetic peptide
<400> 42
   Leu Val Glu Ser Gly Gly Gly Leu Val Gln Pro Gly Gly Ser Leu
   1               5                   10                  15
<210> 43
   <211> 15
   <212> PRT
   <213> Artificial Sequence
<220>
   <223> Description of Artificial Sequence: Synthetic peptide
<400> 43
   Ser Gly Gly Gly Leu Val Gln Pro Gly Gly Ser Leu Arg Leu Ser
   1               5                   10                  15
<210> 44
   <211> 15
   <212> PRT
   <213> Artificial Sequence
<220>
   <223> Description of Artificial Sequence: Synthetic peptide
<400> 44
   Gly Leu Val Gln Pro Gly Gly Ser Leu Arg Leu Ser Cys Ala Ala
   1               5                   10                  15
<210> 45
   <211> 15
   <212> PRT
   <213> Artificial Sequence
<220>
   <223> Description of Artificial Sequence: Synthetic peptide
<400> 45
   Gln Pro Gly Gly Ser Leu Arg Leu Ser Cys Ala Ala Ser Gly Phe
   1 5 10 15
<210> 46
   <211> 15
   <212> PRT
   <213> Artificial Sequence
<220>
   <223> Description of Artificial Sequence: Synthetic peptide
<400> 46
   Gly Ser Leu Arg Leu Ser Cys Ala Ala Ser Gly Phe Asn Ile Lys
   1 5 10 15
<210> 47
   <211> 15
   <212> PRT
   <213> Artificial Sequence
<220>
   <223> Description of Artificial Sequence: Synthetic peptide
<400> 47
   Arg Leu Ser Cys Ala Ala Ser Gly Phe Asn Ile Lys Asp Thr Tyr
   1 5 10 15
<210> 48
   <211> 15
   <212> PRT
   <213> Artificial Sequence
<220>
   <223> Description of Artificial Sequence: Synthetic peptide
<400> 48
   Cys Ala Ala Ser Gly Phe Asn Ile Lys Asp Thr Tyr Ile His Trp
   1 5 10 15
<210> 49
   <211> 15
   <212> PRT
   <213> Artificial Sequence
<220>
   <223> Description of Artificial Sequence: Synthetic peptide
<400> 49
   Ser Gly Phe Asn Ile Lys Asp Thr Tyr Ile His Trp Val Arg Gln
   1 5 10 15
<210> 50
   <211> 15
   <212> PRT
   <213> Artificial Sequence
<220>
   <223> Description of Artificial Sequence: Synthetic peptide
<400> 50
   Asn Ile Lys Asp Thr Tyr Ile His Trp Val Arg Gln Ala Pro Gly
   1               5                   10                  15
<210> 51
   <211> 15
   <212> PRT
   <213> Artificial Sequence
<220>
   <223> Description of Artificial Sequence: Synthetic peptide
<400> 51
   Asp Thr Tyr Ile His Trp Val Arg Gln Ala Pro Gly Lys Gly Leu
   1               5                   10                  15
<210> 52
   <211> 15
   <212> PRT
   <213> Artificial Sequence
<220>
   <223> Description of Artificial Sequence: Synthetic peptide
<400> 52
   Ile His Trp Val Arg Gln Ala Pro Gly Lys Gly Leu Glu Trp Val
   1               5                   10                  15
<210> 53
   <211> 15
   <212> PRT
   <213> Artificial Sequence
<220>
   <223> Description of Artificial Sequence: Synthetic peptide
<400> 53
   Val Arg Gln Ala Pro Gly Lys Gly Leu Glu Trp Val Ala Arg Ile
   1               5                   10                  15
<210> 54
   <211> 15
   <212> PRT
   <213> Artificial Sequence
<220>
   <223> Description of Artificial Sequence: Synthetic peptide
<400> 54
   Ala Pro Gly Lys Gly Leu Glu Trp Val Ala Arg Ile Tyr Pro Thr
   1               5                   10                  15
<210> 55
   <211> 15
   <212> PRT
   <213> Artificial Sequence
<220>
   <223> Description of Artificial Sequence: Synthetic peptide
<400> 55
   Lys Gly Leu Glu Trp Val Ala Arg Ile Tyr Pro Thr Asn Gly Tyr
   1               5                   10                  15
<210> 56
   <211> 15
   <212> PRT
   <213> Artificial Sequence
<220>
   <223> Description of Artificial Sequence: Synthetic peptide
<400> 56
   Glu Trp Val Ala Arg Ile Tyr Pro Thr Asn Gly Tyr Thr Arg Tyr
   1               5                   10                  15
<210> 57
   <211> 15
   <212> PRT
   <213> Artificial Sequence
<220>
   <223> Description of Artificial Sequence: Synthetic peptide
<400> 57
   Ala Arg Ile Tyr Pro Thr Asn Gly Tyr Thr Arg Tyr Ala Asp Ser
   1               5                   10                  15
<210> 58
   <211> 15
   <212> PRT
   <213> Artificial Sequence
<220>
   <223> Description of Artificial Sequence: Synthetic peptide
<400> 58
   Tyr Pro Thr Asn Gly Tyr Thr Arg Tyr Ala Asp Ser Val Lys Gly
   1               5                   10                  15
<210> 59
   <211> 15
   <212> PRT
   <213> Artificial Sequence
<220>
   <223> Description of Artificial Sequence: Synthetic peptide
<400> 59
   Asn Gly Tyr Thr Arg Tyr Ala Asp Ser Val Lys Gly Arg Phe Thr
   1               5                   10                  15
<210> 60
   <211> 15
   <212> PRT
   <213> Artificial Sequence
<220>
   <223> Description of Artificial Sequence: Synthetic peptide
<400> 60
   Thr Arg Tyr Ala Asp Ser Val Lys Gly Arg Phe Thr Ile Ser Ala
   1               5                   10                  15
<210> 61
   <211> 15
   <212> PRT
   <213> Artificial Sequence
<220>
   <223> Description of Artificial Sequence: Synthetic peptide
<400> 61
   Ala Asp Ser Val Lys Gly Arg Phe Thr Ile Ser Ala Asp Thr Ser
   1               5                   10                  15
<210> 62
   <211> 15
   <212> PRT
   <213> Artificial Sequence
<220>
   <223> Description of Artificial Sequence: Synthetic peptide
<400> 62
   Val Lys Gly Arg Phe Thr Ile Ser Ala Asp Thr Ser Lys Asn Thr
   1               5                   10                  15
<210> 63
   <211> 15
   <212> PRT
   <213> Artificial Sequence
<220>
   <223> Description of Artificial Sequence: Synthetic peptide
<400> 63
   Arg Phe Thr Ile Ser Ala Asp Thr Ser Lys Asn Thr Ala Tyr Leu
   1 5 10 15
<210> 64
   <211> 15
   <212> PRT
   <213> Artificial Sequence
<220>
   <223> Description of Artificial Sequence: Synthetic peptide
<400> 64
   Ile Ser Ala Asp Thr Ser Lys Asn Thr Ala Tyr Leu Gln Met Asn
   1 5 10 15
<210> 65
   <211> 15
   <212> PRT
   <213> Artificial Sequence
<220>
   <223> Description of Artificial Sequence: Synthetic peptide
<400> 65
   Asp Thr Ser Lys Asn Thr Ala Tyr Leu Gln Met Asn Ser Leu Arg
   1 5 10 15
<210> 66
   <211> 15
   <212> PRT
   <213> Artificial Sequence
<220>
   <223> Description of Artificial Sequence: Synthetic peptide
<400> 66
   Lys Asn Thr Ala Tyr Leu Gln Met Asn Ser Leu Arg Ala Glu Asp
   1 5 10 15
<210> 67
   <211> 15
   <212> PRT
   <213> Artificial Sequence
<220>
   <223> Description of Artificial Sequence: Synthetic peptide
<400> 67
   Ala Tyr Leu Gln Met Asn Ser Leu Arg Ala Glu Asp Thr Ala Val
   1 5 10 15
<210> 68
   <211> 15
   <212> PRT
   <213> Artificial Sequence
<220>
   <223> Description of Artificial Sequence: Synthetic peptide
<400> 68
   Gln Met Asn Ser Leu Arg Ala Glu Asp Thr Ala Val Tyr Tyr Cys
   1               5                   10                  15
<210> 69
   <211> 15
   <212> PRT
   <213> Artificial Sequence
<220>
   <223> Description of Artificial Sequence: Synthetic peptide
<400> 69
   Ser Leu Arg Ala Glu Asp Thr Ala Val Tyr Tyr Cys Ser Arg Trp
   1               5                   10                  15
<210> 70
   <211> 15
   <212> PRT
   <213> Artificial Sequence
<220>
   <223> Description of Artificial Sequence: Synthetic peptide
<400> 70
   Ala Glu Asp Thr Ala Val Tyr Tyr Cys Ser Arg Trp Gly Gly Asp
   1               5                   10                  15
<210> 71
   <211> 15
   <212> PRT
   <213> Artificial Sequence
<220>
   <223> Description of Artificial Sequence: Synthetic peptide
<400> 71
   Thr Ala Val Tyr Tyr Cys Ser Arg Trp Gly Gly Asp Gly Phe Tyr
   1               5                   10                  15
<210> 72
   <211> 15
   <212> PRT
   <213> Artificial Sequence
<220>
   <223> Description of Artificial Sequence: Synthetic peptide
<400> 72
   Tyr Tyr Cys Ser Arg Trp Gly Gly Asp Gly Phe Tyr Ala Met Asp
   1               5                   10                  15
<210> 73
   <211> 15
   <212> PRT
   <213> Artificial Sequence
<220>
   <223> Description of Artificial Sequence: Synthetic peptide
<400> 73
   Ser Arg Trp Gly Gly Asp Gly Phe Tyr Ala Met Asp Tyr Trp Gly
   1               5                   10                  15
<210> 74
   <211> 15
   <212> PRT
   <213> Artificial Sequence
<220>
   <223> Description of Artificial Sequence: Synthetic peptide
<400> 74
   Gly Gly Asp Gly Phe Tyr Ala Met Asp Tyr Trp Gly Gln Gly Thr
   1               5                   10                  15
<210> 75
   <211> 15
   <212> PRT
   <213> Artificial Sequence
<220>
   <223> Description of Artificial Sequence: Synthetic peptide
<400> 75
   Gly Phe Tyr Ala Met Asp Tyr Trp Gly Gln Gly Thr Leu Val Thr
   1               5                   10                  15
<210> 76
   <211> 15
   <212> PRT
   <213> Artificial Sequence
<220>
   <223> Description of Artificial Sequence: Synthetic peptide
<400> 76
   Ala Met Asp Tyr Trp Gly Gln Gly Thr Leu Val Thr Val Ser Ser
   1 5 10 15
<210> 77
   <211> 25
   <212> PRT
   <213> Artificial Sequence
<220>
   <223> Description of Artificial Sequence: Synthetic peptide
<400> 77 Ser Leu Arg Leu Ser Cys Ala Ala Ser 20 25
<210> 78
   <211> 14
   <212> PRT
   <213> Artificial Sequence
<220>
   <223> Description of Artificial Sequence: Synthetic peptide
<400> 78
   Trp Val Arg Gln Ala Pro Gly Lys Gly Leu Glu Trp Val Ala
   1 5 10
<210> 79
   <211> 32
   <212> PRT
   <213> Artificial Sequence
<220>
   <223> Description of Artificial Sequence: Synthetic polypeptide
<400> 79 Met Asn Ser Leu Arg Ala Glu Asp Thr Ala Val Tyr Tyr Cys Ser Arg 20 25 30
<210> 80
   <211> 11
   <212> PRT
   <213> Artificial Sequence
<220>
   <223> Description of Artificial Sequence: Synthetic peptide
<400> 80
   Trp Gly Gln Gly Thr Leu Val Thr Val Ser Ser
   1 5 10
<210> 81
   <211> 23
   <212> PRT
   <213> Artificial Sequence
<220>
   <223> Description of Artificial Sequence: Synthetic peptide
<400> 81 Asp Arg Val Thr Ile Thr Cys 20
<210> 82
   <211> 15
   <212> PRT
   <213> Artificial Sequence
<220>
   <223> Description of Artificial Sequence: Synthetic peptide
<400> 82
   Trp Tyr Gln Gln Lys Pro Gly Lys Ala Pro Lys Leu Leu Ile Tyr
   1 5 10 15
<210> 83
   <211> 32
   <212> PRT
   <213> Artificial Sequence
<220>
   <223> Description of Artificial Sequence: Synthetic polypeptide
<400> 83
   Gly Val Pro Ser Arg Phe Ser Gly Ser Arg Ser Gly Thr Asp Phe Thr
   1 5 10 15
   Leu Thr Ile Ser Ser Leu Gln Pro Glu Asp Phe Ala Thr Tyr Tyr Cys
   20 25 30
<210> 84
   <211> 10
   <212> PRT
   <213> Artificial Sequence
<220>
   <223> Description of Artificial Sequence: Synthetic peptide
<400> 84
   Phe Gly Gln Gly Thr Lys Leu Glu Ile Lys
   1 5 10
<210> 85
   <211> 15
   <212> PRT
   <213> Artificial Sequence
<220>
   <223> Description of Artificial Sequence: Synthetic peptide
<400> 85
   Gly Gly Gly Gly Ser Gly Gly Gly Gly Ser Gly Gly Gly Gly Ser
   1 5 10 15
<210> 86
   <211> 10
   <212> PRT
   <213> Artificial Sequence
<220>
   <223> Description of Artificial Sequence: Synthetic peptide
<400> 86
   Gly Phe Asn Ile Lys Leu Thr Tyr Ile His
   1 5 10
<210> 87
   <211> 10
   <212> PRT
   <213> Artificial Sequence
<220>
   <223> Description of Artificial Sequence: Synthetic peptide
<400> 87
   Gly Phe Asn Ile Lys Arg Thr Tyr Ile His
   1 5 10
<210> 88
   <211> 10
   <212> PRT
   <213> Artificial Sequence
<220>
   <223> Description of Artificial Sequence: Synthetic peptide
<400> 88
   Gly Phe Asn Ile Lys Val Thr Tyr Ile His
   1 5 10
<210> 89
   <211> 10
   <212> PRT
   <213> Artificial Sequence
<220>
   <223> Description of Artificial Sequence: Synthetic peptide
<400> 89
   Gly Phe Asn Ile Lys Asp Lys Tyr Ile His
   1               5                   10
<210> 90
   <211> 10
   <212> PRT
   <213> Artificial Sequence
<220>
   <223> Description of Artificial Sequence: Synthetic peptide
<400> 90
   Gly Phe Asn Ile Lys Asp Arg Tyr Ile His
   1               5                   10
<210> 91
   <211> 10
   <212> PRT
   <213> Artificial Sequence
<220>
   <223> Description of Artificial Sequence: Synthetic peptide
<400> 91
   Gly Phe Asn Ile Lys Asp Thr Tyr Asp His
   1               5                   10
<210> 92
   <211> 17
   <212> PRT
   <213> Artificial Sequence
<220>
   <223> Description of Artificial Sequence: Synthetic peptide
<400> 92
   Arg Ile Tyr Pro Leu Asn Gly Tyr Thr Arg Tyr Ala Asp Ser Val Lys
   1               5                   10                  15
   Gly
<210> 93
   <211> 17
   <212> PRT
   <213> Artificial Sequence
<220>
   <223> Description of Artificial Sequence: Synthetic peptide
<400> 93
   Arg Ile Tyr Pro Arg Asn Gly Tyr Thr Arg Tyr Ala Asp Ser Val Lys
   1               5                   10                  15
   Gly
<210> 94
   <211> 17
   <212> PRT
   <213> Artificial Sequence
<220>
   <223> Description of Artificial Sequence: Synthetic peptide
<400> 94
   Arg Ile Tyr Pro Thr Asn Gly Tyr Thr Arg Tyr Ala Asp Ser Val Arg
   1               5                   10                  15
   Gly
<210> 95
   <211> 17
   <212> PRT
   <213> Artificial Sequence
<220>
   <223> Description of Artificial Sequence: Synthetic peptide
<400> 95
   Arg Ile Tyr Pro Thr Asn Gly Tyr Thr Arg Tyr Ala Asp Ser Val Ser
   1               5                   10                  15
   Gly
<210> 96
   <211> 11
   <212> PRT
   <213> Artificial Sequence
<220>
   <223> Description of Artificial Sequence: Synthetic peptide
<400> 96
   Arg Ala Ser Leu Asp Val Asn Thr Ala Val Ala
   1               5                   10
<210> 97
   <211> 11
   <212> PRT
   <213> Artificial Sequence
<220>
   <223> Description of Artificial Sequence: Synthetic peptide
<400> 97
   Arg Ala Ser Val Asp Val Asn Thr Ala Val Ala
   1               5                   10
<210> 98
   <211> 11
   <212> PRT
   <213> Artificial Sequence
<220>
   <223> Description of Artificial Sequence: Synthetic peptide
<400> 98
   Arg Ala Ser Ile Asp Val Asn Thr Ala Val Ala
   1               5                   10
<210> 99
   <211> 11
   <212> PRT
   <213> Artificial Sequence
<220>
   <223> Description of Artificial Sequence: Synthetic peptide
<400> 99
   Arg Ala Ser Tyr Asp Val Asn Thr Ala Val Ala
   1               5                   10
<210> 100
   <211> 11
   <212> PRT
   <213> Artificial Sequence
<220>
   <223> Description of Artificial Sequence: Synthetic peptide
<400> 100
   Arg Ala Ser Gln Leu Val Asn Thr Ala Val Ala
   1               5                   10
<210> 101
   <211> 11
   <212> PRT
   <213> Artificial Sequence
<220>
   <223> Description of Artificial Sequence: Synthetic peptide
<400> 101
   Arg Ala Ser Gln Ala Val Asn Thr Ala Val Ala
   1 5 10
<210> 102
   <211> 11
   <212> PRT
   <213> Artificial Sequence
<220>
   <223> Description of Artificial Sequence: Synthetic peptide
<400> 102
   Arg Ala Ser Gln Met Val Asn Thr Ala Val Ala
   1 5 10
<210> 103
   <211> 11
   <212> PRT
   <213> Artificial Sequence
<220>
   <223> Description of Artificial Sequence: Synthetic peptide
<400> 103
   Arg Ala Ser Gln Trp Val Asn Thr Ala Val Ala
   1 5 10
<210> 104
   <211> 11
   <212> PRT
   <213> Artificial Sequence
<220>
   <223> Description of Artificial Sequence: Synthetic peptide
<400> 104
   Arg Ala Ser Gln Asp Val Asn Thr Ala Val Val
   1 5 10
<210> 105
   <211> 11
   <212> PRT
   <213> Artificial Sequence
<220>
   <223> Description of Artificial Sequence: Synthetic peptide
<400> 105
   Arg Ala Ser Gln Asp Val Asn Thr Ala Val Gly
   1 5 10
<210> 106
   <211> 11
   <212> PRT
   <213> Artificial Sequence
<220>
   <223> Description of Artificial Sequence: Synthetic peptide
<400> 106
   Arg Ala Ser Gln Asp Val Asn Thr Ala Val Asp
   1               5                   10
<210> 107
   <211> 11
   <212> PRT
   <213> Artificial Sequence
<220>
   <223> Description of Artificial Sequence: Synthetic peptide
<400> 107
   Arg Ala Ser Gln Asp Val Asn Thr Ala Val Ser
   1               5                   10
<210> 108
   <211> 11
   <212> PRT
   <213> Artificial Sequence
<220>
   <223> Description of Artificial Sequence: Synthetic peptide
<400> 108
   Arg Ala Ser Gln Asp Val Asn Thr Ala Val Glu
   1               5                   10
<210> 109
   <211> 10
   <212> PRT
   <213> Artificial Sequence
<220>
   <223> Description of Artificial Sequence: Synthetic peptide
<400> 109
   Gly Phe Ser Ile Lys Asp Thr Tyr Ile His
   1               5                   10
<210> 110
   <211> 10
   <212> PRT
   <213> Artificial Sequence
<220>
   <223> Description of Artificial Sequence: Synthetic peptide
<400> 110
   Gly Phe Trp Ile Lys Asp Thr Tyr Ile His
   1 5 10
<210> 111
   <211> 10
   <212> PRT
   <213> Artificial Sequence
<220>
   <223> Description of Artificial Sequence: Synthetic peptide
<400> 111
   Gly Phe Asn Ile Trp Asp Thr Tyr Ile His
   1 5 10
<210> 112
   <211> 10
   <212> PRT
   <213> Artificial Sequence
<220>
   <223> Description of Artificial Sequence: Synthetic peptide
<400> 112
   Gly Phe Asn Ile Ala Asp Thr Tyr Ile His
   1 5 10
<210> 113
   <211> 10
   <212> PRT
   <213> Artificial Sequence
<220>
   <223> Description of Artificial Sequence: Synthetic peptide
<400> 113
   Gly Phe Asn Ile Lys Trp Thr Tyr Ile His
   1 5 10
<210> 114
   <211> 10
   <212> PRT
   <213> Artificial Sequence
<220>
   <223> Description of Artificial Sequence: Synthetic peptide
<400> 114
   Gly Phe Asn Ile Lys Ala Thr Tyr Ile His
   1 5 10
<210> 115
   <211> 10
   <212> PRT
   <213> Artificial Sequence
<220>
   <223> Description of Artificial Sequence: Synthetic peptide
<400> 115
   Gly Phe Asn Ile Lys Gly Thr Tyr Ile His
   1               5                   10
<210> 116
   <211> 10
   <212> PRT
   <213> Artificial Sequence
<220>
   <223> Description of Artificial Sequence: Synthetic peptide
<400> 116
   Gly Phe Asn Ile Lys Ser Thr Tyr Ile His
   1               5                   10
<210> 117
   <211> 17
   <212> PRT
   <213> Artificial Sequence
<220>
   <223> Description of Artificial Sequence: Synthetic peptide
<400> 117
   Arg Ile Arg Pro Thr Asn Gly Tyr Thr Arg Tyr Ala Asp Ser Val Lys
   1               5                   10                  15
   Gly
<210> 118
   <211> 17
   <212> PRT
   <213> Artificial Sequence
<220>
   <223> Description of Artificial Sequence: Synthetic peptide
<400> 118
   Arg Ile Ser Pro Thr Asn Gly Tyr Thr Arg Tyr Ala Asp Ser Val Lys
   1               5                   10                  15
   Gly
<210> 119
   <211> 17
   <212> PRT
   <213> Artificial Sequence
<220>
   <223> Description of Artificial Sequence: Synthetic peptide
<400> 119
   Arg Ile Tyr Pro Ser Asn Gly Tyr Thr Arg Tyr Ala Asp Ser Val Lys
   1               5                   10                  15
   Gly
<210> 120
   <211> 17
   <212> PRT
   <213> Artificial Sequence
<220>
   <223> Description of Artificial Sequence: Synthetic peptide
<400> 120
   Arg Ile Tyr Pro Lys Asn Gly Tyr Thr Arg Tyr Ala Asp Ser Val Lys
   1               5                   10                  15
   Gly
<210> 121
   <211> 17
   <212> PRT
   <213> Artificial Sequence
<220>
   <223> Description of Artificial Sequence: Synthetic peptide
<400> 121
   Arg Ile Tyr Pro Gly Asn Gly Tyr Thr Arg Tyr Ala Asp Ser Val Lys
   1               5                   10                  15
   Gly
<210> 122
   <211> 17
   <212> PRT
   <213> Artificial Sequence
<220>
   <223> Description of Artificial Sequence: Synthetic peptide
<400> 122
   Arg Ile Tyr Pro Thr Ser Gly Tyr Thr Arg Tyr Ala Asp Ser Val Lys
   1 5 10 15
   Gly
<210> 123
   <211> 17
   <212> PRT
   <213> Artificial Sequence
<220>
   <223> Description of Artificial Sequence: Synthetic peptide
<400> 123
   Arg Ile Tyr Pro Thr Asn Gly Tyr Ser Arg Tyr Ala Asp Ser Val Lys
   1 5 10 15
   Gly
<210> 124
   <211> 17
   <212> PRT
   <213> Artificial Sequence
<220>
   <223> Description of Artificial Sequence: Synthetic peptide
<400> 124
   Arg Ile Tyr Pro Thr Asn Gly Tyr Thr Arg Tyr Ala Asp Arg Val Lys
   1 5 10 15
   Gly
<210> 125
   <211> 17
   <212> PRT
   <213> Artificial Sequence
<220>
   <223> Description of Artificial Sequence: Synthetic peptide
<400> 125
   Arg Ile Tyr Pro Thr Asn Gly Tyr Thr Arg Tyr Ala Asp Ser Val Gln
   1 5 10 15
   Gly
<210> 126
   <211> 11
   <212> PRT
   <213> Artificial Sequence
<220>
   <223> Description of Artificial Sequence: Synthetic peptide
<400> 126
   Tyr Gly Gly Asp Gly Phe Tyr Ala Met Asp Tyr
   1               5                   10
<210> 127
   <211> 11
   <212> PRT
   <213> Artificial Sequence
<220>
   <223> Description of Artificial Sequence: Synthetic peptide
<400> 127
   Phe Gly Gly Asp Gly Phe Tyr Ala Met Asp Tyr
   1               5                   10
<210> 128
   <211> 11
   <212> PRT
   <213> Artificial Sequence
<220>
   <223> Description of Artificial Sequence: Synthetic peptide
<400> 128
   Trp Gly Arg Asp Gly Phe Tyr Ala Met Asp Tyr
   1               5                   10
<210> 129
   <211> 11
   <212> PRT
   <213> Artificial Sequence
<220>
   <223> Description of Artificial Sequence: Synthetic peptide
<400> 129
   Trp Gly Thr Asp Gly Phe Tyr Ala Met Asp Tyr
   1               5                   10
<210> 130
   <211> 11
   <212> PRT
   <213> Artificial Sequence
<220>
   <223> Description of Artificial Sequence: Synthetic peptide
<400> 130
   Trp Gly Gly Gly Gly Phe Tyr Ala Met Asp Tyr
   1 5 10
<210> 131
   <211> 11
   <212> PRT
   <213> Artificial Sequence
<220>
   <223> Description of Artificial Sequence: Synthetic peptide
<400> 131
   Trp Gly Gly Thr Gly Phe Tyr Ala Met Asp Tyr
   1 5 10
<210> 132
   <211> 11
   <212> PRT
   <213> Artificial Sequence
<220>
   <223> Description of Artificial Sequence: Synthetic peptide
<400> 132
   Trp Gly Gly Ala Gly Phe Tyr Ala Met Asp Tyr
   1 5 10
<210> 133
   <211> 11
   <212> PRT
   <213> Artificial Sequence
<220>
   <223> Description of Artificial Sequence: Synthetic peptide
<400> 133
   Trp Gly Gly Arg Gly Phe Tyr Ala Met Asp Tyr
   1 5 10
<210> 134
   <211> 11
   <212> PRT
   <213> Artificial Sequence
<220>
   <223> Description of Artificial Sequence: Synthetic peptide
<400> 134
   Trp Gly Gly Gln Gly Phe Tyr Ala Met Asp Tyr
   1 5 10
<210> 135
   <211> 11
   <212> PRT
   <213> Artificial Sequence
<220>
   <223> Description of Artificial Sequence: Synthetic peptide
<400> 135
   Trp Gly Gly Glu Gly Phe Tyr Ala Met Asp Tyr
   1               5                   10
<210> 136
   <211> 11
   <212> PRT
   <213> Artificial Sequence
<220>
   <223> Description of Artificial Sequence: Synthetic peptide
<400> 136
   Trp Gly Gly Ser Gly Phe Tyr Ala Met Asp Tyr
   1               5                   10
<210> 137
   <211> 11
   <212> PRT
   <213> Artificial Sequence
<220>
   <223> Description of Artificial Sequence: Synthetic peptide
<400> 137
   Trp Gly Gly Tyr Gly Phe Tyr Ala Met Asp Tyr
   1               5                   10
<210> 138
   <211> 11
   <212> PRT
   <213> Artificial Sequence
<220>
   <223> Description of Artificial Sequence: Synthetic peptide
<400> 138
   Trp Gly Gly Phe Gly Phe Tyr Ala Met Asp Tyr
   1               5                   10
<210> 139
   <211> 11
   <212> PRT
   <213> Artificial Sequence
<220>
   <223> Description of Artificial Sequence: Synthetic peptide
<400> 139
   Trp Gly Gly Leu Gly Phe Tyr Ala Met Asp Tyr
   1 5 10
<210> 140
   <211> 11
   <212> PRT
   <213> Artificial Sequence
<220>
   <223> Description of Artificial Sequence: Synthetic peptide
<400> 140
   Trp Gly Gly Pro Gly Phe Tyr Ala Met Asp Tyr
   1 5 10
<210> 141
   <211> 11
   <212> PRT
   <213> Artificial Sequence
<220>
   <223> Description of Artificial Sequence: Synthetic peptide
<400> 141
   Trp Gly Gly His Gly Phe Tyr Ala Met Asp Tyr
   1 5 10
<210> 142
   <211> 11
   <212> PRT
   <213> Artificial Sequence
<220>
   <223> Description of Artificial Sequence: Synthetic peptide
<400> 142
   Trp Gly Gly Asp Gly Phe Tyr Val Met Asp Tyr
   1 5 10
<210> 143
   <211> 11
   <212> PRT
   <213> Artificial Sequence
<220>
   <223> Description of Artificial Sequence: Synthetic peptide
<400> 143
   Trp Gly Gly Asp Gly Phe Tyr Ser Met Asp Tyr
   1 5 10
<210> 144
   <211> 11
   <212> PRT
   <213> Artificial Sequence
<220>
   <223> Description of Artificial Sequence: Synthetic peptide
<400> 144
   Trp Gly Gly Asp Gly Phe Tyr Glu Met Asp Tyr
   1               5                   10
<210> 145
   <211> 11
   <212> PRT
   <213> Artificial Sequence
<220>
   <223> Description of Artificial Sequence: Synthetic peptide
<400> 145
   Trp Gly Gly Asp Gly Phe Tyr Ala Phe Asp Tyr
   1               5                   10
<210> 146
   <211> 11
   <212> PRT
   <213> Artificial Sequence
<220>
   <223> Description of Artificial Sequence: Synthetic peptide
<400> 146
   Trp Gly Gly Asp Gly Phe Tyr Ala Met Asp Gly
   1               5                   10
<210> 147
   <211> 11
   <212> PRT
   <213> Artificial Sequence
<220>
   <223> Description of Artificial Sequence: Synthetic peptide
<400> 147
   Trp Gly Gly Asp Gly Phe Tyr Ala Met Asp Ser
   1               5                   10
<210> 148
   <211> 11
   <212> PRT
   <213> Artificial Sequence
<220>
   <223> Description of Artificial Sequence: Synthetic peptide
<400> 148
   Trp Gly Gly Asp Gly Phe Tyr Ala Met Asp Lys
   1 5 10
<210> 149
   <211> 11
   <212> PRT
   <213> Artificial Sequence
<220>
   <223> Description of Artificial Sequence: Synthetic peptide
<400> 149
   Trp Gly Gly Asp Gly Phe Tyr Ala Met Asp His
   1 5 10
<210> 150
   <211> 11
   <212> PRT
   <213> Artificial Sequence
<220>
   <223> Description of Artificial Sequence: Synthetic peptide
<400> 150
   Lys Ala Ser Gln Asp Val Asn Thr Ala Val Ala
   1 5 10
<210> 151
   <211> 11
   <212> PRT
   <213> Artificial Sequence
<220>
   <223> Description of Artificial Sequence: Synthetic peptide
<400> 151
   Arg Ser Ser Gln Asp Val Asn Thr Ala Val Ala
   1 5 10
<210> 152
   <211> 11
   <212> PRT
   <213> Artificial Sequence
<220>
   <223> Description of Artificial Sequence: Synthetic peptide
<400> 152
   Arg Ala Ser Arg Asp Val Asn Thr Ala Val Ala
   1 5 10
<210> 153
   <211> 11
   <212> PRT
   <213> Artificial Sequence
<220>
   <223> Description of Artificial Sequence: Synthetic peptide
<400> 153
   Arg Ala Ser Ser Asp Val Asn Thr Ala Val Ala
   1               5                   10
<210> 154
   <211> 11
   <212> PRT
   <213> Artificial Sequence
<220>
   <223> Description of Artificial Sequence: Synthetic peptide
<400> 154
   Arg Ala Ser Phe Asp Val Asn Thr Ala Val Ala
   1               5                   10
<210> 155
   <211> 11
   <212> PRT
   <213> Artificial Sequence
<220>
   <223> Description of Artificial Sequence: Synthetic peptide
<400> 155
   Arg Ala Ser Lys Asp Val Asn Thr Ala Val Ala
   1               5                   10
<210> 156
   <211> 11
   <212> PRT
   <213> Artificial Sequence
<220>
   <223> Description of Artificial Sequence: Synthetic peptide
<400> 156
   Arg Ala Ser Gln Gly Val Asn Thr Ala Val Ala
   1               5                   10
<210> 157
   <211> 11
   <212> PRT
   <213> Artificial Sequence
<220>
   <223> Description of Artificial Sequence: Synthetic peptide
<400> 157
   Arg Ala Ser Gln Asn Val Asn Thr Ala Val Ala
   1 5 10
<210> 158
   <211> 11
   <212> PRT
   <213> Artificial Sequence
<220>
   <223> Description of Artificial Sequence: Synthetic peptide
<400> 158
   Arg Ala Ser Gln Arg Val Asn Thr Ala Val Ala
   1 5 10
<210> 159
   <211> 11
   <212> PRT
   <213> Artificial Sequence
<220>
   <223> Description of Artificial Sequence: Synthetic peptide
<400> 159
   Arg Ala Ser Gln Pro Val Asn Thr Ala Val Ala
   1 5 10
<210> 160
   <211> 11
   <212> PRT
   <213> Artificial Sequence
<220>
   <223> Description of Artificial Sequence: Synthetic peptide
<400> 160
   Arg Ala Ser Gln Ser Val Asn Thr Ala Val Ala
   1 5 10
<210> 161
   <211> 11
   <212> PRT
   <213> Artificial Sequence
<220>
   <223> Description of Artificial Sequence: Synthetic peptide
<400> 161
   Arg Ala Ser Gln Asp Ile Asn Thr Ala Val Ala
   1 5 10
<210> 162
   <211> 11
   <212> PRT
   <213> Artificial Sequence
<220>
   <223> Description of Artificial Sequence: Synthetic peptide
<400> 162
   Arg Ala Ser Gln Asp Val Asn Ser Ala Val Ala
   1               5                   10
<210> 163
   <211> 11
   <212> PRT
   <213> Artificial Sequence
<220>
   <223> Description of Artificial Sequence: Synthetic peptide
<400> 163
   Arg Ala Ser Gln Asp Val Asn Thr Ala Val Asn
   1               5                   10
<210> 164
   <211> 7
   <212> PRT
   <213> Artificial Sequence
<220>
   <223> Description of Artificial Sequence: Synthetic peptide
<400> 164
   Ser Gly Ser Phe Leu Tyr Ser
   1               5
<210> 165
   <211> 7
   <212> PRT
   <213> Artificial Sequence
<220>
   <223> Description of Artificial Sequence: Synthetic peptide
<400> 165
   Ser Ala Ser Trp Leu Tyr Ser
   1               5
<210> 166
   <211> 7
   <212> PRT
   <213> Artificial Sequence
<220>
   <223> Description of Artificial Sequence: Synthetic peptide
<400> 166
   Ser Ala Ser Tyr Leu Tyr Ser
   1 5
<210> 167
   <211> 7
   <212> PRT
   <213> Artificial Sequence
<220>
   <223> Description of Artificial Sequence: Synthetic peptide
<400> 167
   Ser Ala Ser Phe Ser Tyr Ser
   1 5
<210> 168
   <211> 7
   <212> PRT
   <213> Artificial Sequence
<220>
   <223> Description of Artificial Sequence: Synthetic peptide
<400> 168
   Ser Ala Ser Phe Leu Leu Ser
   1 5
<210> 169
   <211> 7
   <212> PRT
   <213> Artificial Sequence
<220>
   <223> Description of Artificial Sequence: Synthetic peptide
<400> 169
   Ser Ala Ser Phe Leu Ala Ser
   1 5
<210> 170
   <211> 9
   <212> PRT
   <213> Artificial Sequence
<220>
   <223> Description of Artificial Sequence: Synthetic peptide
<400> 170
   Gln Gln Trp Tyr Thr Thr Pro Pro Thr
   1 5
<210> 171
   <211> 9
   <212> PRT
   <213> Artificial Sequence
<220>
   <223> Description of Artificial Sequence: Synthetic peptide
<400> 171
   Gln Gln Tyr Tyr Thr Thr Pro Pro Thr
   1               5
<210> 172
   <211> 9
   <212> PRT
   <213> Artificial Sequence
<220>
   <223> Description of Artificial Sequence: Synthetic peptide
<400> 172
   Gln Gln His Tyr Ser Thr Pro Pro Thr
   1               5
<210> 173
   <211> 9
   <212> PRT
   <213> Artificial Sequence
<220>
   <223> Description of Artificial Sequence: Synthetic peptide
<400> 173
   Gln Gln His Tyr Ala Thr Pro Pro Thr
   1               5
<210> 174
   <211> 9
   <212> PRT
   <213> Artificial Sequence
<220>
   <223> Description of Artificial Sequence: Synthetic peptide
<400> 174
   Gln Gln His Tyr Thr Ser Pro Pro Thr
   1               5
<210> 175
   <211> 9
   <212> PRT
   <213> Artificial Sequence
<220>
   <223> Description of Artificial Sequence: Synthetic peptide
<400> 175
   Gln Gln His Tyr Thr Thr Pro Ser Thr
   1               5
<210> 176
   <211> 10
   <212> PRT
   <213> Artificial Sequence
<220>
   <223> Description of Artificial Sequence: Synthetic peptide
<400> 176
   Cys Phe Asn Ile Lys Asp Thr Tyr Ile His
   1               5                   10
<210> 177
   <211> 10
   <212> PRT
   <213> Artificial Sequence
<220>
   <223> Description of Artificial Sequence: Synthetic peptide
<400> 177
   Gly Phe Gly Ile Lys Asp Thr Tyr Ile His
   1               5                   10
<210> 178
   <211> 10
   <212> PRT
   <213> Artificial Sequence
<220>
   <223> Description of Artificial Sequence: Synthetic peptide
<400> 178
   Gly Phe Asn Ile Arg Asp Thr Tyr Ile His
   1               5                   10
<210> 179
   <211> 10
   <212> PRT
   <213> Artificial Sequence
<220>
   <223> Description of Artificial Sequence: Synthetic peptide
<400> 179
   Gly Phe Asn Ile Val Asp Thr Tyr Ile His
   1               5                   10
<210> 180
   <211> 10
   <212> PRT
   <213> Artificial Sequence
<220>
   <223> Description of Artificial Sequence: Synthetic peptide
<400> 180
   Gly Phe Asn Ile Lys His Thr Tyr Ile His
   1               5                   10
<210> 181
   <211> 10
   <212> PRT
   <213> Artificial Sequence
<220>
   <223> Description of Artificial Sequence: Synthetic peptide
<400> 181
   Gly Phe Asn Ile Lys Gln Thr Tyr Ile His
   1               5                   10
<210> 182
   <211> 10
   <212> PRT
   <213> Artificial Sequence
<220>
   <223> Description of Artificial Sequence: Synthetic peptide
<400> 182
   Gly Phe Asn Ile Lys Thr Thr Tyr Ile His
   1               5                   10
<210> 183
   <211> 10
   <212> PRT
   <213> Artificial Sequence
<220>
   <223> Description of Artificial Sequence: Synthetic peptide
<400> 183
   Gly Phe Asn Ile Lys Asp Val Tyr Ile His
   1               5                   10
<210> 184
   <211> 17
   <212> PRT
   <213> Artificial Sequence
<220>
   <223> Description of Artificial Sequence: Synthetic peptide
<400> 184
   Arg Ile Lys Pro Thr Asn Gly Tyr Thr Arg Tyr Ala Asp Ser Val Lys
   1               5                   10                  15
   Gly
<210> 185
   <211> 17
   <212> PRT
   <213> Artificial Sequence
<220>
   <223> Description of Artificial Sequence: Synthetic peptide
<400> 185
   Arg Ile Gln Pro Thr Asn Gly Tyr Thr Arg Tyr Ala Asp Ser Val Lys
   1               5                   10                  15
   Gly
<210> 186
   <211> 17
   <212> PRT
   <213> Artificial Sequence
<220>
   <223> Description of Artificial Sequence: Synthetic peptide
<400> 186
   Arg Ile Tyr Pro Thr Asn Gly Tyr Gly Arg Tyr Ala Asp Ser Val Lys
   1               5                   10                  15
   Gly
<210> 187
   <211> 17
   <212> PRT
   <213> Artificial Sequence
<220>
   <223> Description of Artificial Sequence: Synthetic peptide
<400> 187
   Arg Ile Tyr Pro Thr Asn Gly Tyr Val Arg Tyr Ala Asp Ser Val Lys
   1               5                   10                  15
   Gly
<210> 188
   <211> 17
   <212> PRT
   <213> Artificial Sequence
<220>
   <223> Description of Artificial Sequence: Synthetic peptide
<400> 188
   Arg Ile Tyr Pro Thr Asn Gly Tyr Thr Arg Tyr Lys Asp Ser Val Lys
   1               5                   10                  15
   Gly
<210> 189
   <211> 17
   <212> PRT
   <213> Artificial Sequence
<220>
   <223> Description of Artificial Sequence: Synthetic peptide
<400> 189
   Arg Ile Tyr Pro Thr Asn Gly Tyr Thr Arg Tyr Ala Ser Ser Val Lys
   1               5                   10                  15
   Gly
<210> 190
   <211> 17
   <212> PRT
   <213> Artificial Sequence
<220>
   <223> Description of Artificial Sequence: Synthetic peptide
<400> 190
   Arg Ile Tyr Pro Thr Asn Gly Tyr Thr Arg Tyr Ala Glu Ser Val Lys
   1               5                   10                  15
   Gly
<210> 191
   <211> 17
   <212> PRT
   <213> Artificial Sequence
<220>
   <223> Description of Artificial Sequence: Synthetic peptide
<400> 191
   Arg Ile Tyr Pro Thr Asn Gly Tyr Thr Arg Tyr Ala Asp Glu Val Lys
   1 5 10 15
   Gly
<210> 192
   <211> 17
   <212> PRT
   <213> Artificial Sequence
<220>
   <223> Description of Artificial Sequence: Synthetic peptide
<400> 192
   Arg Ile Tyr Pro Thr Asn Gly Tyr Thr Arg Tyr Ala Asp Gly Val Lys
   1 5 10 15
   Gly
<210> 193
   <211> 17
   <212> PRT
   <213> Artificial Sequence
<220>
   <223> Description of Artificial Sequence: Synthetic peptide
<400> 193
   Arg Ile Tyr Pro Thr Asn Gly Tyr Thr Arg Tyr Ala Asp Ala Val Lys
   1 5 10 15
   Gly
<210> 194
   <211> 17
   <212> PRT
   <213> Artificial Sequence
<220>
   <223> Description of Artificial Sequence: Synthetic peptide
<400> 194
   Arg Ile Tyr Pro Thr Asn Gly Tyr Thr Arg Tyr Ala Asp Ser Ala Lys
   1 5 10 15
   Gly
<210> 195
   <211> 17
   <212> PRT
   <213> Artificial Sequence
<220>
   <223> Description of Artificial Sequence: Synthetic peptide
<400> 195
   Arg Ile Tyr Pro Thr Asn Gly Tyr Thr Arg Tyr Ala Asp Ser Val Ala
   1               5                   10                  15
   Gly
<210> 196
   <211> 17
   <212> PRT
   <213> Artificial Sequence
<220>
   <223> Description of Artificial Sequence: Synthetic peptide
<400> 196
   Arg Ile Tyr Pro Thr Asn Gly Tyr Thr Arg Tyr Ala Asp Ser Val Lys
   1               5                   10                  15
   Val
<210> 197
   <211> 17
   <212> PRT
   <213> Artificial Sequence
<220>
   <223> Description of Artificial Sequence: Synthetic peptide
<400> 197
   Arg Ile Tyr Pro Thr Asn Gly Tyr Thr Arg Tyr Ala Asp Ser Val Lys
   1               5                   10                  15
   Glu
<210> 198
   <211> 17
   <212> PRT
   <213> Artificial Sequence
<220>
   <223> Description of Artificial Sequence: Synthetic peptide
<400> 198
   Arg Ile Tyr Pro Thr Asn Gly Tyr Thr Arg Tyr Ala Asp Ser Val Lys
   1               5                   10                  15
   Ile
<210> 199
   <211> 11
   <212> PRT
   <213> Artificial Sequence
<220>
   <223> Description of Artificial Sequence: Synthetic peptide
<400> 199
   Trp Gly Gly Val Gly Phe Tyr Ala Met Asp Tyr
   1               5                   10
<210> 200
   <211> 11
   <212> PRT
   <213> Artificial Sequence
<220>
   <223> Description of Artificial Sequence: Synthetic peptide
<400> 200
   Trp Gly Gly Met Gly Phe Tyr Ala Met Asp Tyr
   1               5                   10
<210> 201
   <211> 11
   <212> PRT
   <213> Artificial Sequence
<220>
   <223> Description of Artificial Sequence: Synthetic peptide
<400> 201
   Trp Gly Gly Asp Gly Phe Tyr Ala Met Leu Tyr
   1               5                   10
<210> 202
   <211> 11
   <212> PRT
   <213> Artificial Sequence
<220>
   <223> Description of Artificial Sequence: Synthetic peptide
<400> 202
   Trp Gly Gly Asp Gly Phe Tyr Ala Met Asp Arg
   1               5                   10
<210> 203
   <211> 11
   <212> PRT
   <213> Artificial Sequence
<220>
   <223> Description of Artificial Sequence: Synthetic peptide
<400> 203
   His Ala Ser Gln Asp Val Asn Thr Ala Val Ala
   1               5                   10
<210> 204
   <211> 11
   <212> PRT
   <213> Artificial Sequence
<220>
   <223> Description of Artificial Sequence: Synthetic peptide
<400> 204
   Glu Ala Ser Gln Asp Val Asn Thr Ala Val Ala
   1               5                   10
<210> 205
   <211> 11
   <212> PRT
   <213> Artificial Sequence
<220>
   <223> Description of Artificial Sequence: Synthetic peptide
<400> 205
   Cys Ala Ser Gln Asp Val Asn Thr Ala Val Ala
   1               5                   10
<210> 206
   <211> 11
   <212> PRT
   <213> Artificial Sequence
<220>
   <223> Description of Artificial Sequence: Synthetic peptide
<400> 206
   Val Ala Ser Gln Asp Val Asn Thr Ala Val Ala
   1               5                   10
<210> 207
   <211> 11
   <212> PRT
   <213> Artificial Sequence
<220>
   <223> Description of Artificial Sequence: Synthetic peptide
<400> 207
   Arg Cys Ser Gln Asp Val Asn Thr Ala Val Ala
   1               5                   10
<210> 208
   <211> 11
   <212> PRT
   <213> Artificial Sequence
<220>
   <223> Description of Artificial Sequence: Synthetic peptide
<400> 208
   Arg Gly Ser Gln Asp Val Asn Thr Ala Val Ala
   1               5                   10
<210> 209
   <211> 11
   <212> PRT
   <213> Artificial Sequence
<220>
   <223> Description of Artificial Sequence: Synthetic peptide
<400> 209
   Arg Pro Ser Gln Asp Val Asn Thr Ala Val Ala
   1               5                   10
<210> 210
   <211> 11
   <212> PRT
   <213> Artificial Sequence
<220>
   <223> Description of Artificial Sequence: Synthetic peptide
<400> 210
   Arg Ala Ser Gln Asp Ala Asn Thr Ala Val Ala
   1               5                   10
<210> 211
   <211> 11
   <212> PRT
   <213> Artificial Sequence
<220>
   <223> Description of Artificial Sequence: Synthetic peptide
<400> 211
   Arg Ala Ser Gln Asp Ser Asn Thr Ala Val Ala
   1               5                   10
<210> 212
   <211> 7
   <212> PRT
   <213> Artificial Sequence
<220>
   <223> Description of Artificial Sequence: Synthetic peptide
<400> 212
   Ser Ser Ser Phe Leu Tyr Ser
   1 5
<210> 213
   <211> 7
   <212> PRT
   <213> Artificial Sequence
<220>
   <223> Description of Artificial Sequence: Synthetic peptide
<400> 213
   Ser Ala Trp Phe Leu Tyr Ser
   1 5
<210> 214
   <211> 7
   <212> PRT
   <213> Artificial Sequence
<220>
   <223> Description of Artificial Sequence: Synthetic peptide
<400> 214
   Ser Ala Met Phe Leu Tyr Ser
   1 5
<210> 215
   <211> 7
   <212> PRT
   <213> Artificial Sequence
<220>
   <223> Description of Artificial Sequence: Synthetic peptide
<400> 215
   Ser Ala Gln Phe Leu Tyr Ser
   1 5
<210> 216
   <211> 7
   <212> PRT
   <213> Artificial Sequence
<220>
   <223> Description of Artificial Sequence: Synthetic peptide
<400> 216
   Ser Ala His Phe Leu Tyr Ser
   1 5
<210> 217
   <211> 7
   <212> PRT
   <213> Artificial Sequence
<220>
   <223> Description of Artificial Sequence: Synthetic peptide
<400> 217
   Ser Ala Gly Phe Leu Tyr Ser
   1 5
<210> 218
   <211> 7
   <212> PRT
   <213> Artificial Sequence
<220>
   <223> Description of Artificial Sequence: Synthetic peptide
<400> 218
   Ser Ala Arg Phe Leu Tyr Ser
   1 5
<210> 219
   <211> 7
   <212> PRT
   <213> Artificial Sequence
<220>
   <223> Description of Artificial Sequence: Synthetic peptide
<400> 219
   Ser Ala Ser Phe Ile Tyr Ser
   1 5
<210> 220
   <211> 7
   <212> PRT
   <213> Artificial Sequence
<220>
   <223> Description of Artificial Sequence: Synthetic peptide
<400> 220
   Ser Ala Ser Phe Gly Tyr Ser
   1 5
<210> 221
   <211> 7
   <212> PRT
   <213> Artificial Sequence
<220>
   <223> Description of Artificial Sequence: Synthetic peptide
<400> 221
   Ser Ala Ser Phe Val Tyr Ser
   1               5
<210> 222
   <211> 7
   <212> PRT
   <213> Artificial Sequence
<220>
   <223> Description of Artificial Sequence: Synthetic peptide
<400> 222
   Ser Ala Ser Phe Leu Tyr Ala
   1               5
<210> 223
   <211> 7
   <212> PRT
   <213> Artificial Sequence
<220>
   <223> Description of Artificial Sequence: Synthetic peptide
<400> 223
   Ser Ala Ser Phe Leu Tyr Pro
   1               5
<210> 224
   <211> 7
   <212> PRT
   <213> Artificial Sequence
<220>
   <223> Description of Artificial Sequence: Synthetic peptide
<400> 224
   Ser Ala Ser Phe Leu Tyr Gly
   1               5
<210> 225
   <211> 7
   <212> PRT
   <213> Artificial Sequence
<220>
   <223> Description of Artificial Sequence: Synthetic peptide
<400> 225
   Ser Ala Ser Phe Leu Tyr His
   1 5
<210> 226
   <211> 7
   <212> PRT
   <213> Artificial Sequence
<220>
   <223> Description of Artificial Sequence: Synthetic peptide
<400> 226
   Ser Ala Ser Phe Leu Tyr Tyr
   1 5
<210> 227
   <211> 7
   <212> PRT
   <213> Artificial Sequence
<220>
   <223> Description of Artificial Sequence: Synthetic peptide
<400> 227
   Ser Ala Ser Phe Leu Tyr Phe
   1 5
<210> 228
   <211> 7
   <212> PRT
   <213> Artificial Sequence
<220>
   <223> Description of Artificial Sequence: Synthetic peptide
<400> 228
   Ser Ala Ser Phe Leu Tyr Asn
   1 5
<210> 229
   <211> 7
   <212> PRT
   <213> Artificial Sequence
<220>
   <223> Description of Artificial Sequence: Synthetic peptide
<400> 229
   Ser Ala Ser Phe Leu Tyr Met
   1               5
<210> 230
   <211> 9
   <212> PRT
   <213> Artificial Sequence
<220>
   <223> Description of Artificial Sequence: Synthetic peptide
<400> 230
   Gln Gln His Met Thr Thr Pro Pro Thr
   1               5
<210> 231
   <211> 9
   <212> PRT
   <213> Artificial Sequence
<220>
   <223> Description of Artificial Sequence: Synthetic peptide
<400> 231
   Gln Gln His Tyr Leu Thr Pro Pro Thr
   1               5
<210> 232
   <211> 9
   <212> PRT
   <213> Artificial Sequence
<220>
   <223> Description of Artificial Sequence: Synthetic peptide
<400> 232
   Gln Gln His Tyr Met Thr Pro Pro Thr
   1               5
<210> 233
   <211> 9
   <212> PRT
   <213> Artificial Sequence
<220>
   <223> Description of Artificial Sequence: Synthetic peptide
<400> 233
   Gln Gln His Tyr Gly Thr Pro Pro Thr
   1               5
<210> 234
   <211> 9
   <212> PRT
   <213> Artificial Sequence
<220>
   <223> Description of Artificial Sequence: Synthetic peptide
<400> 234
   Gln Gln His Tyr Val Thr Pro Pro Thr
   1 5
<210> 235
   <211> 9
   <212> PRT
   <213> Artificial Sequence
<220>
   <223> Description of Artificial Sequence: Synthetic peptide
<400> 235
   Gln Gln His Tyr Thr Thr Pro Gly Thr
   1 5
<210> 236
   <211> 9
   <212> PRT
   <213> Artificial Sequence
<220>
   <223> Description of Artificial Sequence: Synthetic peptide
<400> 236
   Gln Gln His Tyr Thr Thr Pro Pro Asp
   1 5
<210> 237
   <211> 32
   <212> PRT
   <213> Artificial Sequence
<220>
   <223> Description of Artificial Sequence: Synthetic polypeptide
<400> 237 Met Asn Ser Leu Arg Ala Glu Asp Thr Gln Val Tyr Tyr Cys Ser Arg 20 25 30
<210> 238
   <211> 32
   <212> PRT
   <213> Artificial Sequence
<220>
   <223> Description of Artificial Sequence: Synthetic polypeptide
<400> 238
   Arg Phe Thr Ile Ser Ala Asp Thr Ser Lys Asn Thr Ala Tyr Leu Gln
   1               5                   10                  15
   Met Asn Ser Leu Arg Ala Glu Asp Thr Trp Val Tyr Tyr Cys Ser Arg
               20                  25                  30
<210> 239
   <211> 32
   <212> PRT
   <213> Artificial Sequence
<220>
   <223> Description of Artificial Sequence: Synthetic polypeptide
<400> 239
   Arg Phe Thr Ile Ser Ala Asp Thr Ser Lys Asn Thr Ala Tyr Leu Gln
   1               5                   10                  15
   Met Asn Ser Leu Arg Ala Glu Asp Thr Glu Val Tyr Tyr Cys Ser Arg
               20                  25                  30
<210> 240
   <211> 32
   <212> PRT
   <213> Artificial Sequence
<220>
   <223> Description of Artificial Sequence: Synthetic polypeptide
<400> 240
   Arg Phe Thr Ile Ser Ala Asp Thr Ser Lys Asn Thr Ala Tyr Leu Gln
   1               5                   10                  15
   Met Asn Ser Leu Arg Ala Glu Asp Thr Ala Arg Tyr Tyr Cys Ser Arg
               20                  25                  30
<210> 241
   <211> 32
   <212> PRT
   <213> Artificial Sequence
<220>
   <223> Description of Artificial Sequence: Synthetic polypeptide
<400> 241
   Arg Phe Thr Ile Ser Ala Asp Thr Ser Lys Asn Thr Ala Tyr Leu Gln
   1               5                   10                  15
   Met Asn Ser Leu Arg Ala Glu Asp Thr Ala Tyr Tyr Tyr Cys Ser Arg
                   20                  25                  30
<210> 242
   <211> 32
   <212> PRT
   <213> Artificial Sequence
<220>
   <223> Description of Artificial Sequence: Synthetic polypeptide
<400> 242
   Arg Phe Thr Ile Ser Ala Asp Thr Ser Lys Asn Thr Ala Tyr Leu Gln
   1               5                   10                  15
   Met Asn Ser Leu Arg Ala Glu Asp Thr Ala Val Tyr Tyr Cys Asn Arg
                   20                  25                  30
<210> 243
   <211> 32
   <212> PRT
   <213> Artificial Sequence
<220>
   <223> Description of Artificial Sequence: Synthetic polypeptide
<400> 243
   Arg Phe Thr Ile Ser Ala Asp Thr Ser Lys Asn Thr Ala Tyr Leu Gln
   1               5                   10                  15
   Met Asn Ser Leu Arg Ala Glu Asp Thr Ala Val Tyr Tyr Cys Pro Arg
                   20                  25                  30
<210> 244
   <211> 32
   <212> PRT
   <213> Artificial Sequence
<220>
   <223> Description of Artificial Sequence: Synthetic polypeptide
<400> 244
   Arg Phe Thr Ile Ser Ala Asp Thr Ser Lys Asn Thr Ala Tyr Leu Gln
   1               5                   10                  15
   Met Asn Ser Leu Arg Ala Glu Asp Thr Ala Val Tyr Tyr Cys Tyr Arg
                   20                  25                  30
<210> 245
   <211> 32
   <212> PRT
   <213> Artificial Sequence
<220>
   <223> Description of Artificial Sequence: Synthetic polypeptide
<400> 245
   Arg Phe Thr Ile Ser Ala Asp Thr Ser Lys Asn Thr Ala Tyr Leu Gln
   1               5                   10                  15
   Met Asn Ser Leu Arg Ala Glu Asp Thr Ala Val Tyr Tyr Cys Asp Arg
               20                  25                  30
<210> 246
   <211> 32
   <212> PRT
   <213> Artificial Sequence
<220>
   <223> Description of Artificial Sequence: Synthetic polypeptide
<400> 246
   Arg Phe Thr Ile Ser Ala Asp Thr Ser Lys Asn Thr Cys Tyr Leu Gln
   1               5                   10                  15
   Met Asn Ser Leu Arg Ala Glu Asp Thr Ala Val Tyr Tyr Cys Ser Arg
               20                  25                  30
<210> 247
   <211> 32
   <212> PRT
   <213> Artificial Sequence
<220>
   <223> Description of Artificial Sequence: Synthetic polypeptide
<400> 247
   Arg Phe Thr Ile Ser Ala Asp Thr Ser Lys Asn Thr Trp Tyr Leu Gln
   1               5                   10                  15
   Met Asn Ser Leu Arg Ala Glu Asp Thr Ala Val Tyr Tyr Cys Ser Arg
               20                  25                  30
<210> 248
   <211> 32
   <212> PRT
   <213> Artificial Sequence
<220>
   <223> Description of Artificial Sequence: Synthetic polypeptide
<400> 248
   Arg Phe Thr Ile Ser Ala Asp Thr Ser Lys Asn Thr Gln Tyr Leu Gln
   1               5                   10                  15
   Met Asn Ser Leu Arg Ala Glu Asp Thr Ala Val Tyr Tyr Cys Ser Arg
               20                  25                  30
<210> 249
   <211> 32
   <212> PRT
   <213> Artificial Sequence
<220>
   <223> Description of Artificial Sequence: Synthetic polypeptide
<400> 249
   Arg Phe Thr Ile Ser Ala Asp Thr Ser Lys Asn Thr Ala Tyr Leu Gln
   1               5                   10                  15
   Met Asn Val Leu Arg Ala Glu Asp Thr Ala Val Tyr Tyr Cys Ser Arg
               20                  25                  30
<210> 250
   <211> 32
   <212> PRT
   <213> Artificial Sequence
<220>
   <223> Description of Artificial Sequence: Synthetic polypeptide
<400> 250
   Arg Phe Thr Ile Ser Ala Asp Thr Ser Lys Asn Thr Ala Tyr Leu Gln
   1               5                   10                  15
   Met Asn Leu Leu Arg Ala Glu Asp Thr Ala Val Tyr Tyr Cys Ser Arg
               20                  25                  30
<210> 251
   <211> 32
   <212> PRT
   <213> Artificial Sequence
<220>
   <223> Description of Artificial Sequence: Synthetic polypeptide
<400> 251
   Arg Phe Thr Ile Ser Ala Asp Thr Ser Lys Asn Thr Ala Tyr Leu Gln
   1 5 10 15
   Met Asn Ser Val Arg Ala Glu Asp Thr Ala Val Tyr Tyr Cys Ser Arg
   20 25 30
<210> 252
   <211> 32
   <212> PRT
   <213> Artificial Sequence
<220>
   <223> Description of Artificial Sequence: Synthetic polypeptide
<400> 252
   Arg Phe Thr Ile Ser Ala Asp Thr Ser Lys Asn Thr Ala Tyr Leu Gln
   1 5 10 15
   Met Asn Ser Ala Arg Ala Glu Asp Thr Ala Val Tyr Tyr Cys Ser Arg
   20 25 30
<210> 253
   <211> 32
   <212> PRT
   <213> Artificial Sequence
<220>
   <223> Description of Artificial Sequence: Synthetic polypeptide
<400> 253
   Arg Phe Thr Ile Ser Ala Asp Thr Ser Lys Asn Thr Ala Tyr Leu Gln
   1 5 10 15
   Met Asn Ser Glu Arg Ala Glu Asp Thr Ala Val Tyr Tyr Cys Ser Arg
   20 25 30
<210> 254
   <211> 32
   <212> PRT
   <213> Artificial Sequence
<220>
   <223> Description of Artificial Sequence: Synthetic polypeptide
<400> 254
   Arg Phe Thr Ile Ser Ala Asp Thr Ser Lys Asn Thr Ala Tyr Leu Gln
   1 5 10 15
   Met Asn Ser Gly Arg Ala Glu Asp Thr Ala Val Tyr Tyr Cys Ser Arg
   20 25 30
<210> 255
   <211> 32
   <212> PRT
   <213> Artificial Sequence
<220>
   <223> Description of Artificial Sequence: Synthetic polypeptide
<400> 255
   Arg Phe Thr Ile Ser Ala Asp Thr Ser Lys Asn Thr Ala Tyr Leu Gln
   1               5                   10                  15
   Met Asn Ser Leu Val Ala Glu Asp Thr Ala Val Tyr Tyr Cys Ser Arg
               20                  25                  30
<210> 256
   <211> 32
   <212> PRT
   <213> Artificial Sequence
<220>
   <223> Description of Artificial Sequence: Synthetic polypeptide
<400> 256
   Arg Phe Thr Ile Ser Ala Asp Thr Ser Lys Asn Thr Ala Tyr Leu Gln
   1               5                   10                  15
   Met Asn Ser Leu Leu Ala Glu Asp Thr Ala Val Tyr Tyr Cys Ser Arg
               20                  25                  30
<210> 257
   <211> 32
   <212> PRT
   <213> Artificial Sequence
<220>
   <223> Description of Artificial Sequence: Synthetic polypeptide
<400> 257
   Arg Phe Thr Ile Ser Ala Asp Thr Ser Lys Asn Thr Ala Tyr Leu Gln
   1               5                   10                  15
   Met Asn Ser Leu Gly Ala Glu Asp Thr Ala Val Tyr Tyr Cys Ser Arg
               20                  25                  30
<210> 258
   <211> 32
   <212> PRT
   <213> Artificial Sequence
<220>
   <223> Description of Artificial Sequence: Synthetic polypeptide
<400> 258
   Arg Phe Thr Ile Ser Ala Asp Thr Ser Lys Asn Thr Ala Tyr Leu Gln
   1               5                   10                  15
   Met Asn Ser Leu Lys Ala Glu Asp Thr Ala Val Tyr Tyr Cys Ser Arg
               20                  25                  30
<210> 259
   <211> 32
   <212> PRT
   <213> Artificial Sequence
<220>
   <223> Description of Artificial Sequence: Synthetic polypeptide
<400> 259
   Arg Phe Thr Ile Ser Ala Asp Thr Ser Lys Asn Thr Ala Tyr Leu Gln
   1               5                   10                  15
   Met Asn Ser Leu Arg Met Glu Asp Thr Ala Val Tyr Tyr Cys Ser Arg
               20                  25                  30
<210> 260
   <211> 32
   <212> PRT
   <213> Artificial Sequence
<220>
   <223> Description of Artificial Sequence: Synthetic polypeptide
<400> 260
   Arg Phe Thr Ile Ser Ala Asp Thr Ser Lys Asn Thr Ala Tyr Leu Gln
   1               5                   10                  15
   Met Asn Ser Leu Arg Trp Glu Asp Thr Ala Val Tyr Tyr Cys Ser Arg
               20                  25                  30
<210> 261
   <211> 32
   <212> PRT
   <213> Artificial Sequence
<220>
   <223> Description of Artificial Sequence: Synthetic polypeptide
<400> 261
   Arg Phe Thr Ile Ser Ala Asp Thr Ser Lys Asn Thr Ala Tyr Leu Gln
   1               5                   10                  15
   Met Asn Ser Leu Arg Lys Glu Asp Thr Ala Val Tyr Tyr Cys Ser Arg
               20                  25                  30
<210> 262
   <211> 32
   <212> PRT
   <213> Artificial Sequence
<220>
   <223> Description of Artificial Sequence: Synthetic polypeptide
<400> 262
   Arg Phe Thr Ile Ser Ala Asp Thr Ser Lys Asn Thr Ala Tyr Leu Gln
   1               5                   10                  15
   Met Asn Ser Leu Arg Gly Glu Asp Thr Ala Val Tyr Tyr Cys Ser Arg
               20                  25                  30
<210> 263
   <211> 32
   <212> PRT
   <213> Artificial Sequence
<220>
   <223> Description of Artificial Sequence: Synthetic polypeptide
<400> 263
   Arg Phe Thr Ile Ser Ala Asp Thr Ser Lys Asn Thr Ala Tyr Leu Gln
   1               5                   10                  15
   Met Asn Ser Leu Arg Gln Glu Asp Thr Ala Val Tyr Tyr Cys Ser Arg
               20                  25                  30
<210> 264
   <211> 32
   <212> PRT
   <213> Artificial Sequence
<220>
   <223> Description of Artificial Sequence: Synthetic polypeptide
<400> 264
   Arg Phe Thr Ile Ser Ala Asp Thr Ser Lys Asn Thr Ala Tyr Leu Gln
   1               5                   10                  15
   Met Asn Ser Leu Arg Arg Glu Asp Thr Ala Val Tyr Tyr Cys Ser Arg
               20                  25                  30
<210> 265
   <211> 32
   <212> PRT
   <213> Artificial Sequence
<220>
   <223> Description of Artificial Sequence: Synthetic polypeptide
<400> 265
   Arg Phe Thr Ile Ser Ala Asp Thr Ser Lys Asn Thr Ala Tyr Leu Gln
   1               5                   10                  15
   Met Asn Ser Leu Arg Ala Leu Asp Thr Ala Val Tyr Tyr Cys Ser Arg
               20                  25                  30
<210> 266
   <211> 32
   <212> PRT
   <213> Artificial Sequence
<220>
   <223> Description of Artificial Sequence: Synthetic polypeptide
<400> 266
   Arg Phe Thr Ile Ser Ala Asp Thr Ser Lys Asn Thr Ala Tyr Leu Gln
   1               5                   10                  15
   Met Asn Ser Leu Arg Ala Gly Asp Thr Ala Val Tyr Tyr Cys Ser Arg
               20                  25                  30
<210> 267
   <211> 32
   <212> PRT
   <213> Artificial Sequence
<220>
   <223> Description of Artificial Sequence: Synthetic polypeptide
<400> 267
   Arg Phe Thr Ile Ser Ala Asp Thr Ser Lys Asn Thr Ala Tyr Leu Gln
   1               5                   10                  15
   Met Asn Ser Leu Arg Ala Ala Asp Thr Ala Val Tyr Tyr Cys Ser Arg
               20                  25                  30
<210> 268
   <211> 32
   <212> PRT
   <213> Artificial Sequence
<220>
   <223> Description of Artificial Sequence: Synthetic polypeptide
<400> 268
   Arg Phe Thr Ile Ser Ala Asp Thr Ser Lys Asn Thr Ala Tyr Leu Gln
   1               5                   10                  15
   Met Asn Ser Leu Arg Ala Ser Asp Thr Ala Val Tyr Tyr Cys Ser Arg
               20                  25                  30
<210> 269
   <211> 32
   <212> PRT
   <213> Artificial Sequence
<220>
   <223> Description of Artificial Sequence: Synthetic polypeptide
<400> 269
   Arg Phe Thr Ile Ser Ala Asp Thr Ser Lys Asn Thr Ala Tyr Leu Gln
   1               5                   10                  15
   Met Asn Ser Leu Arg Ala Asn Asp Thr Ala Val Tyr Tyr Cys Ser Arg
               20                  25                  30
<210> 270
   <211> 32
   <212> PRT
   <213> Artificial Sequence
<220>
   <223> Description of Artificial Sequence: Synthetic polypeptide
<400> 270
   Arg Phe Thr Ile Ser Ala Asp Thr Ser Lys Asn Thr Ala Tyr Leu Gln
   1               5                   10                  15
   Met Asn Ser Leu Arg Ala His Asp Thr Ala Val Tyr Tyr Cys Ser Arg
               20                  25                  30
<210> 271
   <211> 32
   <212> PRT
   <213> Artificial Sequence
<220>
   <223> Description of Artificial Sequence: Synthetic polypeptide
<400> 271
   Arg Phe Thr Ile Ser Ala Asp Thr Ser Lys Asn Thr Ala Tyr Leu Gln
   1               5                   10                  15
   Met Asn Ser Leu Arg Ala Glu Ser Thr Ala Val Tyr Tyr Cys Ser Arg
               20                  25                  30
<210> 272
   <211> 32
   <212> PRT
   <213> Artificial Sequence
<220>
   <223> Description of Artificial Sequence: Synthetic polypeptide
<400> 272
   Arg Phe Thr Ile Ser Ala Asp Thr Ser Lys Asn Thr Ala Tyr Leu Gln
   1               5                   10                  15
   Met Asn Ser Leu Arg Ala Glu Gly Thr Ala Val Tyr Tyr Cys Ser Arg
               20                  25                  30
<210> 273
   <211> 32
   <212> PRT
   <213> Artificial Sequence
<220>
   <223> Description of Artificial Sequence: Synthetic polypeptide
<400> 273
   Arg Phe Thr Ile Ser Ala Asp Thr Ser Lys Asn Thr Ala Tyr Leu Gln
   1               5                   10                  15
   Met Asn Ser Leu Arg Ala Glu Asp Trp Ala Val Tyr Tyr Cys Ser Arg
               20                  25                  30
<210> 274
   <211> 32
   <212> PRT
   <213> Artificial Sequence
<220>
   <223> Description of Artificial Sequence: Synthetic polypeptide
<400> 274
   Arg Phe Thr Ile Ser Ala Asp Thr Ser Lys Asn Thr Ala Tyr Leu Gln
   1               5                   10                  15
   Met Asn Ser Leu Arg Ala Glu Asp Glu Ala Val Tyr Tyr Cys Ser Arg
               20                  25                  30
<210> 275
   <211> 32
   <212> PRT
   <213> Artificial Sequence
<220>
   <223> Description of Artificial Sequence: Synthetic polypeptide
<400> 275
   Arg Phe Thr Ile Ser Ala Asp Thr Ser Lys Asn Thr Ala Tyr Leu Gln
   1               5                   10                  15
   Met Asn Ser Leu Arg Ala Glu Asp Leu Ala Val Tyr Tyr Cys Ser Arg
               20                  25                  30
<210> 276
   <211> 32
   <212> PRT
   <213> Artificial Sequence
<220>
   <223> Description of Artificial Sequence: Synthetic polypeptide
<400> 276
   Arg Phe Thr Ile Ser Ala Asp Thr Ser Lys Asn Thr Ala Tyr Leu Gln
   1               5                   10                  15
   Met Asn Ser Leu Arg Ala Glu Asp Phe Ala Val Tyr Tyr Cys Ser Arg
               20                  25                  30
<210> 277
   <211> 32
   <212> PRT
   <213> Artificial Sequence
<220>
   <223> Description of Artificial Sequence: Synthetic polypeptide
<400> 277
   Arg Phe Thr Ile Ser Ala Asp Thr Ser Lys Asn Thr Ala Tyr Leu Gln
   1               5                   10                  15
   Met Asn Ser Leu Arg Ala Glu Asp His Ala Val Tyr Tyr Cys Ser Arg
               20                  25                  30
<210> 278
   <211> 32
   <212> PRT
   <213> Artificial Sequence
<220>
   <223> Description of Artificial Sequence: Synthetic polypeptide
<400> 278
   Arg Phe Thr Ile Ser Ala Asp Thr Ser Lys Asn Thr Ala Tyr Leu Gln
   1               5                   10                  15
   Met Asn Ser Leu Arg Ala Glu Asp Asp Ala Val Tyr Tyr Cys Ser Arg
               20                  25                  30
<210> 279
   <211> 32
   <212> PRT
   <213> Artificial Sequence
<220>
   <223> Description of Artificial Sequence: Synthetic polypeptide
<400> 279
   Arg Phe Thr Ile Ser Ala Asp Thr Ser Lys Asn Thr Ala Tyr Leu Gln
   1               5                   10                  15
   Met Asn Ser Leu Arg Ala Glu Asp Thr Gly Val Tyr Tyr Cys Ser Arg
               20                  25                  30
<210> 280
   <211> 32
   <212> PRT
   <213> Artificial Sequence
<220>
   <223> Description of Artificial Sequence: Synthetic polypeptide
<400> 280
   Arg Phe Thr Ile Ser Ala Asp Thr Ser Lys Asn Thr Ala Tyr Leu Gln
   1               5                   10                  15
   Met Asn Ser Leu Arg Ala Glu Asp Thr Arg Val Tyr Tyr Cys Ser Arg
               20                  25                  30
<210> 281
   <211> 32
   <212> PRT
   <213> Artificial Sequence
<220>
   <223> Description of Artificial Sequence: Synthetic polypeptide
<400> 281
   Arg Phe Thr Ile Ser Ala Asp Thr Ser Lys Asn Thr Ala Tyr Leu Gln
   1 5 10 15
   Met Asn Ser Leu Arg Ala Glu Asp Thr Lys Val Tyr Tyr Cys Ser Arg
   20 25 30
<210> 282
   <211> 32
   <212> PRT
   <213> Artificial Sequence
<220>
   <223> Description of Artificial Sequence: Synthetic polypeptide
<400> 282
   Arg Phe Thr Ile Ser Ala Asp Thr Ser Lys Asn Thr Ala Tyr Leu Gln
   1 5 10 15
   Met Asn Ser Leu Arg Ala Glu Asp Thr Val Val Tyr Tyr Cys Ser Arg
   20 25 30
<210> 283
   <211> 32
   <212> PRT
   <213> Artificial Sequence
<220>
   <223> Description of Artificial Sequence: Synthetic polypeptide
<400> 283
   Arg Phe Thr Ile Ser Ala Asp Thr Ser Lys Asn Thr Ala Tyr Leu Gln
   1 5 10 15
   Met Asn Ser Leu Arg Ala Glu Asp Thr Ala Lys Tyr Tyr Cys Ser Arg
   20 25 30
<210> 284
   <211> 32
   <212> PRT
   <213> Artificial Sequence
<220>
   <223> Description of Artificial Sequence: Synthetic polypeptide
<400> 284
   Arg Phe Thr Ile Ser Ala Asp Thr Ser Lys Asn Thr Ala Tyr Leu Gln
   1 5 10 15
   Met Asn Ser Leu Arg Ala Glu Asp Thr Ala Ser Tyr Tyr Cys Ser Arg
   20 25 30
<210> 285
   <211> 32
   <212> PRT
   <213> Artificial Sequence
<220>
   <223> Description of Artificial Sequence: Synthetic polypeptide
<400> 285
   Arg Phe Thr Ile Ser Ala Asp Thr Ser Lys Asn Thr Ala Tyr Leu Gln
   1               5                   10                  15
   Met Asn Ser Leu Arg Ala Glu Asp Thr Ala Trp Tyr Tyr Cys Ser Arg
               20                  25                  30
<210> 286
   <211> 32
   <212> PRT
   <213> Artificial Sequence
<220>
   <223> Description of Artificial Sequence: Synthetic polypeptide
<400> 286
   Arg Phe Thr Ile Ser Ala Asp Thr Ser Lys Asn Thr Ala Tyr Leu Gln
   1               5                   10                  15
   Met Asn Ser Leu Arg Ala Glu Asp Thr Ala Gly Tyr Tyr Cys Ser Arg
               20                  25                  30
<210> 287
   <211> 32
   <212> PRT
   <213> Artificial Sequence
<220>
   <223> Description of Artificial Sequence: Synthetic polypeptide
<400> 287
   Arg Phe Thr Ile Ser Ala Asp Thr Ser Lys Asn Thr Ala Tyr Leu Gln
   1               5                   10                  15
   Met Asn Ser Leu Arg Ala Glu Asp Thr Ala Val Val Tyr Cys Ser Arg
               20                  25                  30
<210> 288
   <211> 32
   <212> PRT
   <213> Artificial Sequence
<220>
   <223> Description of Artificial Sequence: Synthetic polypeptide
<400> 288
   Arg Phe Thr Ile Ser Ala Asp Thr Ser Lys Asn Thr Ala Tyr Leu Gln
   1               5                   10                  15
   Met Asn Ser Leu Arg Ala Glu Asp Thr Ala Val Leu Tyr Cys Ser Arg
               20                  25                  30
<210> 289
   <211> 32
   <212> PRT
   <213> Artificial Sequence
<220>
   <223> Description of Artificial Sequence: Synthetic polypeptide
<400> 289
   Arg Phe Thr Ile Ser Ala Asp Thr Ser Lys Asn Thr Ala Tyr Leu Gln
   1               5                   10                  15
   Met Asn Ser Leu Arg Ala Glu Asp Thr Ala Val Ile Tyr Cys Ser Arg
               20                  25                  30
<210> 290
   <211> 32
   <212> PRT
   <213> Artificial Sequence
<220>
   <223> Description of Artificial Sequence: Synthetic polypeptide
<400> 290
   Arg Phe Thr Ile Ser Ala Asp Thr Ser Lys Asn Thr Ala Tyr Leu Gln
   1               5                   10                  15
   Met Asn Ser Leu Arg Ala Glu Asp Thr Ala Val Tyr Leu Cys Ser Arg
               20                  25                  30
<210> 291
   <211> 32
   <212> PRT
   <213> Artificial Sequence
<220>
   <223> Description of Artificial Sequence: Synthetic polypeptide
<400> 291
   Arg Phe Thr Ile Ser Ala Asp Thr Ser Lys Asn Thr Ala Tyr Leu Gln
   1 5 10 15
   Met Asn Ser Leu Arg Ala Glu Asp Thr Ala Val Tyr Trp Cys Ser Arg
   20 25 30
<210> 292
   <211> 32
   <212> PRT
   <213> Artificial Sequence
<220>
   <223> Description of Artificial Sequence: Synthetic polypeptide
<400> 292
   Arg Phe Thr Ile Ser Ala Asp Thr Ser Lys Asn Thr Ala Tyr Leu Gln
   1 5 10 15
   Met Asn Ser Leu Arg Ala Glu Asp Thr Ala Val Tyr Tyr Gly Ser Arg
   20 25 30
<210> 293
   <211> 23
   <212> PRT
   <213> Artificial Sequence
<220>
   <223> Description of Artificial Sequence: Synthetic peptide
<400> 293
   Asp Ile Gln Met Thr Gln Ser Pro Ser Ser Leu Ser Ala Ser Val Gly
   1 5 10 15
   Asp Arg Val Thr Ile Gly Cys
   20
<210> 294
   <211> 23
   <212> PRT
   <213> Artificial Sequence
<220>
   <223> Description of Artificial Sequence: Synthetic peptide
<400> 294
   Asp Ile Gln Met Thr Gln Ser Pro Ser Ser Leu Ser Ala Ser Val Gly
   1 5 10 15
   Asp Arg Val Thr Ile Ser Cys
   20
<210> 295
   <211> 23
   <212> PRT
   <213> Artificial Sequence
<220>
   <223> Description of Artificial Sequence: Synthetic peptide
<400> 295
   Asp Ile Gln Met Thr Gln Ser Pro Ser Ser Leu Ser Ala Ser Val Gly
   1 5 10 15
   Asp Arg Val Thr Ile Thr Ala
   20
<210> 296
   <211> 23
   <212> PRT
   <213> Artificial Sequence
<220>
   <223> Description of Artificial Sequence: Synthetic peptide
<400> 296
   Asp Ile Gln Met Thr Gln Ser Pro Ser Ser Leu Ser Ala Ser Val Gly
   1 5 10 15
   Asp Arg Val Thr Ile Thr Asn
   20
<210> 297
   <211> 15
   <212> PRT
   <213> Artificial Sequence
<220>
   <223> Description of Artificial Sequence: Synthetic peptide
<400> 297
   His Tyr Gln Gln Lys Pro Gly Lys Ala Pro Lys Leu Leu Ile Tyr
   1 5 10 15
<210> 298
   <211> 15
   <212> PRT
   <213> Artificial Sequence
<220>
   <223> Description of Artificial Sequence: Synthetic peptide
<400> 298
   Phe Tyr Gln Gln Lys Pro Gly Lys Ala Pro Lys Leu Leu Ile Tyr
   1               5                   10                  15
<210> 299
   <211> 32
   <212> PRT
   <213> Artificial Sequence
<220>
   <223> Description of Artificial Sequence: Synthetic polypeptide
<400> 299
   Gly Val Pro Ser Arg Phe Ser Gly Ser Lys Ser Gly Thr Asp Phe Thr
   1               5                   10                  15
   Leu Thr Ile Ser Ser Leu Gln Pro Glu Asp Phe Ala Thr Tyr Tyr Cys
               20                  25                  30
<210> 300
   <211> 32
   <212> PRT
   <213> Artificial Sequence
<220>
   <223> Description of Artificial Sequence: Synthetic polypeptide
<400> 300
   Arg Phe Thr Ile Ser Ala Asp Thr Ser Lys Asn Thr Val Tyr Leu Gln
   1               5                   10                  15
   Met Asn Ser Leu Arg Ala Glu Asp Thr Ala Val Tyr Tyr Cys Ser Arg
               20                  25                  30
<210> 301
   <211> 32
   <212> PRT
   <213> Artificial Sequence
<220>
   <223> Description of Artificial Sequence: Synthetic polypeptide
<400> 301
   Arg Phe Thr Ile Ser Ala Asp Thr Ser Lys Asn Thr Ala Tyr Leu Gln
   1               5                   10                  15
   Met Asn Ser Leu Arg Ala Glu Asp Thr Cys Val Tyr Tyr Cys Ser Arg
               20                  25                  30
<210> 302
   <211> 32
   <212> PRT
   <213> Artificial Sequence
<220>
   <223> Description of Artificial Sequence: Synthetic polypeptide
<400> 302
   Arg Phe Thr Ile Ser Ala Asp Thr Ser Lys Asn Thr Ala Tyr Leu Gln
   1               5                   10                  15
   Met Asn Ser Leu Arg Ala Glu Asp Thr Ala Val Tyr Phe Cys Ser Arg
               20                  25                  30
<210> 303
   <211> 32
   <212> PRT
   <213> Artificial Sequence
<220>
   <223> Description of Artificial Sequence: Synthetic polypeptide
<400> 303
   Arg Phe Thr Ile Ser Ala Asp Thr Ser Lys Asn Thr Ala Tyr Leu Gln
   1               5                   10                  15
   Met Asn Ser Leu Arg Ala Glu Asp Thr Ala Val Tyr Tyr Cys Val Arg
               20                  25                  30
<210> 304
   <211> 32
   <212> PRT
   <213> Artificial Sequence
<220>
   <223> Description of Artificial Sequence: Synthetic polypeptide
<400> 304
   Arg Phe Thr Ile Ser Ala Asp Thr Ser Lys Asn Thr Ala Tyr Leu Gln
   1               5                   10                  15
   Met Asn Ser Leu Arg Ala Glu Asp Thr Ala Val Tyr Tyr Cys Ala Arg
               20                  25                  30
<210> 305
   <211> 32
   <212> PRT
   <213> Artificial Sequence
<220>
   <223> Description of Artificial Sequence: Synthetic polypeptide
<400> 305
   Arg Phe Thr Ile Ser Ala Asp Thr Ser Lys Asn Thr Ala Tyr Leu Gln
   1 5 10 15
   Met Asn Ser Leu Arg Ala Glu Asp Thr Ala Val Tyr Tyr Cys Leu Arg
   20 25 30
<210> 306
   <211> 32
   <212> PRT
   <213> Artificial Sequence
<220>
   <223> Description of Artificial Sequence: Synthetic polypeptide
<400> 306
   Arg Phe Thr Ile Ser Ala Asp Thr Ser Lys Asn Thr Ala Tyr Leu Gln
   1 5 10 15
   Met Asn Ser Leu Arg Ala Glu Asp Thr Ala Val Tyr Tyr Cys Gln Arg
   20 25 30
<210> 307
   <211> 32
   <212> PRT
   <213> Artificial Sequence
<220>
   <223> Description of Artificial Sequence: Synthetic polypeptide
<400> 307
   Arg Phe Thr Ile Ser Ala Asp Thr Ser Lys Asn Thr Ala Tyr Leu Gln
   1 5 10 15
   Met Asn Ser Leu Arg Ala Glu Asp Thr Ala Val Tyr Tyr Cys Thr Arg
   20 25 30
<210> 308
   <211> 32
   <212> PRT
   <213> Artificial Sequence
<220>
   <223> Description of Artificial Sequence: Synthetic polypeptide
<400> 308
   Arg Phe Thr Ile Ser Ala Asp Thr Ser Lys Asn Thr Ala Tyr Leu Gln
   1 5 10 15
   Met Asn Ser Leu Arg Ala Glu Asp Thr Ala Val Tyr Tyr Cys Asn Arg
   20 25 30
<210> 309
   <211> 32
   <212> PRT
   <213> Artificial Sequence
<220>
   <223> Description of Artificial Sequence: Synthetic polypeptide
<400> 309
   Arg Phe Thr Ile Ser Ala Asp Thr Ser Lys Asn Thr Ala Tyr Leu Gln
   1               5                   10                  15
   Met Asn Ser Leu Arg Ala Glu Asp Thr Ala Val Tyr Tyr Cys Asp Arg
               20                  25                  30
<210> 310
   <211> 32
   <212> PRT
   <213> Artificial Sequence
<220>
   <223> Description of Artificial Sequence: Synthetic polypeptide
<400> 310
   Arg Phe Thr Ile Ser Ala Asp Thr Ser Lys Asn Thr Ala Tyr Leu Gln
   1               5                   10                  15
   Met Asn Ser Leu Arg Ala Glu Asp Thr Ala Val Tyr Tyr Cys Ala Arg
               20                  25                  30
<210> 311
   <211> 32
   <212> PRT
   <213> Artificial Sequence
<220>
   <223> Description of Artificial Sequence: Synthetic polypeptide
<400> 311
   Arg Phe Thr Ile Ser Ala Asp Thr Ser Lys Asn Thr Ala Tyr Leu Gln
   1               5                   10                  15
   Met Asn Ser Leu Arg Ala Glu Asp Thr Ala Val Tyr Tyr Cys Val Arg
               20                  25                  30
<210> 312
   <211> 32
   <212> PRT
   <213> Artificial Sequence
<220>
   <223> Description of Artificial Sequence: Synthetic polypeptide
<400> 312
   Arg Phe Thr Ile Ser Ala Asp Thr Ser Lys Asn Thr Ala Tyr Leu Gln
   1 5 10 15
   Met Asn Ser Leu Arg Ala Glu Asp Thr Ala Val Tyr Tyr Cys Leu Arg
   20 25 30
<210> 313
   <211> 32
   <212> PRT
   <213> Artificial Sequence
<220>
   <223> Description of Artificial Sequence: Synthetic polypeptide
<400> 313
   Arg Phe Thr Ile Ser Ala Asp Thr Ser Lys Asn Thr Ala Tyr Leu Gln
   1 5 10 15
   Met Asn Ser Leu Arg Ala Glu Asp Thr Ala Val Tyr Tyr Cys Asn Arg
   20 25 30
<210> 314
   <211> 32
   <212> PRT
   <213> Artificial Sequence
<220>
   <223> Description of Artificial Sequence: Synthetic polypeptide
<400> 314
   Arg Phe Thr Ile Ser Ala Asp Thr Ser Lys Asn Thr Ala Tyr Leu Gln
   1 5 10 15
   Met Asn Ser Leu Arg Ala Glu Asp Thr Ala Val Tyr Tyr Cys Asp Arg
   20 25 30
<210> 315
   <211> 32
   <212> PRT
   <213> Artificial Sequence
<220>
   <223> Description of Artificial Sequence: Synthetic polypeptide
<400> 315
   Arg Phe Thr Ile Ser Ala Asp Thr Ser Lys Asn Thr Ala Tyr Leu Gln
   1 5 10 15
   Met Asn Ser Leu Arg Ala Glu Asp Thr Ala Val Tyr Tyr Cys Ala Arg
   20 25 30
<210> 316
   <211> 32
   <212> PRT
   <213> Artificial Sequence
<220>
   <223> Description of Artificial Sequence: Synthetic polypeptide
<400> 316
   Arg Phe Thr Ile Ser Ala Asp Thr Ser Lys Asn Thr Ala Tyr Leu Gln
   1 5 10 15
   Met Asn Ser Leu Arg Ala Glu Asp Thr Ala Val Tyr Tyr Cys Val Arg
   20 25 30
<210> 317
   <211> 32
   <212> PRT
   <213> Artificial Sequence
<220>
   <223> Description of Artificial Sequence: Synthetic polypeptide
<400> 317
   Arg Phe Thr Ile Ser Ala Asp Thr Ser Lys Asn Thr Ala Tyr Leu Gln
   1 5 10 15
   Met Asn Ser Leu Arg Ala Glu Asp Thr Ala Val Tyr Tyr Cys Leu Arg
   20 25 30
<210> 318
   <211> 11
   <212> PRT
   <213> Artificial Sequence
<220>
   <223> Description of Artificial Sequence: Synthetic peptide
<400> 318
   Arg Ala Ser Ser Asp Val Asn Thr Ala Val Gly
   1 5 10
<210> 319
   <211> 11
   <212> PRT
   <213> Artificial Sequence
<220>
   <223> Description of Artificial Sequence: Synthetic peptide
<400> 319
   Arg Ala Ser Lys Asp Val Asn Thr Ala Val Gly
   1               5                   10
<210> 320
   <211> 11
   <212> PRT
   <213> Artificial Sequence
<220>
   <223> Description of Artificial Sequence: Synthetic peptide
<400> 320
   Arg Ala Ser Val Asp Val Asn Thr Ala Val Gly
   1               5                   10
<210> 321
   <211> 11
   <212> PRT
   <213> Artificial Sequence
<220>
   <223> Description of Artificial Sequence: Synthetic peptide
<400> 321
   Arg Ala Ser Leu Asp Val Asn Thr Ala Val Gly
   1               5                   10
<210> 322
   <211> 11
   <212> PRT
   <213> Artificial Sequence
<220>
   <223> Description of Artificial Sequence: Synthetic peptide
<400> 322
   Arg Ala Ser Phe Asp Val Asn Thr Ala Val Gly
   1               5                   10
<210> 323
   <211> 11
   <212> PRT
   <213> Artificial Sequence
<220>
   <223> Description of Artificial Sequence: Synthetic peptide
<400> 323
   Arg Ala Ser Ile Asp Val Asn Thr Ala Val Gly
   1 5 10
<210> 324
   <211> 11
   <212> PRT
   <213> Artificial Sequence
<220>
   <223> Description of Artificial Sequence: Synthetic peptide
<400> 324
   Arg Ala Ser Tyr Asp Val Asn Thr Ala Val Gly
   1 5 10
<210> 325
   <211> 11
   <212> PRT
   <213> Artificial Sequence
<220>
   <223> Description of Artificial Sequence: Synthetic peptide
<400> 325
   Arg Ala Ser Arg Asp Val Asn Thr Ala Val Gly
   1 5 10
<210> 326
   <211> 11
   <212> PRT
   <213> Artificial Sequence
<220>
   <223> Description of Artificial Sequence: Synthetic peptide
<400> 326
   Arg Ala Ser Ser Asp Val Asn Thr Ala Val Asp
   1 5 10
<210> 327
   <211> 11
   <212> PRT
   <213> Artificial Sequence
<220>
   <223> Description of Artificial Sequence: Synthetic peptide
<400> 327
   Arg Ala Ser Lys Asp Val Asn Thr Ala Val Asp
   1 5 10
<210> 328
   <211> 11
   <212> PRT
   <213> Artificial Sequence
<220>
   <223> Description of Artificial Sequence: Synthetic peptide
<400> 328
   Arg Ala Ser Val Asp Val Asn Thr Ala Val Asp
   1               5                   10
<210> 329
   <211> 11
   <212> PRT
   <213> Artificial Sequence
<220>
   <223> Description of Artificial Sequence: Synthetic peptide
<400> 329
   Arg Ala Ser Leu Asp Val Asn Thr Ala Val Asp
   1               5                   10
<210> 330
   <211> 11
   <212> PRT
   <213> Artificial Sequence
<220>
   <223> Description of Artificial Sequence: Synthetic peptide
<400> 330
   Arg Ala Ser Phe Asp Val Asn Thr Ala Val Asp
   1               5                   10
<210> 331
   <211> 11
   <212> PRT
   <213> Artificial Sequence
<220>
   <223> Description of Artificial Sequence: Synthetic peptide
<400> 331
   Arg Ala Ser Ile Asp Val Asn Thr Ala Val Asp
   1               5                   10
<210> 332
   <211> 11
   <212> PRT
   <213> Artificial Sequence
<220>
   <223> Description of Artificial Sequence: Synthetic peptide
<400> 332
   Arg Ala Ser Tyr Asp Val Asn Thr Ala Val Asp
   1               5                   10
<210> 333
   <211> 11
   <212> PRT
   <213> Artificial Sequence
<220>
   <223> Description of Artificial Sequence: Synthetic peptide
<400> 333
   Arg Ala Ser Arg Asp Val Asn Thr Ala Val Asp
   1               5                   10
<210> 334
   <211> 11
   <212> PRT
   <213> Artificial Sequence
<220>
   <223> Description of Artificial Sequence: Synthetic peptide
<400> 334
   Arg Ala Ser Ser Asp Val Asn Thr Ala Val Val
   1               5                   10
<210> 335
   <211> 11
   <212> PRT
   <213> Artificial Sequence
<220>
   <223> Description of Artificial Sequence: Synthetic peptide
<400> 335
   Arg Ala Ser Lys Asp Val Asn Thr Ala Val Val
   1               5                   10
<210> 336
   <211> 11
   <212> PRT
   <213> Artificial Sequence
<220>
   <223> Description of Artificial Sequence: Synthetic peptide
<400> 336
   Arg Ala Ser Val Asp Val Asn Thr Ala Val Val
   1 5 10
<210> 337
   <211> 11
   <212> PRT
   <213> Artificial Sequence
<220>
   <223> Description of Artificial Sequence: Synthetic peptide
<400> 337
   Arg Ala Ser Leu Asp Val Asn Thr Ala Val Val
   1 5 10
<210> 338
   <211> 11
   <212> PRT
   <213> Artificial Sequence
<220>
   <223> Description of Artificial Sequence: Synthetic peptide
<400> 338
   Arg Ala Ser Phe Asp Val Asn Thr Ala Val Val
   1 5 10
<210> 339
   <211> 11
   <212> PRT
   <213> Artificial Sequence
<220>
   <223> Description of Artificial Sequence: Synthetic peptide
<400> 339
   Arg Ala Ser Ile Asp Val Asn Thr Ala Val Val
   1 5 10
<210> 340
   <211> 11
   <212> PRT
   <213> Artificial Sequence
<220>
   <223> Description of Artificial Sequence: Synthetic peptide
<400> 340
   Arg Ala Ser Tyr Asp Val Asn Thr Ala Val Val
   1 5 10
<210> 341
   <211> 11
   <212> PRT
   <213> Artificial Sequence
<220>
   <223> Description of Artificial Sequence: Synthetic peptide
<400> 341
   Arg Ala Ser Arg Asp Val Asn Thr Ala Val Val
   1               5                   10
<210> 342
   <211> 11
   <212> PRT
   <213> Artificial Sequence
<220>
   <223> Description of Artificial Sequence: Synthetic peptide
<400> 342
   Arg Ala Ser Gln Gly Val Asn Thr Ala Val Gly
   1               5                   10
<210> 343
   <211> 11
   <212> PRT
   <213> Artificial Sequence
<220>
   <223> Description of Artificial Sequence: Synthetic peptide
<400> 343
   Arg Ala Ser Gln Ala Val Asn Thr Ala Val Gly
   1               5                   10
<210> 344
   <211> 11
   <212> PRT
   <213> Artificial Sequence
<220>
   <223> Description of Artificial Sequence: Synthetic peptide
<400> 344
   Arg Ala Ser Gln Asn Val Asn Thr Ala Val Gly
   1               5                   10
<210> 345
   <211> 11
   <212> PRT
   <213> Artificial Sequence
<220>
   <223> Description of Artificial Sequence: Synthetic peptide
<400> 345
   Arg Ala Ser Gln Leu Val Asn Thr Ala Val Gly
   1 5 10
<210> 346
   <211> 11
   <212> PRT
   <213> Artificial Sequence
<220>
   <223> Description of Artificial Sequence: Synthetic peptide
<400> 346
   Arg Ala Ser Gln Gly Val Asn Thr Ala Val Asp
   1 5 10
<210> 347
   <211> 11
   <212> PRT
   <213> Artificial Sequence
<220>
   <223> Description of Artificial Sequence: Synthetic peptide
<400> 347
   Arg Ala Ser Gln Ala Val Asn Thr Ala Val Asp
   1 5 10
<210> 348
   <211> 11
   <212> PRT
   <213> Artificial Sequence
<220>
   <223> Description of Artificial Sequence: Synthetic peptide
<400> 348
   Arg Ala Ser Gln Asn Val Asn Thr Ala Val Asp
   1 5 10
<210> 349
   <211> 11
   <212> PRT
   <213> Artificial Sequence
<220>
   <223> Description of Artificial Sequence: Synthetic peptide
<400> 349
   Arg Ala Ser Gln Leu Val Asn Thr Ala Val Asp
   1 5 10
<210> 350
   <211> 11
   <212> PRT
   <213> Artificial Sequence
<220>
   <223> Description of Artificial Sequence: Synthetic peptide
<400> 350
   Arg Ala Ser Gln Gly Val Asn Thr Ala Val Val
   1               5                   10
<210> 351
   <211> 11
   <212> PRT
   <213> Artificial Sequence
<220>
   <223> Description of Artificial Sequence: Synthetic peptide
<400> 351
   Arg Ala Ser Gln Ala Val Asn Thr Ala Val Val
   1               5                   10
<210> 352
   <211> 11
   <212> PRT
   <213> Artificial Sequence
<220>
   <223> Description of Artificial Sequence: Synthetic peptide
<400> 352
   Arg Ala Ser Gln Asn Val Asn Thr Ala Val Val
   1               5                   10
<210> 353
   <211> 11
   <212> PRT
   <213> Artificial Sequence
<220>
   <223> Description of Artificial Sequence: Synthetic peptide
<400> 353
   Arg Ala Ser Gln Leu Val Asn Thr Ala Val Val
   1               5                   10
<210> 354
   <211> 16
   <212> PRT
   <213> Artificial Sequence
<220>
   <223> Description of Artificial Sequence: Synthetic peptide
<400> 354
   Arg Ile Tyr Pro Thr Asn Gly Tyr Thr Arg Tyr Asp Pro Lys Phe Gln
   1               5                   10                  15
<210> 355
   <211> 17
   <212> PRT
   <213> Artificial Sequence
<220>
   <223> Description of Artificial Sequence: Synthetic peptide
<400> 355
   Arg Ile Tyr Pro Thr Asn Gly Tyr Thr Arg Tyr Asp Pro Lys Phe Gln
   1               5                   10                  15
   Asp
<210> 356
   <211> 11
   <212> PRT
   <213> Artificial Sequence
<220>
   <223> Description of Artificial Sequence: Synthetic peptide
<400> 356
   Trp Gly Gly Lys Gly Phe Tyr Ala Met Asp Tyr
   1               5                   10
<210> 357
   <211> 11
   <212> PRT
   <213> Artificial Sequence
<220>
   <223> Description of Artificial Sequence: Synthetic peptide
<400> 357
   Trp Gly Gly Asp Gly Phe Tyr Ala Met Asp Val
   1               5                   10
<210> 358
   <211> 11
   <212> PRT
   <213> Artificial Sequence
<220>
   <223> Description of Artificial Sequence: Synthetic peptide
<400> 358
   Trp Gly Gly Asp Gly Pro Tyr Ala Met Asp Lys
   1 5 10
<210> 359
   <211> 11
   <212> PRT
   <213> Artificial Sequence
<220>
   <223> Description of Artificial Sequence: Synthetic peptide
<400> 359
   Trp Gly Gly Asp Gly Pro Tyr Ala Met Asp Leu
   1 5 10
<210> 360
   <211> 11
   <212> PRT
   <213> Artificial Sequence
<220>
   <223> Description of Artificial Sequence: Synthetic peptide
<400> 360
   Arg Ala Ser Gln Asp Val Asp Thr Ala Val Ala
   1 5 10
<210> 361
   <211> 11
   <212> PRT
   <213> Artificial Sequence
<220>
   <223> Description of Artificial Sequence: Synthetic peptide
<400> 361
   Arg Ala Ser Lys Asp Val Asn Thr Ala Val Ala
   1 5 10
<210> 362
   <211> 11
   <212> PRT
   <213> Artificial Sequence
<220>
   <223> Description of Artificial Sequence: Synthetic peptide
<400> 362
   Arg Ala Ser Gln Lys Val Asn Thr Ala Val Ala
   1 5 10
<210> 363
   <211> 11
   <212> PRT
   <213> Artificial Sequence
<220>
   <223> Description of Artificial Sequence: Synthetic peptide
<400> 363
   Arg Ala Ser Gln Asp Val Ser Thr Ala Val Ala
   1 5 10
<210> 364
   <211> 11
   <212> PRT
   <213> Artificial Sequence
<220>
   <223> Description of Artificial Sequence: Synthetic peptide
<400> 364
   Lys Ala Ser Gln Asp Val Asn Thr Ala Val Ala
   1 5 10
<210> 365
   <211> 7
   <212> PRT
   <213> Artificial Sequence
<220>
   <223> Description of Artificial Sequence: Synthetic peptide
<400> 365
   Ser Ala Lys Phe Leu Tyr Ser
   1 5
<210> 366
   <211> 7
   <212> PRT
   <213> Artificial Sequence
<220>
   <223> Description of Artificial Sequence: Synthetic peptide
<400> 366
   Ser Ala Ser Phe Leu Tyr Lys
   1 5
<210> 367
   <211> 7
   <212> PRT
   <213> Artificial Sequence
<220>
   <223> Description of Artificial Sequence: Synthetic peptide
<400> 367
   Ser Ala Ser Phe Leu Glu Ser
   1               5
<210> 368
   <211> 7
   <212> PRT
   <213> Artificial Sequence
<220>
   <223> Description of Artificial Sequence: Synthetic peptide
<400> 368
   Ser Ala Ser Phe Arg Tyr Thr
   1               5
<210> 369
   <211> 7
   <212> PRT
   <213> Artificial Sequence
<220>
   <223> Description of Artificial Sequence: Synthetic peptide
<400> 369
   Ser Ala Ser Trp Leu Trp Ser
   1               5
<210> 370
   <211> 9
   <212> PRT
   <213> Artificial Sequence
<220>
   <223> Description of Artificial Sequence: Synthetic peptide
<400> 370
   Gln Gln Ala Tyr Thr Thr Pro Pro Thr
   1               5
<210> 371
   <211> 9
   <212> PRT
   <213> Artificial Sequence
<220>
   <223> Description of Artificial Sequence: Synthetic peptide
<400> 371
   Gln Gln His Ala Thr Thr Pro Pro Thr
   1 5
<210> 372
   <211> 9
   <212> PRT
   <213> Artificial Sequence
<220>
   <223> Description of Artificial Sequence: Synthetic peptide
<400> 372
   Gln Gln Ser Tyr Thr Thr Pro Pro Thr
   1 5
<210> 373
   <211> 9
   <212> PRT
   <213> Artificial Sequence
<220>
   <223> Description of Artificial Sequence: Synthetic peptide
<400> 373
   Gln Gln Tyr Tyr Thr Thr Pro Pro Leu
   1 5
<210> 374
   <211> 9
   <212> PRT
   <213> Artificial Sequence
<220>
   <223> Description of Artificial Sequence: Synthetic peptide
<400> 374
   Gln Gln Phe Trp Thr Thr Pro Pro Thr
   1 5
<210> 375
   <211> 10
   <212> PRT
   <213> Artificial Sequence
<220>
   <223> Description of Artificial Sequence: Synthetic peptide
<400> 375
   Gly Phe Tyr Ile Ser Ser Ser Tyr Ile His
   1 5 10
<210> 376
   <211> 10
   <212> PRT
   <213> Artificial Sequence
<220>
   <223> Description of Artificial Sequence: Synthetic peptide
<400> 376
   Gly Phe Ser Ile Ser Ser Ser Tyr Ile His
   1               5                   10
<210> 377
   <211> 10
   <212> PRT
   <213> Artificial Sequence
<220>
   <223> Description of Artificial Sequence: Synthetic peptide
<400> 377
   Gly Phe Ser Ile Ser Tyr Ser Tyr Ile His
   1               5                   10
<210> 378
   <211> 10
   <212> PRT
   <213> Artificial Sequence
<220>
   <223> Description of Artificial Sequence: Synthetic peptide
<400> 378
   Gly Phe Ser Ile Tyr Ser Ser Tyr Ile His
   1               5                   10
<210> 379
   <211> 10
   <212> PRT
   <213> Artificial Sequence
<220>
   <223> Description of Artificial Sequence: Synthetic peptide
<400> 379
   Gly Phe Ser Ile Tyr Tyr Ser Tyr Ile His
   1               5                   10
<210> 380
   <211> 10
   <212> PRT
   <213> Artificial Sequence
<220>
   <223> Description of Artificial Sequence: Synthetic peptide
<400> 380
   Gly Phe Ser Ile Tyr Tyr Ser Tyr Ile His
   1 5 10
<210> 381
   <211> 10
   <212> PRT
   <213> Artificial Sequence
<220>
   <223> Description of Artificial Sequence: Synthetic peptide
<400> 381
   Gly Phe Tyr Ile Tyr Ser Ser Tyr Ile His
   1 5 10
<210> 382
   <211> 17
   <212> PRT
   <213> Artificial Sequence
<220>
   <223> Description of Artificial Sequence: Synthetic peptide
<400> 382
   Ser Ile Tyr Pro Tyr Ser Gly Tyr Thr Arg Tyr Ala Asp Ser Val Lys
   1 5 10 15
   Gly
<210> 383
   <211> 17
   <212> PRT
   <213> Artificial Sequence
<220>
   <223> Description of Artificial Sequence: Synthetic peptide
<400> 383
   Ser Ile Tyr Pro Ser Ser Gly Tyr Thr Arg Tyr Ala Asp Ser Val Lys
   1 5 10 15
   Gly
<210> 384
   <211> 8
   <212> PRT
   <213> Artificial Sequence
<220>
   <223> Description of Artificial Sequence: Synthetic peptide
<400> 384
   Tyr Tyr Ser Tyr Ala Phe Asp Tyr
   1 5
<210> 385
   <211> 8
   <212> PRT
   <213> Artificial Sequence
<220>
   <223> Description of Artificial Sequence: Synthetic peptide
<400> 385
   Tyr Tyr Ser Ser Gly Phe Asp Tyr
   1 5
<210> 386
   <211> 8
   <212> PRT
   <213> Artificial Sequence
<220>
   <223> Description of Artificial Sequence: Synthetic peptide
<400> 386
   Tyr Tyr Ser Tyr Ala Leu Asp Tyr
   1 5
<210> 387
   <211> 8
   <212> PRT
   <213> Artificial Sequence
<220>
   <223> Description of Artificial Sequence: Synthetic peptide
<400> 387
   Trp Trp Ser Ser Ala Phe Asp Tyr
   1 5
<210> 388
   <211> 8
   <212> PRT
   <213> Artificial Sequence
<220>
   <223> Description of Artificial Sequence: Synthetic peptide
<400> 388
   Trp Trp Ser Ser Ala Met Asp Tyr
   1 5
<210> 389
   <211> 8
   <212> PRT
   <213> Artificial Sequence
<220>
   <223> Description of Artificial Sequence: Synthetic peptide
<400> 389
   Trp Trp Ser Trp Gly Phe Asp Tyr
   1 5
<210> 390
   <211> 8
   <212> PRT
   <213> Artificial Sequence
<220>
   <223> Description of Artificial Sequence: Synthetic peptide
<400> 390
   Trp Trp Ser Ser Ala Phe Asp Tyr
   1 5
<210> 391
   <211> 8
   <212> PRT
   <213> Artificial Sequence
<220>
   <223> Description of Artificial Sequence: Synthetic peptide
<400> 391
   Trp Trp Ser Ser Gly Phe Asp Tyr
   1 5
<210> 392
   <211> 8
   <212> PRT
   <213> Artificial Sequence
<220>
   <223> Description of Artificial Sequence: Synthetic peptide
<400> 392
   Trp Trp Ser Trp Ala Phe Asp Tyr
   1 5
<210> 393
   <211> 8
   <212> PRT
   <213> Artificial Sequence
<220>
   <223> Description of Artificial Sequence: Synthetic peptide
<400> 393
   Trp Trp Ser Ser Ala Phe Asp Tyr
   1 5
<210> 394
   <211> 8
   <212> PRT
   <213> Artificial Sequence
<220>
   <223> Description of Artificial Sequence: Synthetic peptide
<400> 394
   Trp Trp Ser Ser Ala Leu Asp Tyr
   1 5
<210> 395
   <211> 10
   <212> PRT
   <213> Artificial Sequence
<220>
   <223> Description of Artificial Sequence: Synthetic peptide
<400> 395
   Gly Phe Ser Ile Tyr Ser Ser Tyr Ile His
   1 5 10
<210> 396
   <211> 10
   <212> PRT
   <213> Artificial Sequence
<220>
   <223> Description of Artificial Sequence: Synthetic peptide
<400> 396
   Gly Phe Ser Ile Ser Ser Ser Tyr Ile His
   1 5 10
<210> 397
   <211> 10
   <212> PRT
   <213> Artificial Sequence
<220>
   <223> Description of Artificial Sequence: Synthetic peptide
<400> 397
   Gly Phe Tyr Ile Ser Tyr Ser Tyr Ile His
   1 5 10
<210> 398
   <211> 10
   <212> PRT
   <213> Artificial Sequence
<220>
   <223> Description of Artificial Sequence: Synthetic peptide
<400> 398
   Gly Phe Ser Ile Ser Tyr Ser Tyr Ile His
   1               5                   10
<210> 399
   <211> 10
   <212> PRT
   <213> Artificial Sequence
<220>
   <223> Description of Artificial Sequence: Synthetic peptide
<400> 399
   Gly Phe Ser Ile Ser Tyr Ser Ser Ile His
   1               5                   10
<210> 400
   <211> 10
   <212> PRT
   <213> Artificial Sequence
<220>
   <223> Description of Artificial Sequence: Synthetic peptide
<400> 400
   Gly Phe Ser Ile Ser Ser Ser Ser Ile His
   1               5                   10
<210> 401
   <211> 10
   <212> PRT
   <213> Artificial Sequence
<220>
   <223> Description of Artificial Sequence: Synthetic peptide
<400> 401
   Gly Phe Ser Ile Trp Trp Ser Trp Ile His
   1               5                   10
<210> 402
   <211> 10
   <212> PRT
   <213> Artificial Sequence
<220>
   <223> Description of Artificial Sequence: Synthetic peptide
<400> 402
   Gly Phe Ser Ile Trp Ser Ser Trp Ile His
   1 5 10
<210> 403
   <211> 10
   <212> PRT
   <213> Artificial Sequence
<220>
   <223> Description of Artificial Sequence: Synthetic peptide
<400> 403
   Gly Phe Ser Ile Ser Trp Ser Trp Ile His
   1 5 10
<210> 404
   <211> 10
   <212> PRT
   <213> Artificial Sequence
<220>
   <223> Description of Artificial Sequence: Synthetic peptide
<400> 404
   Gly Phe Trp Ile Trp Ser Ser Ser Ile His
   1 5 10
<210> 405
   <211> 10
   <212> PRT
   <213> Artificial Sequence
<220>
   <223> Description of Artificial Sequence: Synthetic peptide
<400> 405
   Gly Phe Ser Ile Ser Ser Ser Trp Ile His
   1 5 10
<210> 406
   <211> 17
   <212> PRT
   <213> Artificial Sequence
<220>
   <223> Description of Artificial Sequence: Synthetic peptide
<400> 406
   Ser Ile Tyr Pro Tyr Ser Gly Tyr Thr Arg Tyr Ala Asp Ser Val Lys
   1 5 10 15
   Gly
<210> 407
   <211> 17
   <212> PRT
   <213> Artificial Sequence
<220>
   <223> Description of Artificial Sequence: Synthetic peptide
<400> 407
   Ser Ile Tyr Pro Tyr Tyr Gly Tyr Thr Arg Tyr Ala Asp Ser Val Lys
   1               5                   10                  15
   Gly
<210> 408
   <211> 17
   <212> PRT
   <213> Artificial Sequence
<220>
   <223> Description of Artificial Sequence: Synthetic peptide
<400> 408
   Tyr Ile Ser Pro Ser Ser Gly Tyr Thr Arg Tyr Ala Asp Ser Val Lys
   1               5                   10                  15
   Gly
<210> 409
   <211> 17
   <212> PRT
   <213> Artificial Sequence
<220>
   <223> Description of Artificial Sequence: Synthetic peptide
<400> 409
   Ser Ile Ser Pro Ser Ser Gly Trp Thr Ser Tyr Ala Asp Ser Val Lys
   1               5                   10                  15
   Gly
<210> 410
   <211> 17
   <212> PRT
   <213> Artificial Sequence
<220>
   <223> Description of Artificial Sequence: Synthetic peptide
<400> 410
   Ser Ile Tyr Pro Ser Ser Gly Tyr Thr Ser Tyr Ala Asp Ser Val Lys
   1 5 10 15
   Gly
<210> 411
   <211> 17
   <212> PRT
   <213> Artificial Sequence
<220>
   <223> Description of Artificial Sequence: Synthetic peptide
<400> 411
   Ser Ile Ser Pro Ser Ser Gly Trp Thr Ser Tyr Ala Asp Ser Val Lys
   1 5 10 15
   Gly
<210> 412
   <211> 17
   <212> PRT
   <213> Artificial Sequence
<220>
   <223> Description of Artificial Sequence: Synthetic peptide
<400> 412
   Ser Ile Trp Pro Ser Ser Gly Trp Thr Ser Tyr Ala Asp Ser Val Lys
   1 5 10 15
   Gly
<210> 413
   <211> 17
   <212> PRT
   <213> Artificial Sequence
<220>
   <223> Description of Artificial Sequence: Synthetic peptide
<400> 413
   Ser Ile Ser Pro Ser Ser Gly Trp Thr Ser Tyr Ala Asp Ser Val Lys
   1 5 10 15
   Gly
<210> 414
   <211> 17
   <212> PRT
   <213> Artificial Sequence
<220>
   <223> Description of Artificial Sequence: Synthetic peptide
<400> 414
   Trp Ile Ser Pro Ser Ser Gly Trp Thr Ser Tyr Ala Asp Ser Val Lys
   1               5                   10                  15
   Gly
<210> 415
   <211> 17
   <212> PRT
   <213> Artificial Sequence
<220>
   <223> Description of Artificial Sequence: Synthetic peptide
<400> 415
   Trp Ile Ser Pro Ser Ser Gly Ser Thr Trp Tyr Ala Asp Ser Val Lys
   1               5                   10                  15
   Gly
<210> 416
   <211> 17
   <212> PRT
   <213> Artificial Sequence
<220>
   <223> Description of Artificial Sequence: Synthetic peptide
<400> 416
   Trp Ile Ser Pro Ser Ser Gly Ser Thr Trp Tyr Ala Asp Ser Val Lys
   1               5                   10                  15
   Gly
<210> 417
   <211> 17
   <212> PRT
   <213> Artificial Sequence
<220>
   <223> Description of Artificial Sequence: Synthetic peptide
<400> 417
   Trp Ile Trp Pro Ser Ser Gly Trp Thr Ser Tyr Ala Asp Ser Val Lys
   1 5 10 15
   Gly
<210> 418
   <211> 17
   <212> PRT
   <213> Artificial Sequence
<220>
   <223> Description of Artificial Sequence: Synthetic peptide
<400> 418
   Trp Ile Ser Pro Ser Trp Gly Ser Thr Trp Tyr Ala Asp Ser Val Lys
   1 5 10 15
   Gly
<210> 419
   <211> 17
   <212> PRT
   <213> Artificial Sequence
<220>
   <223> Description of Artificial Sequence: Synthetic peptide
<400> 419
   Trp Ile Ser Pro Ser Ser Gly Ser Thr Trp Tyr Ala Asp Ser Val Lys
   1 5 10 15
   Gly
<210> 420
   <211> 8
   <212> PRT
   <213> Artificial Sequence
<220>
   <223> Description of Artificial Sequence: Synthetic peptide
<400> 420
   Trp Ser Ser Ser Gly Phe Asp Tyr
   1 5
<210> 421
   <211> 8
   <212> PRT
   <213> Artificial Sequence
<220>
   <223> Description of Artificial Sequence: Synthetic peptide
<400> 421
   Trp Trp Ser Ser Ala Met Asp Tyr
   1               5
<210> 422
   <211> 8
   <212> PRT
   <213> Artificial Sequence
<220>
   <223> Description of Artificial Sequence: Synthetic peptide
<400> 422
   Trp Trp Ser Ser Ala Leu Asp Tyr
   1               5
<210> 423
   <211> 12
   <212> PRT
   <213> Artificial Sequence
<220>
   <223> Description of Artificial Sequence: Synthetic peptide
<400> 423
   Phe Ser Phe Ser Ser Ser Ser Phe Ala Met Asp Tyr
   1               5                   10
<210> 424
   <211> 12
   <212> PRT
   <213> Artificial Sequence
<220>
   <223> Description of Artificial Sequence: Synthetic peptide
<400> 424
   Phe Ser Phe Ser Ser Ser Ser Phe Ala Ile Asp Tyr
   1               5                   10
<210> 425
   <211> 13
   <212> PRT
   <213> Artificial Sequence
<220>
   <223> Description of Artificial Sequence: Synthetic peptide
<400> 425
   Phe Phe Phe Phe Ser Ser Ser Phe Phe Gly Phe Asp Tyr
   1               5                   10
<210> 426
   <211> 15
   <212> PRT
   <213> Artificial Sequence
<220>
   <223> Description of Artificial Sequence: Synthetic peptide
<400> 426
   Tyr Tyr Ser Ser Tyr Tyr Ser Tyr Tyr Ser Phe Gly Leu Asp Tyr
   1               5                   10                  15
<210> 427
   <211> 8
   <212> PRT
   <213> Artificial Sequence
<220>
   <223> Description of Artificial Sequence: Synthetic peptide
<400> 427
   Trp Trp Ser Ser Ala Ile Asp Tyr
   1               5
<210> 428
   <211> 8
   <212> PRT
   <213> Artificial Sequence
<220>
   <223> Description of Artificial Sequence: Synthetic peptide
<400> 428
   Trp Trp Ser Ser Ala Leu Asp Tyr
   1               5
<210> 429
   <211> 8
   <212> PRT
   <213> Artificial Sequence
<220>
   <223> Description of Artificial Sequence: Synthetic peptide
<400> 429
   Trp Trp Ser Ser Gly Met Asp Tyr
   1               5
<210> 430
   <211> 8
   <212> PRT
   <213> Artificial Sequence
<220>
   <223> Description of Artificial Sequence: Synthetic peptide
<400> 430
   Trp Trp Ser Ser Ala Phe Asp Tyr
   1               5
<210> 431
   <211> 8
   <212> PRT
   <213> Artificial Sequence
<220>
   <223> Description of Artificial Sequence: Synthetic peptide
<400> 431
   Trp Ser Ser Ser Ala Phe Asp Tyr
   1               5
<210> 432
   <211> 8
   <212> PRT
   <213> Artificial Sequence
<220>
   <223> Description of Artificial Sequence: Synthetic peptide
<400> 432
   Trp Trp Ser Ser Ala Met Asp Tyr
   1               5
<210> 433
   <211> 8
   <212> PRT
   <213> Artificial Sequence
<220>
   <223> Description of Artificial Sequence: Synthetic peptide
<400> 433
   Trp Ser Ser Trp Gly Leu Asp Tyr
   1               5
<210> 434
   <211> 12
   <212> PRT
   <213> Artificial Sequence
<220>
   <223> Description of Artificial Sequence: Synthetic peptide
<400> 434
   Ser Ser Trp Ser Ser Trp Ser Ser Ala Leu Asp Tyr
   1               5                   10
<210> 435
   <211> 12
   <212> PRT
   <213> Artificial Sequence
<220>
   <223> Description of Artificial Sequence: Synthetic peptide
<400> 435
   Ser Ser Ser Ser Ser Trp Trp Ser Ala Ile Asp Tyr
   1 5 10
<210> 436
   <211> 12
   <212> PRT
   <213> Artificial Sequence
<220>
   <223> Description of Artificial Sequence: Synthetic peptide
<400> 436
   Ser Ser Trp Ser Ser Trp Ser Trp Ala Leu Asp Tyr
   1 5 10
<210> 437
   <211> 12
   <212> PRT
   <213> Artificial Sequence
<220>
   <223> Description of Artificial Sequence: Synthetic peptide
<400> 437
   Ser Ser Trp Ser Ser Trp Ser Ser Ala Met Asp Tyr
   1 5 10
<210> 438
   <211> 10
   <212> PRT
   <213> Artificial Sequence
<220>
   <223> Description of Artificial Sequence: Synthetic peptide
<400> 438
   Gly Phe Ser Ile Trp Ser Ser Ser Ile His
   1 5 10
<210> 439
   <211> 10
   <212> PRT
   <213> Artificial Sequence
<220>
   <223> Description of Artificial Sequence: Synthetic peptide
<400> 439
   Gly Phe Ser Ile Tyr Ser Tyr Tyr Ile His
   1               5                   10
<210> 440
   <211> 10
   <212> PRT
   <213> Artificial Sequence
<220>
   <223> Description of Artificial Sequence: Synthetic peptide
<400> 440
   Gly Phe Tyr Ile Ser Tyr Ser Tyr Ile His
   1               5                   10
<210> 441
   <211> 10
   <212> PRT
   <213> Artificial Sequence
<220>
   <223> Description of Artificial Sequence: Synthetic peptide
<400> 441
   Gly Phe Tyr Ile Ser Ser Ser Tyr Ile His
   1               5                   10
<210> 442
   <211> 10
   <212> PRT
   <213> Artificial Sequence
<220>
   <223> Description of Artificial Sequence: Synthetic peptide
<400> 442
   Gly Phe Ser Ile Ser Ser Ser Tyr Ile His
   1               5                   10
<210> 443
   <211> 10
   <212> PRT
   <213> Artificial Sequence
<220>
   <223> Description of Artificial Sequence: Synthetic peptide
<400> 443
   Gly Phe Ser Ile Tyr Ser Ser Tyr Ile His
   1 5 10
<210> 444
   <211> 10
   <212> PRT
   <213> Artificial Sequence
<220>
   <223> Description of Artificial Sequence: Synthetic peptide
<400> 444
   Gly Phe Tyr Ile Ser Ser Ser Tyr Ile His
   1 5 10
<210> 445
   <211> 10
   <212> PRT
   <213> Artificial Sequence
<220>
   <223> Description of Artificial Sequence: Synthetic peptide
<400> 445
   Gly Phe Tyr Ile Ser Tyr Ser Ser Ile His
   1 5 10
<210> 446
   <211> 10
   <212> PRT
   <213> Artificial Sequence
<220>
   <223> Description of Artificial Sequence: Synthetic peptide
<400> 446
   Gly Phe Tyr Ile Ser Ser Ser Ser Ile His
   1 5 10
<210> 447
   <211> 10
   <212> PRT
   <213> Artificial Sequence
<220>
   <223> Description of Artificial Sequence: Synthetic peptide
<400> 447
   Gly Phe Ser Ile Tyr Tyr Ser Ser Ile His
   1 5 10
<210> 448
   <211> 10
   <212> PRT
   <213> Artificial Sequence
<220>
   <223> Description of Artificial Sequence: Synthetic peptide
<400> 448
   Gly Phe Tyr Ile Ser Ser Ser Ser Ile His
   1               5                   10
<210> 449
   <211> 10
   <212> PRT
   <213> Artificial Sequence
<220>
   <223> Description of Artificial Sequence: Synthetic peptide
<400> 449
   Gly Phe Ser Ile Ser Tyr Ser Ser Ile His
   1               5                   10
<210> 450
   <211> 10
   <212> PRT
   <213> Artificial Sequence
<220>
   <223> Description of Artificial Sequence: Synthetic peptide
<400> 450
   Gly Phe Ser Ile Ser Tyr Ser Tyr Ile His
   1               5                   10
<210> 451
   <211> 10
   <212> PRT
   <213> Artificial Sequence
<220>
   <223> Description of Artificial Sequence: Synthetic peptide
<400> 451
   Gly Phe Ser Ile Lys Ser Ser Tyr Ile His
   1               5                   10
<210> 452
   <211> 10
   <212> PRT
   <213> Artificial Sequence
<220>
   <223> Description of Artificial Sequence: Synthetic peptide
<400> 452
   Gly Phe Ser Ile Lys Tyr Ser Tyr Ile His
   1               5                   10
<210> 453
   <211> 10
   <212> PRT
   <213> Artificial Sequence
<220>
   <223> Description of Artificial Sequence: Synthetic peptide
<400> 453
   Gly Phe Ser Ile Lys Ser Ser Tyr Ile His
   1               5                   10
<210> 454
   <211> 10
   <212> PRT
   <213> Artificial Sequence
<220>
   <223> Description of Artificial Sequence: Synthetic peptide
<400> 454
   Gly Phe Tyr Ile Lys Ser Ser Tyr Ile His
   1               5                   10
<210> 455
   <211> 10
   <212> PRT
   <213> Artificial Sequence
<220>
   <223> Description of Artificial Sequence: Synthetic peptide
<400> 455
   Gly Phe Ser Ile Lys Ser Ser Tyr Ile His
   1               5                   10
<210> 456
   <211> 17
   <212> PRT
   <213> Artificial Sequence
<220>
   <223> Description of Artificial Sequence: Synthetic peptide
<400> 456
   Ser Ile Ser Pro Ser Ser Gly Ser Thr Trp Tyr Ala Asp Ser Val Lys
   1               5                   10                  15
   Gly
<210> 457
   <211> 17
   <212> PRT
   <213> Artificial Sequence
<220>
   <223> Description of Artificial Sequence: Synthetic peptide
<400> 457
   Ser Ile Tyr Pro Ser Ser Gly Tyr Thr Ser Tyr Ala Asp Ser Val Lys
   1 5 10 15
   Gly
<210> 458
   <211> 17
   <212> PRT
   <213> Artificial Sequence
<220>
   <223> Description of Artificial Sequence: Synthetic peptide
<400> 458
   Ser Ile Tyr Pro Tyr Ser Gly Tyr Thr Ser Tyr Ala Asp Ser Val Lys
   1 5 10 15
   Gly
<210> 459
   <211> 17
   <212> PRT
   <213> Artificial Sequence
<220>
   <223> Description of Artificial Sequence: Synthetic peptide
<400> 459
   Ser Ile Ser Pro Tyr Ser Gly Tyr Thr Tyr Tyr Ala Asp Ser Val Lys
   1 5 10 15
   Gly
<210> 460
   <211> 17
   <212> PRT
   <213> Artificial Sequence
<220>
   <223> Description of Artificial Sequence: Synthetic peptide
<400> 460
   Tyr Ile Ser Pro Ser Ser Gly Tyr Thr Tyr Tyr Ala Asp Ser Val Lys
   1 5 10 15
   Gly
<210> 461
   <211> 17
   <212> PRT
   <213> Artificial Sequence
<220>
   <223> Description of Artificial Sequence: Synthetic peptide
<400> 461
   Ser Ile Tyr Pro Tyr Ser Gly Ser Thr Tyr Tyr Ala Asp Ser Val Lys
   1 5 10 15
   Gly
<210> 462
   <211> 17
   <212> PRT
   <213> Artificial Sequence
<220>
   <223> Description of Artificial Sequence: Synthetic peptide
<400> 462
   Ser Ile Tyr Pro Ser Ser Gly Ser Thr Tyr Tyr Ala Asp Ser Val Lys
   1 5 10 15
   Gly
<210> 463
   <211> 17
   <212> PRT
   <213> Artificial Sequence
<220>
   <223> Description of Artificial Sequence: Synthetic peptide
<400> 463
   Tyr Ile Tyr Pro Ser Ser Gly Tyr Thr Arg Tyr Ala Asp Ser Val Lys
   1 5 10 15
   Gly
<210> 464
   <211> 17
   <212> PRT
   <213> Artificial Sequence
<220>
   <223> Description of Artificial Sequence: Synthetic peptide
<400> 464
   Ser Ile Tyr Pro Tyr Tyr Gly Tyr Thr Arg Tyr Ala Asp Ser Val Lys
   1               5                   10                  15
   Gly
<210> 465
   <211> 17
   <212> PRT
   <213> Artificial Sequence
<220>
   <223> Description of Artificial Sequence: Synthetic peptide
<400> 465
   Ser Ile Tyr Pro Ser Ser Gly Tyr Thr Arg Tyr Ala Asp Ser Val Lys
   1               5                   10                  15
   Gly
<210> 466
   <211> 17
   <212> PRT
   <213> Artificial Sequence
<220>
   <223> Description of Artificial Sequence: Synthetic peptide
<400> 466
   Ser Ile Tyr Pro Tyr Ser Gly Tyr Thr Ser Tyr Ala Asp Ser Val Lys
   1               5                   10                  15
   Gly
<210> 467
   <211> 17
   <212> PRT
   <213> Artificial Sequence
<220>
   <223> Description of Artificial Sequence: Synthetic peptide
<400> 467
   Ser Ile Tyr Pro Ser Ser Gly Tyr Thr Ser Tyr Ala Asp Ser Val Lys
   1               5                   10                  15
   Gly
<210> 468
   <211> 12
   <212> PRT
   <213> Artificial Sequence
<220>
   <223> Description of Artificial Sequence: Synthetic peptide
<400> 468
   Ser Ser Trp Ser Ser Trp Ser Ser Ala Met Asp Tyr
   1               5                   10
<210> 469
   <211> 8
   <212> PRT
   <213> Artificial Sequence
<220>
   <223> Description of Artificial Sequence: Synthetic peptide
<400> 469
   Tyr Tyr Ser Tyr Ala Leu Asp Tyr
   1               5
<210> 470
   <211> 8
   <212> PRT
   <213> Artificial Sequence
<220>
   <223> Description of Artificial Sequence: Synthetic peptide
<400> 470
   Tyr Tyr Ser Tyr Ala Phe Asp Tyr
   1               5
<210> 471
   <211> 8
   <212> PRT
   <213> Artificial Sequence
<220>
   <223> Description of Artificial Sequence: Synthetic peptide
<400> 471
   Tyr Tyr Ser Ser Ala Phe Asp Tyr
   1               5
<210> 472
   <211> 8
   <212> PRT
   <213> Artificial Sequence
<220>
   <223> Description of Artificial Sequence: Synthetic peptide
<400> 472
   Tyr Tyr Ser Tyr Ala Leu Asp Tyr
   1               5
<210> 473
   <211> 8
   <212> PRT
   <213> Artificial Sequence
<220>
   <223> Description of Artificial Sequence: Synthetic peptide
<400> 473
   Tyr Tyr Ser Tyr Ala Phe Asp Tyr
   1               5
<210> 474
   <211> 8
   <212> PRT
   <213> Artificial Sequence
<220>
   <223> Description of Artificial Sequence: Synthetic peptide
<400> 474
   Tyr Tyr Ser Tyr Ala Leu Asp Tyr
   1               5
<210> 475
   <211> 14
   <212> PRT
   <213> Artificial Sequence
<220>
   <223> Description of Artificial Sequence: Synthetic peptide
<400> 475
   Ser Ser Tyr Ser Tyr Tyr Tyr Tyr Ser Tyr Ala Leu Asp Tyr
   1               5                   10
<210> 476
   <211> 15
   <212> PRT
   <213> Artificial Sequence
<220>
   <223> Description of Artificial Sequence: Synthetic peptide
<400> 476
   Tyr Tyr Ser Ser Tyr Tyr Ser Tyr Tyr Ser Tyr Ala Leu Asp Tyr
   1               5                   10                  15
<210> 477
   <211> 18
   <212> PRT
   <213> Artificial Sequence
<220>
   <223> Description of Artificial Sequence: Synthetic peptide
<400> 477
   Tyr Tyr Ser Ser Tyr Ser Ser Ser Ser Ser Tyr Tyr Tyr Tyr Ala Phe
   1               5                   10                  15
   Asp Tyr
<210> 478
   <211> 19
   <212> PRT
   <213> Artificial Sequence
<220>
   <223> Description of Artificial Sequence: Synthetic peptide
<400> 478
   Tyr Tyr Ser Ser Ser Ser Ser Tyr Ser Ser Ser Tyr Tyr Tyr Tyr Ala
   1               5                   10                  15
   Phe Asp Tyr
<210> 479
   <211> 12
   <212> PRT
   <213> Artificial Sequence
<220>
   <223> Description of Artificial Sequence: Synthetic peptide
<400> 479
   Gly Tyr Tyr Tyr Ser Tyr Tyr Ser Gly Tyr Ala Leu
   1               5                   10
<210> 480
   <211> 8
   <212> PRT
   <213> Artificial Sequence
<220>
   <223> Description of Artificial Sequence: Synthetic peptide
<400> 480
   Gly Gly Gly Tyr Gly Tyr Gly Ile
   1               5
<210> 481
   <211> 15
   <212> PRT
   <213> Artificial Sequence
<220>
   <223> Description of Artificial Sequence: Synthetic peptide
<400> 481
   Glu Tyr Tyr Gln Gly Tyr Gly Pro Tyr Arg Ser Thr Tyr Gly Leu
   1               5                   10                  15
<210> 482
   <211> 8
   <212> PRT
   <213> Artificial Sequence
<220>
   <223> Description of Artificial Sequence: Synthetic peptide
<400> 482
   Tyr Tyr Ser Ser Gly Phe Asp Tyr
   1               5
<210> 483
   <211> 8
   <212> PRT
   <213> Artificial Sequence
<220>
   <223> Description of Artificial Sequence: Synthetic peptide
<400> 483
   Tyr Tyr Gly Tyr Gly Met Asp Tyr
   1               5
<210> 484
   <211> 8
   <212> PRT
   <213> Artificial Sequence
<220>
   <223> Description of Artificial Sequence: Synthetic peptide
<400> 484
   Tyr Tyr Gly Tyr Gly Leu Asp Tyr
   1               5
<210> 485
   <211> 8
   <212> PRT
   <213> Artificial Sequence
<220>
   <223> Description of Artificial Sequence: Synthetic peptide
<400> 485
   Tyr Tyr Gly Tyr Gly Phe Asp Tyr
   1               5
<210> 486
   <211> 8
   <212> PRT
   <213> Artificial Sequence
<220>
   <223> Description of Artificial Sequence: Synthetic peptide
<400> 486
   Tyr Tyr Gly Ser Gly Phe Asp Tyr
   1               5
<210> 487
   <211> 8
   <212> PRT
   <213> Artificial Sequence
<220>
   <223> Description of Artificial Sequence: Synthetic peptide
<400> 487
   Tyr Tyr Gly Gly Ala Phe Asp Tyr
   1               5
<210> 488
   <211> 10
   <212> PRT
   <213> Artificial Sequence
<220>
   <223> Description of Artificial Sequence: Synthetic peptide
<400> 488
   Gly Phe Ser Ile Lys Tyr Ser Tyr Ile His
   1               5                   10
<210> 489
   <211> 10
   <212> PRT
   <213> Artificial Sequence
<220>
   <223> Description of Artificial Sequence: Synthetic peptide
<400> 489
   Gly Phe Ser Ile Lys Ser Ser Tyr Ile His
   1               5                   10
<210> 490
   <211> 10
   <212> PRT
   <213> Artificial Sequence
<220>
   <223> Description of Artificial Sequence: Synthetic peptide
<400> 490
   Gly Phe Ser Ile Lys Tyr Ser Tyr Ile His
   1               5                   10
<210> 491
   <211> 10
   <212> PRT
   <213> Artificial Sequence
<220>
   <223> Description of Artificial Sequence: Synthetic peptide
<400> 491
   Gly Phe Ser Ile Lys Tyr Ser Ser Ile His
   1               5                   10
<210> 492
   <211> 10
   <212> PRT
   <213> Artificial Sequence
<220>
   <223> Description of Artificial Sequence: Synthetic peptide
<400> 492
   Gly Phe Ser Ile Lys Ser Tyr Ser Ile His
   1               5                   10
<210> 493
   <211> 10
   <212> PRT
   <213> Artificial Sequence
<220>
   <223> Description of Artificial Sequence: Synthetic peptide
<400> 493
   Gly Phe Ser Ile Lys Ser Ser Ser Ile His
   1               5                   10
<210> 494
   <211> 10
   <212> PRT
   <213> Artificial Sequence
<220>
   <223> Description of Artificial Sequence: Synthetic peptide
<400> 494
   Gly Phe Tyr Ile Lys Ser Ser Ser Ile His
   1 5 10
<210> 495
   <211> 10
   <212> PRT
   <213> Artificial Sequence
<220>
   <223> Description of Artificial Sequence: Synthetic peptide
<400> 495
   Gly Phe Tyr Ile Lys Ser Tyr Ser Ile His
   1 5 10
<210> 496
   <211> 17
   <212> PRT
   <213> Artificial Sequence
<220>
   <223> Description of Artificial Sequence: Synthetic peptide
<400> 496 Gly
<210> 497
   <211> 17
   <212> PRT
   <213> Artificial Sequence
<220>
   <223> Description of Artificial Sequence: Synthetic peptide
<400> 497
   Ser Ile Tyr Pro Tyr Ser Gly Tyr Thr Ser Tyr Ala Asp Ser Val Lys
   1 5 10 15
   Gly
<210> 498
   <211> 17
   <212> PRT
   <213> Artificial Sequence
<220>
   <223> Description of Artificial Sequence: Synthetic peptide
<400> 498
   Ser Ile Tyr Pro Ser Ser Gly Ser Thr Tyr Tyr Ala Asp Ser Val Lys
   1               5                   10                  15
   Gly
<210> 499
   <211> 17
   <212> PRT
   <213> Artificial Sequence
<220>
   <223> Description of Artificial Sequence: Synthetic peptide
<400> 499
   Ser Ile Tyr Pro Tyr Ser Gly Ser Thr Ser Tyr Ala Asp Ser Val Lys
   1               5                   10                  15
   Gly
<210> 500
   <211> 17
   <212> PRT
   <213> Artificial Sequence
<220>
   <223> Description of Artificial Sequence: Synthetic peptide
<400> 500
   Ser Ile Tyr Pro Tyr Tyr Gly Tyr Thr Ser Tyr Ala Asp Ser Val Lys
   1               5                   10                  15
   Gly
<210> 501
   <211> 17
   <212> PRT
   <213> Artificial Sequence
<220>
   <223> Description of Artificial Sequence: Synthetic peptide
<400> 501
   Ser Ile Tyr Pro Tyr Tyr Gly Ser Thr Tyr Tyr Ala Asp Ser Val Lys
   1 5 10 15
   Gly
<210> 502
   <211> 17
   <212> PRT
   <213> Artificial Sequence
<220>
   <223> Description of Artificial Sequence: Synthetic peptide
<400> 502
   Ser Ile Tyr Pro Tyr Tyr Gly Tyr Thr Ser Tyr Ala Asp Ser Val Lys
   1 5 10 15
   Gly
<210> 503
   <211> 17
   <212> PRT
   <213> Artificial Sequence
<220>
   <223> Description of Artificial Sequence: Synthetic peptide
<400> 503
   Ser Ile Tyr Pro Ser Tyr Gly Tyr Thr Ser Tyr Ala Asp Ser Val Lys
   1 5 10 15
   Gly
<210> 504
   <211> 17
   <212> PRT
   <213> Artificial Sequence
<220>
   <223> Description of Artificial Sequence: Synthetic peptide
<400> 504
   Ser Ile Tyr Pro Tyr Tyr Gly Ser Thr Tyr Tyr Ala Asp Ser Val Lys
   1 5 10 15
   Gly
<210> 505
   <211> 17
   <212> PRT
   <213> Artificial Sequence
<220>
   <223> Description of Artificial Sequence: Synthetic peptide
<400> 505
   Ser Ile Tyr Pro Tyr Tyr Gly Tyr Thr Ser Tyr Ala Asp Ser Val Lys
   1               5                   10                  15
   Gly
<210> 506
   <211> 17
   <212> PRT
   <213> Artificial Sequence
<220>
   <223> Description of Artificial Sequence: Synthetic peptide
<400> 506
   Ser Ile Tyr Pro Ser Tyr Gly Tyr Thr Ser Tyr Ala Asp Ser Val Lys
   1               5                   10                  15
   Gly
<210> 507
   <211> 17
   <212> PRT
   <213> Artificial Sequence
<220>
   <223> Description of Artificial Sequence: Synthetic peptide
<400> 507
   Ser Ile Tyr Pro Tyr Tyr Gly Tyr Thr Ser Tyr Ala Asp Ser Val Lys
   1               5                   10                  15
   Gly
<210> 508
   <211> 9
   <212> PRT
   <213> Artificial Sequence
<220>
   <223> Description of Artificial Sequence: Synthetic peptide
<400> 508
   Tyr Tyr Gly Arg Gly Ala Met Asp Tyr
   1               5
<210> 509
   <211> 9
   <212> PRT
   <213> Artificial Sequence
<220>
   <223> Description of Artificial Sequence: Synthetic peptide
<400> 509
   Tyr Ser Tyr Ser Gly Ala Leu Asp Tyr
   1               5
<210> 510
   <211> 9
   <212> PRT
   <213> Artificial Sequence
<220>
   <223> Description of Artificial Sequence: Synthetic peptide
<400> 510
   Tyr Arg Tyr Tyr Gly Ala Leu Asp Tyr
   1               5
<210> 511
   <211> 9
   <212> PRT
   <213> Artificial Sequence
<220>
   <223> Description of Artificial Sequence: Synthetic peptide
<400> 511
   Tyr Arg Tyr Gly Gly Ala Met Asp Tyr
   1               5
<210> 512
   <211> 10
   <212> PRT
   <213> Artificial Sequence
<220>
   <223> Description of Artificial Sequence: Synthetic peptide
<400> 512
   Tyr Tyr Gly Gly Tyr Tyr Gly Met Asp Tyr
   1               5                   10
<210> 513
   <211> 10
   <212> PRT
   <213> Artificial Sequence
<220>
   <223> Description of Artificial Sequence: Synthetic peptide
<400> 513
   Tyr Ser Tyr Tyr Tyr Tyr Ala Leu Asp Tyr
   1 5 10
<210> 514
   <211> 10
   <212> PRT
   <213> Artificial Sequence
<220>
   <223> Description of Artificial Sequence: Synthetic peptide
<400> 514
   Ser Ser Tyr Tyr Tyr Tyr Gly Met Asp Tyr
   1 5 10
<210> 515
   <211> 10
   <212> PRT
   <213> Artificial Sequence
<220>
   <223> Description of Artificial Sequence: Synthetic peptide
<400> 515
   Ser Ser Tyr Tyr Tyr Tyr Gly Phe Asp Tyr
   1 5 10
<210> 516
   <211> 10
   <212> PRT
   <213> Artificial Sequence
<220>
   <223> Description of Artificial Sequence: Synthetic peptide
<400> 516
   Ser Ser Tyr Tyr Tyr Tyr Gly Leu Asp Tyr
   1 5 10
<210> 517
   <211> 10
   <212> PRT
   <213> Artificial Sequence
<220>
   <223> Description of Artificial Sequence: Synthetic peptide
<400> 517
   Ser Gly Gly Tyr Ser Arg Gly Ile Asp Tyr
   1 5 10
<210> 518
   <211> 10
   <212> PRT
   <213> Artificial Sequence
<220>
   <223> Description of Artificial Sequence: Synthetic peptide
<400> 518
   Gly Tyr Ser Tyr Ser Arg Gly Ile Asp Tyr
   1               5                   10
<210> 519
   <211> 10
   <212> PRT
   <213> Artificial Sequence
<220>
   <223> Description of Artificial Sequence: Synthetic peptide
<400> 519
   Gly Tyr Ser Tyr Ser Arg Gly Met Asp Tyr
   1               5                   10
<210> 520
   <211> 10
   <212> PRT
   <213> Artificial Sequence
<220>
   <223> Description of Artificial Sequence: Synthetic peptide
<400> 520
   Gly Ser Ser Tyr Ser Tyr Gly Met Asp Tyr
   1               5                   10
<210> 521
   <211> 10
   <212> PRT
   <213> Artificial Sequence
<220>
   <223> Description of Artificial Sequence: Synthetic peptide
<400> 521
   Gly Ser Gly Tyr Gly Tyr Gly Met Asp Tyr
   1               5                   10
<210> 522
   <211> 10
   <212> PRT
   <213> Artificial Sequence
<220>
   <223> Description of Artificial Sequence: Synthetic peptide
<400> 522
   Gly Ser Gly Tyr Gly Tyr Gly Ile Asp Tyr
   1 5 10
<210> 523
   <211> 10
   <212> PRT
   <213> Artificial Sequence
<220>
   <223> Description of Artificial Sequence: Synthetic peptide
<400> 523
   Gly Arg Arg Tyr Ser Arg Gly Ile Asp Tyr
   1 5 10
<210> 524
   <211> 10
   <212> PRT
   <213> Artificial Sequence
<220>
   <223> Description of Artificial Sequence: Synthetic peptide
<400> 524
   Gly Gly Ser Tyr Gly Tyr Gly Met Asp Tyr
   1 5 10
<210> 525
   <211> 10
   <212> PRT
   <213> Artificial Sequence
<220>
   <223> Description of Artificial Sequence: Synthetic peptide
<400> 525
   Gly Gly Ser Tyr Gly Tyr Gly Met Asp Tyr
   1 5 10
<210> 526
   <211> 10
   <212> PRT
   <213> Artificial Sequence
<220>
   <223> Description of Artificial Sequence: Synthetic peptide
<400> 526
   Gly Gly Arg Tyr Gly Tyr Gly Leu Asp Tyr
   1 5 10
<210> 527
   <211> 10
   <212> PRT
   <213> Artificial Sequence
<220>
   <223> Description of Artificial Sequence: Synthetic peptide
<400> 527
   Gly Phe Tyr Ile Lys Ser Ser Ser Ile His
   1 5 10
<210> 528
   <211> 10
   <212> PRT
   <213> Artificial Sequence
<220>
   <223> Description of Artificial Sequence: Synthetic peptide
<400> 528
   Gly Phe Ser Ile Lys Ser Ser Ser Ile His
   1 5 10
<210> 529
   <211> 10
   <212> PRT
   <213> Artificial Sequence
<220>
   <223> Description of Artificial Sequence: Synthetic peptide
<400> 529
   Gly Phe Ser Ile Lys Tyr Ser Ser Ile His
   1 5 10
<210> 530
   <211> 10
   <212> PRT
   <213> Artificial Sequence
<220>
   <223> Description of Artificial Sequence: Synthetic peptide
<400> 530
   Gly Phe Ser Ile Lys Ser Tyr Ser Ile His
   1 5 10
<210> 531
   <211> 10
   <212> PRT
   <213> Artificial Sequence
<220>
   <223> Description of Artificial Sequence: Synthetic peptide
<400> 531
   Gly Phe Tyr Ile Lys Ser Ser Tyr Ile His
   1 5 10
<210> 532
   <211> 10
   <212> PRT
   <213> Artificial Sequence
<220>
   <223> Description of Artificial Sequence: Synthetic peptide
<400> 532
   Gly Phe Tyr Ile Lys Ser Tyr Ser Ile His
   1 5 10
<210> 533
   <211> 17
   <212> PRT
   <213> Artificial Sequence
<220>
   <223> Description of Artificial Sequence: Synthetic peptide
<400> 533
   Ser Ile Tyr Pro Tyr Tyr Gly Ser Thr Tyr Tyr Ala Asp Ser Val Lys
   1 5 10 15
   Gly
<210> 534
   <211> 17
   <212> PRT
   <213> Artificial Sequence
<220>
   <223> Description of Artificial Sequence: Synthetic peptide
<400> 534
   Ser Ile Tyr Pro Tyr Tyr Gly Tyr Thr Ser Tyr Ala Asp Ser Val Lys
   1 5 10 15
   Gly
<210> 535
   <211> 17
   <212> PRT
   <213> Artificial Sequence
<220>
   <223> Description of Artificial Sequence: Synthetic peptide
<400> 535
   Ser Ile Tyr Pro Ser Tyr Gly Ser Thr Tyr Tyr Ala Asp Ser Val Lys
   1               5                   10                  15
   Gly
<210> 536
   <211> 17
   <212> PRT
   <213> Artificial Sequence
<220>
   <223> Description of Artificial Sequence: Synthetic peptide
<400> 536
   Tyr Ile Tyr Pro Ser Ser Gly Ser Thr Tyr Tyr Ala Asp Ser Val Lys
   1               5                   10                  15
   Gly
<210> 537
   <211> 17
   <212> PRT
   <213> Artificial Sequence
<220>
   <223> Description of Artificial Sequence: Synthetic peptide
<400> 537
   Ser Ile Ser Pro Tyr Tyr Gly Ser Thr Tyr Tyr Ala Asp Ser Val Lys
   1               5                   10                  15
   Gly
<210> 538
   <211> 17
   <212> PRT
   <213> Artificial Sequence
<220>
   <223> Description of Artificial Sequence: Synthetic peptide
<400> 538
   Ser Ile Ser Pro Tyr Ser Gly Ser Thr Ser Tyr Ala Asp Ser Val Lys
   1               5                   10                  15
   Gly
<210> 539
   <211> 17
   <212> PRT
   <213> Artificial Sequence
<220>
   <223> Description of Artificial Sequence: Synthetic peptide
<400> 539
   Ser Ile Ser Pro Tyr Ser Gly Tyr Thr Ser Tyr Ala Asp Ser Val Lys
   1               5                   10                  15
   Gly
<210> 540
   <211> 17
   <212> PRT
   <213> Artificial Sequence
<220>
   <223> Description of Artificial Sequence: Synthetic peptide
<400> 540
   Tyr Ile Ser Pro Ser Ser Gly Tyr Thr Tyr Tyr Ala Asp Ser Val Lys
   1               5                   10                  15
   Gly
<210> 541
   <211> 17
   <212> PRT
   <213> Artificial Sequence
<220>
   <223> Description of Artificial Sequence: Synthetic peptide
<400> 541
   Ser Ile Tyr Pro Ser Ser Gly Tyr Thr Ser Tyr Ala Asp Ser Val Lys
   1               5                   10                  15
   Gly
<210> 542
   <211> 17
   <212> PRT
   <213> Artificial Sequence
<220>
   <223> Description of Artificial Sequence: Synthetic peptide
<400> 542
   Ser Ile Ser Pro Ser Ser Gly Tyr Thr Ser Tyr Ala Asp Ser Val Lys
   1 5 10 15
   Gly
<210> 543
   <211> 17
   <212> PRT
   <213> Artificial Sequence
<220>
   <223> Description of Artificial Sequence: Synthetic peptide
<400> 543
   Ser Ile Tyr Pro Ser Ser Gly Tyr Thr Tyr Tyr Ala Asp Ser Val Lys
   1 5 10 15
   Gly
<210> 544
   <211> 17
   <212> PRT
   <213> Artificial Sequence
<220>
   <223> Description of Artificial Sequence: Synthetic peptide
<400> 544
   Ser Ile Tyr Pro Ser Ser Gly Tyr Thr Ser Tyr Ala Asp Ser Val Lys
   1 5 10 15
   Gly
<210> 545
   <211> 17
   <212> PRT
   <213> Artificial Sequence
<220>
   <223> Description of Artificial Sequence: Synthetic peptide
<400> 545
   Tyr Ile Tyr Pro Ser Ser Gly Tyr Thr Ser Tyr Ala Asp Ser Val Lys
   1 5 10 15
   Gly
<210> 546
   <211> 17
   <212> PRT
   <213> Artificial Sequence
<220>
   <223> Description of Artificial Sequence: Synthetic peptide
<400> 546
   Tyr Ile Tyr Pro Ser Ser Gly Tyr Thr Ser Tyr Ala Asp Ser Val Lys
   1               5                   10                  15
   Gly
<210> 547
   <211> 17
   <212> PRT
   <213> Artificial Sequence
<220>
   <223> Description of Artificial Sequence: Synthetic peptide
<400> 547
   Tyr Ile Ser Pro Tyr Ser Gly Tyr Thr Ser Tyr Ala Asp Ser Val Lys
   1               5                   10                  15
   Gly
<210> 548
   <211> 17
   <212> PRT
   <213> Artificial Sequence
<220>
   <223> Description of Artificial Sequence: Synthetic peptide
<400> 548
   Ser Ile Tyr Pro Ser Ser Gly Tyr Thr Ser Tyr Ala Asp Ser Val Lys
   1               5                   10                  15
   Gly
<210> 549
   <211> 10
   <212> PRT
   <213> Artificial Sequence
<220>
   <223> Description of Artificial Sequence: Synthetic peptide
<400> 549
   Gly Gly Arg Tyr Gly Arg Gly Ile Asp Tyr
   1               5                   10
<210> 550
   <211> 10
   <212> PRT
   <213> Artificial Sequence
<220>
   <223> Description of Artificial Sequence: Synthetic peptide
<400> 550
   Gly Gly Gly Tyr Gly Tyr Gly Ile Asp Tyr
   1 5 10
<210> 551
   <211> 12
   <212> PRT
   <213> Artificial Sequence
<220>
   <223> Description of Artificial Sequence: Synthetic peptide
<400> 551
   Tyr Tyr Tyr Ser Gly Gly Ser Tyr Gly Met Asp Tyr
   1 5 10
<210> 552
   <211> 12
   <212> PRT
   <213> Artificial Sequence
<220>
   <223> Description of Artificial Sequence: Synthetic peptide
<400> 552
   Ser Tyr Ser Ser Tyr Tyr Ser Ser Gly Met Asp Tyr
   1 5 10
<210> 553
   <211> 12
   <212> PRT
   <213> Artificial Sequence
<220>
   <223> Description of Artificial Sequence: Synthetic peptide
<400> 553
   Arg Tyr Tyr Tyr Tyr Tyr Tyr Ser Ala Phe Asp Tyr
   1 5 10
<210> 554
   <211> 14
   <212> PRT
   <213> Artificial Sequence
<220>
   <223> Description of Artificial Sequence: Synthetic peptide
<400> 554
   Tyr Tyr Tyr Tyr Ser Tyr Tyr Ser Gly Gly Ala Ile Asp Tyr
   1 5 10
<210> 555
   <211> 14
   <212> PRT
   <213> Artificial Sequence
<220>
   <223> Description of Artificial Sequence: Synthetic peptide
<400> 555
   Ser Tyr Tyr Tyr Ser Tyr Tyr Ser Gly Tyr Gly Met Asp Tyr
   1 5 10
<210> 556
   <211> 14
   <212> PRT
   <213> Artificial Sequence
<220>
   <223> Description of Artificial Sequence: Synthetic peptide
<400> 556
   Ser Tyr Tyr Tyr Ser Tyr Tyr Ser Gly Tyr Gly Leu Asp Tyr
   1 5 10
<210> 557
   <211> 14
   <212> PRT
   <213> Artificial Sequence
<220>
   <223> Description of Artificial Sequence: Synthetic peptide
<400> 557
   Ser Tyr Tyr Tyr Ser Tyr Tyr Tyr Gly Tyr Gly Met Asp Tyr
   1 5 10
<210> 558
   <211> 14
   <212> PRT
   <213> Artificial Sequence
<220>
   <223> Description of Artificial Sequence: Synthetic peptide
<400> 558
   Ser Tyr Tyr Gly Ser Tyr Tyr Tyr Gly Tyr Gly Phe Asp Tyr
   1 5 10
<210> 559
   <211> 14
   <212> PRT
   <213> Artificial Sequence
<220>
   <223> Description of Artificial Sequence: Synthetic peptide
<400> 559
   Ser Asp Tyr Tyr Gly Tyr Tyr Ser Gly Tyr Gly Ile Asp Tyr
   1 5 10
<210> 560
   <211> 14
   <212> PRT
   <213> Artificial Sequence
<220>
   <223> Description of Artificial Sequence: Synthetic peptide
<400> 560
   Ser Asp Tyr Tyr Ser Tyr Tyr Ser Gly Tyr Gly Leu Asp Tyr
   1 5 10
<210> 561
   <211> 14
   <212> PRT
   <213> Artificial Sequence
<220>
   <223> Description of Artificial Sequence: Synthetic peptide
<400> 561
   Gly Tyr Tyr Tyr Ser Tyr Tyr Tyr Gly Tyr Gly Met Asp Tyr
   1 5 10
<210> 562
   <211> 14
   <212> PRT
   <213> Artificial Sequence
<220>
   <223> Description of Artificial Sequence: Synthetic peptide
<400> 562
   Gly Tyr Tyr Tyr Ser Tyr Tyr Tyr Gly Ser Ala Met Asp Tyr
   1 5 10
<210> 563
   <211> 14
   <212> PRT
   <213> Artificial Sequence
<220>
   <223> Description of Artificial Sequence: Synthetic peptide
<400> 563
   Gly Tyr Tyr Tyr Ser Tyr Tyr Ser Gly Tyr Gly Met Asp Tyr
   1               5                   10
<210> 564
   <211> 14
   <212> PRT
   <213> Artificial Sequence
<220>
   <223> Description of Artificial Sequence: Synthetic peptide
<400> 564
   Gly Tyr Tyr Tyr Ser Tyr Tyr Ser Gly Tyr Ala Leu Asp Tyr
   1               5                   10
<210> 565
   <211> 14
   <212> PRT
   <213> Artificial Sequence
<220>
   <223> Description of Artificial Sequence: Synthetic peptide
<400> 565
   Gly Tyr Tyr Tyr Ser Tyr Tyr Ser Gly Tyr Ala Ile Asp Tyr
   1               5                   10
<210> 566
   <211> 14
   <212> PRT
   <213> Artificial Sequence
<220>
   <223> Description of Artificial Sequence: Synthetic peptide
<400> 566
   Gly Tyr Tyr Tyr Ser Tyr Tyr Ser Gly Tyr Ala Met Asp Tyr
   1               5                   10
<210> 567
   <211> 10
   <212> PRT
   <213> Artificial Sequence
<220>
   <223> Description of Artificial Sequence: Synthetic peptide
<400> 567
   Gly Phe Asn Tyr Lys Asp Ser Ser Ser His
   1               5                   10
<210> 568
   <211> 10
   <212> PRT
   <213> Artificial Sequence
<220>
   <223> Description of Artificial Sequence: Synthetic peptide
<400> 568
   Gly Phe Asn Tyr Lys Asp Ser Tyr Ser His
   1               5                   10
<210> 569
   <211> 10
   <212> PRT
   <213> Artificial Sequence
<220>
   <223> Description of Artificial Sequence: Synthetic peptide
<400> 569
   Gly Phe Asn Ser Lys Asp Ser Tyr Ser His
   1               5                   10
<210> 570
   <211> 10
   <212> PRT
   <213> Artificial Sequence
<220>
   <223> Description of Artificial Sequence: Synthetic peptide
<400> 570
   Gly Phe Asn Tyr Lys Asp Ser Tyr Ile His
   1               5                   10
<210> 571
   <211> 10
   <212> PRT
   <213> Artificial Sequence
<220>
   <223> Description of Artificial Sequence: Synthetic peptide
<400> 571
   Gly Phe Asn Ser Lys Asp Ser Ser Ser His
   1 5 10
<210> 572
   <211> 18
   <212> PRT
   <213> Artificial Sequence
<220>
   <223> Description of Artificial Sequence: Synthetic peptide
<400> 572
   Arg Ser Tyr Pro Thr Ser Asn Ser Tyr Ser Arg Ser Ala Asp Ser Val
   1 5 10 15
   Lys Gly
<210> 573
   <211> 18
   <212> PRT
   <213> Artificial Sequence
<220>
   <223> Description of Artificial Sequence: Synthetic peptide
<400> 573
   Arg Tyr Tyr Ser Thr Ser Asn Ser Tyr Thr Arg Ser Ala Asp Ser Val
   1 5 10 15
   Lys Gly
<210> 574
   <211> 18
   <212> PRT
   <213> Artificial Sequence
<220>
   <223> Description of Artificial Sequence: Synthetic peptide
<400> 574
   Arg Ser Tyr Pro Thr Ser Asn Ser Tyr Thr Arg Ser Ala Asp Ser Val
   1 5 10 15
   Lys Gly
<210> 575
   <211> 18
   <212> PRT
   <213> Artificial Sequence
<220>
   <223> Description of Artificial Sequence: Synthetic peptide
<400> 575
   Arg Ser Tyr Ser Thr Ser Asn Ser Tyr Thr Arg Ser Ala Asp Ser Val
   1               5                   10                  15
   Lys Gly
<210> 576
   <211> 18
   <212> PRT
   <213> Artificial Sequence
<220>
   <223> Description of Artificial Sequence: Synthetic peptide
<400> 576
   Arg Ser Tyr Pro Thr Ser Asn Ser Tyr Thr Arg Tyr Ala Asp Ser Val
   1               5                   10                  15
   Lys Gly
<210> 577
   <211> 18
   <212> PRT
   <213> Artificial Sequence
<220>
   <223> Description of Artificial Sequence: Synthetic peptide
<400> 577
   Arg Tyr Tyr Pro Thr Ser Asn Ser Tyr Thr Arg Ser Ala Asp Ser Val
   1               5                   10                  15
   Lys Gly
<210> 578
   <211> 18
   <212> PRT
   <213> Artificial Sequence
<220>
   <223> Description of Artificial Sequence: Synthetic peptide
<400> 578
   Arg Ser Tyr Pro Thr Ser Asn Tyr Tyr Thr Arg Ser Ala Asp Ser Val
   1               5                   10                  15
   Lys Gly
<210> 579
   <211> 18
   <212> PRT
   <213> Artificial Sequence
<220>
   <223> Description of Artificial Sequence: Synthetic peptide
<400> 579
   Arg Tyr Tyr Pro Thr Ser Asn Ser Tyr Ser Arg Ser Ala Asp Ser Val
   1               5                   10                  15
   Lys Gly
<210> 580
   <211> 18
   <212> PRT
   <213> Artificial Sequence
<220>
   <223> Description of Artificial Sequence: Synthetic peptide
<400> 580
   Arg Ser Tyr Pro Thr Ser Asn Ser Tyr Thr Arg Tyr Ala Asp Ser Val
   1               5                   10                  15
   Lys Gly
<210> 581
   <211> 18
   <212> PRT
   <213> Artificial Sequence
<220>
   <223> Description of Artificial Sequence: Synthetic peptide
<400> 581
   Arg Tyr Tyr Pro Thr Ser Asn Ser Tyr Thr Arg Tyr Ala Asp Ser Val
   1               5                   10                  15
   Lys Gly
<210> 582
   <211> 14
   <212> PRT
   <213> Artificial Sequence
<220>
   <223> Description of Artificial Sequence: Synthetic peptide
<400> 582
   Gly Tyr Tyr Tyr Ser Tyr Tyr Ser Gly Tyr Gly Ile Asp Tyr
   1 5 10
<210> 583
   <211> 14
   <212> PRT
   <213> Artificial Sequence
<220>
   <223> Description of Artificial Sequence: Synthetic peptide
<400> 583
   Gly Tyr Tyr Tyr Ser Tyr Tyr Ser Gly Tyr Gly Leu Asp Tyr
   1 5 10
<210> 584
   <211> 14
   <212> PRT
   <213> Artificial Sequence
<220>
   <223> Description of Artificial Sequence: Synthetic peptide
<400> 584
   Gly Tyr Tyr Tyr Ser Tyr Tyr Ser Gly Tyr Gly Met Asp Tyr
   1 5 10
<210> 585
   <211> 14
   <212> PRT
   <213> Artificial Sequence
<220>
   <223> Description of Artificial Sequence: Synthetic peptide
<400> 585
   Gly Tyr Tyr Tyr Ser Tyr Tyr Gly Gly Ser Gly Leu Asp Tyr
   1 5 10
<210> 586
   <211> 14
   <212> PRT
   <213> Artificial Sequence
<220>
   <223> Description of Artificial Sequence: Synthetic peptide
<400> 586
   Gly Tyr Tyr Tyr Ser Tyr Tyr Gly Gly Tyr Gly Met Asp Tyr
   1 5 10
<210> 587
   <211> 14
   <212> PRT
   <213> Artificial Sequence
<220>
   <223> Description of Artificial Sequence: Synthetic peptide
<400> 587
   Gly Tyr Tyr Tyr Ser Tyr Tyr Gly Gly Tyr Gly Ile Asp Tyr
   1               5                   10
<210> 588
   <211> 14
   <212> PRT
   <213> Artificial Sequence
<220>
   <223> Description of Artificial Sequence: Synthetic peptide
<400> 588
   Gly Tyr Tyr Tyr Ser Tyr Tyr Gly Gly Tyr Gly Met Asp Tyr
   1               5                   10
<210> 589
   <211> 14
   <212> PRT
   <213> Artificial Sequence
<220>
   <223> Description of Artificial Sequence: Synthetic peptide
<400> 589
   Gly Tyr Tyr Tyr Ser Tyr Tyr Gly Gly Tyr Gly Met Asp Tyr
   1               5                   10
<210> 590
   <211> 14
   <212> PRT
   <213> Artificial Sequence
<220>
   <223> Description of Artificial Sequence: Synthetic peptide
<400> 590
   Gly Tyr Tyr Tyr Ser Tyr Tyr Gly Gly Tyr Gly Met Asp Tyr
   1               5                   10
<210> 591
   <211> 14
   <212> PRT
   <213> Artificial Sequence
<220>
   <223> Description of Artificial Sequence: Synthetic peptide
<400> 591
   Gly Tyr Tyr Tyr Ser Tyr Tyr Gly Gly Tyr Gly Leu Asp Tyr
   1               5                   10
<210> 592
   <211> 14
   <212> PRT
   <213> Artificial Sequence
<220>
   <223> Description of Artificial Sequence: Synthetic peptide
<400> 592
   Gly Tyr Tyr Tyr Ser Tyr Tyr Gly Gly Tyr Gly Met Asp Tyr
   1               5                   10
<210> 593
   <211> 14
   <212> PRT
   <213> Artificial Sequence
<220>
   <223> Description of Artificial Sequence: Synthetic peptide
<400> 593
   Gly Tyr Tyr Tyr Ser Tyr Tyr Gly Gly Tyr Gly Met Asp Tyr
   1               5                   10
<210> 594
   <211> 14
   <212> PRT
   <213> Artificial Sequence
<220>
   <223> Description of Artificial Sequence: Synthetic peptide
<400> 594
   Gly Tyr Tyr Tyr Gly Tyr Tyr Ser Gly Tyr Gly Ile Asp Tyr
   1               5                   10
<210> 595
   <211> 14
   <212> PRT
   <213> Artificial Sequence
<220>
   <223> Description of Artificial Sequence: Synthetic peptide
<400> 595
   Gly Ser Tyr Tyr Ser Tyr Tyr Ser Gly Tyr Gly Met Asp Tyr
   1               5                   10
<210> 596
   <211> 14
   <212> PRT
   <213> Artificial Sequence
<220>
   <223> Description of Artificial Sequence: Synthetic peptide
<400> 596
   Gly Ser Tyr Tyr Ser Tyr Tyr Ser Gly Tyr Ala Ile Asp Tyr
   1               5                   10
<210> 597
   <211> 14
   <212> PRT
   <213> Artificial Sequence
<220>
   <223> Description of Artificial Sequence: Synthetic peptide
<400> 597
   Gly Ser Tyr Tyr Ser Tyr Tyr Ser Gly Tyr Ala Phe Asp Tyr
   1               5                   10
<210> 598
   <211> 14
   <212> PRT
   <213> Artificial Sequence
<220>
   <223> Description of Artificial Sequence: Synthetic peptide
<400> 598
   Gly Ser Tyr Tyr Ser Tyr Tyr Ser Gly Ser Gly Ile Asp Tyr
   1               5                   10
<210> 599
   <211> 14
   <212> PRT
   <213> Artificial Sequence
<220>
   <223> Description of Artificial Sequence: Synthetic peptide
<400> 599
   Gly Ser Tyr Tyr Gly Tyr Tyr Tyr Gly Ser Ala Ile Asp Tyr
   1               5                   10
<210> 600
   <211> 14
   <212> PRT
   <213> Artificial Sequence
<220>
   <223> Description of Artificial Sequence: Synthetic peptide
<400> 600
   Gly Ser Tyr Tyr Gly Tyr Tyr Tyr Gly Ser Gly Met Asp Tyr
   1 5 10
<210> 601
   <211> 10
   <212> PRT
   <213> Artificial Sequence
<220>
   <223> Description of Artificial Sequence: Synthetic peptide
<400> 601
   Gly Phe Tyr Ile Lys Ser Tyr Ser Ile His
   1 5 10
<210> 602
   <211> 10
   <212> PRT
   <213> Artificial Sequence
<220>
   <223> Description of Artificial Sequence: Synthetic peptide
<400> 602
   Gly Phe Tyr Ile Lys Ser Ser Tyr Ile His
   1 5 10
<210> 603
   <211> 10
   <212> PRT
   <213> Artificial Sequence
<220>
   <223> Description of Artificial Sequence: Synthetic peptide
<400> 603
   Gly Phe Tyr Ile Lys Ser Ser Ser Ile His
   1 5 10
<210> 604
   <211> 10
   <212> PRT
   <213> Artificial Sequence
<220>
   <223> Description of Artificial Sequence: Synthetic peptide
<400> 604
   Gly Phe Ser Ile Lys Tyr Ser Ser Ile His
   1 5 10
<210> 605
   <211> 10
   <212> PRT
   <213> Artificial Sequence
<220>
   <223> Description of Artificial Sequence: Synthetic peptide
<400> 605
   Gly Phe Ser Ile Lys Ser Ser Ser Ile His
   1 5 10
<210> 606
   <211> 10
   <212> PRT
   <213> Artificial Sequence
<220>
   <223> Description of Artificial Sequence: Synthetic peptide
<400> 606
   Gly Phe Ser Ile Lys Tyr Ser Ser Ile His
   1 5 10
<210> 607
   <211> 17
   <212> PRT
   <213> Artificial Sequence
<220>
   <223> Description of Artificial Sequence: Synthetic peptide
<400> 607
   Ser Ile Tyr Pro Ser Ser Gly Ser Thr Ser Tyr Ala Asp Ser Val Lys
   1 5 10 15
   Gly
<210> 608
   <211> 17
   <212> PRT
   <213> Artificial Sequence
<220>
   <223> Description of Artificial Sequence: Synthetic peptide
<400> 608
   Ser Ile Ser Pro Ser Ser Gly Tyr Thr Ser Tyr Ala Asp Ser Val Lys
   1 5 10 15
   Gly
<210> 609
   <211> 17
   <212> PRT
   <213> Artificial Sequence
<220>
   <223> Description of Artificial Sequence: Synthetic peptide
<400> 609
   Ser Ile Tyr Pro Ser Ser Gly Tyr Thr Ser Tyr Ala Asp Ser Val Lys
   1               5                   10                  15
   Gly
<210> 610
   <211> 17
   <212> PRT
   <213> Artificial Sequence
<220>
   <223> Description of Artificial Sequence: Synthetic peptide
<400> 610
   Tyr Ile Ser Pro Ser Ser Gly Tyr Thr Ser Tyr Ala Asp Ser Val Lys
   1               5                   10                  15
   Gly
<210> 611
   <211> 17
   <212> PRT
   <213> Artificial Sequence
<220>
   <223> Description of Artificial Sequence: Synthetic peptide
<400> 611
   Ser Ile Ser Pro Tyr Ser Gly Ser Thr Ser Tyr Ala Asp Ser Val Lys
   1               5                   10                  15
   Gly
<210> 612
   <211> 17
   <212> PRT
   <213> Artificial Sequence
<220>
   <223> Description of Artificial Sequence: Synthetic peptide
<400> 612
   Tyr Ile Tyr Pro Ser Ser Gly Tyr Thr Ser Tyr Ala Asp Ser Val Lys
   1 5 10 15
   Gly
<210> 613
   <211> 17
   <212> PRT
   <213> Artificial Sequence
<220>
   <223> Description of Artificial Sequence: Synthetic peptide
<400> 613
   Ser Ile Tyr Pro Tyr Ser Gly Ser Thr Ser Tyr Ala Asp Ser Val Lys
   1 5 10 15
   Gly
<210> 614
   <211> 17
   <212> PRT
   <213> Artificial Sequence
<220>
   <223> Description of Artificial Sequence: Synthetic peptide
<400> 614
   Ser Ile Ser Pro Tyr Ser Gly Tyr Thr Ser Tyr Ala Asp Ser Val Lys
   1 5 10 15
   Gly
<210> 615
   <211> 17
   <212> PRT
   <213> Artificial Sequence
<220>
   <223> Description of Artificial Sequence: Synthetic peptide
<400> 615
   Ser Ile Tyr Pro Tyr Tyr Gly Ser Thr Ser Tyr Ala Asp Ser Val Lys
   1 5 10 15
   Gly
<210> 616
   <211> 17
   <212> PRT
   <213> Artificial Sequence
<220>
   <223> Description of Artificial Sequence: Synthetic peptide
<400> 616
   Tyr Ile Tyr Pro Ser Ser Gly Ser Thr Ser Tyr Ala Asp Ser Val Lys
   1               5                   10                  15
   Gly
<210> 617
   <211> 17
   <212> PRT
   <213> Artificial Sequence
<220>
   <223> Description of Artificial Sequence: Synthetic peptide
<400> 617
   Tyr Ile Ser Pro Ser Ser Gly Tyr Thr Tyr Tyr Ala Asp Ser Val Lys
   1               5                   10                  15
   Gly
<210> 618
   <211> 14
   <212> PRT
   <213> Artificial Sequence
<220>
   <223> Description of Artificial Sequence: Synthetic peptide
<400> 618
   Gly Ser Tyr Tyr Gly Tyr Tyr Ser Gly Ser Gly Met Asp Tyr
           1               5                   10
<210> 619
   <211> 14
   <212> PRT
   <213> Artificial Sequence
<220>
   <223> Description of Artificial Sequence: Synthetic peptide
<400> 619
   Gly Ser Gly Tyr Ser Tyr Tyr Gly Gly Tyr Gly Leu Asp Tyr
   1               5                   10
<210> 620
   <211> 14
   <212> PRT
   <213> Artificial Sequence
<220>
   <223> Description of Artificial Sequence: Synthetic peptide
<400> 620
   Gly Ser Tyr Ser Gly Tyr Tyr Tyr Gly Tyr Gly Leu Asp Tyr
   1               5                   10
<210> 621
   <211> 14
   <212> PRT
   <213> Artificial Sequence
<220>
   <223> Description of Artificial Sequence: Synthetic peptide
<400> 621
   Gly Arg Tyr Ser Gly Tyr Tyr Gly Gly Tyr Gly Met Asp Tyr
   1               5                   10
<210> 622
   <211> 15
   <212> PRT
   <213> Artificial Sequence
<220>
   <223> Description of Artificial Sequence: Synthetic peptide
<400> 622
   Tyr Tyr Tyr Ser Ser Gly Tyr Tyr Tyr Tyr Tyr Ala Phe Asp Tyr
   1               5                   10                  15
<210> 623
   <211> 15
   <212> PRT
   <213> Artificial Sequence
<220>
   <223> Description of Artificial Sequence: Synthetic peptide
<400> 623
   Tyr Ser Tyr Ser Tyr Tyr Gly Tyr Tyr Gly Ser Gly Met Asp Tyr
   1               5                   10                  15
<210> 624
   <211> 15
   <212> PRT
   <213> Artificial Sequence
<220>
   <223> Description of Artificial Sequence: Synthetic peptide
<400> 624
   Tyr Arg Ser Tyr Tyr Ser Tyr Arg Tyr Gly Tyr Gly Met Asp Tyr
   1 5 10 15
<210> 625
   <211> 15
   <212> PRT
   <213> Artificial Sequence
<220>
   <223> Description of Artificial Sequence: Synthetic peptide
<400> 625
   Tyr Gly Tyr Tyr Tyr Ser Tyr Tyr Gly Gly Ser Gly Leu Asp Tyr
   1 5 10 15
<210> 626
   <211> 15
   <212> PRT
   <213> Artificial Sequence
<220>
   <223> Description of Artificial Sequence: Synthetic peptide
<400> 626
   Tyr Gly Tyr Tyr Tyr Ser Tyr Tyr Gly Gly Ser Gly Leu Asp Tyr
   1 5 10 15
<210> 627
   <211> 15
   <212> PRT
   <213> Artificial Sequence
<220>
   <223> Description of Artificial Sequence: Synthetic peptide
<400> 627
   Tyr Gly Tyr Ser Tyr Ser Tyr Ser Ser Gly Ser Ala Leu Asp Tyr
   1 5 10 15
<210> 628
   <211> 15
   <212> PRT
   <213> Artificial Sequence
<220>
   <223> Description of Artificial Sequence: Synthetic peptide
<400> 628
   Ser Tyr Tyr Tyr Gly Gly Tyr Tyr Ser Gly Tyr Gly Met Asp Tyr
   1 5 10 15
<210> 629
   <211> 15
   <212> PRT
   <213> Artificial Sequence
<220>
   <223> Description of Artificial Sequence: Synthetic peptide
<400> 629
   Arg Arg Ser Tyr Tyr Ser Tyr Arg Tyr Ser Tyr Gly Leu Asp Tyr
   1               5                   10                  15
<210> 630
   <211> 15
   <212> PRT
   <213> Artificial Sequence
<220>
   <223> Description of Artificial Sequence: Synthetic peptide
<400> 630
   Arg Arg Ser Tyr Tyr Ser Tyr Ser Arg Ser Tyr Ala Met Asp Tyr
   1               5                   10                  15
<210> 631
   <211> 16
   <212> PRT
   <213> Artificial Sequence
<220>
   <223> Description of Artificial Sequence: Synthetic peptide
<400> 631
   Tyr Tyr Tyr Gly Tyr Tyr Ser Tyr Tyr Ser Gly Tyr Gly Leu Asp Tyr
   1               5                   10                  15
<210> 632
   <211> 16
   <212> PRT
   <213> Artificial Sequence
<220>
   <223> Description of Artificial Sequence: Synthetic peptide
<400> 632
   Tyr Tyr Tyr Gly Tyr Tyr Ser Tyr Tyr Gly Gly Ser Gly Leu Asp Tyr
   1               5                   10                  15
<210> 633
   <211> 16
   <212> PRT
   <213> Artificial Sequence
<220>
   <223> Description of Artificial Sequence: Synthetic peptide
<400> 633
   Tyr Tyr Tyr Gly Tyr Tyr Ser Tyr Tyr Gly Gly Ser Ala Met Asp Tyr
   1               5                   10                  15
<210> 634
   <211> 16
   <212> PRT
   <213> Artificial Sequence
<220>
   <223> Description of Artificial Sequence: Synthetic peptide
<400> 634
   Tyr Tyr Tyr Ser Gly Gly Ser Tyr Tyr Tyr Tyr Tyr Ala Phe Asp Tyr
   1               5                   10                  15
<210> 635
   <211> 16
   <212> PRT
   <213> Artificial Sequence
<220>
   <223> Description of Artificial Sequence: Synthetic peptide
<400> 635
   Tyr Tyr Tyr Ser Tyr Tyr Ser Tyr Tyr Ser Tyr Gly Gly Ile Asp Tyr
   1               5                   10                  15
<210> 636
   <211> 16
   <212> PRT
   <213> Artificial Sequence
<220>
   <223> Description of Artificial Sequence: Synthetic peptide
<400> 636
   Tyr Tyr Ser Ser Tyr Tyr Ser Tyr Tyr Tyr Tyr Gly Gly Met Asp Tyr
   1               5                   10                  15
<210> 637
   <211> 10
   <212> PRT
   <213> Artificial Sequence
<220>
   <223> Description of Artificial Sequence: Synthetic peptide
<400> 637
   Gly Phe Tyr Ile Lys Ser Ser Ser Ile His
   1 5 10
<210> 638
   <211> 10
   <212> PRT
   <213> Artificial Sequence
<220>
   <223> Description of Artificial Sequence: Synthetic peptide
<400> 638
   Gly Phe Ser Ile Lys Ser Ser Ser Ile His
   1 5 10
<210> 639
   <211> 10
   <212> PRT
   <213> Artificial Sequence
<220>
   <223> Description of Artificial Sequence: Synthetic peptide
<400> 639
   Gly Phe Ser Ile Lys Tyr Ser Ser Ile His
   1 5 10
<210> 640
   <211> 10
   <212> PRT
   <213> Artificial Sequence
<220>
   <223> Description of Artificial Sequence: Synthetic peptide
<400> 640
   Gly Phe Tyr Ile Lys Tyr Ser Ser Ile His
   1 5 10
<210> 641
   <211> 10
   <212> PRT
   <213> Artificial Sequence
<220>
   <223> Description of Artificial Sequence: Synthetic peptide
<400> 641
   Gly Phe Ser Ile Lys Ser Ser Tyr Ile His
   1 5 10
<210> 642
   <211> 10
   <212> PRT
   <213> Artificial Sequence
<220>
   <223> Description of Artificial Sequence: Synthetic peptide
<400> 642
   Gly Phe Phe Ile Lys Tyr Tyr Tyr Ile His
   1               5                   10
<210> 643
   <211> 10
   <212> PRT
   <213> Artificial Sequence
<220>
   <223> Description of Artificial Sequence: Synthetic peptide
<400> 643
   Gly Phe Ser Ile Lys Ser Ser Tyr Ile His
   1               5                   10
<210> 644
   <211> 10
   <212> PRT
   <213> Artificial Sequence
<220>
   <223> Description of Artificial Sequence: Synthetic peptide
<400> 644
   Gly Phe Tyr Ile Lys Ser Tyr Ser Ile His
   1               5                   10
<210> 645
   <211> 17
   <212> PRT
   <213> Artificial Sequence
<220>
   <223> Description of Artificial Sequence: Synthetic peptide
<400> 645
   Ser Ile Ser Pro Ser Ser Gly Tyr Thr Ser Tyr Ala Asp Ser Val Lys
   1               5                   10                  15
   Gly
<210> 646
   <211> 17
   <212> PRT
   <213> Artificial Sequence
<220>
   <223> Description of Artificial Sequence: Synthetic peptide
<400> 646
   Tyr Ile Ser Pro Tyr Ser Gly Tyr Thr Tyr Tyr Ala Asp Ser Val Lys
   1               5                   10                  15
   Gly
<210> 647
   <211> 17
   <212> PRT
   <213> Artificial Sequence
<220>
   <223> Description of Artificial Sequence: Synthetic peptide
<400> 647
   Tyr Ile Tyr Pro Tyr Ser Gly Ser Thr Ser Tyr Ala Asp Ser Val Lys
   1               5                   10                  15
   Gly
<210> 648
   <211> 17
   <212> PRT
   <213> Artificial Sequence
<220>
   <223> Description of Artificial Sequence: Synthetic peptide
<400> 648
   Ser Ile Ser Pro Ser Tyr Gly Tyr Thr Ser Tyr Ala Asp Ser Val Lys
   1               5                   10                  15
   Gly
<210> 649
   <211> 17
   <212> PRT
   <213> Artificial Sequence
<220>
   <223> Description of Artificial Sequence: Synthetic peptide
<400> 649
   Ser Ile Tyr Pro Tyr Ser Gly Tyr Thr Ser Tyr Ala Asp Ser Val Lys
   1               5                   10                  15
   Gly
<210> 650
   <211> 17
   <212> PRT
   <213> Artificial Sequence
<220>
   <223> Description of Artificial Sequence: Synthetic peptide
<400> 650
   Ser Ile Ser Pro Tyr Ser Gly Tyr Thr Ser Tyr Ala Asp Ser Val Lys
   1               5                   10                  15
   Gly
<210> 651
   <211> 17
   <212> PRT
   <213> Artificial Sequence
<220>
   <223> Description of Artificial Sequence: Synthetic peptide
<400> 651
   Tyr Ile Tyr Pro Tyr Ser Gly Tyr Thr Tyr Tyr Ala Asp Ser Val Lys
   1               5                   10                  15
   Gly
<210> 652
   <211> 17
   <212> PRT
   <213> Artificial Sequence
<220>
   <223> Description of Artificial Sequence: Synthetic peptide
<400> 652
   Tyr Ile Ser Pro Ser Ser Gly Tyr Thr Ser Tyr Ala Asp Ser Val Lys
   1               5                   10                  15
   Gly
<210> 653
   <211> 17
   <212> PRT
   <213> Artificial Sequence
<220>
   <223> Description of Artificial Sequence: Synthetic peptide
<400> 653
   Tyr Ile Ser Pro Ser Tyr Gly Ser Thr Tyr Tyr Ala Asp Ser Val Lys
   1 5 10 15
   Gly
<210> 654
   <211> 17
   <212> PRT
   <213> Artificial Sequence
<220>
   <223> Description of Artificial Sequence: Synthetic peptide
<400> 654
   Ser Ile Tyr Pro Tyr Tyr Gly Ser Thr Tyr Tyr Ala Asp Ser Val Lys
   1 5 10 15
   Gly
<210> 655
   <211> 17
   <212> PRT
   <213> Artificial Sequence
<220>
   <223> Description of Artificial Sequence: Synthetic peptide
<400> 655
   Ser Ile Tyr Pro Tyr Tyr Gly Tyr Thr Ser Tyr Ala Asp Ser Val Lys
   1 5 10 15
   Gly
<210> 656
   <211> 17
   <212> PRT
   <213> Artificial Sequence
<220>
   <223> Description of Artificial Sequence: Synthetic peptide
<400> 656
   Ser Ile Tyr Pro Tyr Tyr Gly Ser Thr Tyr Tyr Ala Asp Ser Val Lys
   1 5 10 15
   Gly
<210> 657
   <211> 17
   <212> PRT
   <213> Artificial Sequence
<220>
   <223> Description of Artificial Sequence: Synthetic peptide
<400> 657
   Ser Ile Tyr Pro Ser Tyr Gly Tyr Thr Ser Tyr Ala Asp Ser Val Lys
   1               5                   10                  15
   Gly
<210> 658
   <211> 16
   <212> PRT
   <213> Artificial Sequence
<220>
   <223> Description of Artificial Sequence: Synthetic peptide
<400> 658
   Tyr Ser Tyr Tyr Tyr Tyr Ser Tyr Tyr Tyr Gly Ser Ala Met Asp Tyr
   1               5                   10                  15
<210> 659
   <211> 16
   <212> PRT
   <213> Artificial Sequence
<220>
   <223> Description of Artificial Sequence: Synthetic peptide
<400> 659
   Tyr Ser Tyr Tyr Tyr Tyr Ser Tyr Tyr Ser Gly Tyr Gly Phe Asp Tyr
   1               5                   10                  15
<210> 660
   <211> 16
   <212> PRT
   <213> Artificial Sequence
<220>
   <223> Description of Artificial Sequence: Synthetic peptide
<400> 660
   Tyr Ser Tyr Tyr Tyr Tyr Ser Tyr Tyr Ser Gly Tyr Gly Phe Asp Tyr
   1               5                   10                  15
<210> 661
   <211> 16
   <212> PRT
   <213> Artificial Sequence
<220>
   <223> Description of Artificial Sequence: Synthetic peptide
<400> 661
   Tyr Ser Tyr Tyr Tyr Tyr Ser Tyr Tyr Gly Gly Ser Ala Phe Asp Tyr
   1               5                   10                  15
<210> 662
   <211> 16
   <212> PRT
   <213> Artificial Sequence
<220>
   <223> Description of Artificial Sequence: Synthetic peptide
<400> 662
   Tyr Ser Tyr Tyr Tyr Tyr Ser Tyr Tyr Gly Gly Ser Gly Met Asp Tyr
   1               5                   10                  15
<210> 663
   <211> 16
   <212> PRT
   <213> Artificial Sequence
<220>
   <223> Description of Artificial Sequence: Synthetic peptide
<400> 663
   Tyr Ser Tyr Tyr Tyr Tyr Ser Tyr Tyr Gly Gly Ser Gly Leu Asp Tyr
   1               5                   10                  15
<210> 664
   <211> 16
   <212> PRT
   <213> Artificial Sequence
<220>
   <223> Description of Artificial Sequence: Synthetic peptide
<400> 664
   Tyr Ser Tyr Ser Tyr Tyr Gly Tyr Tyr Tyr Gly Ser Gly Leu Asp Tyr
   1               5                   10                  15
<210> 665
   <211> 16
   <212> PRT
   <213> Artificial Sequence
<220>
   <223> Description of Artificial Sequence: Synthetic peptide
<400> 665
   Tyr Ser Ser Tyr Tyr Tyr Ser Tyr Tyr Ser Gly Ser Gly Leu Asp Tyr
   1               5                   10                  15
<210> 666
   <211> 16
   <212> PRT
   <213> Artificial Sequence
<220>
   <223> Description of Artificial Sequence: Synthetic peptide
<400> 666
   Ser Ser Tyr Tyr Tyr Tyr Ser Tyr Tyr Gly Gly Ser Gly Ile Asp Tyr
   1               5                   10                  15
<210> 667
   <211> 16
   <212> PRT
   <213> Artificial Sequence
<220>
   <223> Description of Artificial Sequence: Synthetic peptide
<400> 667
   Arg Ser Tyr Tyr Tyr Tyr Ser Tyr Tyr Tyr Ser Arg Ala Met Asp Tyr
   1               5                   10                  15
<210> 668
   <211> 17
   <212> PRT
   <213> Artificial Sequence
<220>
   <223> Description of Artificial Sequence: Synthetic peptide
<400> 668
   Ser Tyr Ser Ser Tyr Tyr Ser Tyr Tyr Ser Ser Tyr Gly Gly Met Asp
   1               5                   10                  15
   Tyr
<210> 669
   <211> 21
   <212> PRT
   <213> Artificial Sequence
<220>
   <223> Description of Artificial Sequence: Synthetic peptide
<400> 669
   Tyr Arg Tyr Tyr Tyr Ser Arg Tyr Gly Tyr Arg Tyr Tyr Tyr Tyr Tyr
   1               5                   10                  15
   Arg Ala Leu Asp Tyr
               20
<210> 670
   <211> 8
   <212> PRT
   <213> Artificial Sequence
<220>
   <223> Description of Artificial Sequence: Synthetic peptide
<400> 670
   Arg Tyr Ser Ser Gly Met Asp Tyr
   1               5
<210> 671
   <211> 9
   <212> PRT
   <213> Artificial Sequence
<220>
   <223> Description of Artificial Sequence: Synthetic peptide
<400> 671
   Tyr Ser His Ser Gly Ala Phe Asp Tyr
   1               5
<210> 672
   <211> 10
   <212> PRT
   <213> Artificial Sequence
<220>
   <223> Description of Artificial Sequence: Synthetic peptide
<400> 672
   Gly Tyr Ser Tyr Gly His Gly Ile Asp Tyr
   1               5                   10
<210> 673
   <211> 10
   <212> PRT
   <213> Artificial Sequence
<220>
   <223> Description of Artificial Sequence: Synthetic peptide
<400> 673
   Gly Ser Ser Phe Gly Arg Gly Phe Asp Tyr
   1               5                   10
<210> 674
   <211> 10
   <212> PRT
   <213> Artificial Sequence
<220>
   <223> Description of Artificial Sequence: Synthetic peptide
<400> 674
   Gly Ser Ser Tyr Ser Trp Gly Leu Asp Tyr
   1               5                   10
<210> 675
   <211> 10
   <212> PRT
   <213> Artificial Sequence
<220>
   <223> Description of Artificial Sequence: Synthetic peptide
<400> 675
   Gly Ser Arg Tyr Ser His Gly Met Asp Tyr
   1               5                   10
<210> 676
   <211> 10
   <212> PRT
   <213> Artificial Sequence
<220>
   <223> Description of Artificial Sequence: Synthetic peptide
<400> 676
   Gly Ala Ser Tyr Gly Tyr Gly Met Asp Tyr
   1               5                   10
<210> 677
   <211> 10
   <212> PRT
   <213> Artificial Sequence
<220>
   <223> Description of Artificial Sequence: Synthetic peptide
<400> 677
   Gly Phe Tyr Ile Lys Tyr Ser Ser Ile His
   1               5                   10
<210> 678
   <211> 10
   <212> PRT
   <213> Artificial Sequence
<220>
   <223> Description of Artificial Sequence: Synthetic peptide
<400> 678
   Gly Phe Ser Ile Lys Tyr Ser Ser Ile His
   1               5                   10
<210> 679
   <211> 10
   <212> PRT
   <213> Artificial Sequence
<220>
   <223> Description of Artificial Sequence: Synthetic peptide
<400> 679
   Gly Phe Ser Ile Lys Tyr Ser Tyr Ile His
   1               5                   10
<210> 680
   <211> 10
   <212> PRT
   <213> Artificial Sequence
<220>
   <223> Description of Artificial Sequence: Synthetic peptide
<400> 680
   Gly Phe Ser Ile Lys Tyr Ser Tyr Ile His
   1               5                   10
<210> 681
   <211> 10
   <212> PRT
   <213> Artificial Sequence
<220>
   <223> Description of Artificial Sequence: Synthetic peptide
<400> 681
   Gly Phe Tyr Ile Lys Tyr Ser Tyr Ile His
   1               5                   10
<210> 682
   <211> 17
   <212> PRT
   <213> Artificial Sequence
<220>
   <223> Description of Artificial Sequence: Synthetic peptide
<400> 682
   Ser Ile Tyr Pro Tyr Tyr Gly Tyr Thr Ser Tyr Ala Asp Ser Val Lys
   1               5                   10                  15
   Gly
<210> 683
   <211> 17
   <212> PRT
   <213> Artificial Sequence
<220>
   <223> Description of Artificial Sequence: Synthetic peptide
<400> 683
   Ser Ile Tyr Pro Tyr Tyr Gly Ser Thr Tyr Tyr Ala Asp Ser Val Lys
   1               5                   10                  15
   Gly
<210> 684
   <211> 17
   <212> PRT
   <213> Artificial Sequence
<220>
   <223> Description of Artificial Sequence: Synthetic peptide
<400> 684
   Ser Ile Tyr Pro Ser Ser Gly Tyr Thr Ser Tyr Ala Asp Ser Val Lys
   1               5                   10                  15
   Gly
<210> 685
   <211> 17
   <212> PRT
   <213> Artificial Sequence
<220>
   <223> Description of Artificial Sequence: Synthetic peptide
<400> 685
   Arg Ile Tyr Pro Thr Asn Gly Ala Thr Arg Tyr Ala Asp Ser Val Lys
   1               5                   10                  15
   Gly
<210> 686
   <211> 17
   <212> PRT
   <213> Artificial Sequence
<220>
   <223> Description of Artificial Sequence: Synthetic peptide
<400> 686
   Arg Ile Tyr Pro Thr Asn Gly Trp Thr Ile Tyr Ala Asp Ser Val Lys
   1 5 10 15
   Gly
<210> 687
   <211> 10
   <212> PRT
   <213> Artificial Sequence
<220>
   <223> Description of Artificial Sequence: Synthetic peptide
<400> 687
   Gly Met Ser Tyr Gly Tyr Gly Met Asp Tyr
   1 5 10
<210> 688
   <211> 10
   <212> PRT
   <213> Artificial Sequence
<220>
   <223> Description of Artificial Sequence: Synthetic peptide
<400> 688
   Gly Gly Arg Tyr Asn His Gly Leu Asp Tyr
   1 5 10
<210> 689
   <211> 17
   <212> PRT
   <213> Artificial Sequence
<220>
   <223> Description of Artificial Sequence: Synthetic peptide
<400> 689
   Glu Tyr Tyr Gln Gly Tyr Gly Pro Tyr Arg Ser Thr Tyr Gly Leu Asp
   1 5 10 15
   Tyr
<210> 690
   <211> 19
   <212> PRT
   <213> Artificial Sequence
<220>
   <223> Description of Artificial Sequence: Synthetic peptide
<400> 690
   Ser Ser Trp Ser Ser Arg Gly Val Ser Tyr Ser Arg Thr Ala Gly Gly
   1 5 10 15
   Met Asp Tyr
<210> 691
   <211> 20
   <212> PRT
   <213> Artificial Sequence
<220>
   <223> Description of Artificial Sequence: Synthetic peptide
<400> 691
   Glu Gly Tyr Tyr Ser Val Ser Gly Ser Tyr Ser Tyr Ser Thr Arg Gly
   1 5 10 15
   Gly Pro Asp Tyr
   20
<210> 692
   <211> 11
   <212> PRT
   <213> Artificial Sequence
<220>
   <223> Description of Artificial Sequence: Synthetic peptide
<400> 692
   Trp Gly Gly Asp Gly Phe Phe Ala Met Asp Tyr
   1 5 10
<210> 693
   <211> 11
   <212> PRT
   <213> Artificial Sequence
<220>
   <223> Description of Artificial Sequence: Synthetic peptide
<400> 693
   Trp Gly Gly Trp Gly Phe Phe Ala Met Asp Val
   1 5 10
<210> 694
   <211> 11
   <212> PRT
   <213> Artificial Sequence
<220>
   <223> Description of Artificial Sequence: Synthetic peptide
<400> 694
   Trp Gly Gly Asp Gly Phe Tyr Ala Met Asp Tyr
   1 5 10
<210> 695
   <211> 11
   <212> PRT
   <213> Artificial Sequence
<220>
   <223> Description of Artificial Sequence: Synthetic peptide
<400> 695
   Trp Gly Gly Asp Gly Phe Tyr Ala Met Asp Tyr
   1 5 10
<210> 696
   <211> 12
   <212> PRT
   <213> Artificial Sequence
<220>
   <223> Description of Artificial Sequence: Synthetic peptide
<400> 696
   Trp Gly His Asp Gly Met Phe Tyr Ala Met Asp Tyr
   1 5 10
<210> 697
   <211> 11
   <212> PRT
   <213> Artificial Sequence
<220>
   <223> Description of Artificial Sequence: Synthetic peptide
<400> 697
   Trp Gly Ala Asp Gly Asn Tyr Ala Met Asp Tyr
   1 5 10
<210> 698
   <211> 12
   <212> PRT
   <213> Artificial Sequence
<220>
   <223> Description of Artificial Sequence: Synthetic peptide
<400> 698
   Trp Gly Arg Ser Gly Tyr Phe Tyr Ala Met Asp Tyr
   1 5 10
<210> 699
   <211> 12
   <212> PRT
   <213> Artificial Sequence
<220>
   <223> Description of Artificial Sequence: Synthetic peptide
<400> 699
   Trp Gly Ala Ser Gly Leu Phe Tyr Ala Met Asp Tyr
   1               5                   10
<210> 700
   <211> 12
   <212> PRT
   <213> Artificial Sequence
<220>
   <223> Description of Artificial Sequence: Synthetic peptide
<400> 700
   Trp Gly Arg Asp Gly Leu Phe Tyr Ala Met Asp Tyr
   1               5                   10
<210> 701
   <211> 11
   <212> PRT
   <213> Artificial Sequence
<220>
   <223> Description of Artificial Sequence: Synthetic peptide
<400> 701
   Trp Gly Ser Asp Gly Phe Tyr Ala Met Asp Tyr
   1               5                   10
<210> 702
   <211> 12
   <212> PRT
   <213> Artificial Sequence
<220>
   <223> Description of Artificial Sequence: Synthetic peptide
<400> 702
   Trp Gly Thr Asp Gly Ala Phe Tyr Ala Met Asp Tyr
   1               5                   10
<210> 703
   <211> 11
   <212> PRT
   <213> Artificial Sequence
<220>
   <223> Description of Artificial Sequence: Synthetic peptide
<400> 703
   Trp Gly Lys Asp Gly Phe Tyr Ala Met Asp Tyr
   1 5 10
<210> 704
   <211> 12
   <212> PRT
   <213> Artificial Sequence
<220>
   <223> Description of Artificial Sequence: Synthetic peptide
<400> 704
   Trp Gly Leu Asp Gly His Phe Tyr Ala Met Asp Tyr
   1 5 10
<210> 705
   <211> 12
   <212> PRT
   <213> Artificial Sequence
<220>
   <223> Description of Artificial Sequence: Synthetic peptide
<400> 705
   Trp Gly His Leu Gly Tyr Phe Tyr Ala Met Asp Tyr
   1 5 10
<210> 706
   <211> 12
   <212> PRT
   <213> Artificial Sequence
<220>
   <223> Description of Artificial Sequence: Synthetic peptide
<400> 706
   Trp Gly Gly Asp Gly Pro Phe Tyr Ala Met Lys Tyr
   1 5 10
<210> 707
   <211> 12
   <212> PRT
   <213> Artificial Sequence
<220>
   <223> Description of Artificial Sequence: Synthetic peptide
<400> 707
   Trp Gly Gly Asp Gly Pro Phe Tyr Ala Met Leu Tyr
   1 5 10
<210> 708
   <211> 12
   <212> PRT
   <213> Artificial Sequence
<220>
   <223> Description of Artificial Sequence: Synthetic peptide
<400> 708
   Trp Gly Gly Asp Gly Pro Phe Tyr Ala Met Trp Tyr
   1               5                   10
<210> 709
   <211> 11
   <212> PRT
   <213> Artificial Sequence
<220>
   <223> Description of Artificial Sequence: Synthetic peptide
<400> 709
   Trp Gly Gly Asp Gly Trp Tyr Ala Met Asp Met
   1               5                   10
<210> 710
   <211> 11
   <212> PRT
   <213> Artificial Sequence
<220>
   <223> Description of Artificial Sequence: Synthetic peptide
<400> 710
   Trp Gly Gly Asp Gly Trp Tyr Ala Met Asp Leu
   1               5                   10
<210> 711
   <211> 11
   <212> PRT
   <213> Artificial Sequence
<220>
   <223> Description of Artificial Sequence: Synthetic peptide
<400> 711
   Trp Gly Gly Asp Gly His Tyr Ala Met Asp Leu
   1               5                   10
<210> 712
   <211> 11
   <212> PRT
   <213> Artificial Sequence
<220>
   <223> Description of Artificial Sequence: Synthetic peptide
<400> 712
   Trp Gly Gly Asp Gly Phe Tyr Ala Met Asp Trp
   1               5                   10
<210> 713
   <211> 11
   <212> PRT
   <213> Artificial Sequence
<220>
   <223> Description of Artificial Sequence: Synthetic peptide
<400> 713
   Trp Gly Gly Asp Gly Pro Tyr Ala Met Asp His
   1               5                   10
<210> 714
   <211> 11
   <212> PRT
   <213> Artificial Sequence
<220>
   <223> Description of Artificial Sequence: Synthetic peptide
<400> 714
   Trp Gly Gly Asp Gly Pro Tyr Ala Met Asp Lys
   1               5                   10
<210> 715
   <211> 11
   <212> PRT
   <213> Artificial Sequence
<220>
   <223> Description of Artificial Sequence: Synthetic peptide
<400> 715
   Trp Gly Gly Asp Gly Pro Tyr Ala Met Asp Leu
   1               5                   10
<210> 716
   <211> 11
   <212> PRT
   <213> Artificial Sequence
<220>
   <223> Description of Artificial Sequence: Synthetic peptide
<400> 716
   Trp Gly Gly Asp Gly Pro Tyr Ala Met Asp Lys
   1               5                   10
<210> 717
   <211> 11
   <212> PRT
   <213> Artificial Sequence
<220>
   <223> Description of Artificial Sequence: Synthetic peptide
<400> 717
   Trp Gly Gly Asp Gly Met Tyr Ala Met Asp Ala
   1 5 10
<210> 718
   <211> 11
   <212> PRT
   <213> Artificial Sequence
<220>
   <223> Description of Artificial Sequence: Synthetic peptide
<400> 718
   Trp Gly Gly Asp Gly Leu Tyr Ala Met Asp Ser
   1 5 10
<210> 719
   <211> 11
   <212> PRT
   <213> Artificial Sequence
<220>
   <223> Description of Artificial Sequence: Synthetic peptide
<400> 719
   Trp Gly Gly Asp Gly Leu Tyr Ala Met Asp Thr
   1 5 10
<210> 720
   <211> 11
   <212> PRT
   <213> Artificial Sequence
<220>
   <223> Description of Artificial Sequence: Synthetic peptide
<400> 720
   Trp Gly Gly Asp Gly Arg Tyr Ala Met Asp Gly
   1 5 10
<210> 721
   <211> 11
   <212> PRT
   <213> Artificial Sequence
<220>
   <223> Description of Artificial Sequence: Synthetic peptide
<400> 721
   Trp Gly Gly Asp Gly Met Tyr Ala Met Asp Gly
   1               5                   10
<210> 722
   <211> 11
   <212> PRT
   <213> Artificial Sequence
<220>
   <223> Description of Artificial Sequence: Synthetic peptide
<400> 722
   Trp Gly Gly Asp Gly Arg Tyr Ala Met Asp Leu
   1               5                   10
<210> 723
   <211> 11
   <212> PRT
   <213> Artificial Sequence
<220>
   <223> Description of Artificial Sequence: Synthetic peptide
<400> 723
   Trp Gly Gly Asp Gly Pro Tyr Ala Met Asp Ala
   1               5                   10
<210> 724
   <211> 11
   <212> PRT
   <213> Artificial Sequence
<220>
   <223> Description of Artificial Sequence: Synthetic peptide
<400> 724
   Trp Gly Gly Asp Gly Pro Tyr Ala Met Asp Val
   1               5                   10
<210> 725
   <211> 11
   <212> PRT
   <213> Artificial Sequence
<220>
   <223> Description of Artificial Sequence: Synthetic peptide
<400> 725
   Trp Gly Gly Asp Gly Pro Tyr Ala Met Asp Lys
   1               5                   10
<210> 726
   <211> 11
   <212> PRT
   <213> Artificial Sequence
<220>
   <223> Description of Artificial Sequence: Synthetic peptide
<400> 726
   Trp Gly Gly Asp Gly Pro Tyr Ala Met Asp Leu
   1               5                   10
<210> 727
   <211> 11
   <212> PRT
   <213> Artificial Sequence
<220>
   <223> Description of Artificial Sequence: Synthetic peptide
<400> 727
   Trp Gly Gly Asp Gly Leu Tyr Ala Met Asp Tyr
   1               5                   10
<210> 728
   <211> 11
   <212> PRT
   <213> Artificial Sequence
<220>
   <223> Description of Artificial Sequence: Synthetic peptide
<400> 728
   Trp Gly Gly Trp Gly Phe Tyr Ala Met Asp Tyr
   1               5                   10
<210> 729
   <211> 11
   <212> PRT
   <213> Artificial Sequence
<220>
   <223> Description of Artificial Sequence: Synthetic peptide
<400> 729
   Trp Gly Gly Asp Gly Phe Phe Ala Met Asp Tyr
   1               5                   10
<210> 730
   <211> 11
   <212> PRT
   <213> Artificial Sequence
<220>
   <223> Description of Artificial Sequence: Synthetic peptide
<400> 730
   Trp Gly Gly Trp Gly Pro Tyr Ala Met Asp Lys
   1 5 10
<210> 731
   <211> 11
   <212> PRT
   <213> Artificial Sequence
<220>
   <223> Description of Artificial Sequence: Synthetic peptide
<400> 731
   Trp Gly Gly Trp Gly Pro Tyr Ala Met Asp Leu
   1 5 10
<210> 732
   <211> 11
   <212> PRT
   <213> Artificial Sequence
<220>
   <223> Description of Artificial Sequence: Synthetic peptide
<400> 732
   Trp Gly Gly Asp Gly Phe Tyr Ala Met Asp Val
   1 5 10
<210> 733
   <211> 11
   <212> PRT
   <213> Artificial Sequence
<220>
   <223> Description of Artificial Sequence: Synthetic peptide
<400> 733
   Trp Gly Gly Asp Gly Pro Tyr Ala Met Asp Lys
   1 5 10
<210> 734
   <211> 9
   <212> PRT
   <213> Artificial Sequence
<220>
   <223> Description of Artificial Sequence: Synthetic peptide
<400> 734
   Gln Gln Ser Tyr Tyr Ser Pro Ser Thr
   1 5
<210> 735
   <211> 9
   <212> PRT
   <213> Artificial Sequence
<220>
   <223> Description of Artificial Sequence: Synthetic peptide
<400> 735
   Gln Gln Ser Tyr Tyr Tyr Pro Ser Thr
   1               5
<210> 736
   <211> 9
   <212> PRT
   <213> Artificial Sequence
<220>
   <223> Description of Artificial Sequence: Synthetic peptide
<400> 736
   Gln Gln Tyr Tyr Tyr Ser Pro Ser Thr
   1               5
<210> 737
   <211> 9
   <212> PRT
   <213> Artificial Sequence
<220>
   <223> Description of Artificial Sequence: Synthetic peptide
<400> 737
   Gln Gln Tyr Tyr Tyr Tyr Pro Tyr Thr
   1               5
<210> 738
   <211> 9
   <212> PRT
   <213> Artificial Sequence
<220>
   <223> Description of Artificial Sequence: Synthetic peptide
<400> 738
   Gln Gln Tyr Tyr Tyr Tyr Pro Ser Thr
   1               5
<210> 739
   <211> 9
   <212> PRT
   <213> Artificial Sequence
<220>
   <223> Description of Artificial Sequence: Synthetic peptide
<400> 739
   Gln Gln Ser Tyr Tyr Tyr Pro Ser Thr
   1 5
<210> 740
   <211> 9
   <212> PRT
   <213> Artificial Sequence
<220>
   <223> Description of Artificial Sequence: Synthetic peptide
<400> 740
   Gln Gln Trp Ser Ser Ser Pro Ser Thr
   1 5
<210> 741
   <211> 9
   <212> PRT
   <213> Artificial Sequence
<220>
   <223> Description of Artificial Sequence: Synthetic peptide
<400> 741
   Gln Gln Trp Ser Ser Trp Pro Ser Thr
   1 5
<210> 742
   <211> 9
   <212> PRT
   <213> Artificial Sequence
<220>
   <223> Description of Artificial Sequence: Synthetic peptide
<400> 742
   Gln Gln Ser Tyr Tyr Ser Pro Ser Thr
   1 5
<210> 743
   <211> 9
   <212> PRT
   <213> Artificial Sequence
<220>
   <223> Description of Artificial Sequence: Synthetic peptide
<400> 743
   Gln Gln Tyr Tyr Tyr Ser Pro Ser Thr
   1 5
<210> 744
   <211> 9
   <212> PRT
   <213> Artificial Sequence
<220>
   <223> Description of Artificial Sequence: Synthetic peptide
<400> 744
   Gln Gln Ser Tyr Tyr Tyr Pro Ser Thr
   1 5
<210> 745
   <211> 9
   <212> PRT
   <213> Artificial Sequence
<220>
   <223> Description of Artificial Sequence: Synthetic peptide
<400> 745
   Gln Gln Ser Ser Ser Ser Pro Ser Thr
   1 5
<210> 746
   <211> 9
   <212> PRT
   <213> Artificial Sequence
<220>
   <223> Description of Artificial Sequence: Synthetic peptide
<400> 746
   Gln Gln Ser Tyr Tyr Tyr Pro Tyr Thr
   1 5
<210> 747
   <211> 9
   <212> PRT
   <213> Artificial Sequence
<220>
   <223> Description of Artificial Sequence: Synthetic peptide
<400> 747
   Gln Gln Tyr Tyr Tyr Ser Pro Tyr Thr
   1 5
<210> 748
   <211> 9
   <212> PRT
   <213> Artificial Sequence
<220>
   <223> Description of Artificial Sequence: Synthetic peptide
<400> 748
   Gln Gln Ser Tyr Ser Ser Pro Ser Thr
   1               5
<210> 749
   <211> 9
   <212> PRT
   <213> Artificial Sequence
<220>
   <223> Description of Artificial Sequence: Synthetic peptide
<400> 749
   Gln Gln Tyr Ser Tyr Tyr Pro Tyr Thr
   1               5
<210> 750
   <211> 9
   <212> PRT
   <213> Artificial Sequence
<220>
   <223> Description of Artificial Sequence: Synthetic peptide
<400> 750
   Gln Gln Tyr Ser Ser Ser Pro Ser Thr
   1               5
<210> 751
   <211> 9
   <212> PRT
   <213> Artificial Sequence
<220>
   <223> Description of Artificial Sequence: Synthetic peptide
<400> 751
   Gln Gln Ser Ser Tyr Tyr Pro Ser Thr
   1               5
<210> 752
   <211> 9
   <212> PRT
   <213> Artificial Sequence
<220>
   <223> Description of Artificial Sequence: Synthetic peptide
<400> 752
   Gln Gln Ser Tyr Tyr Ser Pro Ser Thr
   1 5
<210> 753
   <211> 9
   <212> PRT
   <213> Artificial Sequence
<220>
   <223> Description of Artificial Sequence: Synthetic peptide
<400> 753
   Gln Gln Ser Tyr Tyr Tyr Pro Ser Thr
   1 5
<210> 754
   <211> 9
   <212> PRT
   <213> Artificial Sequence
<220>
   <223> Description of Artificial Sequence: Synthetic peptide
<400> 754
   Gln Gln His Tyr Tyr Tyr Pro Tyr Thr
   1 5
<210> 755
   <211> 9
   <212> PRT
   <213> Artificial Sequence
<220>
   <223> Description of Artificial Sequence: Synthetic peptide
<400> 755
   Gln Gln His Tyr Tyr Ser Pro Ser Thr
   1 5
<210> 756
   <211> 9
   <212> PRT
   <213> Artificial Sequence
<220>
   <223> Description of Artificial Sequence: Synthetic peptide
<400> 756
   Gln Gln Ser Tyr Tyr Tyr Pro Tyr Thr
   1 5
<210> 757
   <211> 9
   <212> PRT
   <213> Artificial Sequence
<220>
   <223> Description of Artificial Sequence: Synthetic peptide
<220>
   <221> MOD_RES
   <222> (3)..(6)
   <223> Any amino acid
<220>
   <221> MOD_RES
   <222> (8)..(8)
   <223> Any amino acid
<400> 757
   Gln Gln Xaa Xaa Xaa Xaa Pro Xaa Thr
   1               5
<210> 758
   <211> 9
   <212> PRT
   <213> Artificial Sequence
<220>
   <223> Description of Artificial Sequence: Synthetic peptide
<400> 758
   Gln Gln His Ser Tyr Tyr Pro Tyr Thr
   1               5
<210> 759
   <211> 9
   <212> PRT
   <213> Artificial Sequence
<220>
   <223> Description of Artificial Sequence: Synthetic peptide
<400> 759
   Gln Gln His Ser Tyr Tyr Pro Ser Thr
   1               5
<210> 760
   <211> 9
   <212> PRT
   <213> Artificial Sequence
<220>
   <223> Description of Artificial Sequence: Synthetic peptide
<400> 760
   Gln Gln Ser Ser Tyr Ser Pro Ser Thr
   1 5
<210> 761
   <211> 9
   <212> PRT
   <213> Artificial Sequence
<220>
   <223> Description of Artificial Sequence: Synthetic peptide
<400> 761
   Gln Gln His Ser Tyr Tyr Pro Tyr Thr
   1 5
<210> 762
   <211> 9
   <212> PRT
   <213> Artificial Sequence
<220>
   <223> Description of Artificial Sequence: Synthetic peptide
<400> 762
   Gln Gln His Ser Tyr Tyr Pro Ser Thr
   1 5
<210> 763
   <211> 9
   <212> PRT
   <213> Artificial Sequence
<220>
   <223> Description of Artificial Sequence: Synthetic peptide
<400> 763
   Gln Gln Ser Ser Tyr Ser Pro Ser Thr
   1 5
<210> 764
   <211> 9
   <212> PRT
   <213> Artificial Sequence
<220>
   <223> Description of Artificial Sequence: Synthetic peptide
<400> 764
   Gln Gln Ser Ser Tyr Tyr Pro Tyr Thr
   1 5
<210> 765
   <211> 9
   <212> PRT
   <213> Artificial Sequence
<220>
   <223> Description of Artificial Sequence: Synthetic peptide
<400> 765
   Gln Gln Ser Tyr Tyr Tyr Pro Ser Thr
   1 5
<210> 766
   <211> 9
   <212> PRT
   <213> Artificial Sequence
<220>
   <223> Description of Artificial Sequence: Synthetic peptide
<400> 766
   Gln Gln Ser Phe Ser Ser Pro Tyr Thr
   1 5
<210> 767
   <211> 9
   <212> PRT
   <213> Artificial Sequence
<220>
   <223> Description of Artificial Sequence: Synthetic peptide
<400> 767
   Gln Gln Ser Tyr Ser Ser Pro Tyr Thr
   1 5
<210> 768
   <211> 9
   <212> PRT
   <213> Artificial Sequence
<220>
   <223> Description of Artificial Sequence: Synthetic peptide
<400> 768
   Gln Gln Ser Tyr Ser Tyr Pro Ser Thr
   1 5
<210> 769
   <211> 9
   <212> PRT
   <213> Artificial Sequence
<220>
   <223> Description of Artificial Sequence: Synthetic peptide
<400> 769
   Gln Gln Ser Phe Ser Tyr Pro Tyr Thr
   1               5
<210> 770
   <211> 9
   <212> PRT
   <213> Artificial Sequence
<220>
   <223> Description of Artificial Sequence: Synthetic peptide
<400> 770
   Gln Gln Ser Tyr Ser Ser Pro Ser Thr
   1               5
<210> 771
   <211> 9
   <212> PRT
   <213> Artificial Sequence
<220>
   <223> Description of Artificial Sequence: Synthetic peptide
<400> 771
   Gln Gln Ser Tyr Ser Ser Pro Tyr Thr
   1               5
<210> 772
   <211> 9
   <212> PRT
   <213> Artificial Sequence
<220>
   <223> Description of Artificial Sequence: Synthetic peptide
<400> 772
   Gln Gln Ser Tyr Ser Ser Pro Ser Thr
   1               5
<210> 773
   <211> 9
   <212> PRT
   <213> Artificial Sequence
<220>
   <223> Description of Artificial Sequence: Synthetic peptide
<400> 773
   Gln Gln Ser Phe Ser Ser Pro Ser Thr
   1               5
<210> 774
   <211> 9
   <212> PRT
   <213> Artificial Sequence
<220>
   <223> Description of Artificial Sequence: Synthetic peptide
<400> 774
   Gln Gln Ser Ser Ser Ser Pro Tyr Thr
   1               5
<210> 775
   <211> 9
   <212> PRT
   <213> Artificial Sequence
<220>
   <223> Description of Artificial Sequence: Synthetic peptide
<400> 775
   Gln Gln Ser Ser Ser Tyr Pro Tyr Thr
   1               5
<210> 776
   <211> 9
   <212> PRT
   <213> Artificial Sequence
<220>
   <223> Description of Artificial Sequence: Synthetic peptide
<400> 776
   Gln Gln Ser Tyr Ser Tyr Pro Ser Thr
   1               5
<210> 777
   <211> 9
   <212> PRT
   <213> Artificial Sequence
<220>
   <223> Description of Artificial Sequence: Synthetic peptide
<400> 777
   Gln Gln Ser Ser Ser Ser Pro Tyr Thr
   1               5
<210> 778
   <211> 9
   <212> PRT
   <213> Artificial Sequence
<220>
   <223> Description of Artificial Sequence: Synthetic peptide
<400> 778
   Gln Gln Ser Ser Ser Tyr Pro Ser Thr
   1 5
<210> 779
   <211> 9
   <212> PRT
   <213> Artificial Sequence
<220>
   <223> Description of Artificial Sequence: Synthetic peptide
<400> 779
   Gln Gln Ser Ser Tyr Tyr Pro Ser Thr
   1 5
<210> 780
   <211> 9
   <212> PRT
   <213> Artificial Sequence
<220>
   <223> Description of Artificial Sequence: Synthetic peptide
<400> 780
   Gln Gln Ser Ser Phe Ser Pro Tyr Thr
   1 5
<210> 781
   <211> 9
   <212> PRT
   <213> Artificial Sequence
<220>
   <223> Description of Artificial Sequence: Synthetic peptide
<400> 781
   Gln Gln Ser Ser Ser Tyr Pro Ser Thr
   1 5
<210> 782
   <211> 9
   <212> PRT
   <213> Artificial Sequence
<220>
   <223> Description of Artificial Sequence: Synthetic peptide
<400> 782
   Gln Gln Ser Ser Ser Ser Pro Tyr Thr
   1 5
<210> 783
   <211> 9
   <212> PRT
   <213> Artificial Sequence
<220>
   <223> Description of Artificial Sequence: Synthetic peptide
<400> 783
   Gln Gln Ser Tyr Ser Ser Pro Tyr Thr
   1 5
<210> 784
   <211> 9
   <212> PRT
   <213> Artificial Sequence
<220>
   <223> Description of Artificial Sequence: Synthetic peptide
<400> 784
   Gln Gln Ser Tyr Ser Ser Pro Ser Thr
   1 5
<210> 785
   <211> 9
   <212> PRT
   <213> Artificial Sequence
<220>
   <223> Description of Artificial Sequence: Synthetic peptide
<400> 785
   Gln Gln Ser Ser Ser Tyr Pro Tyr Thr
   1 5
<210> 786
   <211> 9
   <212> PRT
   <213> Artificial Sequence
<220>
   <223> Description of Artificial Sequence: Synthetic peptide
<400> 786
   Gln Gln Ser Phe Ser Ser Pro Tyr Thr
   1 5
<210> 787
   <211> 9
   <212> PRT
   <213> Artificial Sequence
<220>
   <223> Description of Artificial Sequence: Synthetic peptide
<400> 787
   Gln Gln Ser Tyr Ser Tyr Pro Ser Thr
   1 5
<210> 788
   <211> 9
   <212> PRT
   <213> Artificial Sequence
<220>
   <223> Description of Artificial Sequence: Synthetic peptide
<400> 788
   Gln Gln His Tyr Ser Ser Pro Tyr Thr
   1 5
<210> 789
   <211> 9
   <212> PRT
   <213> Artificial Sequence
<220>
   <223> Description of Artificial Sequence: Synthetic peptide
<400> 789
   Gln Gln Ser Ser Ser Tyr Pro Ser Thr
   1 5
<210> 790
   <211> 9
   <212> PRT
   <213> Artificial Sequence
<220>
   <223> Description of Artificial Sequence: Synthetic peptide
<400> 790
   Gln Gln His Tyr Ser Ser Pro Ser Thr
   1 5
<210> 791
   <211> 9
   <212> PRT
   <213> Artificial Sequence
<220>
   <223> Description of Artificial Sequence: Synthetic peptide
<400> 791
   Gln Gln Ser Ser Tyr Tyr Pro Ser Thr
   1 5
<210> 792
   <211> 9
   <212> PRT
   <213> Artificial Sequence
<220>
   <223> Description of Artificial Sequence: Synthetic peptide
<400> 792
   Gln Gln Ser Ser Phe Tyr Pro Ser Thr
   1               5
<210> 793
   <211> 9
   <212> PRT
   <213> Artificial Sequence
<220>
   <223> Description of Artificial Sequence: Synthetic peptide
<400> 793
   Gln Gln Ser Ser Tyr Ser Pro Tyr Thr
   1               5
<210> 794
   <211> 9
   <212> PRT
   <213> Artificial Sequence
<220>
   <223> Description of Artificial Sequence: Synthetic peptide
<400> 794
   Gln Gln Ser Ser Ser Ser Pro Ser Thr
   1               5
<210> 795
   <211> 9
   <212> PRT
   <213> Artificial Sequence
<220>
   <223> Description of Artificial Sequence: Synthetic peptide
<400> 795
   Gln Gln His Tyr Tyr Tyr Pro Tyr Thr
   1               5
<210> 796
   <211> 9
   <212> PRT
   <213> Artificial Sequence
<220>
   <223> Description of Artificial Sequence: Synthetic peptide
<400> 796
   Gln Gln Ser Ser Tyr Ser Pro Tyr Thr
   1 5
<210> 797
   <211> 9
   <212> PRT
   <213> Artificial Sequence
<220>
   <223> Description of Artificial Sequence: Synthetic peptide
<400> 797
   Gln Gln His Tyr Ser Ser Pro Tyr Thr
   1 5
<210> 798
   <211> 9
   <212> PRT
   <213> Artificial Sequence
<220>
   <223> Description of Artificial Sequence: Synthetic peptide
<400> 798
   Gln Gln His Tyr Ser Tyr Pro Ser Thr
   1 5
<210> 799
   <211> 9
   <212> PRT
   <213> Artificial Sequence
<220>
   <223> Description of Artificial Sequence: Synthetic peptide
<400> 799
   Gln Gln Ser Ser Tyr Tyr Pro Ser Thr
   1 5
<210> 800
   <211> 9
   <212> PRT
   <213> Artificial Sequence
<220>
   <223> Description of Artificial Sequence: Synthetic peptide
<400> 800
   Gln Gln Ser Ser Ser Ser Pro Tyr Thr
   1 5
<210> 801
   <211> 9
   <212> PRT
   <213> Artificial Sequence
<220>
   <223> Description of Artificial Sequence: Synthetic peptide
<400> 801
   Gln Gln Ser Ser Tyr Tyr Pro Tyr Thr
   1 5
<210> 802
   <211> 9
   <212> PRT
   <213> Artificial Sequence
<220>
   <223> Description of Artificial Sequence: Synthetic peptide
<400> 802
   Gln Gln Ser Tyr Tyr Tyr Pro Tyr Thr
   1 5
<210> 803
   <211> 9
   <212> PRT
   <213> Artificial Sequence
<220>
   <223> Description of Artificial Sequence: Synthetic peptide
<400> 803
   Gln Gln His Tyr Tyr Ser Pro Ser Thr
   1 5
<210> 804
   <211> 11
   <212> PRT
   <213> Artificial Sequence
<220>
   <223> Description of Artificial Sequence: Synthetic peptide
<400> 804
   Arg Ala Ser Gln Asp Val Ser Thr Ala Val Ala
   1 5 10
<210> 805
   <211> 11
   <212> PRT
   <213> Artificial Sequence
<220>
   <223> Description of Artificial Sequence: Synthetic peptide
<400> 805
   Arg Ala Ser Gln Asp Val Asn Thr Ala Tyr Ala
   1 5 10
<210> 806
   <211> 11
   <212> PRT
   <213> Artificial Sequence
<220>
   <223> Description of Artificial Sequence: Synthetic peptide
<400> 806
   Arg Ala Ser Gln Asp Val Asn Thr Ala Trp Ala
   1 5 10
<210> 807
   <211> 7
   <212> PRT
   <213> Artificial Sequence
<220>
   <223> Description of Artificial Sequence: Synthetic peptide
<400> 807
   Arg Ala Ser Phe Leu Tyr Ser
   1 5
<210> 808
   <211> 9
   <212> PRT
   <213> Artificial Sequence
<220>
   <223> Description of Artificial Sequence: Synthetic peptide
<400> 808
   Gln Gln His Ser Tyr Tyr Pro Tyr Thr
   1 5
<210> 809
   <211> 9
   <212> PRT
   <213> Artificial Sequence
<220>
   <223> Description of Artificial Sequence: Synthetic peptide
<400> 809
   Gln Gln Ser Ser Tyr Tyr Pro Tyr Thr
   1 5
<210> 810
   <211> 9
   <212> PRT
   <213> Artificial Sequence
<220>
   <223> Description of Artificial Sequence: Synthetic peptide
<400> 810
   Gln Gln His Ser Tyr Tyr Pro Ser Thr
   1               5
<210> 811
   <211> 9
   <212> PRT
   <213> Artificial Sequence
<220>
   <223> Description of Artificial Sequence: Synthetic peptide
<400> 811
   Gln Gln His Ser Ser Ser Pro Ser Thr
   1               5
<210> 812
   <211> 9
   <212> PRT
   <213> Artificial Sequence
<220>
   <223> Description of Artificial Sequence: Synthetic peptide
<400> 812
   Gln Gln His Ser Ser Ser Pro Tyr Thr
   1               5
<210> 813
   <211> 9
   <212> PRT
   <213> Artificial Sequence
<220>
   <223> Description of Artificial Sequence: Synthetic peptide
<400> 813
   Gln Gln Ser Tyr Ser Ser Pro Ser Thr
   1               5
<210> 814
   <211> 9
   <212> PRT
   <213> Artificial Sequence
<220>
   <223> Description of Artificial Sequence: Synthetic peptide
<400> 814
   Gln Gln Phe Trp Thr Ser Pro Pro Thr
   1 5
<210> 815
   <211> 11
   <212> PRT
   <213> Artificial Sequence
<220>
   <223> Description of Artificial Sequence: Synthetic peptide
<400> 815
   Arg Ala Ser Gln Asp Val Asn Thr Ala Tyr Ala
   1 5 10
<210> 816
   <211> 11
   <212> PRT
   <213> Artificial Sequence
<220>
   <223> Description of Artificial Sequence: Synthetic peptide
<400> 816
   Arg Ala Ser Gln Asp Val Asn Thr Ala Trp Ala
   1 5 10
<210> 817
   <211> 11
   <212> PRT
   <213> Artificial Sequence
<220>
   <223> Description of Artificial Sequence: Synthetic peptide
<400> 817
   Arg Ala Ser Gln Asp Val Asn Thr Ala Phe Ala
   1 5 10
<210> 818
   <211> 11
   <212> PRT
   <213> Artificial Sequence
<220>
   <223> Description of Artificial Sequence: Synthetic peptide
<400> 818
   Arg Ala Ser Gln Asp Val Ser Thr Ala Val Ala
   1 5 10
<210> 819
   <211> 7
   <212> PRT
   <213> Artificial Sequence
<220>
   <223> Description of Artificial Sequence: Synthetic peptide
<400> 819
   Arg Ala Ser Phe Leu Tyr Ser
   1 5
<210> 820
   <211> 9
   <212> PRT
   <213> Artificial Sequence
<220>
   <223> Description of Artificial Sequence: Synthetic peptide
<400> 820
   Gln Gln Phe Trp Thr Thr Pro Pro Thr
   1 5
<210> 821
   <211> 9
   <212> PRT
   <213> Artificial Sequence
<220>
   <223> Description of Artificial Sequence: Synthetic peptide
<400> 821
   Gln Gln Tyr Trp Thr Thr Pro Pro Thr
   1 5
<210> 822
   <211> 9
   <212> PRT
   <213> Artificial Sequence
<220>
   <223> Description of Artificial Sequence: Synthetic peptide
<400> 822
   Gln Gln His Trp Thr Thr Pro Pro Thr
   1 5
<210> 823
   <211> 9
   <212> PRT
   <213> Artificial Sequence
<220>
   <223> Description of Artificial Sequence: Synthetic peptide
<400> 823
   Gln Gln Phe Phe Thr Thr Pro Pro Thr
   1               5
<210> 824
   <211> 9
   <212> PRT
   <213> Artificial Sequence
<220>
   <223> Description of Artificial Sequence: Synthetic peptide
<400> 824
   Gln Gln Phe Gly Thr Thr Pro Pro Thr
   1               5
<210> 825
   <211> 11
   <212> PRT
   <213> Artificial Sequence
<220>
   <223> Description of Artificial Sequence: Synthetic peptide
<400> 825
   Arg Ala Ser Gln Asp Val Ser Thr Ala Val Ala
   1               5                   10
<210> 826
   <211> 7
   <212> PRT
   <213> Artificial Sequence
<220>
   <223> Description of Artificial Sequence: Synthetic peptide
<400> 826
   Ser Ala Ser Val Leu Tyr Ser
   1               5
<210> 827
   <211> 7
   <212> PRT
   <213> Artificial Sequence
<220>
   <223> Description of Artificial Sequence: Synthetic peptide
<400> 827
   Ser Ala Ser Trp Leu Tyr Ser
   1               5
<210> 828
   <211> 7
   <212> PRT
   <213> Artificial Sequence
<220>
   <223> Description of Artificial Sequence: Synthetic peptide
<400> 828
   Ser Ala Ser Leu Leu Tyr Ser
   1 5
<210> 829
   <211> 7
   <212> PRT
   <213> Artificial Sequence
<220>
   <223> Description of Artificial Sequence: Synthetic peptide
<400> 829
   Ser Ala Ser Trp Leu Trp Ser
   1 5
<210> 830
   <211> 9
   <212> PRT
   <213> Artificial Sequence
<220>
   <223> Description of Artificial Sequence: Synthetic peptide
<400> 830
   Gln Gln Phe Gly Thr Thr Pro Pro Thr
   1 5
<210> 831
   <211> 9
   <212> PRT
   <213> Artificial Sequence
<220>
   <223> Description of Artificial Sequence: Synthetic peptide
<400> 831
   Gln Gln Ile Trp Thr Thr Pro Pro Thr
   1 5
<210> 832
   <211> 9
   <212> PRT
   <213> Artificial Sequence
<220>
   <223> Description of Artificial Sequence: Synthetic peptide
<400> 832
   Gln Gln Phe Trp Thr Thr Pro Pro Thr
   1               5
<210> 833
   <211> 9
   <212> PRT
   <213> Artificial Sequence
<220>
   <223> Description of Artificial Sequence: Synthetic peptide
<400> 833
   Gln Gln Tyr Tyr Thr Thr Pro Pro Thr
   1               5
<210> 834
   <211> 9
   <212> PRT
   <213> Artificial Sequence
<220>
   <223> Description of Artificial Sequence: Synthetic peptide
<400> 834
   Gln Gln Phe Tyr Thr Thr Pro Pro Thr
   1               5
<210> 835
   <211> 9
   <212> PRT
   <213> Artificial Sequence
<220>
   <223> Description of Artificial Sequence: Synthetic peptide
<400> 835
   Gln Gln Thr Tyr Thr Thr Pro Pro Thr
   1               5
<210> 836
   <211> 9
   <212> PRT
   <213> Artificial Sequence
<220>
   <223> Description of Artificial Sequence: Synthetic peptide
<400> 836
   Gln Gln Trp Tyr Thr Thr Pro Pro Thr
   1               5
<210> 837
   <211> 7
   <212> PRT
   <213> Artificial Sequence
<220>
   <223> Description of Artificial Sequence: Synthetic peptide
<400> 837
   Ser Ala Ser Trp Leu Trp Ser
   1 5
<210> 838
   <211> 7
   <212> PRT
   <213> Artificial Sequence
<220>
   <223> Description of Artificial Sequence: Synthetic peptide
<400> 838
   Ser Ala Ser Thr Leu Trp Ser
   1 5
<210> 839
   <211> 7
   <212> PRT
   <213> Artificial Sequence
<220>
   <223> Description of Artificial Sequence: Synthetic peptide
<400> 839
   Ser Ala Ser Phe Leu Trp Ser
   1 5
<210> 840
   <211> 7
   <212> PRT
   <213> Artificial Sequence
<220>
   <223> Description of Artificial Sequence: Synthetic peptide
<400> 840
   Ser Ala Ser His Leu Trp Ser
   1 5
<210> 841
   <211> 7
   <212> PRT
   <213> Artificial Sequence
<220>
   <223> Description of Artificial Sequence: Synthetic peptide
<400> 841
   Ser Ala Ser Val Leu Trp Ser
   1 5
<210> 842
   <211> 7
   <212> PRT
   <213> Artificial Sequence
<220>
   <223> Description of Artificial Sequence: Synthetic peptide
<400> 842
   Ser Ala Ser Val Leu Leu Ser
   1 5
<210> 843
   <211> 7
   <212> PRT
   <213> Artificial Sequence
<220>
   <223> Description of Artificial Sequence: Synthetic peptide
<400> 843
   Ser Ala Ser Arg Leu Trp Ser
   1 5
<210> 844
   <211> 7
   <212> PRT
   <213> Artificial Sequence
<220>
   <223> Description of Artificial Sequence: Synthetic peptide
<400> 844
   Ser Ala Ser Gln Leu Phe Ser
   1 5
<210> 845
   <211> 7
   <212> PRT
   <213> Artificial Sequence
<220>
   <223> Description of Artificial Sequence: Synthetic peptide
<400> 845
   Ser Ala Ser Trp Leu Leu Ser
   1 5
<210> 846
   <211> 7
   <212> PRT
   <213> Artificial Sequence
<220>
   <223> Description of Artificial Sequence: Synthetic peptide
<400> 846
   Ser Ala Ser Thr Leu Tyr Ser
   1               5
<210> 847
   <211> 7
   <212> PRT
   <213> Artificial Sequence
<220>
   <223> Description of Artificial Sequence: Synthetic peptide
<400> 847
   Ser Ala Ser Trp Leu Val Ser
   1               5
<210> 848
   <211> 7
   <212> PRT
   <213> Artificial Sequence
<220>
   <223> Description of Artificial Sequence: Synthetic peptide
<400> 848
   Ser Ala Ser Lys Leu Trp Ser
   1               5
<210> 849
   <211> 7
   <212> PRT
   <213> Artificial Sequence
<220>
   <223> Description of Artificial Sequence: Synthetic peptide
<400> 849
   Ser Ala Ser Ala Leu Trp Ser
   1               5
<210> 850
   <211> 7
   <212> PRT
   <213> Artificial Sequence
<220>
   <223> Description of Artificial Sequence: Synthetic peptide
<400> 850
   Ser Ala Ser Arg Leu Val Ser
   1               5
<210> 851
   <211> 7
   <212> PRT
   <213> Artificial Sequence
<220>
   <223> Description of Artificial Sequence: Synthetic peptide
<400> 851
   Ser Ala Ser Lys Leu Ser Ser
   1               5
<210> 852
   <211> 7
   <212> PRT
   <213> Artificial Sequence
<220>
   <223> Description of Artificial Sequence: Synthetic peptide
<400> 852
   Ser Ala Ser Val Leu Trp Ser
   1               5
<210> 853
   <211> 7
   <212> PRT
   <213> Artificial Sequence
<220>
   <223> Description of Artificial Sequence: Synthetic peptide
<400> 853
   Ser Ala Ser Arg Leu Ala Ser
   1               5
<210> 854
   <211> 7
   <212> PRT
   <213> Artificial Sequence
<220>
   <223> Description of Artificial Sequence: Synthetic peptide
<400> 854
   Ser Ala Ser Leu Leu Pro Ser
   1               5
<210> 855
   <211> 7
   <212> PRT
   <213> Artificial Sequence
<220>
   <223> Description of Artificial Sequence: Synthetic peptide
<400> 855
   Ser Ala Ser Met Leu Gly Ser
   1               5
<210> 856
   <211> 13
   <212> PRT
   <213> Artificial Sequence
<220>
   <223> Description of Artificial Sequence: Synthetic peptide
<400> 856
   Arg Ala Ser Gln Ser Asp Gln Asn Ser Thr Ser Gly Ala
   1               5                   10
<210> 857
   <211> 11
   <212> PRT
   <213> Artificial Sequence
<220>
   <223> Description of Artificial Sequence: Synthetic peptide
<400> 857
   Arg Ala Ser Ser Gln Val Ser Gly Gly Val Ala
   1               5                   10
<210> 858
   <211> 11
   <212> PRT
   <213> Artificial Sequence
<220>
   <223> Description of Artificial Sequence: Synthetic peptide
<400> 858
   Arg Ala Ser Arg Gln Val Asn Thr Ala Val Ala
   1               5                   10
<210> 859
   <211> 11
   <212> PRT
   <213> Artificial Sequence
<220>
   <223> Description of Artificial Sequence: Synthetic peptide
<400> 859
   Arg Ala Ser Ala Gln Val Ser Ala Gly Val Ala
   1               5                   10
<210> 860
   <211> 11
   <212> PRT
   <213> Artificial Sequence
<220>
   <223> Description of Artificial Sequence: Synthetic peptide
<400> 860
   Arg Ala Ser Gln Gly Val Ser Ser Gly Val Ala
   1               5                   10
<210> 861
   <211> 11
   <212> PRT
   <213> Artificial Sequence
<220>
   <223> Description of Artificial Sequence: Synthetic peptide
<400> 861
   Arg Ala Ser Gln Gly Val Ser Ser Ala Val Ala
   1               5                   10
<210> 862
   <211> 11
   <212> PRT
   <213> Artificial Sequence
<220>
   <223> Description of Artificial Sequence: Synthetic peptide
<400> 862
   Arg Ala Ser Gln Arg Val Asn Ser Ala Val Ala
   1               5                   10
<210> 863
   <211> 11
   <212> PRT
   <213> Artificial Sequence
<220>
   <223> Description of Artificial Sequence: Synthetic peptide
<400> 863
   Arg Ala Ser Gln Gly Val Ser Ser Ala Val Ala
   1 5 10
<210> 864
   <211> 11
   <212> PRT
   <213> Artificial Sequence
<220>
   <223> Description of Artificial Sequence: Synthetic peptide
<400> 864
   Arg Ala Ser Asn Pro Val Ser Gln Ala Val Ala
   1 5 10
<210> 865
   <211> 11
   <212> PRT
   <213> Artificial Sequence
<220>
   <223> Description of Artificial Sequence: Synthetic peptide
<400> 865
   Arg Ala Ser Ser Ser Val Lys Ala Ser Val Ala
   1 5 10
<210> 866
   <211> 11
   <212> PRT
   <213> Artificial Sequence
<220>
   <223> Description of Artificial Sequence: Synthetic peptide
<400> 866
   Arg Ala Ser Phe Asp Val Asn Ala Cys Val Ala
   1 5 10
<210> 867
   <211> 11
   <212> PRT
   <213> Artificial Sequence
<220>
   <223> Description of Artificial Sequence: Synthetic peptide
<400> 867
   Arg Ala Ser Gln Asp Val Ser Thr Ala Val Ala
   1 5 10
<210> 868
   <211> 9
   <212> PRT
   <213> Artificial Sequence
<220>
   <223> Description of Artificial Sequence: Synthetic peptide
<400> 868
   Gln Gln His Trp Thr Thr Pro Pro Thr
   1 5
<210> 869
   <211> 9
   <212> PRT
   <213> Artificial Sequence
<220>
   <223> Description of Artificial Sequence: Synthetic peptide
<400> 869
   Gln Gln Phe Phe Thr Thr Pro Pro Thr
   1 5
<210> 870
   <211> 9
   <212> PRT
   <213> Artificial Sequence
<220>
   <223> Description of Artificial Sequence: Synthetic peptide
<400> 870
   Gln Gln Tyr Trp Thr Thr Pro Pro Thr
   1 5
<210> 871
   <211> 9
   <212> PRT
   <213> Artificial Sequence
<220>
   <223> Description of Artificial Sequence: Synthetic peptide
<400> 871
   Gln Gln His Phe Thr Thr Pro Pro Thr
   1 5
<210> 872
   <211> 9
   <212> PRT
   <213> Artificial Sequence
<220>
   <223> Description of Artificial Sequence: Synthetic peptide
<400> 872
   Gln Gln Leu Pro Thr Thr Pro Pro Thr
   1 5
<210> 873
   <211> 9
   <212> PRT
   <213> Artificial Sequence
<220>
   <223> Description of Artificial Sequence: Synthetic peptide
<400> 873
   Gln Gln His Gln Thr Thr Pro Pro Thr
   1 5
<210> 874
   <211> 9
   <212> PRT
   <213> Artificial Sequence
<220>
   <223> Description of Artificial Sequence: Synthetic peptide
<400> 874
   Gln Gln Phe Tyr Thr Thr Pro Pro Thr
   1 5
<210> 875
   <211> 9
   <212> PRT
   <213> Artificial Sequence
<220>
   <223> Description of Artificial Sequence: Synthetic peptide
<400> 875
   Gln Gln His Tyr Thr Ser Pro Pro Thr
   1 5
<210> 876
   <211> 11
   <212> PRT
   <213> Artificial Sequence
<220>
   <223> Description of Artificial Sequence: Synthetic peptide
<400> 876
   Arg Ala Ser Gln Asp Val Trp Lys Trp Val Ala
   1 5 10
<210> 877
   <211> 12
   <212> PRT
   <213> Artificial Sequence
<220>
   <223> Description of Artificial Sequence: Synthetic peptide
<400> 877
   Arg Ala Ser Gln Asp Ile Lys Asn Gly Ser Val Ala
   1               5                   10
<210> 878
   <211> 12
   <212> PRT
   <213> Artificial Sequence
<220>
   <223> Description of Artificial Sequence: Synthetic peptide
<400> 878
   Arg Ala Ser Gln Asp Ile Leu Gly Gly Ser Val Ala
   1               5                   10
<210> 879
   <211> 12
   <212> PRT
   <213> Artificial Sequence
<220>
   <223> Description of Artificial Sequence: Synthetic peptide
<400> 879
   Arg Ala Ser Gln Asp Ile Met Ser Gly Ser Val Ala
   1               5                   10
<210> 880
   <211> 12
   <212> PRT
   <213> Artificial Sequence
<220>
   <223> Description of Artificial Sequence: Synthetic peptide
<400> 880
   Arg Ala Ser Gln Asp Ile Arg Ala Gly Ser Val Ala
   1               5                   10
<210> 881
   <211> 12
   <212> PRT
   <213> Artificial Sequence
<220>
   <223> Description of Artificial Sequence: Synthetic peptide
<400> 881
   Arg Ala Ser Gln Asp Ile Arg Gly Gly Ser Val Ala
   1 5 10
<210> 882
   <211> 12
   <212> PRT
   <213> Artificial Sequence
<220>
   <223> Description of Artificial Sequence: Synthetic peptide
<400> 882
   Arg Ala Ser Gln Asp Val Arg Gln Gly Ser Val Ala
   1 5 10
<210> 883
   <211> 12
   <212> PRT
   <213> Artificial Sequence
<220>
   <223> Description of Artificial Sequence: Synthetic peptide
<400> 883
   Arg Ala Ser Gln Asp Ile Ala Ala Gly Ser Val Ala
   1 5 10
<210> 884
   <211> 12
   <212> PRT
   <213> Artificial Sequence
<220>
   <223> Description of Artificial Sequence: Synthetic peptide
<400> 884
   Arg Ala Ser Gln Asp Ile Ala Gly Gly Ser Val Ala
   1 5 10
<210> 885
   <211> 12
   <212> PRT
   <213> Artificial Sequence
<220>
   <223> Description of Artificial Sequence: Synthetic peptide
<400> 885
   Arg Ala Ser Gln Asp Ile Ala His Gly Ser Val Ala
   1 5 10
<210> 886
   <211> 12
   <212> PRT
   <213> Artificial Sequence
<220>
   <223> Description of Artificial Sequence: Synthetic peptide
<400> 886
   Arg Ala Ser Gln Asp Ile Ala Lys Gly Ser Val Ala
   1               5                   10
<210> 887
   <211> 12
   <212> PRT
   <213> Artificial Sequence
<220>
   <223> Description of Artificial Sequence: Synthetic peptide
<400> 887
   Arg Ala Ser Gln Asp Ile Gly Ala Gly Ser Val Ala
   1               5                   10
<210> 888
   <211> 12
   <212> PRT
   <213> Artificial Sequence
<220>
   <223> Description of Artificial Sequence: Synthetic peptide
<400> 888
   Arg Ala Ser Gln Asp Ile Gly Gly Gly Ser Val Ala
   1               5                   10
<210> 889
   <211> 12
   <212> PRT
   <213> Artificial Sequence
<220>
   <223> Description of Artificial Sequence: Synthetic peptide
<400> 889
   Arg Ala Ser Gln Asp Ile Gly Leu Gly Ser Val Ala
   1               5                   10
<210> 890
   <211> 12
   <212> PRT
   <213> Artificial Sequence
<220>
   <223> Description of Artificial Sequence: Synthetic peptide
<400> 890
   Arg Ala Ser Gln Asp Ile Gly Met Gly Ser Val Ala
   1 5 10
<210> 891
   <211> 12
   <212> PRT
   <213> Artificial Sequence
<220>
   <223> Description of Artificial Sequence: Synthetic peptide
<400> 891
   Arg Ala Ser Gln Asp Ile Lys His Gly Ser Val Ala
   1 5 10
<210> 892
   <211> 12
   <212> PRT
   <213> Artificial Sequence
<220>
   <223> Description of Artificial Sequence: Synthetic peptide
<400> 892
   Arg Ala Ser Gln Asp Ile Leu Ala Gly Ser Val Ala
   1 5 10
<210> 893
   <211> 7
   <212> PRT
   <213> Artificial Sequence
<220>
   <223> Description of Artificial Sequence: Synthetic peptide
<400> 893
   Ala Ala Ser Ser Leu Tyr Ser
   1 5
<210> 894
   <211> 7
   <212> PRT
   <213> Artificial Sequence
<220>
   <223> Description of Artificial Sequence: Synthetic peptide
<400> 894
   Trp Ala Ser Phe Leu Tyr Ser
   1 5
<210> 895
   <211> 7
   <212> PRT
   <213> Artificial Sequence
<220>
   <223> Description of Artificial Sequence: Synthetic peptide
<400> 895
   Trp Gly Ser Phe Leu Tyr Ser
   1               5
<210> 896
   <211> 7
   <212> PRT
   <213> Artificial Sequence
<220>
   <223> Description of Artificial Sequence: Synthetic peptide
<400> 896
   Trp Ala Ser Tyr Leu Tyr Ser
   1               5
<210> 897
   <211> 7
   <212> PRT
   <213> Artificial Sequence
<220>
   <223> Description of Artificial Sequence: Synthetic peptide
<400> 897
   Trp Gly Ser Phe Leu Tyr Ser
   1               5
<210> 898
   <211> 12
   <212> PRT
   <213> Artificial Sequence
<220>
   <223> Description of Artificial Sequence: Synthetic peptide
<400> 898
   Arg Ala Ser Gln Asp Ile Leu Gly Gly Ser Val Ala
   1               5                   10
<210> 899
   <211> 12
   <212> PRT
   <213> Artificial Sequence
<220>
   <223> Description of Artificial Sequence: Synthetic peptide
<400> 899
   Arg Ala Ser Gln Asp Ile Leu Ile Gly Ser Val Ala
   1               5                   10
<210> 900
   <211> 12
   <212> PRT
   <213> Artificial Sequence
<220>
   <223> Description of Artificial Sequence: Synthetic peptide
<400> 900
   Arg Ala Ser Gln Asp Ile Leu Thr Gly Ser Val Ala
   1               5                   10
<210> 901
   <211> 12
   <212> PRT
   <213> Artificial Sequence
<220>
   <223> Description of Artificial Sequence: Synthetic peptide
<400> 901
   Arg Ala Ser Gln Asp Ile Met Leu Gly Ser Val Ala
   1               5                   10
<210> 902
   <211> 12
   <212> PRT
   <213> Artificial Sequence
<220>
   <223> Description of Artificial Sequence: Synthetic peptide
<400> 902
   Arg Ala Ser Gln Asp Ile Gln Ser Gly Ser Val Ala
   1               5                   10
<210> 903
   <211> 12
   <212> PRT
   <213> Artificial Sequence
<220>
   <223> Description of Artificial Sequence: Synthetic peptide
<400> 903
   Arg Ala Ser Gln Asp Ile Arg Ile Gly Ser Val Ala
   1               5                   10
<210> 904
   <211> 12
   <212> PRT
   <213> Artificial Sequence
<220>
   <223> Description of Artificial Sequence: Synthetic peptide
<400> 904
   Arg Ala Ser Gln Asp Ile Arg Met Gly Ser Val Ala
   1               5                   10
<210> 905
   <211> 12
   <212> PRT
   <213> Artificial Sequence
<220>
   <223> Description of Artificial Sequence: Synthetic peptide
<400> 905
   Arg Ala Ser Gln Asp Ile Arg Gln Gly Ser Val Ala
   1               5                   10
<210> 906
   <211> 12
   <212> PRT
   <213> Artificial Sequence
<220>
   <223> Description of Artificial Sequence: Synthetic peptide
<400> 906
   Arg Ala Ser Gln Asp Ile Arg Thr Gly Ser Val Ala
   1               5                   10
<210> 907
   <211> 12
   <212> PRT
   <213> Artificial Sequence
<220>
   <223> Description of Artificial Sequence: Synthetic peptide
<400> 907
   Arg Ala Ser Gln Asp Ile Arg Val Gly Ser Val Ala
   1               5                   10
<210> 908
   <211> 12
   <212> PRT
   <213> Artificial Sequence
<220>
   <223> Description of Artificial Sequence: Synthetic peptide
<400> 908
   Arg Ala Ser Gln Asp Ile Ser Met Gly Ser Val Ala
   1               5                   10
<210> 909
   <211> 12
   <212> PRT
   <213> Artificial Sequence
<220>
   <223> Description of Artificial Sequence: Synthetic peptide
<400> 909
   Arg Ala Ser Gln Asp Ile Ser Arg Gly Ser Val Ala
   1               5                   10
<210> 910
   <211> 12
   <212> PRT
   <213> Artificial Sequence
<220>
   <223> Description of Artificial Sequence: Synthetic peptide
<400> 910
   Arg Ala Ser Gln Asp Ile Ser Val Gly Ser Val Ala
   1               5                   10
<210> 911
   <211> 12
   <212> PRT
   <213> Artificial Sequence
<220>
   <223> Description of Artificial Sequence: Synthetic peptide
<400> 911
   Arg Ala Ser Gln Asp Ile Val Ser Gly Ser Val Ala
   1               5                   10
<210> 912
   <211> 11
   <212> PRT
   <213> Artificial Sequence
<220>
   <223> Description of Artificial Sequence: Synthetic peptide
<400> 912
   Arg Ala Ser Gln Asp Ile Trp His Trp Val Ala
   1               5                   10
<210> 913
   <211> 12
   <212> PRT
   <213> Artificial Sequence
<220>
   <223> Description of Artificial Sequence: Synthetic peptide
<400> 913
   Arg Ala Ser Gln Asn Ile Ala Gln Gly Ser Val Ala
   1               5                   10
<210> 914
   <211> 12
   <212> PRT
   <213> Artificial Sequence
<220>
   <223> Description of Artificial Sequence: Synthetic peptide
<400> 914
   Arg Ala Ser Gln Asp Ile Ala Phe Gly Ser Leu Ala
   1               5                   10
<210> 915
   <211> 12
   <212> PRT
   <213> Artificial Sequence
<220>
   <223> Description of Artificial Sequence: Synthetic peptide
<400> 915
   Arg Ala Ser Gln Asp Ile Ala Met Gly Ser Val Ala
   1               5                   10
<210> 916
   <211> 12
   <212> PRT
   <213> Artificial Sequence
<220>
   <223> Description of Artificial Sequence: Synthetic peptide
<400> 916
   Arg Ala Ser Gln Asp Ile Ala Arg Gly Ser Val Ala
   1               5                   10
<210> 917
   <211> 12
   <212> PRT
   <213> Artificial Sequence
<220>
   <223> Description of Artificial Sequence: Synthetic peptide
<400> 917
   Arg Ala Ser Gln Asp Ile Ala Ser Gly Ser Val Ala
   1               5                   10
<210> 918
   <211> 12
   <212> PRT
   <213> Artificial Sequence
<220>
   <223> Description of Artificial Sequence: Synthetic peptide
<400> 918
   Arg Ala Ser Gln Asp Ile Ala Ser Gly Ser Val Ala
   1               5                   10
<210> 919
   <211> 7
   <212> PRT
   <213> Artificial Sequence
<220>
   <223> Description of Artificial Sequence: Synthetic peptide
<400> 919
   Ala Gly Ser Ser Leu Tyr Ser
   1               5
<210> 920
   <211> 7
   <212> PRT
   <213> Artificial Sequence
<220>
   <223> Description of Artificial Sequence: Synthetic peptide
<400> 920
   Trp Ala Ser Phe Leu Tyr Ser
   1               5
<210> 921
   <211> 7
   <212> PRT
   <213> Artificial Sequence
<220>
   <223> Description of Artificial Sequence: Synthetic peptide
<400> 921
   Trp Gly Ser Tyr Leu Tyr Ser
   1               5
<210> 922
   <211> 7
   <212> PRT
   <213> Artificial Sequence
<220>
   <223> Description of Artificial Sequence: Synthetic peptide
<400> 922
   Trp Gly Ser Met Leu Tyr Ser
   1               5
<210> 923
   <211> 7
   <212> PRT
   <213> Artificial Sequence
<220>
   <223> Description of Artificial Sequence: Synthetic peptide
<400> 923
   Trp Gly Ser Leu Leu Tyr Ser
   1               5
<210> 924
   <211> 7
   <212> PRT
   <213> Artificial Sequence
<220>
   <223> Description of Artificial Sequence: Synthetic peptide
<400> 924
   Trp Gly Ser Ser Leu Tyr Ser
   1               5
<210> 925
   <211> 12
   <212> PRT
   <213> Artificial Sequence
<220>
   <223> Description of Artificial Sequence: Synthetic peptide
<400> 925
   Arg Ala Ser Gln Asp Ile Gly Ser Gly Ser Val Ala
   1               5                   10
<210> 926
   <211> 12
   <212> PRT
   <213> Artificial Sequence
<220>
   <223> Description of Artificial Sequence: Synthetic peptide
<400> 926
   Arg Ala Ser Gln Asp Ile Ile Gly Gly Ser Val Ala
   1               5                   10
<210> 927
   <211> 12
   <212> PRT
   <213> Artificial Sequence
<220>
   <223> Description of Artificial Sequence: Synthetic peptide
<400> 927
   Arg Ala Ser Gln Asp Ile Lys Ala Gly Ser Val Ala
   1               5                   10
<210> 928
   <211> 12
   <212> PRT
   <213> Artificial Sequence
<220>
   <223> Description of Artificial Sequence: Synthetic peptide
<400> 928
   Arg Ala Ser Gln Asp Ile Lys Phe Gly Ser Val Ala
   1               5                   10
<210> 929
   <211> 12
   <212> PRT
   <213> Artificial Sequence
<220>
   <223> Description of Artificial Sequence: Synthetic peptide
<400> 929
   Arg Ala Ser Gln Asp Ile Lys Leu Gly Ser Val Ala
   1               5                   10
<210> 930
   <211> 12
   <212> PRT
   <213> Artificial Sequence
<220>
   <223> Description of Artificial Sequence: Synthetic peptide
<400> 930
   Arg Ala Ser Gln Asp Ile Lys Leu Gly Ser Val Ala
   1 5 10
<210> 931
   <211> 12
   <212> PRT
   <213> Artificial Sequence
<220>
   <223> Description of Artificial Sequence: Synthetic peptide
<400> 931
   Arg Ala Ser Gln Asp Ile Lys Ser Gly Ser Val Ala
   1 5 10
<210> 932
   <211> 12
   <212> PRT
   <213> Artificial Sequence
<220>
   <223> Description of Artificial Sequence: Synthetic peptide
<400> 932
   Arg Ala Ser Gln Asp Ile Lys Val Gly Ser Val Ala
   1 5 10
<210> 933
   <211> 12
   <212> PRT
   <213> Artificial Sequence
<220>
   <223> Description of Artificial Sequence: Synthetic peptide
<400> 933
   Arg Ala Ser Gln Asp Ile Lys Trp Gly Ser Val Ala
   1 5 10
<210> 934
   <211> 12
   <212> PRT
   <213> Artificial Sequence
<220>
   <223> Description of Artificial Sequence: Synthetic peptide
<400> 934
   Arg Ala Ser Gln Asp Ile Leu Lys Gly Ser Val Ala
   1 5 10
<210> 935
   <211> 12
   <212> PRT
   <213> Artificial Sequence
<220>
   <223> Description of Artificial Sequence: Synthetic peptide
<400> 935
   Arg Ala Ser Gln Asp Ile Leu Ser Gly Ser Val Ala
   1               5                   10
<210> 936
   <211> 12
   <212> PRT
   <213> Artificial Sequence
<220>
   <223> Description of Artificial Sequence: Synthetic peptide
<400> 936
   Arg Ala Ser Gln Asp Ile Gln Arg Gly Ser Val Ala
   1               5                   10
<210> 937
   <211> 12
   <212> PRT
   <213> Artificial Sequence
<220>
   <223> Description of Artificial Sequence: Synthetic peptide
<400> 937
   Arg Ala Ser Gln Asp Ile Gln Ser Gly Ser Val Ala
   1               5                   10
<210> 938
   <211> 12
   <212> PRT
   <213> Artificial Sequence
<220>
   <223> Description of Artificial Sequence: Synthetic peptide
<400> 938
   Arg Ala Ser Gln Asp Ile Gln Thr Gly Ser Val Ala
   1               5                   10
<210> 939
   <211> 12
   <212> PRT
   <213> Artificial Sequence
<220>
   <223> Description of Artificial Sequence: Synthetic peptide
<400> 939
   Arg Ala Ser Gln Asp Ile Arg Glu Gly Ser Val Ala
   1               5                   10
<210> 940
   <211> 12
   <212> PRT
   <213> Artificial Sequence
<220>
   <223> Description of Artificial Sequence: Synthetic peptide
<400> 940
   Arg Ala Ser Gln Asp Ile Arg Phe Gly Ser Val Ala
   1               5                   10
<210> 941
   <211> 12
   <212> PRT
   <213> Artificial Sequence
<220>
   <223> Description of Artificial Sequence: Synthetic peptide
<400> 941
   Arg Ala Ser Gln Asp Ile Arg Gly Gly Ser Val Ala
   1               5                   10
<210> 942
   <211> 12
   <212> PRT
   <213> Artificial Sequence
<220>
   <223> Description of Artificial Sequence: Synthetic peptide
<400> 942
   Arg Ala Ser Gln Asp Ile Arg Leu Gly Ser Val Ala
   1               5                   10
<210> 943
   <211> 7
   <212> PRT
   <213> Artificial Sequence
<220>
   <223> Description of Artificial Sequence: Synthetic peptide
<400> 943
   Trp Gly Ser Tyr Leu Tyr Ser
   1               5
<210> 944
   <211> 7
   <212> PRT
   <213> Artificial Sequence
<220>
   <223> Description of Artificial Sequence: Synthetic peptide
<400> 944
   Trp Gly Ser Ser Leu Tyr Ser
   1               5
<210> 945
   <211> 7
   <212> PRT
   <213> Artificial Sequence
<220>
   <223> Description of Artificial Sequence: Synthetic peptide
<400> 945
   Trp Gly Ser Leu Leu Tyr Ser
   1               5
<210> 946
   <211> 7
   <212> PRT
   <213> Artificial Sequence
<220>
   <223> Description of Artificial Sequence: Synthetic peptide
<400> 946
   Trp Gly Ser Met Leu Tyr Ser
   1               5
<210> 947
   <211> 7
   <212> PRT
   <213> Artificial Sequence
<220>
   <223> Description of Artificial Sequence: Synthetic peptide
<400> 947
   Trp Gly Ser Thr Leu Tyr Ser
   1               5
<210> 948
   <211> 7
   <212> PRT
   <213> Artificial Sequence
<220>
   <223> Description of Artificial Sequence: Synthetic peptide
<400> 948
   Trp Gly Ser Trp Leu Tyr Ser
   1 5
<210> 949
   <211> 7
   <212> PRT
   <213> Artificial Sequence
<220>
   <223> Description of Artificial Sequence: Synthetic peptide
<400> 949
   Trp Gly Ser Cys Leu Tyr Ser
   1 5
<210> 950
   <211> 12
   <212> PRT
   <213> Artificial Sequence
<220>
   <223> Description of Artificial Sequence: Synthetic peptide
<400> 950
   Arg Ala Ser Gln Asp Ile Arg Met Gly Ser Val Ala
   1 5 10
<210> 951
   <211> 12
   <212> PRT
   <213> Artificial Sequence
<220>
   <223> Description of Artificial Sequence: Synthetic peptide
<400> 951
   Arg Ala Ser Gln Asp Ile Arg Arg Gly Ser Val Ala
   1 5 10
<210> 952
   <211> 12
   <212> PRT
   <213> Artificial Sequence
<220>
   <223> Description of Artificial Sequence: Synthetic peptide
<400> 952
   Arg Ala Ser Gln Asp Ile Arg Ser Gly Ser Val Ala
   1 5 10
<210> 953
   <211> 12
   <212> PRT
   <213> Artificial Sequence
<220>
   <223> Description of Artificial Sequence: Synthetic peptide
<400> 953
   Arg Ala Ser Gln Asp Ile Arg Val Gly Ser Val Ala
   1 5 10
<210> 954
   <211> 12
   <212> PRT
   <213> Artificial Sequence
<220>
   <223> Description of Artificial Sequence: Synthetic peptide
<400> 954
   Arg Ala Ser Gln Asp Ile Ser Ser Gly Ser Val Ala
   1 5 10
<210> 955
   <211> 12
   <212> PRT
   <213> Artificial Sequence
<220>
   <223> Description of Artificial Sequence: Synthetic peptide
<400> 955
   Arg Ala Ser Gln Asp Ile Thr Met Gly Ser Val Ala
   1 5 10
<210> 956
   <211> 12
   <212> PRT
   <213> Artificial Sequence
<220>
   <223> Description of Artificial Sequence: Synthetic peptide
<400> 956
   Arg Ala Ser Gln Asp Ile Tyr Met Gly Ser Val Ala
   1 5 10
<210> 957
   <211> 12
   <212> PRT
   <213> Artificial Sequence
<220>
   <223> Description of Artificial Sequence: Synthetic peptide
<400> 957
   Arg Ala Ser Gln Asp Ile Ala Thr Gly Ser Leu Ala
   1               5                   10
<210> 958
   <211> 12
   <212> PRT
   <213> Artificial Sequence
<220>
   <223> Description of Artificial Sequence: Synthetic peptide
<400> 958
   Arg Ala Ser Gln Asp Ile Lys Ser Gly Ser Leu Ala
   1               5                   10
<210> 959
   <211> 12
   <212> PRT
   <213> Artificial Sequence
<220>
   <223> Description of Artificial Sequence: Synthetic peptide
<400> 959
   Arg Ala Ser Gln Asp Ile Arg Gly Gly Ser Val Ala
   1               5                   10
<210> 960
   <211> 12
   <212> PRT
   <213> Artificial Sequence
<220>
   <223> Description of Artificial Sequence: Synthetic peptide
<400> 960
   Arg Ala Ser Gln Gly Ile Arg Thr Gly Ser Val Ala
   1               5                   10
<210> 961
   <211> 12
   <212> PRT
   <213> Artificial Sequence
<220>
   <223> Description of Artificial Sequence: Synthetic peptide
<400> 961
   Arg Ala Ser Gln Asn Ile Ala Met Gly Ser Val Ala
   1               5                   10
<210> 962
   <211> 12
   <212> PRT
   <213> Artificial Sequence
<220>
   <223> Description of Artificial Sequence: Synthetic peptide
<400> 962
   Arg Ala Ser Gln Asn Ile Arg Ser Gly Ser Val Ala
   1               5                   10
<210> 963
   <211> 7
   <212> PRT
   <213> Artificial Sequence
<220>
   <223> Description of Artificial Sequence: Synthetic peptide
<400> 963
   Trp Gly Ser Ser Leu Tyr Ser
   1               5
<210> 964
   <211> 7
   <212> PRT
   <213> Artificial Sequence
<220>
   <223> Description of Artificial Sequence: Synthetic peptide
<400> 964
   Trp Gly Ser Tyr Leu Tyr Ser
   1               5
<210> 965
   <211> 7
   <212> PRT
   <213> Artificial Sequence
<220>
   <223> Description of Artificial Sequence: Synthetic peptide
<400> 965
   Trp Gly Ser Ala Leu Tyr Ser
   1               5
<210> 966
   <211> 7
   <212> PRT
   <213> Artificial Sequence
<220>
   <223> Description of Artificial Sequence: Synthetic peptide
<400> 966
   Trp Gly Ser Thr Leu Tyr Ser
   1 5
<210> 967
   <211> 7
   <212> PRT
   <213> Artificial Sequence
<220>
   <223> Description of Artificial Sequence: Synthetic peptide
<400> 967
   Trp Gly Ser Asn Leu Tyr Ser
   1 5
<210> 968
   <211> 7
   <212> PRT
   <213> Artificial Sequence
<220>
   <223> Description of Artificial Sequence: Synthetic peptide
<400> 968
   Trp Gly Ser Glu Leu Tyr Ser
   1 5
<210> 969
   <211> 7
   <212> PRT
   <213> Artificial Sequence
<220>
   <223> Description of Artificial Sequence: Synthetic peptide
<400> 969
   Trp Gly Ser Leu Leu Tyr Ser
   1 5
<210> 970
   <211> 7
   <212> PRT
   <213> Artificial Sequence
<220>
   <223> Description of Artificial Sequence: Synthetic peptide
<400> 970
   Gly Gly Ser Tyr Leu Tyr Ser
   1 5
<210> 971
   <211> 7
   <212> PRT
   <213> Artificial Sequence
<220>
   <223> Description of Artificial Sequence: Synthetic peptide
<400> 971
   Trp Gly Ser Val Leu Tyr Ser
   1               5
<210> 972
   <211> 9
   <212> PRT
   <213> Artificial Sequence
<220>
   <223> Description of Artificial Sequence: Synthetic peptide
<400> 972
   Gln Gln Tyr Tyr Thr Thr Pro Pro Thr
   1               5
<210> 973
   <211> 12
   <212> PRT
   <213> Artificial Sequence
<220>
   <223> Description of Artificial Sequence: Synthetic peptide
<400> 973
   Arg Ala Ser Gln Asn Ile Arg Thr Gly Ser Val Ala
   1               5                   10
<210> 974
   <211> 12
   <212> PRT
   <213> Artificial Sequence
<220>
   <223> Description of Artificial Sequence: Synthetic peptide
<400> 974
   Arg Ala Ser Gln Asp Ile Arg Ala Gly Ser Val Ala
   1               5                   10
<210> 975
   <211> 12
   <212> PRT
   <213> Artificial Sequence
<220>
   <223> Description of Artificial Sequence: Synthetic peptide
<400> 975
   Arg Ala Ser Gln Asn Ile Tyr Ala Gly Ser Leu Ala
   1               5                   10
<210> 976
   <211> 12
   <212> PRT
   <213> Artificial Sequence
<220>
   <223> Description of Artificial Sequence: Synthetic peptide
<400> 976
   Arg Ala Ser Gln Asn Ile Tyr Ser Gly Ser Leu Ala
   1               5                   10
<210> 977
   <211> 15
   <212> PRT
   <213> Artificial Sequence
<220>
   <223> Description of Artificial Sequence: Synthetic peptide
<400> 977
   Arg Ala Ser Gln Asp Ile Pro Arg Ser Ile Ser Gly Tyr Val Ala
   1               5                   10                  15
<210> 978
   <211> 13
   <212> PRT
   <213> Artificial Sequence
<220>
   <223> Description of Artificial Sequence: Synthetic peptide
<400> 978
   Arg Ala Ser Gln Asn Ile Arg Asn Gly Gly Gly Leu Ala
   1               5                   10
<210> 979
   <211> 11
   <212> PRT
   <213> Artificial Sequence
<220>
   <223> Description of Artificial Sequence: Synthetic peptide
<400> 979
   Arg Ala Ser Gln Asn Val Ser Lys His Val Ala
   1               5                   10
<210> 980
   <211> 12
   <212> PRT
   <213> Artificial Sequence
<220>
   <223> Description of Artificial Sequence: Synthetic peptide
<400> 980
   Arg Ala Ser Gln Gln Val Ser Lys Tyr Asp Val Ala
   1 5 10
<210> 981
   <211> 15
   <212> PRT
   <213> Artificial Sequence
<220>
   <223> Description of Artificial Sequence: Synthetic peptide
<400> 981
   Arg Ala Ser Gln Asp Ile Pro Arg Ser Ile Ser Gly Tyr Val Ala
   1 5 10 15
<210> 982
   <211> 12
   <212> PRT
   <213> Artificial Sequence
<220>
   <223> Description of Artificial Sequence: Synthetic peptide
<400> 982
   Arg Ala Ser Gln Asp Ile Gly Leu Gly Ser Val Ala
   1 5 10
<210> 983
   <211> 12
   <212> PRT
   <213> Artificial Sequence
<220>
   <223> Description of Artificial Sequence: Synthetic peptide
<400> 983
   Arg Ala Ser Gln Asp Ile Arg Ser Gly Ser Val Ala
   1 5 10
<210> 984
   <211> 11
   <212> PRT
   <213> Artificial Sequence
<220>
   <223> Description of Artificial Sequence: Synthetic peptide
<400> 984
   Arg Ala Ser Gln Asn Val Ser Lys His Val Ala
   1 5 10
<210> 985
   <211> 13
   <212> PRT
   <213> Artificial Sequence
<220>
   <223> Description of Artificial Sequence: Synthetic peptide
<400> 985
   Arg Ala Ser Gln Asn Ile Arg Asn Gly Gly Gly Val Ala
   1 5 10
<210> 986
   <211> 7
   <212> PRT
   <213> Artificial Sequence
<220>
   <223> Description of Artificial Sequence: Synthetic peptide
<400> 986
   Ser Ala Ser Phe His Tyr Ser
   1 5
<210> 987
   <211> 7
   <212> PRT
   <213> Artificial Sequence
<220>
   <223> Description of Artificial Sequence: Synthetic peptide
<400> 987
   Trp Gly Ser Tyr Ser Tyr Ser
   1 5
<210> 988
   <211> 7
   <212> PRT
   <213> Artificial Sequence
<220>
   <223> Description of Artificial Sequence: Synthetic peptide
<400> 988
   Trp Gly Ser Ser Ser Tyr Ser
   1 5
<210> 989
   <211> 7
   <212> PRT
   <213> Artificial Sequence
<220>
   <223> Description of Artificial Sequence: Synthetic peptide
<400> 989
   Trp Ala Ser Tyr Leu Tyr Ser
   1               5
<210> 990
   <211> 9
   <212> PRT
   <213> Artificial Sequence
<220>
   <223> Description of Artificial Sequence: Synthetic peptide
<400> 990
   Gln Gln His Phe Asn Ala Pro Pro Thr
   1               5
<210> 991
   <211> 9
   <212> PRT
   <213> Artificial Sequence
<220>
   <223> Description of Artificial Sequence: Synthetic peptide
<400> 991
   Gln Gln Ser Tyr Ser Gly Pro Pro Thr
   1               5
<210> 992
   <211> 10
   <212> PRT
   <213> Artificial Sequence
<220>
   <223> Description of Artificial Sequence: Synthetic peptide
<400> 992
   Gln Gln Ser Gly Phe Arg Ser Pro Pro Thr
   1               5                   10
<210> 993
   <211> 9
   <212> PRT
   <213> Artificial Sequence
<220>
   <223> Description of Artificial Sequence: Synthetic peptide
<400> 993
   Gln Gln Ser Tyr Ser Thr Pro Pro Thr
   1 5
<210> 994
   <211> 10
   <212> PRT
   <213> Artificial Sequence
<220>
   <223> Description of Artificial Sequence: Synthetic peptide
<400> 994
   Gly Phe Asn Ile Ala Asp Thr Tyr Ile His
   1 5 10
<210> 995
   <211> 10
   <212> PRT
   <213> Artificial Sequence
<220>
   <223> Description of Artificial Sequence: Synthetic peptide
<400> 995
   Gly Phe Asn Ile Lys Ala Thr Tyr Ile His
   1 5 10
<210> 996
   <211> 10
   <212> PRT
   <213> Artificial Sequence
<220>
   <223> Description of Artificial Sequence: Synthetic peptide
<400> 996
   Gly Phe Asn Ile Lys Asp Ala Tyr Ile His
   1 5 10
<210> 997
   <211> 10
   <212> PRT
   <213> Artificial Sequence
<220>
   <223> Description of Artificial Sequence: Synthetic peptide
<400> 997
   Gly Phe Asn Ile Lys Asp Thr Ala Ile His
   1 5 10
<210> 998
   <211> 17
   <212> PRT
   <213> Artificial Sequence
<220>
   <223> Description of Artificial Sequence: Synthetic peptide
<400> 998
   Ala Ile Tyr Pro Thr Asn Gly Tyr Thr Arg Tyr Ala Asp Ser Val Lys
   1 5 10 15
   Gly
<210> 999
   <211> 17
   <212> PRT
   <213> Artificial Sequence
<220>
   <223> Description of Artificial Sequence: Synthetic peptide
<400> 999
   Arg Ile Ala Pro Thr Asn Gly Tyr Thr Arg Tyr Ala Asp Ser Val Lys
   1 5 10 15
   Gly
<210> 1000
   <211> 17
   <212> PRT
   <213> Artificial Sequence
<220>
   <223> Description of Artificial Sequence: Synthetic peptide
<400> 1000
   Arg Ile Tyr Pro Ala Asn Gly Tyr Thr Arg Tyr Ala Asp Ser Val Lys
   1 5 10 15
   Gly
<210> 1001
   <211> 17
   <212> PRT
   <213> Artificial Sequence
<220>
   <223> Description of Artificial Sequence: Synthetic peptide
<400> 1001
   Arg Ile Tyr Pro Thr Ala Gly Tyr Thr Arg Tyr Ala Asp Ser Val Lys
   1               5                   10                  15
   Gly
<210> 1002
   <211> 17
   <212> PRT
   <213> Artificial Sequence
<220>
   <223> Description of Artificial Sequence: Synthetic peptide
<400> 1002
   Arg Ile Tyr Pro Thr Asn Gly Ala Thr Arg Tyr Ala Asp Ser Val Lys
   1               5                   10                  15
   Gly
<210> 1003
   <211> 11
   <212> PRT
   <213> Artificial Sequence
<220>
   <223> Description of Artificial Sequence: Synthetic peptide
<400> 1003
   Trp Gly Gly Asp Gly Phe Tyr Ala Met Asp Val
   1               5                   10
<210> 1004
   <211> 11
   <212> PRT
   <213> Artificial Sequence
<220>
   <223> Description of Artificial Sequence: Synthetic peptide
<400> 1004
   Ala Gly Gly Asp Gly Phe Tyr Ala Met Asp Tyr
   1               5                   10
<210> 1005
   <211> 11
   <212> PRT
   <213> Artificial Sequence
<220>
   <223> Description of Artificial Sequence: Synthetic peptide
<400> 1005
   Trp Gly Gly Ala Gly Phe Tyr Ala Met Asp Tyr
   1 5 10
<210> 1006
   <211> 11
   <212> PRT
   <213> Artificial Sequence
<220>
   <223> Description of Artificial Sequence: Synthetic peptide
<400> 1006
   Trp Gly Gly Asp Ala Phe Tyr Ala Met Asp Tyr
   1 5 10
<210> 1007
   <211> 11
   <212> PRT
   <213> Artificial Sequence
<220>
   <223> Description of Artificial Sequence: Synthetic peptide
<400> 1007
   Trp Gly Gly Asp Gly Ala Tyr Ala Met Asp Tyr
   1 5 10
<210> 1008
   <211> 11
   <212> PRT
   <213> Artificial Sequence
<220>
   <223> Description of Artificial Sequence: Synthetic peptide
<400> 1008
   Trp Gly Gly Asp Gly Phe Ala Ala Met Asp Tyr
   1 5 10
<210> 1009
   <211> 11
   <212> PRT
   <213> Artificial Sequence
<220>
   <223> Description of Artificial Sequence: Synthetic peptide
<400> 1009
   Trp Gly Gly Asp Gly Phe Phe Ala Met Asp Tyr
   1 5 10
<210> 1010
   <211> 10
   <212> PRT
   <213> Artificial Sequence
<220>
   <223> Description of Artificial Sequence: Synthetic peptide
<400> 1010
   Gly Phe Asn Ile Lys Asp Thr Tyr Met His
   1               5                   10
<210> 1011
   <211> 10
   <212> PRT
   <213> Artificial Sequence
<220>
   <223> Description of Artificial Sequence: Synthetic peptide
<400> 1011
   Gly Phe Thr Phe Ser Asp Tyr Trp Met Asn
   1               5                   10
<210> 1012
   <211> 10
   <212> PRT
   <213> Artificial Sequence
<220>
   <223> Description of Artificial Sequence: Synthetic peptide
<400> 1012
   Gly Phe Thr Phe Ser Asp Tyr Trp Met Asn
   1               5                   10
<210> 1013
   <211> 10
   <212> PRT
   <213> Artificial Sequence
<220>
   <223> Description of Artificial Sequence: Synthetic peptide
<400> 1013
   Gly Phe Asn Ile Gly Lys Ser Ser Ile His
   1               5                   10
<210> 1014
   <211> 10
   <212> PRT
   <213> Artificial Sequence
<220>
   <223> Description of Artificial Sequence: Synthetic peptide
<400> 1014
   Gly Phe Asn Ile Asp Asn Thr Ala Ile His
   1               5                   10
<210> 1015
   <211> 10
   <212> PRT
   <213> Artificial Sequence
<220>
   <223> Description of Artificial Sequence: Synthetic peptide
<400> 1015
   Gly Phe Asn Ile Ala Asp Ser Ala Ile His
   1               5                   10
<210> 1016
   <211> 10
   <212> PRT
   <213> Artificial Sequence
<220>
   <223> Description of Artificial Sequence: Synthetic peptide
<400> 1016
   Gly Phe Thr Ile Gly Asn Ser Trp Ile His
   1               5                   10
<210> 1017
   <211> 10
   <212> PRT
   <213> Artificial Sequence
<220>
   <223> Description of Artificial Sequence: Synthetic peptide
<400> 1017
   Gly Phe Asn Ile Ala Asp Ser Ala Ile His
   1               5                   10
<210> 1018
   <211> 10
   <212> PRT
   <213> Artificial Sequence
<220>
   <223> Description of Artificial Sequence: Synthetic peptide
<400> 1018
   Gly Phe Thr Ile Thr Glu Ser Gly Ile His
   1               5                   10
<210> 1019
   <211> 10
   <212> PRT
   <213> Artificial Sequence
<220>
   <223> Description of Artificial Sequence: Synthetic peptide
<400> 1019
   Gly Phe Asn Ile Thr Ala Tyr Gly Ile His
   1 5 10
<210> 1020
   <211> 10
   <212> PRT
   <213> Artificial Sequence
<220>
   <223> Description of Artificial Sequence: Synthetic peptide
<400> 1020
   Gly Phe Thr Ile Gly Gly Ser Trp Ile His
   1 5 10
<210> 1021
   <211> 10
   <212> PRT
   <213> Artificial Sequence
<220>
   <223> Description of Artificial Sequence: Synthetic peptide
<400> 1021
   Gly Phe Thr Ile Gly Gly Ser Trp Ile His
   1 5 10
<210> 1022
   <211> 10
   <212> PRT
   <213> Artificial Sequence
<220>
   <223> Description of Artificial Sequence: Synthetic peptide
<400> 1022
   Gly Phe Ser Ile Ala Gly Tyr Asp Ile His
   1 5 10
<210> 1023
   <211> 10
   <212> PRT
   <213> Artificial Sequence
<220>
   <223> Description of Artificial Sequence: Synthetic peptide
<400> 1023
   Gly Phe Ser Leu Thr Asn Tyr Gly Val His
   1 5 10
<210> 1024
   <211> 10
   <212> PRT
   <213> Artificial Sequence
<220>
   <223> Description of Artificial Sequence: Synthetic peptide
<400> 1024
   Gly Tyr Thr Phe Thr Ser Tyr Asn Met His
   1 5 10
<210> 1025
   <211> 10
   <212> PRT
   <213> Artificial Sequence
<220>
   <223> Description of Artificial Sequence: Synthetic peptide
<400> 1025
   Gly Phe Thr Phe Thr Asp Phe Tyr Met Asn
   1 5 10
<210> 1026
   <211> 10
   <212> PRT
   <213> Artificial Sequence
<220>
   <223> Description of Artificial Sequence: Synthetic peptide
<400> 1026
   Gly Tyr Thr Phe Thr Asn Tyr Gly Met Asn
   1 5 10
<210> 1027
   <211> 10
   <212> PRT
   <213> Artificial Sequence
<220>
   <223> Description of Artificial Sequence: Synthetic peptide
<400> 1027
   Gly Phe Thr Phe Thr Asp Tyr Thr Met Asp
   1 5 10
<210> 1028
   <211> 12
   <212> PRT
   <213> Artificial Sequence
<220>
   <223> Description of Artificial Sequence: Synthetic peptide
<400> 1028
   Gly Gly Ser Val Ser Ser Gly Asp Tyr Tyr Trp Thr
   1               5                   10
<210> 1029
   <211> 17
   <212> PRT
   <213> Artificial Sequence
<220>
   <223> Description of Artificial Sequence: Synthetic peptide
<400> 1029
   Arg Ile Asp Pro Ala Asn Gly Asn Ser Lys Tyr Val Pro Lys Phe Gln
   1               5                   10                  15
   Gly
<210> 1030
   <211> 17
   <212> PRT
   <213> Artificial Sequence
<220>
   <223> Description of Artificial Sequence: Synthetic peptide
<400> 1030
   Arg Ile Asp Pro Ala Asn Gly Asn Ser Lys Tyr Ala Asp Ser Val Lys
   1               5                   10                  15
   Gly
<210> 1031
   <211> 19
   <212> PRT
   <213> Artificial Sequence
<220>
   <223> Description of Artificial Sequence: Synthetic peptide
<400> 1031
   Gln Ile Arg Asn Lys Pro Tyr Asn Tyr Glu Thr Tyr Tyr Ser Asp Ser
   1               5                   10                  15
   Val Lys Gly
<210> 1032
   <211> 19
   <212> PRT
   <213> Artificial Sequence
<220>
   <223> Description of Artificial Sequence: Synthetic peptide
<400> 1032
   Gln Ile Arg Asn Lys Pro Tyr Asn Tyr Glu Thr Tyr Tyr Ala Asp Ser
   1               5                   10                  15
   Val Lys Gly
<210> 1033
   <211> 17
   <212> PRT
   <213> Artificial Sequence
<220>
   <223> Description of Artificial Sequence: Synthetic peptide
<400> 1033
   Trp Ile Ser Pro Tyr Glu Gly Ala Thr Ala Tyr Ala Asp Ser Val Lys
   1               5                   10                  15
   Gly
<210> 1034
   <211> 17
   <212> PRT
   <213> Artificial Sequence
<220>
   <223> Description of Artificial Sequence: Synthetic peptide
<400> 1034
   Val Ile Ser Pro His Asp Gly Thr Thr Tyr Tyr Ala Asp Ser Val Lys
   1               5                   10                  15
   Gly
<210> 1035
   <211> 17
   <212> PRT
   <213> Artificial Sequence
<220>
   <223> Description of Artificial Sequence: Synthetic peptide
<400> 1035
   Leu Ile Asp Pro Ser Ser Gly Tyr Thr Asp Tyr Ala Asp Ser Val Lys
   1 5 10 15
   Gly
<210> 1036
   <211> 17
   <212> PRT
   <213> Artificial Sequence
<220>
   <223> Description of Artificial Sequence: Synthetic peptide
<400> 1036
   Val Ile Ala Pro Thr Tyr Gly Tyr Thr Asn Tyr Ala Asp Ser Val Lys
   1 5 10 15
   Gly
<210> 1037
   <211> 17
   <212> PRT
   <213> Artificial Sequence
<220>
   <223> Description of Artificial Sequence: Synthetic peptide
<400> 1037
   Tyr Ile Ala Pro Tyr Asp Gly Tyr Thr Tyr Tyr Ala Asp Ser Val Lys
   1 5 10 15
   Gly
<210> 1038
   <211> 17
   <212> PRT
   <213> Artificial Sequence
<220>
   <223> Description of Artificial Sequence: Synthetic peptide
<400> 1038
   Val Ile Tyr Pro His Asp Gly Lys Thr Tyr Tyr Ala Asp Ser Val Lys
   1 5 10 15
   Gly
<210> 1039
   <211> 17
   <212> PRT
   <213> Artificial Sequence
<220>
   <223> Description of Artificial Sequence: Synthetic peptide
<400> 1039
   Val Ile Thr Pro Tyr Asp Gly Asp Thr Thr Tyr Ala Asp Ser Val Lys
   1               5                   10                  15
   Gly
<210> 1040
   <211> 17
   <212> PRT
   <213> Artificial Sequence
<220>
   <223> Description of Artificial Sequence: Synthetic peptide
<400> 1040
   Val Ile Ser Pro Asp Tyr Gly Tyr Thr Asp Tyr Ala Asp Ser Val Lys
   1               5                   10                  15
   Gly
<210> 1041
   <211> 17
   <212> PRT
   <213> Artificial Sequence
<220>
   <223> Description of Artificial Sequence: Synthetic peptide
<400> 1041
   Val Ile Tyr Pro Thr Tyr Gly Tyr Thr Asp Tyr Ala Asp Ser Val Lys
   1               5                   10                  15
   Gly
<210> 1042
   <211> 17
   <212> PRT
   <213> Artificial Sequence
<220>
   <223> Description of Artificial Sequence: Synthetic peptide
<400> 1042
   Leu Ile Ala Pro Tyr Ala Gly Tyr Thr Asn Tyr Ala Asp Ser Val Lys
   1 5 10 15
   Gly
<210> 1043
   <211> 16
   <212> PRT
   <213> Artificial Sequence
<220>
   <223> Description of Artificial Sequence: Synthetic peptide
<400> 1043
   Val Ile Trp Ser Gly Gly Asn Thr Asp Tyr Asn Thr Pro Phe Thr Ser
   1 5 10 15
<210> 1044
   <211> 17
   <212> PRT
   <213> Artificial Sequence
<220>
   <223> Description of Artificial Sequence: Synthetic peptide
<400> 1044
   Ala Ile Tyr Pro Gly Asn Gly Asp Thr Ser Tyr Asn Gln Lys Phe Lys
   1 5 10 15
   Gly
<210> 1045
   <211> 19
   <212> PRT
   <213> Artificial Sequence
<220>
   <223> Description of Artificial Sequence: Synthetic peptide
<400> 1045
   Phe Ile Arg Asp Lys Ala Lys Gly Tyr Thr Thr Glu Tyr Asn Pro Ser
   1 5 10 15
   Val Lys Gly
<210> 1046
   <211> 17
   <212> PRT
   <213> Artificial Sequence
<220>
   <223> Description of Artificial Sequence: Synthetic peptide
<400> 1046
   Trp Ile Asn Thr Tyr Thr Gly Glu Pro Thr Tyr Ala Ala Asp Phe Lys
   1               5                   10                  15
   Arg
<210> 1047
   <211> 17
   <212> PRT
   <213> Artificial Sequence
<220>
   <223> Description of Artificial Sequence: Synthetic peptide
<400> 1047
   Asp Val Asn Pro Asn Ser Gly Gly Ser Ile Tyr Asn Gln Arg Phe Lys
   1               5                   10                  15
   Gly
<210> 1048
   <211> 16
   <212> PRT
   <213> Artificial Sequence
<220>
   <223> Description of Artificial Sequence: Synthetic peptide
<400> 1048
   His Ile Tyr Tyr Ser Gly Asn Thr Asn Tyr Asn Pro Ser Leu Lys Ser
   1               5                   10                  15
<210> 1049
   <211> 12
   <212> PRT
   <213> Artificial Sequence
<220>
   <223> Description of Artificial Sequence: Synthetic peptide
<400> 1049
   Phe Gly Tyr Tyr Val Ser Asp Tyr Ala Met Ala Tyr
   1               5                   10
<210> 1050
   <211> 7
   <212> PRT
   <213> Artificial Sequence
<220>
   <223> Description of Artificial Sequence: Synthetic peptide
<400> 1050
   Ser Tyr Tyr Gly Met Asp Tyr
   1 5
<210> 1051
   <211> 11
   <212> PRT
   <213> Artificial Sequence
<220>
   <223> Description of Artificial Sequence: Synthetic peptide
<400> 1051
   Thr Ser Trp Ser Lys Pro Tyr Ala Met Asp Tyr
   1 5 10
<210> 1052
   <211> 11
   <212> PRT
   <213> Artificial Sequence
<220>
   <223> Description of Artificial Sequence: Synthetic peptide
<400> 1052
   Trp Gly Trp Glu Thr Asp Gly Ala Met Asp Tyr
   1 5 10
<210> 1053
   <211> 11
   <212> PRT
   <213> Artificial Sequence
<220>
   <223> Description of Artificial Sequence: Synthetic peptide
<400> 1053
   Ser Arg Ala Gly Tyr Thr Tyr Ala Met Asp Tyr
   1 5 10
<210> 1054
   <211> 11
   <212> PRT
   <213> Artificial Sequence
<220>
   <223> Description of Artificial Sequence: Synthetic peptide
<400> 1054
   Trp Gly Ala Lys Gly Thr Trp Ala Met Asp Tyr
   1 5 10
<210> 1055
   <211> 11
   <212> PRT
   <213> Artificial Sequence
<220>
   <223> Description of Artificial Sequence: Synthetic peptide
<400> 1055
   Trp Gly Trp Thr Thr Asn Gly Ala Met Asp Tyr
   1               5                   10
<210> 1056
   <211> 11
   <212> PRT
   <213> Artificial Sequence
<220>
   <223> Description of Artificial Sequence: Synthetic peptide
<400> 1056
   Trp Trp Tyr Ser Trp Asn Trp Ala Met Asp Tyr
   1               5                   10
<210> 1057
   <211> 11
   <212> PRT
   <213> Artificial Sequence
<220>
   <223> Description of Artificial Sequence: Synthetic peptide
<400> 1057
   Trp Gly Trp Glu Ala Asn Trp Ala Met Asp Tyr
   1               5                   10
<210> 1058
   <211> 11
   <212> PRT
   <213> Artificial Sequence
<220>
   <223> Description of Artificial Sequence: Synthetic peptide
<400> 1058
   Trp Gly Ser Gly Tyr Thr Trp Ala Met Asp Tyr
   1               5                   10
<210> 1059
   <211> 11
   <212> PRT
   <213> Artificial Sequence
<220>
   <223> Description of Artificial Sequence: Synthetic peptide
<400> 1059
   Trp Gly Ala Gly Gly Thr Trp Ala Met Asp Tyr
   1 5 10
<210> 1060
   <211> 11
   <212> PRT
   <213> Artificial Sequence
<220>
   <223> Description of Artificial Sequence: Synthetic peptide
<400> 1060
   Ala Ala Ala Trp Ala Ser Tyr Ala Met Asp Tyr
   1 5 10
<210> 1061
   <211> 11
   <212> PRT
   <213> Artificial Sequence
<220>
   <223> Description of Artificial Sequence: Synthetic peptide
<400> 1061
   Ala Leu Thr Tyr Tyr Asp Tyr Glu Phe Ala Tyr
   1 5 10
<210> 1062
   <211> 12
   <212> PRT
   <213> Artificial Sequence
<220>
   <223> Description of Artificial Sequence: Synthetic peptide
<400> 1062
   Ser Thr Tyr Tyr Gly Gly Asp Trp Tyr Phe Asn Val
   1 5 10
<210> 1063
   <211> 10
   <212> PRT
   <213> Artificial Sequence
<220>
   <223> Description of Artificial Sequence: Synthetic peptide
<400> 1063
   Glu Gly His Thr Ala Ala Pro Phe Asp Tyr
   1 5 10
<210> 1064
   <211> 14
   <212> PRT
   <213> Artificial Sequence
<220>
   <223> Description of Artificial Sequence: Synthetic peptide
<400> 1064
   Tyr Pro His Tyr Tyr Gly Ser Ser His Trp Tyr Phe Asp Val
   1               5                   10
<210> 1065
   <211> 10
   <212> PRT
   <213> Artificial Sequence
<220>
   <223> Description of Artificial Sequence: Synthetic peptide
<400> 1065
   Asn Leu Gly Pro Ser Phe Tyr Phe Asp Tyr
   1               5                   10
<210> 1066
   <211> 9
   <212> PRT
   <213> Artificial Sequence
<220>
   <223> Description of Artificial Sequence: Synthetic peptide
<400> 1066
   Asp Arg Val Thr Gly Ala Phe Asp Ile
   1               5
<210> 1067
   <211> 11
   <212> PRT
   <213> Artificial Sequence
<220>
   <223> Description of Artificial Sequence: Synthetic peptide
<400> 1067
   Arg Ala Ser Gln Asn Val Asn Thr Ala Val Ala
   1               5                   10
<210> 1068
   <211> 11
   <212> PRT
   <213> Artificial Sequence
<220>
   <223> Description of Artificial Sequence: Synthetic peptide
<400> 1068
   Arg Ala Ser Gln Asp Val Ala Thr Ala Val Ala
   1               5                   10
<210> 1069
   <211> 11
   <212> PRT
   <213> Artificial Sequence
<220>
   <223> Description of Artificial Sequence: Synthetic peptide
<400> 1069
   Arg Ala Ser Gln Asp Val Asn Ala Ala Val Ala
   1               5                   10
<210> 1070
   <211> 11
   <212> PRT
   <213> Artificial Sequence
<220>
   <223> Description of Artificial Sequence: Synthetic peptide
<400> 1070
   Arg Ala Ser Gln Asn Val Trp Asp Trp Val Ala
   1               5                   10
<210> 1071
   <211> 15
   <212> PRT
   <213> Artificial Sequence
<220>
   <223> Description of Artificial Sequence: Synthetic peptide
<400> 1071
   Arg Ala Ser Gln Asp Ile Pro Arg Ser Ile Ser Gly Tyr Val Ala
   1               5                   10                  15
<210> 1072
   <211> 12
   <212> PRT
   <213> Artificial Sequence
<220>
   <223> Description of Artificial Sequence: Synthetic peptide
<400> 1072
   Arg Ala Ser Gln Asp Ile Gly Leu Gly Ser Val Ala
   1               5                   10
<210> 1073
   <211> 12
   <212> PRT
   <213> Artificial Sequence
<220>
   <223> Description of Artificial Sequence: Synthetic peptide
<400> 1073
   Arg Ala Ser Gln Asp Ile Arg Ser Gly Ser Val Ala
   1 5 10
<210> 1074
   <211> 13
   <212> PRT
   <213> Artificial Sequence
<220>
   <223> Description of Artificial Sequence: Synthetic peptide
<400> 1074
   Arg Ala Ser Gln Asp Ile Trp Asn Arg Arg Ala Leu Ala
   1 5 10
<210> 1075
   <211> 11
   <212> PRT
   <213> Artificial Sequence
<220>
   <223> Description of Artificial Sequence: Synthetic peptide
<400> 1075
   Arg Ala Ser Gln Asn Val Gly Arg Pro Val Ala
   1 5 10
<210> 1076
   <211> 11
   <212> PRT
   <213> Artificial Sequence
<220>
   <223> Description of Artificial Sequence: Synthetic peptide
<400> 1076
   Arg Ala Ser Gln Asn Val Ser Lys His Val Ala
   1 5 10
<210> 1077
   <211> 13
   <212> PRT
   <213> Artificial Sequence
<220>
   <223> Description of Artificial Sequence: Synthetic peptide
<400> 1077
   Arg Ala Ser Gln Asn Ile Arg Asn Gly Gly Gly Leu Ala
   1               5                   10
<210> 1078
   <211> 15
   <212> PRT
   <213> Artificial Sequence
<220>
   <223> Description of Artificial Sequence: Synthetic peptide
<400> 1078
   Arg Ala Ser Gln Ser Val Asp Ile Phe Gly Val Gly Phe Leu His
   1               5                   10                  15
<210> 1079
   <211> 15
   <212> PRT
   <213> Artificial Sequence
<220>
   <223> Description of Artificial Sequence: Synthetic peptide
<400> 1079
   Arg Ala Ser Gln Ser Val Asp Ile Phe Gly Val Gly Phe Leu His
   1               5                   10                  15
<210> 1080
   <211> 16
   <212> PRT
   <213> Artificial Sequence
<220>
   <223> Description of Artificial Sequence: Synthetic peptide
<400> 1080
   Arg Ser Ser Gln Ser Leu Val His Ser Gln Gly Asn Thr Tyr Leu Arg
   1               5                   10                  15
<210> 1081
   <211> 16
   <212> PRT
   <213> Artificial Sequence
<220>
   <223> Description of Artificial Sequence: Synthetic peptide
<400> 1081
   Arg Ala Ser Gln Ser Leu Val His Ser Gln Gly Asn Thr Tyr Leu Arg
   1 5 10 15
<210> 1082
   <211> 11
   <212> PRT
   <213> Artificial Sequence
<220>
   <223> Description of Artificial Sequence: Synthetic peptide
<400> 1082
   Arg Ala Ser Gln Ser Ile Gly Thr Asn Ile His
   1 5 10
<210> 1083
   <211> 10
   <212> PRT
   <213> Artificial Sequence
<220>
   <223> Description of Artificial Sequence: Synthetic peptide
<400> 1083
   Arg Ala Ser Ser Ser Val Ser Tyr Ile His
   1 5 10
<210> 1084
   <211> 11
   <212> PRT
   <213> Artificial Sequence
<220>
   <223> Description of Artificial Sequence: Synthetic peptide
<400> 1084
   Lys Ala Ser Gln Asn Ile Asp Lys Tyr Leu Asn
   1 5 10
<210> 1085
   <211> 11
   <212> PRT
   <213> Artificial Sequence
<220>
   <223> Description of Artificial Sequence: Synthetic peptide
<400> 1085
   Ser Ala Ser Gln Asp Ile Ser Asn Tyr Leu Asn
   1 5 10
<210> 1086
   <211> 11
   <212> PRT
   <213> Artificial Sequence
<220>
   <223> Description of Artificial Sequence: Synthetic peptide
<400> 1086
   Lys Ala Ser Gln Asp Val Ser Ile Gly Val Ala
   1               5                   10
<210> 1087
   <211> 11
   <212> PRT
   <213> Artificial Sequence
<220>
   <223> Description of Artificial Sequence: Synthetic peptide
<400> 1087
   Gln Ala Ser Gln Asp Ile Ser Asn Tyr Leu Asn
   1               5                   10
<210> 1088
   <211> 7
   <212> PRT
   <213> Artificial Sequence
<220>
   <223> Description of Artificial Sequence: Synthetic peptide
<400> 1088
   Ser Ala Ser Phe Leu Glu Ser
   1               5
<210> 1089
   <211> 7
   <212> PRT
   <213> Artificial Sequence
<220>
   <223> Description of Artificial Sequence: Synthetic peptide
<400> 1089
   Ala Ala Ser Phe Leu Tyr Ser
   1               5
<210> 1090
   <211> 7
   <212> PRT
   <213> Artificial Sequence
<220>
   <223> Description of Artificial Sequence: Synthetic peptide
<400> 1090
   Ser Ala Ala Phe Leu Tyr Ser
   1 5
<210> 1091
   <211> 7
   <212> PRT
   <213> Artificial Sequence
<220>
   <223> Description of Artificial Sequence: Synthetic peptide
<400> 1091
   Ser Ala Ser Asn Leu Tyr Ser
   1 5
<210> 1092
   <211> 7
   <212> PRT
   <213> Artificial Sequence
<220>
   <223> Description of Artificial Sequence: Synthetic peptide
<400> 1092
   Pro Ala Ser Ser Leu Tyr Ser
   1 5
<210> 1093
   <211> 7
   <212> PRT
   <213> Artificial Sequence
<220>
   <223> Description of Artificial Sequence: Synthetic peptide
<400> 1093
   Trp Gly Ser Tyr Leu Tyr Ser
   1 5
<210> 1094
   <211> 7
   <212> PRT
   <213> Artificial Sequence
<220>
   <223> Description of Artificial Sequence: Synthetic peptide
<400> 1094
   Trp Ala Ser Tyr Leu Tyr Ser
   1 5
<210> 1095
   <211> 7
   <212> PRT
   <213> Artificial Sequence
<220>
   <223> Description of Artificial Sequence: Synthetic peptide
<400> 1095
   Trp Gly Ser Tyr Leu Tyr Ser
   1 5
<210> 1096
   <211> 7
   <212> PRT
   <213> Artificial Sequence
<220>
   <223> Description of Artificial Sequence: Synthetic peptide
<400> 1096
   Glu Gly Ser Phe Leu Tyr Ser
   1 5
<210> 1097
   <211> 7
   <212> PRT
   <213> Artificial Sequence
<220>
   <223> Description of Artificial Sequence: Synthetic peptide
<400> 1097
   Gly Gly Ser Phe Leu Tyr Ser
   1 5
<210> 1098
   <211> 7
   <212> PRT
   <213> Artificial Sequence
<220>
   <223> Description of Artificial Sequence: Synthetic peptide
<400> 1098
   Ser Gly Ser Tyr Leu Tyr Ser
   1 5
<210> 1099
   <211> 7
   <212> PRT
   <213> Artificial Sequence
<220>
   <223> Description of Artificial Sequence: Synthetic peptide
<400> 1099
   Ser Ala Ser Phe Leu Tyr Ser
   1               5
<210> 1100
   <211> 7
   <212> PRT
   <213> Artificial Sequence
<220>
   <223> Description of Artificial Sequence: Synthetic peptide
<400> 1100
   Arg Ala Ser Asn Leu Glu Ser
   1               5
<210> 1101
   <211> 7
   <212> PRT
   <213> Artificial Sequence
<220>
   <223> Description of Artificial Sequence: Synthetic peptide
<400> 1101
   Arg Ala Ser Asn Leu Glu Ser
   1               5
<210> 1102
   <211> 7
   <212> PRT
   <213> Artificial Sequence
<220>
   <223> Description of Artificial Sequence: Synthetic peptide
<400> 1102
   Lys Val Ser Asn Arg Phe Ser
   1               5
<210> 1103
   <211> 7
   <212> PRT
   <213> Artificial Sequence
<220>
   <223> Description of Artificial Sequence: Synthetic peptide
<400> 1103
   Lys Val Ser Asn Arg Phe Ser
   1 5
<210> 1104
   <211> 7
   <212> PRT
   <213> Artificial Sequence
<220>
   <223> Description of Artificial Sequence: Synthetic peptide
<400> 1104
   Tyr Ala Ser Glu Ser Ile Ser
   1 5
<210> 1105
   <211> 7
   <212> PRT
   <213> Artificial Sequence
<220>
   <223> Description of Artificial Sequence: Synthetic peptide
<400> 1105
   Ala Thr Ser Asn Leu Ala Ser
   1 5
<210> 1106
   <211> 7
   <212> PRT
   <213> Artificial Sequence
<220>
   <223> Description of Artificial Sequence: Synthetic peptide
<400> 1106
   Asn Thr Asn Asn Leu Gln Thr
   1 5
<210> 1107
   <211> 7
   <212> PRT
   <213> Artificial Sequence
<220>
   <223> Description of Artificial Sequence: Synthetic peptide
<400> 1107
   Phe Thr Ser Ser Leu His Ser
   1 5
<210> 1108
   <211> 7
   <212> PRT
   <213> Artificial Sequence
<220>
   <223> Description of Artificial Sequence: Synthetic peptide
<400> 1108
   Ser Ala Ser Tyr Arg Tyr Thr
   1 5
<210> 1109
   <211> 7
   <212> PRT
   <213> Artificial Sequence
<220>
   <223> Description of Artificial Sequence: Synthetic peptide
<400> 1109
   Asp Ala Ser Asn Leu Glu Thr
   1 5
<210> 1110
   <211> 9
   <212> PRT
   <213> Artificial Sequence
<220>
   <223> Description of Artificial Sequence: Synthetic peptide
<400> 1110
   Gln Gln Ala Tyr Thr Thr Pro Pro Thr
   1 5
<210> 1111
   <211> 9
   <212> PRT
   <213> Artificial Sequence
<220>
   <223> Description of Artificial Sequence: Synthetic peptide
<400> 1111
   Gln Gln Phe Tyr Thr Thr Pro Pro Thr
   1 5
<210> 1112
   <211> 9
   <212> PRT
   <213> Artificial Sequence
<220>
   <223> Description of Artificial Sequence: Synthetic peptide
<400> 1112
   Gln Gln His Ala Thr Thr Pro Pro Thr
   1 5
<210> 1113
   <211> 9
   <212> PRT
   <213> Artificial Sequence
<220>
   <223> Description of Artificial Sequence: Synthetic peptide
<400> 1113
   Gln Gln His Phe Thr Thr Pro Pro Thr
   1 5
<210> 1114
   <211> 9
   <212> PRT
   <213> Artificial Sequence
<220>
   <223> Description of Artificial Sequence: Synthetic peptide
<400> 1114
   Gln Gln His Tyr Ala Thr Pro Pro Thr
   1 5
<210> 1115
   <211> 9
   <212> PRT
   <213> Artificial Sequence
<220>
   <223> Description of Artificial Sequence: Synthetic peptide
<400> 1115
   Gln Gln His Tyr Thr Ala Pro Pro Thr
   1 5
<210> 1116
   <211> 10
   <212> PRT
   <213> Artificial Sequence
<220>
   <223> Description of Artificial Sequence: Synthetic peptide
<400> 1116
   Gln Gln Gly Trp Tyr Ile Ala Pro Pro Thr
   1 5 10
<210> 1117
   <211> 11
   <212> PRT
   <213> Artificial Sequence
<220>
   <223> Description of Artificial Sequence: Synthetic peptide
<400> 1117
   Gln Gln Gly Gly Ser Tyr Ser Ser Pro Pro Thr
   1               5                   10
<210> 1118
   <211> 11
   <212> PRT
   <213> Artificial Sequence
<220>
   <223> Description of Artificial Sequence: Synthetic peptide
<400> 1118
   Gln Gln Tyr Gly Ser Phe Gly Thr Pro Pro Thr
   1               5                   10
<210> 1119
   <211> 9
   <212> PRT
   <213> Artificial Sequence
<220>
   <223> Description of Artificial Sequence: Synthetic peptide
<400> 1119
   Gln Gln Ser Tyr Ser Gly Pro Pro Thr
   1               5
<210> 1120
   <211> 9
   <212> PRT
   <213> Artificial Sequence
<220>
   <223> Description of Artificial Sequence: Synthetic peptide
<400> 1120
   Gln Gln Thr Asn Glu Asp Pro Tyr Thr
   1               5
<210> 1121
   <211> 9
   <212> PRT
   <213> Artificial Sequence
<220>
   <223> Description of Artificial Sequence: Synthetic peptide
<400> 1121
   Gln Gln Thr Asn Glu Asp Pro Tyr Thr
   1               5
<210> 1122
   <211> 9
   <212> PRT
   <213> Artificial Sequence
<220>
   <223> Description of Artificial Sequence: Synthetic peptide
<400> 1122
   Ser Gln Ser Thr His Val Pro Trp Thr
   1               5
<210> 1123
   <211> 9
   <212> PRT
   <213> Artificial Sequence
<220>
   <223> Description of Artificial Sequence: Synthetic peptide
<400> 1123
   Gln Gln Ser Thr His Val Pro Trp Thr
   1               5
<210> 1124
   <211> 9
   <212> PRT
   <213> Artificial Sequence
<220>
   <223> Description of Artificial Sequence: Synthetic peptide
<400> 1124
   Gln Gln Asn Asn Asn Trp Pro Thr Thr
   1               5
<210> 1125
   <211> 9
   <212> PRT
   <213> Artificial Sequence
<220>
   <223> Description of Artificial Sequence: Synthetic peptide
<400> 1125
   Gln Gln Trp Thr Ser Asn Pro Pro Thr
   1               5
<210> 1126
   <211> 9
   <212> PRT
   <213> Artificial Sequence
<220>
   <223> Description of Artificial Sequence: Synthetic peptide
<400> 1126
   Leu Gln His Ile Ser Arg Pro Arg Thr
   1               5
<210> 1127
   <211> 9
   <212> PRT
   <213> Artificial Sequence
<220>
   <223> Description of Artificial Sequence: Synthetic peptide
<400> 1127
   Gln Gln Tyr Ser Thr Val Pro Trp Thr
   1               5
<210> 1128
   <211> 9
   <212> PRT
   <213> Artificial Sequence
<220>
   <223> Description of Artificial Sequence: Synthetic peptide
<400> 1128
   Gln Gln Tyr Tyr Ile Tyr Pro Tyr Thr
   1               5
<210> 1129
   <211> 9
   <212> PRT
   <213> Artificial Sequence
<220>
   <223> Description of Artificial Sequence: Synthetic peptide
<400> 1129
   Gln His Phe Asp His Leu Pro Leu Ala
   1               5
<210> 1130
   <211> 27
   <212> PRT
   <213> Artificial Sequence
<220>
   <223> Description of Artificial Sequence: Synthetic peptide
<400> 1130
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Ser Arg
               20                  25
<210> 1131
   <211> 27
   <212> PRT
   <213> Artificial Sequence
<220>
   <223> Description of Artificial Sequence: Synthetic peptide
<400> 1131
   Arg Asp Thr Ser Lys Asn Thr Ala Tyr Leu Gln Met Asn Ser Leu Arg
   1               5                   10                  15
   Ala Glu Asp Thr Ala Val Tyr Tyr Cys Ser Arg
               20                  25
<210> 1132
   <211> 27
   <212> PRT
   <213> Artificial Sequence
<220>
   <223> Description of Artificial Sequence: Synthetic peptide
<400> 1132
   Ala Asp Thr Ser Lys Asn Thr Ala Tyr Leu Gln Met Asn Thr Leu Arg
   1               5                   10                  15
   Ala Glu Asp Thr Ala Val Tyr Tyr Cys Ser Arg
               20                  25
<210> 1133
   <211> 27
   <212> PRT
   <213> Artificial Sequence
<220>
   <223> Description of Artificial Sequence: Synthetic peptide
<400> 1133
   Ala Asp Thr Ser Lys Asn Thr Ala Tyr Leu Gln Met Asn Ser Leu Arg
   1 5 10 15
   Ala Glu Asp Thr Cys Val Tyr Tyr Cys Ser Arg
   20 25
<210> 1134
   <211> 27
   <212> PRT
   <213> Artificial Sequence
<220>
   <223> Description of Artificial Sequence: Synthetic peptide
<400> 1134
   Arg Asp Asn Ser Lys Asn Thr Leu Tyr Leu Gln Met Asn Ser Leu Arg
   1 5 10 15
   Ala Glu Asp Thr Ala Val Tyr Tyr Cys Ser Arg
   20 25
<210> 1135
   <211> 27
   <212> PRT
   <213> Artificial Sequence
<220>
   <223> Description of Artificial Sequence: Synthetic peptide
<400> 1135
   Ala Asp Thr Ser Lys Asn Thr Ala Tyr Leu Gln Met Asn Ser Leu Arg
   1 5 10 15
   Ala Glu Asp Thr Ala Val Tyr Tyr Cys Ala Arg
   20 25
<210> 1136
   <211> 27
   <212> PRT
   <213> Artificial Sequence
<220>
   <223> Description of Artificial Sequence: Synthetic peptide
<400> 1136
   Ala Asp Thr Ser Lys Asn Thr Ala Tyr Leu Gln Met Asn Ser Leu Arg
   1 5 10 15
   Ala Glu Asp Thr Ala Thr Tyr Tyr Cys Ser Arg
   20 25
<210> 1137
   <211> 27
   <212> PRT
   <213> Artificial Sequence
<220>
   <223> Description of Artificial Sequence: Synthetic peptide
<400> 1137
   Arg Asp Asp Ser Lys Asn Thr Leu Tyr Leu Gln Met Asn Ser Leu Arg
   1               5                   10                  15
   Ala Glu Asp Thr Ala Val Tyr Tyr Cys Ala Arg
               20                  25
<210> 1138
   <211> 27
   <212> PRT
   <213> Artificial Sequence
<220>
   <223> Description of Artificial Sequence: Synthetic peptide
<400> 1138
   Ala Asp Asp Ser Lys Asn Thr Leu Tyr Leu Gln Met Asn Ser Leu Arg
   1               5                   10                  15
   Ala Glu Asp Thr Ala Val Tyr Tyr Cys Ala Arg
               20                  25
<210> 1139
   <211> 27
   <212> PRT
   <213> Artificial Sequence
<220>
   <223> Description of Artificial Sequence: Synthetic peptide
<400> 1139
   Ala Asp Thr Ser Lys Asn Thr Leu Tyr Leu Gln Met Asn Ser Leu Arg
   1               5                   10                  15
   Ala Glu Asp Thr Ala Val Tyr Tyr Cys Ser Arg
               20                  25
<210> 1140
   <211> 27
   <212> PRT
   <213> Artificial Sequence
<220>
   <223> Description of Artificial Sequence: Synthetic peptide
<400> 1140
   Arg Asp Asp Ser Lys Asn Thr Leu Tyr Leu Gln Met Asn Ser Leu Arg
   1               5                   10                  15
   Ala Glu Asp Thr Ala Val Tyr Tyr Cys Ala Arg
               20                  25
<210> 1141
   <211> 27
   <212> PRT
   <213> Artificial Sequence
<220>
   <223> Description of Artificial Sequence: Synthetic peptide
<400> 1141
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Ala Lys
               20                  25
<210> 1142
   <211> 27
   <212> PRT
   <213> Artificial Sequence
<220>
   <223> Description of Artificial Sequence: Synthetic peptide
<400> 1142
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Ala Arg
               20                  25
<210> 1143
   <211> 27
   <212> PRT
   <213> Artificial Sequence
<220>
   <223> Description of Artificial Sequence: Synthetic peptide
<400> 1143
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Thr Ser
               20                  25
<210> 1144
   <211> 27
   <212> PRT
   <213> Artificial Sequence
<220>
   <223> Description of Artificial Sequence: Synthetic peptide
<400> 1144
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Gly Leu
               20                  25
<210> 1145
   <211> 27
   <212> PRT
   <213> Artificial Sequence
<220>
   <223> Description of Artificial Sequence: Synthetic peptide
<400> 1145
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Gly Val
               20                  25
<210> 1146
   <211> 27
   <212> PRT
   <213> Artificial Sequence
<220>
   <223> Description of Artificial Sequence: Synthetic peptide
<400> 1146
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Arg Thr
               20                  25
<210> 1147
   <211> 27
   <212> PRT
   <213> Artificial Sequence
<220>
   <223> Description of Artificial Sequence: Synthetic peptide
<400> 1147
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Ser Thr
               20                  25
<210> 1148
   <211> 27
   <212> PRT
   <213> Artificial Sequence
<220>
   <223> Description of Artificial Sequence: Synthetic peptide
<400> 1148
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Thr Ser
               20                  25
<210> 1149
   <211> 27
   <212> PRT
   <213> Artificial Sequence
<220>
   <223> Description of Artificial Sequence: Synthetic peptide
<400> 1149
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Gly Pro
               20                  25
<210> 1150
   <211> 27
   <212> PRT
   <213> Artificial Sequence
<220>
   <223> Description of Artificial Sequence: Synthetic peptide
<400> 1150
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Ser Thr
               20                  25
<210> 1151
   <211> 27
   <212> PRT
   <213> Artificial Sequence
<220>
   <223> Description of Artificial Sequence: Synthetic peptide
<400> 1151
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Val Thr
               20                  25
<210> 1152
   <211> 27
   <212> PRT
   <213> Artificial Sequence
<220>
   <223> Description of Artificial Sequence: Synthetic peptide
<400> 1152
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Gly Thr
               20                  25
<210> 1153
   <211> 27
   <212> PRT
   <213> Artificial Sequence
<220>
   <223> Description of Artificial Sequence: Synthetic peptide
<400> 1153
   Ala Asp Thr Ser Lys Asn Thr Ala Tyr Leu Gln Met Asn Ser Leu Arg
   1 5 10 15
   Ala Glu Asp Thr Ala Val Tyr Tyr Cys Leu Ser
   20 25
<210> 1154
   <211> 27
   <212> PRT
   <213> Artificial Sequence
<220>
   <223> Description of Artificial Sequence: Synthetic peptide
<400> 1154
   Ala Asp Thr Ser Lys Asn Thr Ala Tyr Leu Gln Met Asn Ser Leu Arg
   1 5 10 15
   Ala Glu Asp Thr Ala Val Tyr Tyr Cys Ser Ala
   20 25
<210> 1155
   <211> 27
   <212> PRT
   <213> Artificial Sequence
<220>
   <223> Description of Artificial Sequence: Synthetic peptide
<400> 1155
   Ala Asp Thr Ser Lys Asn Thr Ala Tyr Leu Gln Met Asn Ser Leu Arg
   1 5 10 15
   Ala Glu Asp Thr Ala Val Tyr Tyr Cys Met Lys
   20 25
<210> 1156
   <211> 27
   <212> PRT
   <213> Artificial Sequence
<220>
   <223> Description of Artificial Sequence: Synthetic peptide
<400> 1156
   Ala Asp Thr Ser Lys Asn Thr Ala Tyr Leu Gln Met Asn Ser Leu Arg
   1 5 10 15
   Ala Glu Asp Thr Ala Val Tyr Tyr Cys Thr Ser
   20 25
<210> 1157
   <211> 27
   <212> PRT
   <213> Artificial Sequence
<220>
   <223> Description of Artificial Sequence: Synthetic peptide
<400> 1157
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Gly Ser
               20                  25
<210> 1158
   <211> 27
   <212> PRT
   <213> Artificial Sequence
<220>
   <223> Description of Artificial Sequence: Synthetic peptide
<400> 1158
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Ile Arg
               20                  25
<210> 1159
   <211> 27
   <212> PRT
   <213> Artificial Sequence
<220>
   <223> Description of Artificial Sequence: Synthetic peptide
<400> 1159
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Arg Ser
               20                  25
<210> 1160
   <211> 27
   <212> PRT
   <213> Artificial Sequence
<220>
   <223> Description of Artificial Sequence: Synthetic peptide
<400> 1160
   Ala Asp Thr Ser Lys Asn Thr Ala Tyr Leu Gln Met Asn Ser Leu Arg
   1 5 10 15
   Ala Glu Asp Thr Ala Val Tyr Tyr Cys Gly Gly
   20 25
<210> 1161
   <211> 27
   <212> PRT
   <213> Artificial Sequence
<220>
   <223> Description of Artificial Sequence: Synthetic peptide
<400> 1161
   Ala Asp Thr Ser Lys Asn Thr Ala Tyr Leu Gln Met Asn Ser Leu Arg
   1 5 10 15
   Ala Glu Asp Thr Ala Val Tyr Tyr Cys Gly Phe
   20 25
<210> 1162
   <211> 27
   <212> PRT
   <213> Artificial Sequence
<220>
   <223> Description of Artificial Sequence: Synthetic peptide
<400> 1162
   Ala Asp Thr Ser Lys Asn Thr Ala Tyr Leu Gln Met Asn Ser Leu Arg
   1 5 10 15
   Ala Glu Asp Thr Ala Val Tyr Tyr Cys Arg Thr
   20 25
<210> 1163
   <211> 27
   <212> PRT
   <213> Artificial Sequence
<220>
   <223> Description of Artificial Sequence: Synthetic peptide
<400> 1163
   Ala Asp Thr Ser Lys Asn Thr Ala Tyr Leu Gln Met Asn Ser Leu Arg
   1 5 10 15
   Ala Glu Asp Thr Ala Val Tyr Tyr Cys Ser Met
   20 25
<210> 1164
   <211> 27
   <212> PRT
   <213> Artificial Sequence
<220>
   <223> Description of Artificial Sequence: Synthetic peptide
<400> 1164
   Ala Asp Thr Ser Lys Asn Thr Ala Tyr Leu Gln Met Asn Ser Leu Arg
   1 5 10 15
   Ala Glu Asp Thr Ala Val Tyr Ser Cys Gly Ala
   20 25
<210> 1165
   <211> 27
   <212> PRT
   <213> Artificial Sequence
<220>
   <223> Description of Artificial Sequence: Synthetic peptide
<400> 1165
   Ala Asp Thr Ser Lys Asn Thr Ala Tyr Leu Gln Met Asn Ser Leu Arg
   1 5 10 15
   Ala Glu Asp Thr Ala Val Tyr Tyr Cys Arg Ser
   20 25
<210> 1166
   <211> 27
   <212> PRT
   <213> Artificial Sequence
<220>
   <223> Description of Artificial Sequence: Synthetic peptide
<400> 1166
   Ala Asp Thr Ser Lys Asn Thr Ala Tyr Leu Gln Met Asn Ser Leu Arg
   1 5 10 15
   Ala Glu Asp Thr Ala Val Tyr Phe Cys Thr Thr
   20 25
<210> 1167
   <211> 27
   <212> PRT
   <213> Artificial Sequence
<220>
   <223> Description of Artificial Sequence: Synthetic peptide
<400> 1167
   Ala Asp Thr Ser Lys Asn Thr Ala Tyr Leu Gln Met Asn Ser Leu Arg
   1               5                   10                  15
   Ala Glu Asp Thr Ala Val Tyr Asn Cys Thr Thr
               20                  25
<210> 1168
   <211> 27
   <212> PRT
   <213> Artificial Sequence
<220>
   <223> Description of Artificial Sequence: Synthetic peptide
<400> 1168
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Gly Met
               20                  25
<210> 1169
   <211> 27
   <212> PRT
   <213> Artificial Sequence
<220>
   <223> Description of Artificial Sequence: Synthetic peptide
<400> 1169
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Gly Thr
               20                  25
<210> 1170
   <211> 27
   <212> PRT
   <213> Artificial Sequence
<220>
   <223> Description of Artificial Sequence: Synthetic peptide
<400> 1170
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Arg Pro
               20                  25
<210> 1171
   <211> 27
   <212> PRT
   <213> Artificial Sequence
<220>
   <223> Description of Artificial Sequence: Synthetic peptide
<400> 1171
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Arg Thr
               20                  25
<210> 1172
   <211> 27
   <212> PRT
   <213> Artificial Sequence
<220>
   <223> Description of Artificial Sequence: Synthetic peptide
<400> 1172
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Thr Thr
               20                  25
<210> 1173
   <211> 27
   <212> PRT
   <213> Artificial Sequence
<220>
   <223> Description of Artificial Sequence: Synthetic peptide
<400> 1173
   Ala Asp Thr Ser Lys Asn Thr Ala Tyr Leu Gln Met Asn Ser Leu Arg
   1 5 10 15
   Ala Glu Asp Thr Ala Val Tyr Tyr Cys Arg Met
   20 25
<210> 1174
   <211> 27
   <212> PRT
   <213> Artificial Sequence
<220>
   <223> Description of Artificial Sequence: Synthetic peptide
<400> 1174
   Ala Asp Thr Ser Lys Asn Thr Ala Tyr Leu Gln Met Asn Ser Leu Arg
   1 5 10 15
   Ala Glu Asp Thr Ala Val Tyr Arg Cys Ile Thr
   20 25
<210> 1175
   <211> 27
   <212> PRT
   <213> Artificial Sequence
<220>
   <223> Description of Artificial Sequence: Synthetic peptide
<400> 1175
   Ala Asp Thr Ser Lys Asn Thr Ala Tyr Leu Gln Met Asn Ser Leu Arg
   1 5 10 15
   Ala Glu Asp Thr Ala Val Tyr Tyr Cys Gly Ser
   20 25
<210> 1176
   <211> 27
   <212> PRT
   <213> Artificial Sequence
<220>
   <223> Description of Artificial Sequence: Synthetic peptide
<400> 1176
   Ala Asp Thr Ser Lys Asn Thr Ala Tyr Leu Gln Met Asn Ser Leu Arg
   1 5 10 15
   Ala Glu Asp Thr Ala Val Tyr Val Cys Arg Ala
   20 25
<210> 1177
   <211> 27
   <212> PRT
   <213> Artificial Sequence
<220>
   <223> Description of Artificial Sequence: Synthetic peptide
<400> 1177
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Gly Met
               20                  25
<210> 1178
   <211> 27
   <212> PRT
   <213> Artificial Sequence
<220>
   <223> Description of Artificial Sequence: Synthetic peptide
<400> 1178
   Ala Asp Thr Ser Lys Asn Thr Ala Tyr Leu Gln Met Asn Ser Leu Arg
   1               5                   10                  15
   Ala Glu Asp Thr Ala Val Tyr His Cys Val Thr
               20                  25
<210> 1179
   <211> 27
   <212> PRT
   <213> Artificial Sequence
<220>
   <223> Description of Artificial Sequence: Synthetic peptide
<400> 1179
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Thr Arg
               20                  25
<210> 1180
   <211> 27
   <212> PRT
   <213> Artificial Sequence
<220>
   <223> Description of Artificial Sequence: Synthetic peptide
<400> 1180
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Gln Thr
               20                  25
<210> 1181
   <211> 27
   <212> PRT
   <213> Artificial Sequence
<220>
   <223> Description of Artificial Sequence: Synthetic peptide
<400> 1181
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Lys Thr
               20                  25
<210> 1182
   <211> 27
   <212> PRT
   <213> Artificial Sequence
<220>
   <223> Description of Artificial Sequence: Synthetic peptide
<400> 1182
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Arg Ala
               20                  25
<210> 1183
   <211> 27
   <212> PRT
   <213> Artificial Sequence
<220>
   <223> Description of Artificial Sequence: Synthetic peptide
<400> 1183
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Ser Met
               20                  25
<210> 1184
   <211> 27
   <212> PRT
   <213> Artificial Sequence
<220>
   <223> Description of Artificial Sequence: Synthetic peptide
<400> 1184
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Val Ser
               20                  25
<210> 1185
   <211> 27
   <212> PRT
   <213> Artificial Sequence
<220>
   <223> Description of Artificial Sequence: Synthetic peptide
<400> 1185
   Ala Asp Thr Ser Lys Asn Thr Ala Tyr Leu Gln Met Asn Ser Leu Arg
   1               5                   10                  15
   Ala Glu Asp Thr Ala Val Tyr Arg Cys Gly Met
               20                  25
<210> 1186
   <211> 27
   <212> PRT
   <213> Artificial Sequence
<220>
   <223> Description of Artificial Sequence: Synthetic peptide
<400> 1186
   Ala Asp Thr Ser Lys Asn Thr Ala Tyr Leu Gln Met Asn Ser Leu Arg
   1               5                   10                  15
   Ala Glu Asp Thr Ala Val Tyr Lys Cys Arg Thr
               20                  25
<210> 1187
   <211> 27
   <212> PRT
   <213> Artificial Sequence
<220>
   <223> Description of Artificial Sequence: Synthetic peptide
<400> 1187
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Lys Thr
               20                  25
<210> 1188
   <211> 27
   <212> PRT
   <213> Artificial Sequence
<220>
   <223> Description of Artificial Sequence: Synthetic peptide
<400> 1188
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Arg Thr
               20                  25
<210> 1189
   <211> 27
   <212> PRT
   <213> Artificial Sequence
<220>
   <223> Description of Artificial Sequence: Synthetic peptide
<400> 1189
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Arg Ser
               20                  25
<210> 1190
   <211> 27
   <212> PRT
   <213> Artificial Sequence
<220>
   <223> Description of Artificial Sequence: Synthetic peptide
<400> 1190
   Ala Asp Thr Ser Lys Asn Thr Ala Tyr Leu Gln Met Asn Ser Leu Arg
   1 5 10 15
   Ala Glu Asp Thr Ala Val Tyr Phe Cys Ser Thr
   20 25
<210> 1191
   <211> 27
   <212> PRT
   <213> Artificial Sequence
<220>
   <223> Description of Artificial Sequence: Synthetic peptide
<400> 1191 Ala Glu Asp Thr Ala Val Tyr Tyr Cys Gly Thr 20 25
<210> 1192
   <211> 27
   <212> PRT
   <213> Artificial Sequence
<220>
   <223> Description of Artificial Sequence: Synthetic peptide
<400> 1192
   Ala Asp Thr Ser Lys Asn Thr Ala Tyr Leu Gln Met Asn Ser Leu Arg
   1 5 10 15
   Ala Glu Asp Thr Ala Val Tyr Tyr Cys Ser Thr
   20 25
<210> 1193
   <211> 27
   <212> PRT
   <213> Artificial Sequence
<220>
   <223> Description of Artificial Sequence: Synthetic peptide
<400> 1193
   Ala Asp Thr Ser Lys Asn Thr Ala Tyr Leu Gln Met Asn Ser Leu Arg
   1 5 10 15
   Ala Glu Asp Thr Ala Val Tyr Ser Cys Arg Thr
   20 25
<210> 1194
   <211> 27
   <212> PRT
   <213> Artificial Sequence
<220>
   <223> Description of Artificial Sequence: Synthetic peptide
<400> 1194
   Ala Asp Thr Ser Lys Asn Thr Ala Tyr Leu Gln Met Asn Ser Leu Arg
   1               5                   10                  15
   Ala Glu Asp Thr Ala Val Tyr His Cys Ile Thr
               20                  25
<210> 1195
   <211> 27
   <212> PRT
   <213> Artificial Sequence
<220>
   <223> Description of Artificial Sequence: Synthetic peptide
<400> 1195
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Arg Ala
               20                  25
<210> 1196
   <211> 27
   <212> PRT
   <213> Artificial Sequence
<220>
   <223> Description of Artificial Sequence: Synthetic peptide
<400> 1196
   Ala Asp Thr Ser Lys Asn Thr Ala Tyr Leu Gln Met Asn Ser Leu Arg
   1               5                   10                  15
   Ala Glu Asp Thr Ala Val Tyr Ser Cys Arg Met
               20                  25
<210> 1197
   <211> 27
   <212> PRT
   <213> Artificial Sequence
<220>
   <223> Description of Artificial Sequence: Synthetic peptide
<400> 1197
   Ala Asp Thr Ser Lys Asn Thr Ala Tyr Leu Gln Met Asn Ser Leu Arg
   1               5                   10                  15
   Ala Glu Asp Thr Ala Val Tyr Phe Cys Arg Thr
               20                  25
<210> 1198
   <211> 27
   <212> PRT
   <213> Artificial Sequence
<220>
   <223> Description of Artificial Sequence: Synthetic peptide
<400> 1198
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Thr Thr
               20                  25
<210> 1199
   <211> 27
   <212> PRT
   <213> Artificial Sequence
<220>
   <223> Description of Artificial Sequence: Synthetic peptide
<400> 1199
   Ala Asp Thr Ser Lys Asn Thr Ala Tyr Leu Gln Met Asn Ser Leu Arg
   1               5                   10                  15
   Ala Glu Asp Thr Ala Val Tyr Tyr Cys Ser Ile
               20                  25
<210> 1200
   <211> 27
   <212> PRT
   <213> Artificial Sequence
<220>
   <223> Description of Artificial Sequence: Synthetic peptide
<400> 1200
   Ala Asp Thr Ser Lys Asn Thr Ala Tyr Leu Gln Met Asn Ser Leu Arg
   1 5 10 15
   Ala Glu Asp Thr Ala Val Tyr Tyr Cys Ala Thr
   20 25
<210> 1201
   <211> 27
   <212> PRT
   <213> Artificial Sequence
<220>
   <223> Description of Artificial Sequence: Synthetic peptide
<400> 1201
   Ala Asp Thr Ser Lys Asn Thr Ala Tyr Leu Gln Met Asn Ser Leu Arg
   1 5 10 15
   Ala Glu Asp Thr Ala Val Tyr Tyr Cys Val Thr
   20 25
<210> 1202
   <211> 27
   <212> PRT
   <213> Artificial Sequence
<220>
   <223> Description of Artificial Sequence: Synthetic peptide
<400> 1202
   Ala Asp Thr Ser Lys Asn Thr Ala Tyr Leu Gln Met Asn Ser Leu Arg
   1 5 10 15
   Ala Glu Asp Thr Ala Val Tyr Tyr Cys Arg Val
   20 25
<210> 1203
   <211> 27
   <212> PRT
   <213> Artificial Sequence
<220>
   <223> Description of Artificial Sequence: Synthetic peptide
<400> 1203
   Ala Asp Thr Ser Lys Asn Thr Ala Tyr Leu Gln Met Asn Ser Leu Arg
   1 5 10 15
   Ala Glu Asp Thr Ala Val Tyr Tyr Cys Lys Thr
   20 25
<210> 1204
   <211> 27
   <212> PRT
   <213> Artificial Sequence
<220>
   <223> Description of Artificial Sequence: Synthetic peptide
<400> 1204
   Ala Asp Thr Ser Lys Asn Thr Ala Tyr Leu Gln Met Asn Ser Leu Arg
   1 5 10 15
   Ala Glu Asp Thr Ala Val Tyr Tyr Cys Gly Arg
   20 25
<210> 1205
   <211> 27
   <212> PRT
   <213> Artificial Sequence
<220>
   <223> Description of Artificial Sequence: Synthetic peptide
<400> 1205
   Ala Asp Thr Ser Lys Asn Thr Ala Tyr Leu Gln Met Asn Ser Leu Arg
   1 5 10 15
   Ala Glu Asp Thr Ala Val Tyr Phe Cys Arg Ala
   20 25
<210> 1206
   <211> 27
   <212> PRT
   <213> Artificial Sequence
<220>
   <223> Description of Artificial Sequence: Synthetic peptide
<400> 1206
   Ala Asp Thr Ser Lys Asn Thr Ala Tyr Leu Gln Met Asn Ser Leu Arg
   1 5 10 15
   Ala Glu Asp Thr Ala Val Tyr Tyr Cys Lys Met
   20 25
<210> 1207
   <211> 27
   <212> PRT
   <213> Artificial Sequence
<220>
   <223> Description of Artificial Sequence: Synthetic peptide
<400> 1207
   Ala Asp Thr Ser Lys Asn Thr Ala Tyr Leu Gln Met Asn Ser Leu Arg
   1               5                   10                  15
   Ala Glu Asp Thr Ala Val Tyr Ser Cys Lys Thr
               20                  25
<210> 1208
   <211> 27
   <212> PRT
   <213> Artificial Sequence
<220>
   <223> Description of Artificial Sequence: Synthetic peptide
<400> 1208
   Ala Asp Thr Ser Lys Asn Thr Ala Tyr Leu Gln Met Asn Ser Leu Arg
   1               5                   10                  15
   Ala Glu Asp Thr Ala Val Tyr Ser Cys Arg Ser
               20                  25
<210> 1209
   <211> 27
   <212> PRT
   <213> Artificial Sequence
<220>
   <223> Description of Artificial Sequence: Synthetic peptide
<400> 1209
   Ala Asp Thr Ser Lys Asn Thr Ala Tyr Leu Gln Met Asn Ser Leu Arg
   1               5                   10                  15
   Ala Glu Asp Thr Ala Val Tyr His Cys Arg Ile
               20                  25
<210> 1210
   <211> 27
   <212> PRT
   <213> Artificial Sequence
<220>
   <223> Description of Artificial Sequence: Synthetic peptide
<400> 1210
   Ala Asp Thr Ser Lys Asn Thr Ala Tyr Leu Gln Met Asn Ser Leu Arg
   1               5                   10                  15
   Ala Glu Asp Thr Ala Val Tyr Gln Cys Ser Thr
               20                  25
<210> 1211
   <211> 27
   <212> PRT
   <213> Artificial Sequence
<220>
   <223> Description of Artificial Sequence: Synthetic peptide
<400> 1211
   Ala Asp Asn Thr Gln Asn Met Ala Tyr Leu Gln Met Asn Ser Leu Arg
   1               5                   10                  15
   Ala Glu Asp Thr Ala Val Tyr Tyr Cys Ser Arg
               20                  25
<210> 1212
   <211> 27
   <212> PRT
   <213> Artificial Sequence
<220>
   <223> Description of Artificial Sequence: Synthetic peptide
<400> 1212
   Ala Asp Thr Ser Ser Asn Thr Ala Tyr Leu Gln Val Ser Arg Leu Thr
   1               5                   10                  15
   Ser Glu Asp Thr Ala Val Tyr Tyr Cys Ser Arg
               20                  25
<210> 1213
   <211> 27
   <212> PRT
   <213> Artificial Sequence
<220>
   <223> Description of Artificial Sequence: Synthetic peptide
<400> 1213
   Arg Asp Asn Ser Lys Asn Thr Leu Tyr Leu Gln Met Asn Ser Leu Arg
   1               5                   10                  15
   Ala Glu Asp Thr Ala Val Tyr Tyr Cys Ser Arg
               20                  25
<210> 1214
   <211> 27
   <212> PRT
   <213> Artificial Sequence
<220>
   <223> Description of Artificial Sequence: Synthetic peptide
<400> 1214
   Ala Asp Thr Ser Lys Asn Thr Ala Tyr Leu Gln Met Asn Ser Leu Pro
   1               5                   10                  15
   Ala Glu Asp Thr Ala Val Tyr Tyr Cys Ser Arg
               20                  25
<210> 1215
   <211> 27
   <212> PRT
   <213> Artificial Sequence
<220>
   <223> Description of Artificial Sequence: Synthetic peptide
<400> 1215
   Val Asp Arg Ser Ser Arg Ile Val Tyr Met Glu Leu Arg Ser Leu Thr
   1               5                   10                  15
   Phe Glu Asp Thr Ala Val Tyr Tyr Cys Ala Arg
               20                  25
<210> 1216
   <211> 27
   <212> PRT
   <213> Artificial Sequence
<220>
   <223> Description of Artificial Sequence: Synthetic peptide
<400> 1216
   Val Asp Arg Ser Lys Asn Thr Leu Tyr Leu Gln Met Asn Ser Leu Arg
   1               5                   10                  15
   Ala Glu Asp Thr Ala Val Tyr Tyr Cys Ala Arg
               20                  25
<210> 1217
   <211> 27
   <212> PRT
   <213> Artificial Sequence
<220>
   <223> Description of Artificial Sequence: Synthetic peptide
<400> 1217
   Val Asp Thr Ser Lys Asn Thr Ala Tyr Leu Gln Met Asn Ser Leu Arg
   1 5 10 15
   Ala Glu Asp Thr Ala Val Tyr Tyr Cys Ser Arg
   20 25
<210> 1218
   <211> 27
   <212> PRT
   <213> Artificial Sequence
<220>
   <223> Description of Artificial Sequence: Synthetic peptide
<400> 1218
   Ala Asp Arg Ser Lys Asn Thr Ala Tyr Leu Gln Met Asn Ser Leu Arg
   1 5 10 15
   Ala Glu Asp Thr Ala Val Tyr Tyr Cys Ser Arg
   20 25
<210> 1219
   <211> 27
   <212> PRT
   <213> Artificial Sequence
<220>
   <223> Description of Artificial Sequence: Synthetic peptide
<400> 1219
   Val Asp Arg Ser Lys Asn Thr Ala Tyr Leu Gln Met Asn Ser Leu Arg
   1 5 10 15
   Ala Glu Asp Thr Ala Val Tyr Tyr Cys Ser Arg
   20 25
<210> 1220
   <211> 27
   <212> PRT
   <213> Artificial Sequence
<220>
   <223> Description of Artificial Sequence: Synthetic peptide
<400> 1220
   Val Asp Arg Ser Lys Asn Thr Ala Tyr Leu Gln Met Asn Ser Leu Arg
   1               5                   10                  15
   Ala Glu Asp Thr Ala Val Tyr Tyr Cys Ser Arg
               20                  25
<210> 1221
   <211> 27
   <212> PRT
   <213> Artificial Sequence
<220>
   <223> Description of Artificial Sequence: Synthetic peptide
<400> 1221
   Val Asp Arg Ser Lys Asn Thr Ala Tyr Leu Gln Met Asn Ser Leu Arg
   1               5                   10                  15
   Ala Glu Asp Thr Ala Val Tyr Tyr Cys Ser Arg
               20                  25
<210> 1222
   <211> 27
   <212> PRT
   <213> Artificial Sequence
<220>
   <223> Description of Artificial Sequence: Synthetic peptide
<400> 1222
   Val Asp Arg Ser Lys Asn Thr Val Tyr Leu Gln Met Asn Ser Leu Arg
   1               5                   10                  15
   Ala Glu Asp Thr Ala Val Tyr Tyr Cys Ser Arg
               20                  25
<210> 1223
   <211> 27
   <212> PRT
   <213> Artificial Sequence
<220>
   <223> Description of Artificial Sequence: Synthetic peptide
<400> 1223
   Val Asp Arg Ser Lys Asn Thr Ala Tyr Leu Gln Met Asn Ser Leu Arg
   1               5                   10                  15
   Ala Glu Asp Thr Ala Val Tyr Tyr Cys Ser Arg
               20                  25
<210> 1224
   <211> 27
   <212> PRT
   <213> Artificial Sequence
<220>
   <223> Description of Artificial Sequence: Synthetic peptide
<400> 1224
   Val Asp Arg Ser Lys Asn Thr Ala Tyr Leu Gln Met Asn Ser Leu Arg
   1               5                   10                  15
   Ala Glu Asp Thr Ala Val Tyr Tyr Cys Ser Arg
               20                  25
<210> 1225
   <211> 22
   <212> PRT
   <213> Artificial Sequence
<220>
   <223> Description of Artificial Sequence: Synthetic peptide
<400> 1225
   Thr Cys Arg Ala Ser Gln Asp Val Asn Thr Ala Val Ala Trp Tyr Gln
   1               5                   10                  15
   Gln Lys Pro Gly Lys Ala
               20
<210> 1226
   <211> 22
   <212> PRT
   <213> Artificial Sequence
<220>
   <223> Description of Artificial Sequence: Synthetic peptide
<400> 1226
   Asn Cys Lys Ala Ser Gln Asp Val Asn Thr Ala Val Ala Trp Tyr Gln
   1               5                   10                  15
   Gln Lys Pro Gly Lys Ala
               20
<210> 1227
   <211> 22
   <212> PRT
   <213> Artificial Sequence
<220>
   <223> Description of Artificial Sequence: Synthetic peptide
<400> 1227
   Thr Cys Arg Ala Ser Ser Gln Val Ser Ser Gly Val Ala Trp Tyr Gln
   1 5 10 15
   Gln Lys Pro Gly Lys Ala
   20
<210> 1228
   <211> 22
   <212> PRT
   <213> Artificial Sequence
<220>
   <223> Description of Artificial Sequence: Synthetic peptide
<400> 1228
   Thr Cys Arg Ala Ser Arg Gln Val Asn Thr Ala Val Ala Trp Tyr Gln
   1 5 10 15
   Gln Lys Pro Gly Lys Ala
   20
<210> 1229
   <211> 22
   <212> PRT
   <213> Artificial Sequence
<220>
   <223> Description of Artificial Sequence: Synthetic peptide
<400> 1229
   Thr Cys Arg Ala Ser Ala Gln Val Ser Ala Gly Val Ala Trp Tyr Gln
   1 5 10 15
   Gln Lys Pro Gly Lys Ala
   20
<210> 1230
   <211> 22
   <212> PRT
   <213> Artificial Sequence
<220>
   <223> Description of Artificial Sequence: Synthetic peptide
<400> 1230
   Thr Cys Arg Ala Ser Gln Gly Val Ser Ser Gly Val Ala Trp Tyr Gln
   1 5 10 15
   Gln Lys Pro Gly Lys Ala
   20
<210> 1231
   <211> 22
   <212> PRT
   <213> Artificial Sequence
<220>
   <223> Description of Artificial Sequence: Synthetic peptide
<400> 1231
   Thr Cys Arg Ala Ser Gln Gly Val Ser Ser Ala Val Ala Trp Tyr Gln
   1 5 10 15
   Gln Lys Pro Gly Lys Ala
   20
<210> 1232
   <211> 22
   <212> PRT
   <213> Artificial Sequence
<220>
   <223> Description of Artificial Sequence: Synthetic peptide
<400> 1232
   Thr Cys Arg Ala Ser Gln Arg Val Asn Ser Ala Val Ala Trp Tyr Gln
   1 5 10 15
   Gln Lys Pro Gly Lys Ala
   20
<210> 1233
   <211> 22
   <212> PRT
   <213> Artificial Sequence
<220>
   <223> Description of Artificial Sequence: Synthetic peptide
<400> 1233
   Thr Cys Arg Ala Ser Gln Gly Val Ser Ser Ala Val Ala Trp Tyr Gln
   1 5 10 15
   Gln Lys Pro Gly Lys Ala
   20
<210> 1234
   <211> 22
   <212> PRT
   <213> Artificial Sequence
<220>
   <223> Description of Artificial Sequence: Synthetic peptide
<400> 1234
   Thr Cys Arg Ala Ser Asn Pro Val Ser Gln Ala Val Ala Trp Tyr Gln
   1               5                   10                  15
   Gln Lys Pro Gly Lys Ala
               20
<210> 1235
   <211> 22
   <212> PRT
   <213> Artificial Sequence
<220>
   <223> Description of Artificial Sequence: Synthetic peptide
<400> 1235
   Thr Cys Arg Ala Ser Ser Gln Val Ser Lys Ala Val Ala Trp Tyr Gln
   1               5                   10                  15
   Gln Lys Pro Gly Lys Ala
               20
<210> 1236
   <211> 22
   <212> PRT
   <213> Artificial Sequence
<220>
   <223> Description of Artificial Sequence: Synthetic peptide
<400> 1236
   Thr Cys Arg Ala Ser Phe Asp Val Asn Ala Cys Val Ala Trp Tyr Gln
   1               5                   10                  15
   Gln Lys Pro Gly Lys Ala
               20
<210> 1237
   <211> 22
   <212> PRT
   <213> Artificial Sequence
<220>
   <223> Description of Artificial Sequence: Synthetic peptide
<400> 1237
   Thr Cys Arg Ala Ser Gln Ser Val Ser Ser Ala Val Ala Trp Tyr Gln
   1               5                   10                  15
   Gln Lys Pro Gly Lys Ala
               20
<210> 1238
   <211> 22
   <212> PRT
   <213> Artificial Sequence
<220>
   <223> Description of Artificial Sequence: Synthetic peptide
<400> 1238
   Thr Cys Arg Ala Ser Gln Asp Val Asn Ser Tyr Leu Ala Trp Tyr Gln
   1               5                   10                  15
   Gln Lys Pro Gly Lys Ala
               20
<210> 1239
   <211> 22
   <212> PRT
   <213> Artificial Sequence
<220>
   <223> Description of Artificial Sequence: Synthetic peptide
<400> 1239
   Thr Cys Arg Ala Ser Gln Asn Val Asp Lys Tyr Val Ala Trp Tyr Gln
   1               5                   10                  15
   Gln Lys Pro Gly Lys Ala
               20
<210> 1240
   <211> 23
   <212> PRT
   <213> Artificial Sequence
<220>
   <223> Description of Artificial Sequence: Synthetic peptide
<400> 1240
   Pro Lys Leu Leu Ile Tyr Ser Ala Ser Phe Leu Tyr Ser Gly Val Pro
   1               5                   10                  15
   Ser Arg Phe Ser Gly Ser Arg
               20
<210> 1241
   <211> 23
   <212> PRT
   <213> Artificial Sequence
<220>
   <223> Description of Artificial Sequence: Synthetic peptide
<400> 1241
   Pro Lys Leu Leu Ile Tyr Ser Ala Ser Phe Leu Tyr Ser Gly Val Pro
   1 5 10 15
   Ser Arg Phe Ser Gly Ser Gly
   20
<210> 1242
   <211> 23
   <212> PRT
   <213> Artificial Sequence
<220>
   <223> Description of Artificial Sequence: Synthetic peptide
<400> 1242
   Pro Lys Leu Leu Ile Tyr Ser Ala Ser Phe Leu Tyr Ser Gly Val Pro
   1 5 10 15
   Ser Arg Phe Ser Gly Ser Arg
   20
<210> 1243
   <211> 23
   <212> PRT
   <213> Artificial Sequence
<220>
   <223> Description of Artificial Sequence: Synthetic peptide
<400> 1243
   Pro Lys Leu Leu Ile Tyr Ser Ala Ser Phe Leu Tyr Ser Gly Val Pro
   1 5 10 15
   Ser Arg Phe Ser Gly Ser Arg
   20
<210> 1244
   <211> 23
   <212> PRT
   <213> Artificial Sequence
<220>
   <223> Description of Artificial Sequence: Synthetic peptide
<400> 1244
   Pro Lys Leu Leu Ile Tyr Ser Ala Ser Phe Leu Tyr Ser Gly Val Pro
   1 5 10 15
   Ser Arg Phe Ser Gly Ser Arg
   20
<210> 1245
   <211> 23
   <212> PRT
   <213> Artificial Sequence
<220>
   <223> Description of Artificial Sequence: Synthetic peptide
<400> 1245
   Pro Lys Leu Leu Ile Tyr Ser Ala Ser Phe Leu Tyr Ser Gly Val Pro
   1 5 10 15
   Ser Arg Phe Ser Gly Ser Ala
   20
<210> 1246
   <211> 23
   <212> PRT
   <213> Artificial Sequence
<220>
   <223> Description of Artificial Sequence: Synthetic peptide
<400> 1246
   Pro Lys Leu Leu Ile Tyr Ser Ala Ser Phe Leu Tyr Ser Gly Val Pro
   1 5 10 15
   Ser Arg Phe Ser Gly Ser Asn
   20
<210> 1247
   <211> 23
   <212> PRT
   <213> Artificial Sequence
<220>
   <223> Description of Artificial Sequence: Synthetic peptide
<400> 1247
   Pro Lys Leu Leu Ile Tyr Ser Ala Ser Phe Leu Tyr Ser Gly Val Pro
   1 5 10 15
   Ser Arg Phe Ser Gly Ser Arg
   20
<210> 1248
   <211> 23
   <212> PRT
   <213> Artificial Sequence
<220>
   <223> Description of Artificial Sequence: Synthetic peptide
<400> 1248
   Pro Lys Leu Leu Ile Tyr Ser Ala Ser Phe Leu Tyr Ser Gly Val Pro
   1 5 10 15
   Ser Arg Phe Ser Gly Ser Met
   20
<210> 1249
   <211> 23
   <212> PRT
   <213> Artificial Sequence
<220>
   <223> Description of Artificial Sequence: Synthetic peptide
<400> 1249
   Pro Lys Leu Leu Ile Tyr Ser Ala Ser Phe Leu Tyr Ser Gly Val Pro
   1 5 10 15
   Ser Arg Phe Ser Gly Ser Thr
   20
<210> 1250
   <211> 23
   <212> PRT
   <213> Artificial Sequence
<220>
   <223> Description of Artificial Sequence: Synthetic peptide
<400> 1250
   Pro Lys Leu Leu Ile Tyr Ser Ala Ser Phe Leu Tyr Ser Gly Val Pro
   1 5 10 15
   Ser Arg Phe Ser Gly Ser Ser
   20
<210> 1251
   <211> 23
   <212> PRT
   <213> Artificial Sequence
<220>
   <223> Description of Artificial Sequence: Synthetic peptide
<400> 1251
   Pro Lys Leu Leu Ile Tyr Ser Ala Ser Phe Leu Tyr Ser Gly Val Pro
   1               5                   10                  15
   Ser Arg Phe Ser Gly Ser Val
               20
<210> 1252
   <211> 23
   <212> PRT
   <213> Artificial Sequence
<220>
   <223> Description of Artificial Sequence: Synthetic peptide
<400> 1252
   Pro Lys Leu Leu Ile Tyr Ser Ala Ser Phe Leu Tyr Ser Gly Val Pro
   1               5                   10                  15
   Ser Arg Phe Ser Gly Ser Gly
               20
<210> 1253
   <211> 23
   <212> PRT
   <213> Artificial Sequence
<220>
   <223> Description of Artificial Sequence: Synthetic peptide
<400> 1253
   Pro Lys Leu Leu Ile Tyr Ser Ala Ser Ser Leu Tyr Ser Gly Val Pro
   1               5                   10                  15
   Ser Arg Phe Ser Gly Ser Gly
               20
<210> 1254
   <211> 23
   <212> PRT
   <213> Artificial Sequence
<220>
   <223> Description of Artificial Sequence: Synthetic peptide
<400> 1254
   Pro Lys Leu Leu Ile Tyr Ser Ala Ser Phe Leu Glu Ser Gly Val Pro
   1 5 10 15
   Ser Arg Phe Ser Gly Ser Gly
   20
<210> 1255
   <211> 23
   <212> PRT
   <213> Artificial Sequence
<220>
   <223> Description of Artificial Sequence: Synthetic peptide
<400> 1255
   Pro Lys Leu Leu Ile Tyr Ser Ala Ser Phe Leu Glu Ser Gly Val Pro
   1 5 10 15
   Ser Arg Phe Ser Gly Ser Arg
   20
<210> 1256
   <211> 23
   <212> PRT
   <213> Artificial Sequence
<220>
   <223> Description of Artificial Sequence: Synthetic peptide
<400> 1256
   Pro Lys Leu Leu Ile Tyr Ala Ala Ser Ser Leu Tyr Ser Gly Val Pro
   1 5 10 15
   Ser Arg Phe Ser Gly Ser Gly
   20
<210> 1257
   <211> 23
   <212> PRT
   <213> Artificial Sequence
<220>
   <223> Description of Artificial Sequence: Synthetic peptide
<400> 1257
   Pro Lys Leu Leu Ile Tyr Asn Thr Asn Asn Leu Gln Thr Gly Val Pro
   1 5 10 15
   Ser Arg Phe Ser Gly Ser Arg
   20
<210> 1258
   <211> 22
   <212> PRT
   <213> Artificial Sequence
<220>
   <223> Description of Artificial Sequence: Synthetic peptide
<400> 1258
   Thr Cys Arg Ala Ser Gln Asn Val Asp Lys Tyr Val Ala Trp Tyr Gln
   1 5 10 15
   Gln Lys Pro Gly Lys Ala
   20
<210> 1259
   <211> 22
   <212> PRT
   <213> Artificial Sequence
<220>
   <223> Description of Artificial Sequence: Synthetic peptide
<400> 1259
   Thr Cys Arg Ala Ser Gln Asp Val Asn Thr Ala Val Ala Trp Tyr Gln
   1 5 10 15
   Gln Lys Leu Gly Glu Ser
   20
<210> 1260
   <211> 22
   <212> PRT
   <213> Artificial Sequence
<220>
   <223> Description of Artificial Sequence: Synthetic peptide
<400> 1260
   Thr Cys Arg Ala Ser Gln Asp Val Asn Thr Ala Val Ala Trp Tyr Gln
   1               5                   10                  15
   Gln Lys Pro Gly Lys Cys
               20
<210> 1261
   <211> 22
   <212> PRT
   <213> Artificial Sequence
<220>
   <223> Description of Artificial Sequence: Synthetic peptide
<400> 1261
   Thr Cys Arg Ala Ser Gln Asp Val Ser Thr Ala Val Ala Trp Tyr Gln
   1               5                   10                  15
   Gln Lys Pro Gly Lys Ala
               20
<210> 1262
   <211> 23
   <212> PRT
   <213> Artificial Sequence
<220>
   <223> Description of Artificial Sequence: Synthetic peptide
<400> 1262
   Pro Lys Leu Leu Ile Tyr Asn Thr Asn Asn Ser Gln Thr Gly Val Pro
   1               5                   10                  15
   Ser Arg Phe Ser Gly Ser Arg
               20
<210> 1263
   <211> 23
   <212> PRT
   <213> Artificial Sequence
<220>
   <223> Description of Artificial Sequence: Synthetic peptide
<400> 1263
   Pro Lys Leu Leu Ile Trp Ser Ala Ser Val Ser Ala Ser Gly Val Pro
   1               5                   10                  15
   Ser Arg Phe Ser Gly Ser Arg
               20
<210> 1264
   <211> 23
   <212> PRT
   <213> Artificial Sequence
<220>
   <223> Description of Artificial Sequence: Synthetic peptide
<400> 1264
   Pro Lys Leu Leu Ile Trp Ser Ala Ser Phe Ser Ala Ser Gly Val Pro
   1               5                   10                  15
   Ser Arg Phe Ser Gly Ser Arg
               20
<210> 1265
   <211> 23
   <212> PRT
   <213> Artificial Sequence
<220>
   <223> Description of Artificial Sequence: Synthetic peptide
<400> 1265
   Pro Lys Leu Leu Ile Leu Ser Ala Ser Phe Ser Ala Ser Gly Val Pro
   1               5                   10                  15
   Ser Arg Phe Ser Gly Ser Arg
               20
<210> 1266
   <211> 23
   <212> PRT
   <213> Artificial Sequence
<220>
   <223> Description of Artificial Sequence: Synthetic peptide
<400> 1266
   Pro Lys Leu Leu Ile Phe Ser Ala Ser Phe Ser Ala Ser Gly Val Pro
   1               5                   10                  15
   Ser Arg Phe Ser Gly Ser Arg
                                           20
<210> 1267
   <211> 23
   <212> PRT
   <213> Artificial Sequence
<220>
   <223> Description of Artificial Sequence: Synthetic peptide
<400> 1267
   Pro Lys Leu Leu Ile Trp Ser Ala Ser Val Ser Ala Ser Gly Val Pro
   1               5                   10                  15
   Ser Arg Phe Ser Gly Ser Arg
               20
<210> 1268
   <211> 23
   <212> PRT
   <213> Artificial Sequence
<220>
   <223> Description of Artificial Sequence: Synthetic peptide
<400> 1268
   Pro Lys Leu Leu Ile Ser Ser Ala Ser Trp Ser Ala Ser Gly Val Pro
   1               5                   10                  15
   Ser Arg Phe Ser Gly Ser Arg
               20
<210> 1269
   <211> 23
   <212> PRT
   <213> Artificial Sequence
<220>
   <223> Description of Artificial Sequence: Synthetic peptide
<400> 1269
   Pro Lys Leu Leu Ile Lys Ser Ala Ser Phe Ser Ala Ser Gly Val Pro
   1               5                   10                  15
   Ser Arg Phe Ser Gly Ser Arg
               20
<210> 1270
   <211> 23
   <212> PRT
   <213> Artificial Sequence
<220>
   <223> Description of Artificial Sequence: Synthetic peptide
<400> 1270
   Pro Lys Leu Leu Ile Tyr Ser Ala Ser Phe Ser Ala Ser Gly Val Pro
   1               5                   10                  15
   Ser Arg Phe Ser Gly Ser Arg
               20
<210> 1271
   <211> 23
   <212> PRT
   <213> Artificial Sequence
<220>
   <223> Description of Artificial Sequence: Synthetic peptide
<400> 1271
   Pro Lys Leu Leu Ile Val Ser Ala Ser Trp Leu Tyr Ser Gly Val Pro
   1               5                   10                  15
   Ser Arg Phe Ser Gly Ser Arg
               20
<210> 1272
   <211> 23
   <212> PRT
   <213> Artificial Sequence
<220>
   <223> Description of Artificial Sequence: Synthetic peptide
<400> 1272
   Pro Lys Leu Leu Ile Val Ser Ala Ser Phe Leu Tyr Ser Gly Val Pro
   1               5                   10                  15
   Ser Arg Phe Ser Gly Ser Arg
               20
<210> 1273
   <211> 23
   <212> PRT
   <213> Artificial Sequence
<220>
   <223> Description of Artificial Sequence: Synthetic peptide
<400> 1273
   Pro Lys Leu Leu Ile Phe Ser Ala Ser Trp Leu Tyr Ser Gly Val Pro
   1               5                   10                  15
   Ser Arg Phe Ser Gly Ser Arg
               20
<210> 1274
   <211> 23
   <212> PRT
   <213> Artificial Sequence
<220>
   <223> Description of Artificial Sequence: Synthetic peptide
<400> 1274
   Pro Lys Leu Leu Ile Leu Ser Ala Ser Val Leu Tyr Ser Gly Val Pro
   1               5                   10                  15
   Ser Arg Phe Ser Gly Ser Arg
               20
<210> 1275
   <211> 23
   <212> PRT
   <213> Artificial Sequence
<220>
   <223> Description of Artificial Sequence: Synthetic peptide
<400> 1275
   Pro Lys Leu Leu Ile Leu Ser Ala Ser Leu Leu Tyr Ser Gly Val Pro
   1               5                   10                  15
   Ser Arg Phe Ser Gly Ser Arg
               20
<210> 1276
   <211> 23
   <212> PRT
   <213> Artificial Sequence
<220>
   <223> Description of Artificial Sequence: Synthetic peptide
<400> 1276
   Pro Lys Leu Leu Ile Trp Ser Ala Ser Trp Leu Tyr Ser Gly Val Pro
   1               5                   10                  15
   Ser Arg Phe Ser Gly Ser Arg
               20
<210> 1277
   <211> 23
   <212> PRT
   <213> Artificial Sequence
<220>
   <223> Description of Artificial Sequence: Synthetic peptide
<400> 1277
   Pro Lys Leu Leu Ile Asp Ser Ala Ser Trp Leu Trp Ser Gly Val Pro
   1 5 10 15
   Ser Arg Phe Ser Gly Ser Arg
   20
<210> 1278
   <211> 23
   <212> PRT
   <213> Artificial Sequence
<220>
   <223> Description of Artificial Sequence: Synthetic peptide
<400> 1278
   Pro Lys Leu Leu Ile Val Ser Ala Ser Thr Leu Trp Ser Gly Val Pro
   1 5 10 15
   Ser Arg Phe Ser Gly Ser Arg
   20
<210> 1279
   <211> 23
   <212> PRT
   <213> Artificial Sequence
<220>
   <223> Description of Artificial Sequence: Synthetic peptide
<400> 1279
   Pro Lys Leu Leu Ile Tyr Ser Ala Ser Phe Leu Trp Ser Gly Val Pro
   1 5 10 15
   Ser Arg Phe Ser Gly Ser Arg
   20
<210> 1280
   <211> 23
   <212> PRT
   <213> Artificial Sequence
<220>
   <223> Description of Artificial Sequence: Synthetic peptide
<400> 1280
   Pro Lys Leu Leu Ile Phe Ser Ala Ser His Leu Trp Ser Gly Val Pro
   1 5 10 15
   Ser Arg Phe Ser Gly Ser Arg
   20
<210> 1281
   <211> 23
   <212> PRT
   <213> Artificial Sequence
<220>
   <223> Description of Artificial Sequence: Synthetic peptide
<400> 1281
   Pro Lys Leu Leu Ile Val Ser Ala Ser Val Leu Trp Ser Gly Val Pro
   1               5                   10                  15
   Ser Arg Phe Ser Gly Ser Arg
               20
<210> 1282
   <211> 23
   <212> PRT
   <213> Artificial Sequence
<220>
   <223> Description of Artificial Sequence: Synthetic peptide
<400> 1282
   Pro Lys Leu Leu Ile Ala Ser Ala Ser Val Leu Leu Ser Gly Val Pro
   1               5                   10                  15
   Ser Arg Phe Ser Gly Ser Arg
               20
<210> 1283
   <211> 23
   <212> PRT
   <213> Artificial Sequence
<220>
   <223> Description of Artificial Sequence: Synthetic peptide
<400> 1283
   Pro Lys Leu Leu Ile Trp Ser Ala Ser Arg Leu Trp Ser Gly Val Pro
   1               5                   10                  15
   Ser Arg Phe Ser Gly Ser Arg
               20
<210> 1284
   <211> 23
   <212> PRT
   <213> Artificial Sequence
<220>
   <223> Description of Artificial Sequence: Synthetic peptide
<400> 1284
   Pro Lys Leu Leu Ile Trp Ser Ala Ser Gln Leu Phe Ser Gly Val Pro
   1               5                   10                  15
   Ser Arg Phe Ser Gly Ser Arg
               20
<210> 1285
   <211> 23
   <212> PRT
   <213> Artificial Sequence
<220>
   <223> Description of Artificial Sequence: Synthetic peptide
<400> 1285
   Pro Lys Leu Leu Ile Val Ser Ala Ser Trp Leu Leu Ser Gly Val Pro
   1               5                   10                  15
   Ser Arg Phe Ser Gly Ser Arg
               20
<210> 1286
   <211> 23
   <212> PRT
   <213> Artificial Sequence
<220>
   <223> Description of Artificial Sequence: Synthetic peptide
<400> 1286
   Pro Lys Leu Leu Ile Trp Ser Ala Ser Thr Leu Tyr Ser Gly Val Pro
   1               5                   10                  15
   Ser Arg Phe Ser Gly Ser Arg
               20
<210> 1287
   <211> 23
   <212> PRT
   <213> Artificial Sequence
<220>
   <223> Description of Artificial Sequence: Synthetic peptide
<400> 1287
   Pro Lys Leu Leu Ile Asp Ser Ala Ser Trp Leu Trp Ser Gly Val Pro
   1               5                   10                  15
   Ser Arg Phe Ser Gly Ser Arg
               20
<210> 1288
   <211> 23
   <212> PRT
   <213> Artificial Sequence
<220>
   <223> Description of Artificial Sequence: Synthetic peptide
<400> 1288
   Pro Lys Leu Leu Ile Tyr Ser Ala Ser Phe Leu Trp Ser Gly Val Pro
   1               5                   10                  15
   Ser Arg Phe Ser Gly Ser Arg
               20
<210> 1289
   <211> 22
   <212> PRT
   <213> Artificial Sequence
<220>
   <223> Description of Artificial Sequence: Synthetic peptide
<400> 1289
   Thr Cys Arg Ala Ser Gln Asp Val Asn Thr Ala Val Ala Trp Tyr Gln
   1               5                   10                  15
   Gln Lys Pro Gly Lys Ala
               20
<210> 1290
   <211> 22
   <212> PRT
   <213> Artificial Sequence
<220>
   <223> Description of Artificial Sequence: Synthetic peptide
<400> 1290
   Thr Cys Lys Ala Ser Gln Asp Val Asn Thr Ala Val Ala Trp Tyr Gln
   1               5                   10                  15
   Gln Lys Pro Gly His Ser
               20
<210> 1291
   <211> 22
   <212> PRT
   <213> Artificial Sequence
<220>
   <223> Description of Artificial Sequence: Synthetic peptide
<400> 1291
   Thr Cys Lys Ala Ser Gln Asp Val Ser Ile Gly Val Ala Trp Tyr Gln
   1               5                   10                  15
   Gln Arg Pro Gly Gln Ser
               20
<210> 1292
   <211> 22
   <212> PRT
   <213> Artificial Sequence
<220>
   <223> Description of Artificial Sequence: Synthetic peptide
<400> 1292
   Thr Cys Lys Ala Ser Gln Asp Val Ser Ile Gly Val Ala Trp Tyr Gln
   1               5                   10                  15
   Gln Arg Pro Gly Lys Ala
               20
<210> 1293
   <211> 23
   <212> PRT
   <213> Artificial Sequence
<220>
   <223> Description of Artificial Sequence: Synthetic peptide
<400> 1293
   Pro Lys Leu Leu Ile Phe Ser Ala Ser Met Leu Trp Ser Gly Val Pro
   1               5                   10                  15
   Ser Arg Phe Ser Gly Ser Arg
               20
<210> 1294
   <211> 23
   <212> PRT
   <213> Artificial Sequence
<220>
   <223> Description of Artificial Sequence: Synthetic peptide
<400> 1294
   Pro Lys Leu Leu Ile Glu Ser Ala Ser Trp Leu Trp Ser Gly Val Pro
   1 5 10 15
   Ser Arg Phe Ser Gly Ser Arg
   20
<210> 1295
   <211> 23
   <212> PRT
   <213> Artificial Sequence
<220>
   <223> Description of Artificial Sequence: Synthetic peptide
<400> 1295
   Pro Lys Leu Leu Ile Arg Ser Ala Ser Trp Leu Val Ser Gly Val Pro
   1 5 10 15
   Ser Arg Phe Ser Gly Ser Arg
   20
<210> 1296
   <211> 23
   <212> PRT
   <213> Artificial Sequence
<220>
   <223> Description of Artificial Sequence: Synthetic peptide
<400> 1296
   Pro Lys Leu Leu Ile Thr Ser Ala Ser Lys Leu Trp Ser Gly Val Pro
   1 5 10 15
   Ser Arg Phe Ser Gly Ser Arg
   20
<210> 1297
   <211> 23
   <212> PRT
   <213> Artificial Sequence
<220>
   <223> Description of Artificial Sequence: Synthetic peptide
<400> 1297
   Pro Lys Leu Leu Ile Arg Ser Ala Ser Ala Leu Trp Ser Gly Val Pro
   1 5 10 15
   Ser Arg Phe Ser Gly Ser Arg
   20
<210> 1298
   <211> 23
   <212> PRT
   <213> Artificial Sequence
<220>
   <223> Description of Artificial Sequence: Synthetic peptide
<400> 1298
   Pro Lys Leu Leu Ile Thr Ser Ala Ser Arg Leu Val Ser Gly Val Pro
   1               5                   10                  15
   Ser Arg Phe Ser Gly Ser Arg
               20
<210> 1299
   <211> 23
   <212> PRT
   <213> Artificial Sequence
<220>
   <223> Description of Artificial Sequence: Synthetic peptide
<400> 1299
   Pro Lys Leu Leu Ile Val Ser Ala Ser Lys Leu Ser Ser Gly Val Pro
   1               5                   10                  15
   Ser Arg Phe Ser Gly Ser Arg
               20
<210> 1300
   <211> 23
   <212> PRT
   <213> Artificial Sequence
<220>
   <223> Description of Artificial Sequence: Synthetic peptide
<400> 1300
   Pro Lys Leu Leu Ile Ser Ser Ala Ser Val Leu Trp Ser Gly Val Pro
   1               5                   10                  15
   Ser Arg Phe Ser Gly Ser Arg
               20
<210> 1301
   <211> 23
   <212> PRT
   <213> Artificial Sequence
<220>
   <223> Description of Artificial Sequence: Synthetic peptide
<400> 1301
   Pro Lys Leu Leu Ile Tyr Ser Ala Ser Trp Leu Trp Ser Gly Val Pro
   1               5                   10                  15
   Ser Arg Phe Ser Gly Ser Arg
               20
<210> 1302
   <211> 23
   <212> PRT
   <213> Artificial Sequence
<220>
   <223> Description of Artificial Sequence: Synthetic peptide
<400> 1302
   Pro Lys Leu Leu Ile Phe Ser Ala Ser Trp Leu Trp Ser Gly Val Pro
   1               5                   10                  15
   Ser Arg Phe Ser Gly Ser Arg
               20
<210> 1303
   <211> 23
   <212> PRT
   <213> Artificial Sequence
<220>
   <223> Description of Artificial Sequence: Synthetic peptide
<400> 1303
   Pro Lys Leu Leu Ile Val Ser Ala Ser Ala Leu His Ser Gly Val Pro
   1               5                   10                  15
   Ser Arg Phe Ser Gly Ser Arg
               20
<210> 1304
   <211> 23
   <212> PRT
   <213> Artificial Sequence
<220>
   <223> Description of Artificial Sequence: Synthetic peptide
<400> 1304
   Pro Lys Leu Leu Ile Trp Ser Ala Ser Pro Leu His Ser Gly Val Pro
   1               5                   10                  15
   Ser Arg Phe Ser Gly Ser Arg
               20
<210> 1305
   <211> 23
   <212> PRT
   <213> Artificial Sequence
<220>
   <223> Description of Artificial Sequence: Synthetic peptide
<400> 1305
   Pro Lys Leu Leu Ile Leu Ser Ala Ser Gly Leu His Ser Gly Val Pro
   1               5                   10                  15
   Ser Arg Phe Ser Gly Ser Arg
               20
<210> 1306
   <211> 23
   <212> PRT
   <213> Artificial Sequence
<220>
   <223> Description of Artificial Sequence: Synthetic peptide
<400> 1306
   Pro Lys Leu Leu Ile Tyr Ser Ala Ser Phe Arg Glu Thr Gly Val Pro
   1               5                   10                  15
   Asp Arg Phe Thr Gly Asn Arg
               20
<210> 1307
   <211> 23
   <212> PRT
   <213> Artificial Sequence
<220>
   <223> Description of Artificial Sequence: Synthetic peptide
<400> 1307
   Pro Lys Leu Leu Ile Tyr Trp Ala Ser Gly Leu Tyr Ser Gly Val Pro
   1               5                   10                  15
   Ser Arg Phe Ser Gly Ser Arg
               20
<210> 1308
   <211> 23
   <212> PRT
   <213> Artificial Sequence
<220>
   <223> Description of Artificial Sequence: Synthetic peptide
<400> 1308
   Pro Lys Leu Leu Ile Tyr Ser Ala Ser Tyr Arg Tyr Thr Gly Val Pro
   1 5 10 15
   Asp Arg Phe Thr Gly Ser Gly
   20
<210> 1309
   <211> 23
   <212> PRT
   <213> Artificial Sequence
<220>
   <223> Description of Artificial Sequence: Synthetic peptide
<400> 1309
   Pro Lys Leu Leu Ile Tyr Ser Ala Ser Tyr Arg Tyr Thr Gly Val Pro
   1 5 10 15
   Ser Arg Phe Ser Gly Ser Gly
   20
<210> 1310
   <211> 11
   <212> PRT
   <213> Artificial Sequence
<220>
   <223> Description of Artificial Sequence: Synthetic peptide
<400> 1310
   Arg Ala Ser Gln Pro Val Asn Thr Ala Val Ala
   1 5 10
<210> 1311
   <211> 11
   <212> PRT
   <213> Artificial Sequence
<220>
   <223> Description of Artificial Sequence: Synthetic peptide
<400> 1311
   Arg Ala Ser Gln Met Val Asn Thr Ala Val Ala
   1 5 10
<210> 1312
   <211> 11
   <212> PRT
   <213> Artificial Sequence
<220>
   <223> Description of Artificial Sequence: Synthetic peptide
<400> 1312
   Arg Ala Ser Gln Arg Val Asn Thr Ala Val Ala
   1 5 10
<210> 1313
   <211> 9
   <212> PRT
   <213> Artificial Sequence
<220>
   <223> Description of Artificial Sequence: Synthetic peptide
<400> 1313
   Gln Gln His Tyr Asn Thr Pro Pro Thr
   1 5
<210> 1314
   <211> 9
   <212> PRT
   <213> Artificial Sequence
<220>
   <223> Description of Artificial Sequence: Synthetic peptide
<400> 1314
   Gln Gln His Tyr Tyr Thr Pro Pro Thr
   1 5
<210> 1315
   <211> 17
   <212> PRT
   <213> Artificial Sequence
<220>
   <223> Description of Artificial Sequence: Synthetic peptide
<400> 1315
   Arg Ile Tyr Pro Thr Thr Gly Tyr Thr Arg Tyr Ala Asp Ser Val Lys
   1 5 10 15
   Gly
<210> 1316
   <211> 17
   <212> PRT
   <213> Artificial Sequence
<220>
   <223> Description of Artificial Sequence: Synthetic peptide
<400> 1316
   Arg Ile Tyr Pro Thr Lys Gly Tyr Thr Arg Tyr Ala Asp Ser Val Lys
   1 5 10 15
   Gly
<210> 1317
   <211> 17
   <212> PRT
   <213> Artificial Sequence
<220>
   <223> Description of Artificial Sequence: Synthetic peptide
<400> 1317
   Arg Ile Tyr Pro Thr Pro Gly Tyr Thr Arg Tyr Ala Asp Ser Val Lys
   1 5 10 15
   Gly
<210> 1318
   <211> 17
   <212> PRT
   <213> Artificial Sequence
<220>
   <223> Description of Artificial Sequence: Synthetic peptide
<400> 1318 Gly
<210> 1319
   <211> 17
   <212> PRT
   <213> Artificial Sequence
<220>
   <223> Description of Artificial Sequence: Synthetic peptide
<400> 1319
   Arg Ile Tyr Pro Thr Asn Gly Ile Thr Arg Tyr Ala Asp Ser Val Lys
   1 5 10 15
   Gly
<210> 1320
   <211> 17
   <212> PRT
   <213> Artificial Sequence
<220>
   <223> Description of Artificial Sequence: Synthetic peptide
<400> 1320
   Arg Ile Tyr Pro Thr Asn Gly Tyr Thr Thr Tyr Ala Asp Ser Val Lys
   1 5 10 15
   Gly
<210> 1321
   <211> 17
   <212> PRT
   <213> Artificial Sequence
<220>
   <223> Description of Artificial Sequence: Synthetic peptide
<400> 1321
   Arg Ile Tyr Pro Thr Asn Gly Tyr Thr Lys Tyr Ala Asp Ser Val Lys
   1 5 10 15
   Gly
<210> 1322
   <211> 17
   <212> PRT
   <213> Artificial Sequence
<220>
   <223> Description of Artificial Sequence: Synthetic peptide
<400> 1322
   Arg Ile Tyr Pro Thr Asn Gly Tyr Thr Ile Tyr Ala Asp Ser Val Lys
   1 5 10 15
   Gly
<210> 1323
   <211> 11
   <212> PRT
   <213> Artificial Sequence
<220>
   <223> Description of Artificial Sequence: Synthetic peptide
<400> 1323
   Trp Gly Gly Thr Gly Phe Tyr Ala Met Asp Tyr
   1               5                   10
<210> 1324
   <211> 11
   <212> PRT
   <213> Artificial Sequence
<220>
   <223> Description of Artificial Sequence: Synthetic peptide
<400> 1324
   Trp Gly Gly Tyr Gly Phe Tyr Ala Met Asp Tyr
   1               5                   10
<210> 1325
   <211> 11
   <212> PRT
   <213> Artificial Sequence
<220>
   <223> Description of Artificial Sequence: Synthetic peptide
<400> 1325
   Trp Gly Gly Met Gly Phe Tyr Ala Met Asp Tyr
   1               5                   10
<210> 1326
   <211> 11
   <212> PRT
   <213> Artificial Sequence
<220>
   <223> Description of Artificial Sequence: Synthetic peptide
<400> 1326
   Trp Gly Gly Lys Gly Phe Tyr Ala Met Asp Tyr
   1               5                   10
<210> 1327
   <211> 15
   <212> PRT
   <213> Artificial Sequence
<220>
   <223> Description of Artificial Sequence: Synthetic peptide
<400> 1327
   Ser Leu Arg Ala Glu Asp Thr Ala Val Tyr Tyr Cys Ser Arg Trp
   1 5 10 15
<210> 1328
   <211> 15
   <212> PRT
   <213> Artificial Sequence
<220>
   <223> Description of Artificial Sequence: Synthetic peptide
<400> 1328
   Ser Leu Arg Ala Glu Asp Thr Ala Val Tyr Tyr Cys Asn Arg Trp
   1 5 10 15
<210> 1329
   <211> 15
   <212> PRT
   <213> Artificial Sequence
<220>
   <223> Description of Artificial Sequence: Synthetic peptide
<400> 1329
   Ser Leu Arg Ala Glu Asp Thr Ala Val Tyr Tyr Cys Asp Arg Trp
   1 5 10 15
<210> 1330
   <211> 15
   <212> PRT
   <213> Artificial Sequence
<220>
   <223> Description of Artificial Sequence: Synthetic peptide
<400> 1330
   Ser Leu Arg Ala Glu Asp Thr Ala Val Tyr Tyr Cys Ala Arg Trp
   1 5 10 15
<210> 1331
   <211> 15
   <212> PRT
   <213> Artificial Sequence
<220>
   <223> Description of Artificial Sequence: Synthetic peptide
<400> 1331
   Ser Leu Arg Ala Glu Asp Thr Ala Val Tyr Tyr Cys Val Arg Trp
   1 5 10 15
<210> 1332
   <211> 15
   <212> PRT
   <213> Artificial Sequence
<220>
   <223> Description of Artificial Sequence: Synthetic peptide
<400> 1332
   Ser Leu Arg Ala Glu Asp Thr Ala Val Tyr Tyr Cys Leu Arg Trp
   1               5                   10                  15
<210> 1333
   <211> 15
   <212> PRT
   <213> Artificial Sequence
<220>
   <223> Description of Artificial Sequence: Synthetic peptide
<400> 1333
   Ser Leu Arg Ala Glu Asp Thr Ala Val Tyr Tyr Cys Ser Arg Tyr
   1               5                   10                  15
<210> 1334
   <211> 15
   <212> PRT
   <213> Artificial Sequence
<220>
   <223> Description of Artificial Sequence: Synthetic peptide
<400> 1334
   Ser Leu Arg Ala Glu Asp Thr Ala Val Tyr Tyr Cys Asn Arg Tyr
   1               5                   10                  15
<210> 1335
   <211> 15
   <212> PRT
   <213> Artificial Sequence
<220>
   <223> Description of Artificial Sequence: Synthetic peptide
<400> 1335
   Ser Leu Arg Ala Glu Asp Thr Ala Val Tyr Tyr Cys Asp Arg Tyr
   1               5                   10                  15
<210> 1336
   <211> 15
   <212> PRT
   <213> Artificial Sequence
<220>
   <223> Description of Artificial Sequence: Synthetic peptide
<400> 1336
   Ser Leu Arg Ala Glu Asp Thr Ala Val Tyr Tyr Cys Ala Arg Tyr
   1               5                   10                  15
<210> 1337
   <211> 15
   <212> PRT
   <213> Artificial Sequence
<220>
   <223> Description of Artificial Sequence: Synthetic peptide
<400> 1337
   Ser Leu Arg Ala Glu Asp Thr Ala Val Tyr Tyr Cys Val Arg Tyr
   1               5                   10                  15
<210> 1338
   <211> 15
   <212> PRT
   <213> Artificial Sequence
<220>
   <223> Description of Artificial Sequence: Synthetic peptide
<400> 1338
   Ser Leu Arg Ala Glu Asp Thr Ala Val Tyr Tyr Cys Leu Arg Tyr
   1               5                   10                  15
<210> 1339
   <211> 15
   <212> PRT
   <213> Artificial Sequence
<220>
   <223> Description of Artificial Sequence: Synthetic peptide
<400> 1339
   Ser Leu Arg Ala Glu Asp Thr Ala Val Tyr Tyr Cys Ser Arg Phe
   1               5                   10                  15
<210> 1340
   <211> 15
   <212> PRT
   <213> Artificial Sequence
<220>
   <223> Description of Artificial Sequence: Synthetic peptide
<400> 1340
   Ser Leu Arg Ala Glu Asp Thr Ala Val Tyr Tyr Cys Asn Arg Phe
   1               5                   10                  15
<210> 1341
   <211> 15
   <212> PRT
   <213> Artificial Sequence
<220>
   <223> Description of Artificial Sequence: Synthetic peptide
<400> 1341
   Ser Leu Arg Ala Glu Asp Thr Ala Val Tyr Tyr Cys Asp Arg Phe
   1               5                   10                  15
<210> 1342
   <211> 15
   <212> PRT
   <213> Artificial Sequence
<220>
   <223> Description of Artificial Sequence: Synthetic peptide
<400> 1342
   Ser Leu Arg Ala Glu Asp Thr Ala Val Tyr Tyr Cys Ala Arg Phe
   1               5                   10                  15
<210> 1343
   <211> 15
   <212> PRT
   <213> Artificial Sequence
<220>
   <223> Description of Artificial Sequence: Synthetic peptide
<400> 1343
   Ser Leu Arg Ala Glu Asp Thr Ala Val Tyr Tyr Cys Val Arg Phe
   1               5                   10                  15
<210> 1344
   <211> 15
   <212> PRT
   <213> Artificial Sequence
<220>
   <223> Description of Artificial Sequence: Synthetic peptide
<400> 1344
   Ser Leu Arg Ala Glu Asp Thr Ala Val Tyr Tyr Cys Leu Arg Phe
   1               5                   10                  15
<210> 1345
   <211> 15
   <212> PRT
   <213> Artificial Sequence
<220>
   <223> Description of Artificial Sequence: Synthetic peptide
<400> 1345
   Ser Gly Arg Ala Glu Asp Thr Ala Val Tyr Tyr Cys Ser Arg Trp
   1               5                   10                  15
<210> 1346
   <211> 15
   <212> PRT
   <213> Artificial Sequence
<220>
   <223> Description of Artificial Sequence: Synthetic peptide
<400> 1346
   Ser Leu Arg Ala Glu Asp Arg Ala Val Tyr Tyr Cys Ser Arg Trp
   1               5                   10                  15
<210> 1347
   <211> 15
   <212> PRT
   <213> Artificial Sequence
<220>
   <223> Description of Artificial Sequence: Synthetic peptide
<400> 1347
   Val Leu Arg Ala Glu Asp Thr Ala Val Tyr Tyr Cys Ser Arg Trp
   1               5                   10                  15
<210> 1348
   <211> 15
   <212> PRT
   <213> Artificial Sequence
<220>
   <223> Description of Artificial Sequence: Synthetic peptide
<400> 1348
   Ser Leu Lys Ala Glu Asp Thr Ala Val Tyr Tyr Cys Ser Arg Trp
   1               5                   10                  15
<210> 1349
   <211> 15
   <212> PRT
   <213> Artificial Sequence
<220>
   <223> Description of Artificial Sequence: Synthetic peptide
<400> 1349
   Ser Leu Arg Ala Glu Asp Thr Ala Val Tyr Tyr Cys Ser Arg Trp
   1               5                   10                  15
<210> 1350
   <211> 15
   <212> PRT
   <213> Artificial Sequence
<220>
   <223> Description of Artificial Sequence: Synthetic peptide
<400> 1350
   Thr Cys Arg Ala Ser Gln Asp Val Asn Thr Ala Val Ala Trp Tyr
   1               5                   10                  15
<210> 1351
   <211> 15
   <212> PRT
   <213> Artificial Sequence
<220>
   <223> Description of Artificial Sequence: Synthetic peptide
<400> 1351
   Thr Cys Arg Ala Ser Gln Gly Val Asn Thr Ala Val Ala Trp Tyr
   1               5                   10                  15
<210> 1352
   <211> 15
   <212> PRT
   <213> Artificial Sequence
<220>
   <223> Description of Artificial Sequence: Synthetic peptide
<400> 1352
   Thr Cys Arg Ala Ser Gln Ala Val Asn Thr Ala Val Ala Trp Tyr
   1               5                   10                  15
<210> 1353
   <211> 15
   <212> PRT
   <213> Artificial Sequence
<220>
   <223> Description of Artificial Sequence: Synthetic peptide
<400> 1353
   Thr Cys Arg Ala Ser Gln Asn Val Asn Thr Ala Val Ala Trp Tyr
   1               5                   10                  15
<210> 1354
   <211> 15
   <212> PRT
   <213> Artificial Sequence
<220>
   <223> Description of Artificial Sequence: Synthetic peptide
<400> 1354
   Thr Cys Arg Ala Ser Gln Leu Val Asn Thr Ala Val Ala Trp Tyr
   1 5 10 15
<210> 1355
   <211> 15
   <212> PRT
   <213> Artificial Sequence
<220>
   <223> Description of Artificial Sequence: Synthetic peptide
<400> 1355
   Thr Cys Arg Ala Ser Ser Asp Val Asn Thr Ala Val Ala Trp Tyr
   1 5 10 15
<210> 1356
   <211> 15
   <212> PRT
   <213> Artificial Sequence
<220>
   <223> Description of Artificial Sequence: Synthetic peptide
<400> 1356
   Thr Cys Arg Ala Ser Lys Asp Val Asn Thr Ala Val Ala Trp Tyr
   1 5 10 15
<210> 1357
   <211> 15
   <212> PRT
   <213> Artificial Sequence
<220>
   <223> Description of Artificial Sequence: Synthetic peptide
<400> 1357
   Thr Cys Arg Ala Ser Val Asp Val Asn Thr Ala Val Ala Trp Tyr
   1 5 10 15
<210> 1358
   <211> 15
   <212> PRT
   <213> Artificial Sequence
<220>
   <223> Description of Artificial Sequence: Synthetic peptide
<400> 1358
   Thr Cys Arg Ala Ser Leu Asp Val Asn Thr Ala Val Ala Trp Tyr
   1 5 10 15
<210> 1359
   <211> 15
   <212> PRT
   <213> Artificial Sequence
<220>
   <223> Description of Artificial Sequence: Synthetic peptide
<400> 1359
   Thr Cys Arg Ala Ser Phe Asp Val Asn Thr Ala Val Ala Trp Tyr
   1               5                   10                  15
<210> 1360
   <211> 15
   <212> PRT
   <213> Artificial Sequence
<220>
   <223> Description of Artificial Sequence: Synthetic peptide
<400> 1360
   Thr Cys Arg Ala Ser Ile Asp Val Asn Thr Ala Val Ala Trp Tyr
   1               5                   10                  15
<210> 1361
   <211> 15
   <212> PRT
   <213> Artificial Sequence
<220>
   <223> Description of Artificial Sequence: Synthetic peptide
<400> 1361
   Thr Cys Arg Ala Ser Tyr Asp Val Asn Thr Ala Val Ala Trp Tyr
   1               5                   10                  15
<210> 1362
   <211> 15
   <212> PRT
   <213> Artificial Sequence
<220>
   <223> Description of Artificial Sequence: Synthetic peptide
<400> 1362
   Thr Cys Arg Ala Ser Arg Asp Val Asn Thr Ala Val Ala Trp Tyr
   1               5                   10                  15
<210> 1363
   <211> 15
   <212> PRT
   <213> Artificial Sequence
<220>
   <223> Description of Artificial Sequence: Synthetic peptide
<400> 1363
   Thr Cys Arg Ala Ser Gln Asp Val Asn Thr Ala Val Gly Trp Tyr
   1 5 10 15
<210> 1364
   <211> 15
   <212> PRT
   <213> Artificial Sequence
<220>
   <223> Description of Artificial Sequence: Synthetic peptide
<400> 1364
   Thr Cys Arg Ala Ser Gln Gly Val Asn Thr Ala Val Gly Trp Tyr
   1 5 10 15
<210> 1365
   <211> 15
   <212> PRT
   <213> Artificial Sequence
<220>
   <223> Description of Artificial Sequence: Synthetic peptide
<400> 1365
   Thr Cys Arg Ala Ser Gln Ala Val Asn Thr Ala Val Gly Trp Tyr
   1 5 10 15
<210> 1366
   <211> 15
   <212> PRT
   <213> Artificial Sequence
<220>
   <223> Description of Artificial Sequence: Synthetic peptide
<400> 1366
   Thr Cys Arg Ala Ser Gln Asn Val Asn Thr Ala Val Gly Trp Tyr
   1 5 10 15
<210> 1367
   <211> 15
   <212> PRT
   <213> Artificial Sequence
<220>
   <223> Description of Artificial Sequence: Synthetic peptide
<400> 1367
   Thr Cys Arg Ala Ser Gln Leu Val Asn Thr Ala Val Gly Trp Tyr
   1 5 10 15
<210> 1368
   <211> 15
   <212> PRT
   <213> Artificial Sequence
<220>
   <223> Description of Artificial Sequence: Synthetic peptide
<400> 1368
   Thr Cys Arg Ala Ser Ser Asp Val Asn Thr Ala Val Gly Trp Tyr
   1 5 10 15
<210> 1369
   <211> 15
   <212> PRT
   <213> Artificial Sequence
<220>
   <223> Description of Artificial Sequence: Synthetic peptide
<400> 1369
   Thr Cys Arg Ala Ser Lys Asp Val Asn Thr Ala Val Gly Trp Tyr
   1 5 10 15
<210> 1370
   <211> 15
   <212> PRT
   <213> Artificial Sequence
<220>
   <223> Description of Artificial Sequence: Synthetic peptide
<400> 1370
   Thr Cys Arg Ala Ser Val Asp Val Asn Thr Ala Val Gly Trp Tyr
   1 5 10 15
<210> 1371
   <211> 15
   <212> PRT
   <213> Artificial Sequence
<220>
   <223> Description of Artificial Sequence: Synthetic peptide
<400> 1371
   Thr Cys Arg Ala Ser Leu Asp Val Asn Thr Ala Val Gly Trp Tyr
   1 5 10 15
<210> 1372
   <211> 15
   <212> PRT
   <213> Artificial Sequence
<220>
   <223> Description of Artificial Sequence: Synthetic peptide
<400> 1372
   Thr Cys Arg Ala Ser Phe Asp Val Asn Thr Ala Val Gly Trp Tyr
   1 5 10 15
<210> 1373
   <211> 15
   <212> PRT
   <213> Artificial Sequence
<220>
   <223> Description of Artificial Sequence: Synthetic peptide
<400> 1373
   Thr Cys Arg Ala Ser Ile Asp Val Asn Thr Ala Val Gly Trp Tyr
   1 5 10 15
<210> 1374
   <211> 15
   <212> PRT
   <213> Artificial Sequence
<220>
   <223> Description of Artificial Sequence: Synthetic peptide
<400> 1374
   Thr Cys Arg Ala Ser Tyr Asp Val Asn Thr Ala Val Gly Trp Tyr
   1 5 10 15
<210> 1375
   <211> 15
   <212> PRT
   <213> Artificial Sequence
<220>
   <223> Description of Artificial Sequence: Synthetic peptide
<400> 1375
   Thr Cys Arg Ala Ser Arg Asp Val Asn Thr Ala Val Gly Trp Tyr
   1 5 10 15
<210> 1376
   <211> 15
   <212> PRT
   <213> Artificial Sequence
<220>
   <223> Description of Artificial Sequence: Synthetic peptide
<400> 1376
   Thr Cys Arg Ala Ser Gln Asp Val Asn Thr Ala Val Ala Trp Tyr
   1               5                   10                  15
<210> 1377
   <211> 15
   <212> PRT
   <213> Artificial Sequence
<220>
   <223> Description of Artificial Sequence: Synthetic peptide
<400> 1377
   Thr Cys Arg Ala Ser Gln Asp Val Asn Thr Ala Val Ala Trp Tyr
   1               5                   10                  15
<210> 1378
   <211> 15
   <212> PRT
   <213> Artificial Sequence
<220>
   <223> Description of Artificial Sequence: Synthetic peptide
<400> 1378
   Thr Cys Arg Ala Ser Gln Asp Val Asn Thr Ala Val Asp Trp Tyr
   1               5                   10                  15
<210> 1379
   <211> 15
   <212> PRT
   <213> Artificial Sequence
<220>
   <223> Description of Artificial Sequence: Synthetic peptide
<400> 1379
   Thr Cys Arg Ala Ser Gln Gly Val Asn Thr Ala Val Asp Trp Tyr
   1               5                   10                  15
<210> 1380
   <211> 15
   <212> PRT
   <213> Artificial Sequence
<220>
   <223> Description of Artificial Sequence: Synthetic peptide
<400> 1380
   Thr Cys Arg Ala Ser Gln Ala Val Asn Thr Ala Val Asp Trp Tyr
   1               5                   10                  15
<210> 1381
   <211> 15
   <212> PRT
   <213> Artificial Sequence
<220>
   <223> Description of Artificial Sequence: Synthetic peptide
<400> 1381
   Thr Cys Arg Ala Ser Gln Asn Val Asn Thr Ala Val Asp Trp Tyr
   1               5                   10                  15
<210> 1382
   <211> 15
   <212> PRT
   <213> Artificial Sequence
<220>
   <223> Description of Artificial Sequence: Synthetic peptide
<400> 1382
   Thr Cys Arg Ala Ser Gln Leu Val Asn Thr Ala Val Asp Trp Tyr
   1               5                   10                  15
<210> 1383
   <211> 15
   <212> PRT
   <213> Artificial Sequence
<220>
   <223> Description of Artificial Sequence: Synthetic peptide
<400> 1383
   Thr Cys Arg Ala Ser Ser Asp Val Asn Thr Ala Val Asp Trp Tyr
   1               5                   10                  15
<210> 1384
   <211> 15
   <212> PRT
   <213> Artificial Sequence
<220>
   <223> Description of Artificial Sequence: Synthetic peptide
<400> 1384
   Thr Cys Arg Ala Ser Lys Asp Val Asn Thr Ala Val Asp Trp Tyr
   1               5                   10                  15
<210> 1385
   <211> 15
   <212> PRT
   <213> Artificial Sequence
<220>
   <223> Description of Artificial Sequence: Synthetic peptide
<400> 1385
   Thr Cys Arg Ala Ser Val Asp Val Asn Thr Ala Val Asp Trp Tyr
   1 5 10 15
<210> 1386
   <211> 15
   <212> PRT
   <213> Artificial Sequence
<220>
   <223> Description of Artificial Sequence: Synthetic peptide
<400> 1386
   Thr Cys Arg Ala Ser Leu Asp Val Asn Thr Ala Val Asp Trp Tyr
   1 5 10 15
<210> 1387
   <211> 15
   <212> PRT
   <213> Artificial Sequence
<220>
   <223> Description of Artificial Sequence: Synthetic peptide
<400> 1387
   Thr Cys Arg Ala Ser Phe Asp Val Asn Thr Ala Val Asp Trp Tyr
   1 5 10 15
<210> 1388
   <211> 15
   <212> PRT
   <213> Artificial Sequence
<220>
   <223> Description of Artificial Sequence: Synthetic peptide
<400> 1388
   Thr Cys Arg Ala Ser Ile Asp Val Asn Thr Ala Val Asp Trp Tyr
   1 5 10 15
<210> 1389
   <211> 15
   <212> PRT
   <213> Artificial Sequence
<220>
   <223> Description of Artificial Sequence: Synthetic peptide
<400> 1389
   Thr Cys Arg Ala Ser Tyr Asp Val Asn Thr Ala Val Asp Trp Tyr
   1 5 10 15
<210> 1390
   <211> 15
   <212> PRT
   <213> Artificial Sequence
<220>
   <223> Description of Artificial Sequence: Synthetic peptide
<400> 1390
   Thr Cys Arg Ala Ser Arg Asp Val Asn Thr Ala Val Asp Trp Tyr
   1               5                   10                  15
<210> 1391
   <211> 15
   <212> PRT
   <213> Artificial Sequence
<220>
   <223> Description of Artificial Sequence: Synthetic peptide
<400> 1391
   Thr Cys Arg Ala Ser Gln Asp Val Asn Thr Ala Val Val Trp Tyr
   1               5                   10                  15
<210> 1392
   <211> 15
   <212> PRT
   <213> Artificial Sequence
<220>
   <223> Description of Artificial Sequence: Synthetic peptide
<400> 1392
   Thr Cys Arg Ala Ser Gln Gly Val Asn Thr Ala Val Val Trp Tyr
   1               5                   10                  15
<210> 1393
   <211> 15
   <212> PRT
   <213> Artificial Sequence
<220>
   <223> Description of Artificial Sequence: Synthetic peptide
<400> 1393
   Thr Cys Arg Ala Ser Gln Ala Val Asn Thr Ala Val Val Trp Tyr
   1               5                   10                  15
<210> 1394
   <211> 15
   <212> PRT
   <213> Artificial Sequence
<220>
   <223> Description of Artificial Sequence: Synthetic peptide
<400> 1394
   Thr Cys Arg Ala Ser Gln Asn Val Asn Thr Ala Val Val Trp Tyr
   1               5                   10                  15
<210> 1395
   <211> 15
   <212> PRT
   <213> Artificial Sequence
<220>
   <223> Description of Artificial Sequence: Synthetic peptide
<400> 1395
   Thr Cys Arg Ala Ser Gln Leu Val Asn Thr Ala Val Val Trp Tyr
   1               5                   10                  15
<210> 1396
   <211> 15
   <212> PRT
   <213> Artificial Sequence
<220>
   <223> Description of Artificial Sequence: Synthetic peptide
<400> 1396
   Thr Cys Arg Ala Ser Ser Asp Val Asn Thr Ala Val Val Trp Tyr
   1               5                   10                  15
<210> 1397
   <211> 15
   <212> PRT
   <213> Artificial Sequence
<220>
   <223> Description of Artificial Sequence: Synthetic peptide
<400> 1397
   Thr Cys Arg Ala Ser Lys Asp Val Asn Thr Ala Val Val Trp Tyr
   1               5                   10                  15
<210> 1398
   <211> 15
   <212> PRT
   <213> Artificial Sequence
<220>
   <223> Description of Artificial Sequence: Synthetic peptide
<400> 1398
   Thr Cys Arg Ala Ser Val Asp Val Asn Thr Ala Val Val Trp Tyr
   1               5                   10                  15
<210> 1399
   <211> 15
   <212> PRT
   <213> Artificial Sequence
<220>
   <223> Description of Artificial Sequence: Synthetic peptide
<400> 1399
   Thr Cys Arg Ala Ser Leu Asp Val Asn Thr Ala Val Val Trp Tyr
   1               5                   10                  15
<210> 1400
   <211> 15
   <212> PRT
   <213> Artificial Sequence
<220>
   <223> Description of Artificial Sequence: Synthetic peptide
<400> 1400
   Thr Cys Arg Ala Ser Phe Asp Val Asn Thr Ala Val Val Trp Tyr
   1               5                   10                  15
<210> 1401
   <211> 15
   <212> PRT
   <213> Artificial Sequence
<220>
   <223> Description of Artificial Sequence: Synthetic peptide
<400> 1401
   Thr Cys Arg Ala Ser Ile Asp Val Asn Thr Ala Val Val Trp Tyr
   1               5                   10                  15
<210> 1402
   <211> 15
   <212> PRT
   <213> Artificial Sequence
<220>
   <223> Description of Artificial Sequence: Synthetic peptide
<400> 1402
   Thr Cys Arg Ala Ser Tyr Asp Val Asn Thr Ala Val Val Trp Tyr
   1               5                   10                  15
<210> 1403
   <211> 15
   <212> PRT
   <213> Artificial Sequence
<220>
   <223> Description of Artificial Sequence: Synthetic peptide
<400> 1403
   Thr Cys Arg Ala Ser Arg Asp Val Asn Thr Ala Val Val Trp Tyr
   1               5                   10                  15
<210> 1404
   <211> 15
   <212> PRT
   <213> Artificial Sequence
<220>
   <223> Description of Artificial Sequence: Synthetic peptide
<400> 1404
   Thr Cys Arg Ala Ser Gln Asp Val Asn Thr Ala Val Ala Trp Tyr
   1               5                   10                  15
<210> 1405
   <211> 15
   <212> PRT
   <213> Artificial Sequence
<220>
   <223> Description of Artificial Sequence: Synthetic peptide
<400> 1405
   Ser Leu Lys Ala Glu Asp Thr Ala Val Tyr Tyr Cys Ser Arg Trp
   1               5                   10                  15
<210> 1406
   <211> 15
   <212> PRT
   <213> Artificial Sequence
<220>
   <223> Description of Artificial Sequence: Synthetic peptide
<400> 1406
   Ser Leu Arg Ala Glu Asp Thr Ala Val Tyr Tyr Cys Ser Arg Phe
   1               5                   10                  15
<210> 1407
   <211> 15
   <212> PRT
   <213> Artificial Sequence
<220>
   <223> Description of Artificial Sequence: Synthetic peptide
<400> 1407
   Thr Cys Arg Ala Ser Gln Asp Val Asn Thr Ala Val Ala Trp Tyr
   1 5 10 15
<210> 1408
   <211> 15
   <212> PRT
   <213> Artificial Sequence
<220>
   <223> Description of Artificial Sequence: Synthetic peptide
<400> 1408
   Thr Cys Arg Ala Ser Ser Asp Val Asn Thr Ala Val Ala Trp Tyr
   1 5 10 15
<210> 1409
   <211> 15
   <212> PRT
   <213> Artificial Sequence
<220>
   <223> Description of Artificial Sequence: Synthetic peptide
<400> 1409
   Thr Cys Arg Ala Ser Gln Asn Val Asn Thr Ala Val Asp Trp Tyr
   1 5 10 15
<210> 1410
   <211> 15
   <212> PRT
   <213> Artificial Sequence
<220>
   <223> Description of Artificial Sequence: Synthetic peptide
<400> 1410
   Thr Cys Arg Ala Ser Ser Asp Val Asn Thr Ala Val Asp Trp Tyr
   1 5 10 15
<210> 1411
   <211> 15
   <212> PRT
   <213> Artificial Sequence
<220>
   <223> Description of Artificial Sequence: Synthetic peptide
<400> 1411
   Thr Cys Arg Ala Ser Leu Asp Val Asn Thr Ala Val Val Trp Tyr
   1 5 10 15
<210> 1412
   <211> 15
   <212> PRT
   <213> Artificial Sequence
<220>
   <223> Description of Artificial Sequence: Synthetic peptide
<400> 1412
   Thr Cys Arg Ala Ser Gln Leu Val Asn Thr Ala Val Asp Trp Tyr
   1               5                   10                  15
<210> 1413
   <211> 15
   <212> PRT
   <213> Artificial Sequence
<220>
   <223> Description of Artificial Sequence: Synthetic peptide
<400> 1413
   Thr Cys Arg Ala Ser Phe Asp Val Asn Thr Ala Val Asp Trp Tyr
   1               5                   10                  15
<210> 1414
   <211> 15
   <212> PRT
   <213> Artificial Sequence
<220>
   <223> Description of Artificial Sequence: Synthetic peptide
<400> 1414
   Thr Cys Arg Ala Ser Gln Asp Val Asn Thr Ala Val Val Trp Tyr
   1               5                   10                  15